# EUROPEAN PATENT SPECIFICATION

(11) **EP 2 261 334 B1**
(45) Date of publication and mention of the grant of the patent: **26.02.2014**
(21) Application number: 10008162.9
(22) Date of filing: 01.04.2004
(51) Int. Cl.: A61K 31/712, A61K 31/7125, A61K 31/713, C12N 15/113, C07H 21/02

(54) **Modified polynucleotides for use in RNA interference**
Modifizierte Polynukleotide zur Verwendung in RNA-Interferenz
Polynucléotides modifiées à utiliser dans une interférence ARN

(30) Priority: 02.04.2003 US 406908; 01.07.2003 US 613077; 06.02.2004 US 542668 P; 06.02.2004 US 542646 P; 10.02.2004 US 543640 P; 10.02.2004 US 543661 P
(43) Date of publication of application: 15.12.2010
(62) Divisional of application: 04749718.5
(73) Proprietor: Thermo Fisher Scientific Biosciences Inc., Lafayette, CO 80026 (US)
(72) Inventor: Leake, Devin, Denver, CO 80238-2902 (US); Reynolds, Angela, Littleton, CO 80128 (US); Khvorova, Anastasia, Boulder, CO 80305 (US); Marshall, William, Boulder, CO 80303 (US); Fedorov, Yuriy, Superior, CO 80027 (US); Nichols, Kimberly, Louisville, CO 80027 (US)
(74) Representative: Bentham, Andrew

(56) References cited:
- WO-A-02/44321
- WO-A-03/012052
- CHIU Y.-L. AND RANA T.M.: "RNAi in human cells: basic structural and functional features of small interfering RNA", MOLECULAR CELL, CELL PRESS, CAMBRIDGE, MA, US, vol. 10, September 2002 (2002-09), pages 549-561, XP002978510, ISSN: 1097-2765
- HOLEN T ET AL: "Similar behaviour of single-strand and double-strand siRNAs suggests they act through a common RNAi pathway", NUCLEIC ACIDS RESEARCH, OXFORD UNIVERSITY PRESS, SURREY, GB, vol. 31, no. 9, 1 May 2003 (2003-05-01), pages 2401-2407, XP002283906, ISSN: 0305-1048
- HOLEN T. ET AL.: "Positional effects of short interfering RNAs targeting the human coagulation trigger Tissue Factor", NUCLEIC ACIDS RESEARCH, vol. 30, no. 8, 15 April 2002 (2002-04-15) , pages 1757-1766, XP002232890, ISSN: 0305-1048
- AMARZGUIOUI M. ET AL.: "Tolerance for mutations and chemical modifications in a siRNA", NUCLEIC ACIDS RESEARCH, vol. 31, no. 2, 15 January 2003 (2003-01-15), pages 589-595, XP002270887, ISSN: 0305-1048
- CZAUDERNA F ET AL: "Structural variations and stabilising modifications of synthetic siRNAs in mammalian cells", NUCLEIC ACIDS RESEARCH, OXFORD UNIVERSITY PRESS, SURREY, GB, vol. 31, no. 11, 1 June 2003 (2003-06-01), pages 2705-2716, XP002270732, ISSN: 0305-1048
- ELBASHIR S.M. ET AL.: "Functional anatomy of siRNAs for mediating efficient RNAi in Drosophila melanogaster embryo lysate", EMBO JOURNAL, OXFORD UNIVERSITY PRESS, SURREY, GB, vol. 20, no. 23, 3 December 2001 (2001-12-03), pages 6877-6888, XP002225998, ISSN: 0261-4189
- BOUTLA A ET AL: "Short 5'-phosphorylated double-stranded RNAs induce RNA interference in Drosophila.", CURRENT BIOLOGY, vol. 11, no. 22, 13 November 2001 (2001-11-13), pages 1776-1780, XP002619078, ISSN: 0960-9822
- GAIT M.J. ET AL.: "Oligoribonucleotide synthesis", OLIGONUCLEOTIDES AND ANALOGUES - A PRACTICAL APPROACH; ECKSTEIN F., 1 January 1991 (1991-01-01), pages 25-48, XP002325585,
- NYKÄNEN A. ET AL.: "ATP requirements and small interfering RNA structure in the RNA interference pathway", CELL, vol. 107, 2 November 2001 (2001-11-02), pages 309-321, XP002969117, ISSN: 0092-8674
- HARBORTH J. ET AL.: "Sequence, chemical, and structural variation of small interfering RNAs and short hairpin RNAs and the effect on mammalian gene silencing", ANTISENSE & NUCLEIC ACID DRUG DEVELOPMENT, vol. 13, no. 2, April 2003 (2003-04), pages 83-105, XP002284355, ISSN: 1087-2906
- SONG E ET AL: "RNA interference targeting Fas protects mice from fulminant hepatitis", NATURE MEDICINE, NATURE PUBLISHING GROUP, NEW YORK, NY, US, vol. 9, no. 3, 1 March 2003 (2003-03-01), pages 347-351, XP002351920, ISSN: 1078-8956

### SEQUENCE LISTING

<110> DHARMACON Inc.
<120> Modified Polynucleotides for Use in RNA Interference
<130> N95571 AB
<140> EP 04749718.5
   <141> 2004-04-01
<150> PCT/US2004/010343
   <151> 2004-04-01
<150> US 10/406,908
   <151> 2003-04-02
<150> US 10/613,077
   <151> 2003-07-01
<150> US 60/542668
   <151> 2004-02-06
<150> US 60/542646
   <151> 2004-02-06
<150> US 60/542640
   <151> 2004-02-10
<150> US 60/542661
   <151> 2004-02-10
<160> 335
<170> FastSEQ for Windows Version 4.0
<210> 1
   <211> 21
   <212> DNA
   <213> Artificial Sequence
<220>
   <223> RNA/DNA, synthetic, RNA with 2'deoxythymidines at 3' end
<220>
   <221> modified_base
   <222> (20)...(21)
   <223> 2' deoxythymidine
<400> 1
   gugauguaug ucagagagut t 21
<210> 2
   <211> 21
   <212> DNA
   <213> Artificial Sequence
<220>
   <223> RNA/DNA, synthetic, RNA with 2'deoxythymidines at 3' end
<220>
   <221> modified_base
   <222> (20)...(21)
   <223> 2' deoxythymidine
<400> 2
   acucucugac auacaucact t 21
<210> 3
   <211> 21
   <212> DNA
   <213> Artificial Sequence
<220>
   <223> RNA/DNA, synthetic, RNA with 2'deoxythymidines at 3' end
<220>
   <221> modified_base
   <222> (20)...(21)
   <223> 2' deoxythymidine
<220>
   <221> modified_base
   <222> (1)...(19)
   <223> 2' orthoesther
<400> 3
   gugauguaug ucagagagut t 21
<210> 4
   <211> 21
   <212> DNA
   <213> Artificial Sequence
<220>
   <223> RNA/DNA, synthetic, RNA with 2'deoxythymidines at 3' end
<220>
   <221> modified_base
   <222> (20)...(21)
   <223> 2' deoxythymidine
<220>
   <221> modified_base
   <222> (1)...(19)
   <223> 2' orthoesther
<400> 4
   acucucugac auacaucact t 21
<210> 5
   <211> 21
   <212> DNA
   <213> Artificial Sequence
<220>
   <223> RNA/DNA, synthetic, RNA with 2'deoxythymidines at 3' end
<220>
   <221> modified_base
   <222> (2)...(4)
   <223> phosphorothioate modified internucleotide linkages
<220>
   <221> modified_base
   <222> (16)...(18)
   <223> phosphorothioate modified internucleotide linkages
<220>
   <221> modified_base
   <222> (20)...(21)
   <223> 2' deoxythymidine
<400> 5
   acucucugac auacaucact t 21
<210> 6
   <211> 21
   <212> DNA
   <213> Artificial Sequence
<220>
   <223> RNA/DNA, synthetic, RNA with 2'deoxythymidines at 3' end
<220>
   <221> modified_base
   <222> (2)...(6)
   <223> phosphorothioate modified internucleotide linkages
<220>
   <221> modified_base
   <222> (14) ... (18)
   <223> phosphorothioate modified internucleotide linkages
<220>
   <221> modified_base
   <222> (20)...(21)
   <223> 2' deoxythymidine
<400> 6
   acucucugac auacaucact t 21
<210> 7
   <211> 21
   <212> DNA
   <213> Artificial Sequence
<220>
   <223> RNA/DNA, synthetic, RNA with 2'deoxythymidines at 3' end
<220>
   <221> modified_base
   <222> (2) ... (7)
   <223> 2' fluoro
<220>
   <221> modified_base
   <222> 10, 12, 14, 16, 17, 19
   <223> 2' fluoro
<220>
   <221> modified_base
   <222> (2)...(6)
   <223> phosphorothioate modified internucleotide linkages
<220>
   <221> modified_base
   <222> (14)...(18)
   <223> phosphorothioate modified internucleotide linkages
<220>
   <221> modified_base
   <222> (20)...(21)
   <223> 2' deoxythymidine
<400> 7
   acucucugac auacaucact t 21
<210> 8
   <211> 21
   <212> DNA
   <213> Artificial Sequence
<220>
   <223> RNA/DNA, synthetic, RNA with 2'deoxythymidines at 3' end
<220>
   <221> modified_base
   <222> 2,5,7,9,11,12,19
   <223> 2' amino
<220>
   <221> modified_base
   <222> (20)...(21)
   <223> 2' deoxythymidine
<400> 8
   gugauguaug ucagagagut t 21
<210> 9
   <211> 21
   <212> DNA
   <213> Artificial Sequence
<220>
   <223> RNA/DNA, synthetic, RNA with 2'deoxythymidines at 3' end
<220>
   <221> modified_base
   <222> (2)...(6)
   <223> 2' amino

<220>
   <221> modified_base
   <222> (20)...(21)
   <223> 2' deoxythymidine
<400> 9
   acucucugac auacaucact t 21
<210> 10
   <211> 21
   <212> DNA
   <213> Artificial Sequence
<220>
   <223> RNA/DNA, synthetic, RNA with 2'deoxythymidines at 3' end
<220>
   <221> modified_base
   <222> (2)...(18)
   <223> phosphorothioate modified internucleotide linkages
<220>
   <221> modified_base
   <222> (20)...(21)
   <223> 2' deoxythymidine
<400> 10
   gugauguaug ucagagagut t 21
<210> 11
   <211> 21
   <212> DNA
   <213> Artificial Sequence
<220>
   <223> RNA/DNA, synthetic, RNA with 2'deoxythymidines at 3' end
<220>
   <221> modified_base
   <222> (2)...(18)
   <223> phosphorothioate modified internucleotide linkages
<220>
   <221> modified_base
   <222> (0)...(0)
   <223> 2' deoxythymidine
<400> 11
   acucucugac auacaucact t 21
<210> 12
   <211> 21
   <212> DNA
   <213> Artificial Sequence
<220>
   <223> RNA/DNA, synthetic, RNA with 2'deoxythymidines at 3' end
<220>
   <221> modified_base
   <222> (2)...(6)
   <223> phosphorothioate modified internucleotide linkages
<220>
   <221> modified_base
   <222> (14) ... (18)
   <223> phosphorothioate modified internucleotide linkages
<220>
   <221> modified_base
   <222> (20)...(21)
   <223> 2' deoxythymidine
<400> 12
   acucucugac auacaucact t 21
<210> 13
   <211> 21
   <212> DNA
   <213> Artificial Sequence
<220>
   <223> RNA/DNA, synthetic, RNA with 2'deoxythymidines at 3' end
<220>
   <221> modified_base
   <222> 2,5,7,9,11,19
   <223> 2'- O-methyl
<220>
   <221> modified_base
   <222> (20)...(21)
   <223> 2' deoxythymidine
<400> 13
   gugauguaug ucagagagut t 21
<210> 14
   <211> 21
   <212> DNA
   <213> Artificial Sequence
<220>
   <223> RNA/DNA, synthetic, RNA with 2'deoxythymidines at 3' end
<220>
   <221> modified_base
   <222> 2,3,4,5,6,12,14,16,17,19
   <223> 2' omethyl
<220>
   <221> modified_base
   <222> (20)...(21)
   <223> 2' deoxythymidine
<400> 14
   acucucugac auacaucact t 21
<210> 15
   <211> 21
   <212> DNA
   <213> Artificial Sequence
<220>
   <223> RNA/DNA, synthetic, RNA with 2'deoxythymidines at 3' end
<220>
   <221> modified_base
   <222> 1,3,5,7,9,11,13,15,17
   <223> 2'-deoxy
<220>
   <221> modified_base
   <222> (20)...(21)
   <223> 2' deoxythymidine
<400> 15
   gugauguaug ucagagagut t 21
<210> 16
   <211> 21
   <212> DNA
   <213> Artificial Sequence
<220>
   <223> RNA/DNA, synthetic, RNA with 2'deoxythymidines at 3' end
<220>
   <221> modified_base
   <222> 2, 4, 6, 14, 16, 18,
   <223> 2'-deoxy
<220>
   <221> modified_base
   <222> (20)...(21)
   <223> 2' deoxythymidine
<400> 16
   acucucugac auacaucact t 21
<210> 17
   <211> 21
   <212> DNA
   <213> Artificial Sequence
<220>
   <223> RNA/DNA, synthetic, RNA with 2'deoxythymidines at 3' end
<220>
   <221> modified_base
   <222> 2, 5, 7, 9, 11, 12, 19
   <223> 2' fluoro
<220>
   <221> modified_base
   <222> (20)...(21)
   <223> 2' deoxythymidine
<400> 17
   gugauguaug ucagagagut t 21
<210> 18
   <211> 21
   <212> DNA
   <213> Artificial Sequence
<220>
   <223> RNA/DNA, synthetic, RNA with 2'deoxythymidines at 3' end
<220>
   <221> modified_base
   <222> (20)...(21)
   <223> 2' deoxythymidine
<400> 18
   ugguguuugg caaaguucut t 21
<210> 19
   <211> 21
   <212> DNA
   <213> Artificial Sequence
<220>
   <223> RNA/DNA, synthetic, RNA with 2'deoxythymidines at 3' end
<220>
   <221> modified_base
   <222> (20)...(21)
   <223> 2' deoxythymidine
<400> 19
   agaacuuugc caaacaccat t 21
<210> 20
   <211> 21
   <212> DNA
   <213> Artificial Sequence
<220>
   <223> RNA/DNA, synthetic, RNA with 2'deoxythymidines at 3' end
<220>
   <221> modified_base
   <222> 1,4,6,7,8,11,16,17,18,19
   <223> 2' fluoro
<220>
   <221> modified_base
   <222> (20) ... (21)
   <223> 2' deoxythymidine
<400> 20
   ugguguuugg caaaguucut t 21
<210> 21
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(8)
   <223> 2' fluoro
<220>
   <221> modified_base
   <222> 10,11,15,17,18
   <223> 2' fluoro
<220>
   <221> modified_base
   <222> (20)...(21)
   <223> 2' deoxythymidine
<400> 21
   agaacuuugc caaacaccat t 21
<210> 22
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(7)
   <223> 2' fluoro
<220>
   <221> modified_base
   <222> 10, 11, 15, 17, 18
   <223> 2' fluoro
<220>
   <221> modified_base
   <222> (20)...(21)
   <223> 2' deoxythymidine
<400> 22
   agaacuuugc caaacaccat t 21
<210> 23
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(7)
   <223> 2' fluoro
<220>
   <221> modified_base
   <222> 15,17,18
   <223> 2' fluoro
<220>
   <221> modified_base
   <222> (20)...(21)
   <223> 2' deoxythymidine
<400> 23
   agaacuuugc caaacaccat t 21
<210> 24
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2' fluoro
<220>
   <221> modified_base
   <222> (20)...(21)
   <223> 2' deoxythymidine
<400> 24
   agaacuuugc caaacaccat t 21
<210> 25
   <211> 21
   <212> DNA
   <213> Artificial Sequence
<220>
   <223> RNA/DNA, synthetic, RNA with 2'deoxythymidines at 3' end
<220>
   <221> modified_base
   <222> 3, 4, 6, 8, 9, 10, 13, 19, 16, 18
   <223> 2' fluoro
<220>
   <221> modified_base
   <222> (20)...(21)
   <223> 2' deoxythymidine
<400> 25
   gauuaugucc gguuauguat t 21
<210> 26
   <211> 21
   <212> DNA
   <213> Artificial Sequence
<220>
   <223> RNA/DNA, synthetic, RNA with 2'deoxythymidines at 3' end
<220>
   <221> modified_base
   <222> 1, 3, 5, 8, 9, 13, 15, 18, 19
   <223> 2' fluoro
<220>
   <221> modified_base
   <222> (20)...(21)
   <223> 2' deoxythymidine
<400> 26
   uacauaaccg gacauaauct t 21
<210> 27
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-deoxy
<220>
   <221> modified_base
   <222> (20)...(21)
   <223> 2' deoxythymidine
<400> 27
   uuuaugagga ucucucugat t 21
<210> 28
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-deoxy
<220>
   <221> modified_base
   <222> (20) ... (21)
   <223> 2' deoxythymidine
<400> 28
   uuuaugagga ucucucugat t 21
<210> 29
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-deoxy
<220>
   <221> modified_base
   <222> (20)...(21)
   <223> 2' deoxythymidine
<400> 29
   uuuaugagga ucucucugat t 21
<210> 30
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-deoxy
<220>
   <221> modified_base
   <222> (20)...(21)
   <223> 2' deoxythymidine
<400> 30
   uuuaugagga ucucucugat t 21
<210> 31
   <211> 21
   <212> DNA
   <213> Artificial Sequence
<220>
   <223> RNA/DNA, synthetic, RNA with 2'deoxythymidines at 3' end

<220>
   <221> modified_base
   <222> (7)...(9)
   <223> 2'-deoxy
<220>
   <221> modified_base
   <222> (20)...(21)
   <223> 2' deoxythymidine
<400> 31
   uuuaugagga ucucucugat t 21
<210> 32
   <211> 21
   <212> DNA
   <213> Artificial Sequence
<220>
   <223> RNA/DNA, synthetic, RNA with 2'deoxythymidines at 3' end
<220>
   <221> modified_base
   <222> (5) ... (7)
   <223> 2'-deoxy
<220>
   <221> modified_base
   <222> (20)...(21)
   <223> 2' deoxythymidine
<400> 32
   uuuaugagga ucucucugat t 21
<210> 33
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-deoxy
<220>
   <221> modified_base
   <222> (20)...(21)
   <223> 2' deoxythymidine
<400> 33
   uuuaugagga ucucucugat t 21
<210> 34
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-deoxy
<220>
   <221> modified_base
   <222> (20)...(21)
   <223> 2' deoxythymidine
<400> 34
   uuuaugagga ucucucugat t 21
<210> 35
   <211> 21
   <212> DNA
   <213> Artificial Sequence
<220>
   <223> RNA/DNA, synthetic, RNA with 2'deoxythymidines at 3' end
<220>
   <221> modified_base
   <222> (17) ... (18)
   <223> 2'-deoxy
<220>
   <221> modified_base
   <222> (20)...(21)
   <223> 2' deoxythymidine
<400> 35
   uuuaugagga ucucucugat t 21
<210> 36
   <211> 21
   <212> DNA
   <213> Artificial Sequence
<220>
   <223> RNA/DNA, synthetic, RNA with 2'deoxythymidines at 3' end
<220>
   <221> modified_base
   <222> (15)...(16)
   <223> 2'-deoxy
<220>
   <221> modified_base
   <222> (20)...(21)
   <223> 2' deoxythymidine
<400> 36
   uuuaugagga ucucucugat t 21
<210> 37
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(14)
   <223> 2'-deoxy
<220>
   <221> modified_base
   <222> (20)...(21)
   <223> 2' deoxythymidine
<400> 37
   uuuaugagga ucucucugat t 21
<210> 38
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-deoxy
<220>
   <221> modified_base
   <222> (20)...(21)
   <223> 2' deoxythymidine
<400> 38
   uuuaugagga ucucucugat t 21
<210> 39
   <211> 21
   <212> DNA
   <213> Artificial Sequence
<220> .
   <223> RNA/DNA, synthetic, RNA with 2'deoxythymidines at 3' end
<220>
   <221> modified_base
   <222> (9)...(10)
   <223> 2'-deoxy
<220>
   <221> modified_base
   <222> (20)...(21)
   <223> 2' deoxythymidine
<400> 39
   uuuaugagga ucucucugat t 21
<210> 40
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-deoxy
<220>
   <221> modified_base
   <222> (20)...(21)
   <223> 2' deoxythymidine
<400> 40
   uuuaugagga ucucucugat t 21
<210> 41
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-deoxy
<220>
   <221> modified_base
   <222> (20)...(21)
   <223> 2' deoxythymidine
<400> 41
   uuuaugagga ucucucugat t 21
<210> 42
   <211> 21
   <212> DNA
   <213> Artificial Sequence
<220>
   <223> RNA/DNA, synthetic, RNA with 2'deoxythymidines at 3' end
<220>
   <221> modified_base
   <222> (3) ... (4)
   <223> 2'-deoxy
<220>
   <221> modified_base
   <222> (20)...(21)
   <223> 2' deoxythymidine
<400> 42
   uuuaugagga ucucucugat t 21
<210> 43
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-deoxy
<220>
   <221> modified_base
   <222> (20)...(21)
   <223> 2' deoxythymidine
<400> 43
   uuuaugagga ucucucugat t 21
<210> 44
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-deoxy
<220>
   <221> modified_base
   <222> (20)...(21)
   <223> 2' deoxythymidine
<400> 44
   uuuaugagga ucucucugat t 21
<210> 45
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-deoxy
<220>
   <221> modified_base
   <222> (20) ... (21)
   <223> 2' deoxythymidine
<400> 45
   uuuaugagga ucucucugat t 21
<210> 46
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-deoxy
<220>
   <221> modified_base
   <222> (20)...(21)
   <223> 2' deoxythymidine
<400> 46
   uuuaugagga ucucucugat t 21
<210> 47
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-deoxy
<220>
   <221> modified_base
   <222> (20)...(21)
   <223> 2' deoxythymidine
<400> 47
   uuuaugagga ucucucugat t 21
<210> 48
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-deoxy
<220>
   <221> modified_base
   <222> (20)...(21)
   <223> 2' deoxythymidine
<400> 48
   uuuaugagga ucucucugat t 21
<210> 49
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-deoxy
<220>
   <221> modified_base
   <222> (20) ... (21)
   <223> 2' deoxythymidine
<400> 49
   uuuaugagga ucucucugat t 21
<210> 50
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-deoxy
<220>
   <221> modified_base
   <222> (20)...(21)
   <223> 2' deoxythymidine
<400> 50
   uuuaugagga ucucucugat t 21
<210> 51
   <211> 21
   <212> DNA
   <213> Artificial Sequence
<220>
   <223> RNA/DNA, synthetic, RNA with 2'deoxythymidines at 3' end
<220>
   <221> modified_base
   <222> 12
   <223> 2'-deoxy
<220>
   <221> modified_base
   <222> (20)...(21)
   <223> 2' deoxythymidine
<400> 51
   uuuaugagga ucucucugat t 21
<210> 52
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-deoxy
<220>
   <221> modified_base
   <222> (20)...(21)
   <223> 2' deoxythymidine
<400> 52
   uuuaugagga ucucucugat t 21
<210> 53
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-deoxy
<220>
   <221> modified_base
   <222> (20)...(21)
   <223> 2' deoxythymidine
<400> 53
   uuuaugagga ucucucugat t 21
<210> 54
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-deoxy
<220>
   <221> modified_base
   <222> (20)...(21)
   <223> 2' deoxythymidine
<400> 54
   uuuaugagga ucucucugat t 21
<210> 55
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-deoxy
<220>
   <221> modified_base
   <222> (20)...(21)
   <223> 2' deoxythymidine
<400> 55
   uuuaugagga ucucucugat t 21
<210> 56
   <211> 21
   <212> DNA
   <213> Artificial Sequence
<220>
   <223> RNA/DNA, synthetic, RNA with 2'deoxythymidines at 3' end

<220>
   <221> modified_base
   <222> 7
   <223> 2'-deoxy
<220>
   <221> modified_base
   <222> (20)...(21)
   <223> 2' deoxythymidine
<400> 56
   uuuaugagga ucucucugat t 21
<210> 57
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-deoxy
<220>
   <221> modified_base
   <222> (20)...(21)
   <223> 2' deoxythymidine
<400> 57
   uuuaugagga ucucucugat t 21
<210> 58
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-deoxy
<220>
   <221> modified_base
   <222> (20)...(21)
   <223> 2' deoxythymidine
<400> 58
   uuuaugagga ucucucugat t 21
<210> 59
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-deoxy
<220>
   <221> modified_base
   <222> (20)...(21)
   <223> 2' deoxythymidine
<400> 59
   uuuaugagga ucucucugat t 21
<210> 60
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-deoxy
<220>
   <221> modified_base
   <222> (20)...(21)
   <223> 2' deoxythymidine
<400> 60
   uuuaugagga ucucucugat t 21
<210> 61
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-deoxy
<220>
   <221> modified_base
   <222> (20)...(21)
   <223> 2' deoxythymidine
<400> 61
   uuuaugagga ucucucugat t 21
<210> 62
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-deoxy
<220>
   <221> modified_base
   <222> (20)...(21)
   <223> 2' deoxythymidine
<400> 62
   uuuaugagga ucucucugat t 21
<210> 63
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-deoxy
<220>
   <221> modified_base
   <222> (20)...(21)
   <223> 2' deoxythymidine
<400> 63
   ucagagagau ccucauaaat t 21
<210> 64
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-deoxy
<220>
   <221> modified_base
   <222> (20)...(21)
   <223> 2' deoxythymidine
<400> 64
   ucagagagau ccucauaaat t 21
<210> 65
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-deoxy
<220>
   <221> modified_base
   <222> (20)...(21)
   <223> 2' deoxythymidine
<400> 65
   ucagagagau ccucauaaat t 21
<210> 66
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-deoxy
<220>
   <221> modified_base
   <222> (20)...(21)
   <223> 2' deoxythymidine
<400> 66
   ucagagagau ccucauaaat t 21
<210> 67
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(9)
   <223> 2'-deoxy
<220>
   <221> modified_base
   <222> (20)...(21)
   <223> 2' deoxythymidine
<400> 67
   ucagagagau ccucauaaat t 21
<210> 68
   <211> 21
   <212> DNA
   <213> Artificial Sequence
<220>
   <223> RNA/DNA, synthetic, RNA with 2'deoxythymidines at 3' end
<220>
   <221> modified_base
   <222> (4) ... (6)
   <223> 2'-deoxy
<220>
   <221> modified_base
   <222> (20)...(21)
   <223> 2' deoxythymidine
<400> 68
   ucagagagau ccucauaaat t 21
<210> 69
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-deoxy
<220>
   <221> modified_base
   <222> (20)...(21)
   <223> 2' deoxythymidine
<400> 69
   ucagagagau ccucauaaat t 21
<210> 70
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-deoxy
<220>
   <221> modified_base
   <222> (20)...(21)
   <223> 2' deoxythymidine
<400> 70
   ucagagagau ccucauaaat t 21
<210> 71
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(18)
   <223> 2'-deoxy
<220>
   <221> modified_base
   <222> (20) ... (21)
   <223> 2' deoxythymidine
<400> 71
   ucagagagau ccucauaaat t 21
<210> 72
   <211> 21
   <212> DNA
   <213> Artificial Sequence
<220>
   <223> RNA/DNA, synthetic, RNA with 2'deoxythymidines at 3' end
<220>
   <221> modified_base
   <222> (15)...(16)
   <223> 2'-deoxy
<220>
   <221> modified_base
   <222> (20)...(21)
   <223> 2' deoxythymidine
<400> 72
   ucagagagau ccucauaaat t 21
<210> 73
   <211> 21
   <212> DNA
   <213> Artificial Sequence
<220>
   <223> RNA/DNA, synthetic, RNA with 2'deoxythymidines at 3' end
<220>
   <221> modified_base
   <222> (13) ... (14)
   <223> 2'-deoxy
<220>
   <221> modified_base
   <222> (20)...(21)
   <223> 2' deoxythymidine
<400> 73
   ucagagagau ccucauaaat t 21
<210> 74
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-deoxy
<220>
   <221> modified_base
   <222> (20)...(21)
   <223> 2' deoxythymidine
<400> 74
   ucagagagau ccucauaaat t 21
<210> 75
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(10)
   <223> 2'-deoxy
<220>
   <221> modified_base
   <222> (20)...(21)
   <223> 2' deoxythymidine
<400> 75
   ucagagagau ccucauaaat t 21
<210> 76
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-deoxy
<220>
   <221> modified_base
   <222> (20)...(21)
   <223> 2' deoxythymidine
<400> 76
   ucagagagau ccucauaaat t 21
<210> 77
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-deoxy
<220>
   <221> modified_base
   <222> (20)...(21)
   <223> 2' deoxythymidine
<400> 77
   ucagagagau ccucauaaat t 21
<210> 78
   <211> 21
   <212> DNA
   <213> Artificial Sequence
<220>
   <223> RNA/DNA, synthetic, RNA with 2'deoxythymidines at 3' end
<220>
   <221> modified_base
   <222> (3) ... (4)
   <223> 2'-deoxy
<220>
   <221> modified_base
   <222> (20) ... (21)
   <223> 2' deoxythymidine
<400> 78
   ucagagagau ccucauaaat t 21
<210> 79
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-deoxy
<220>
   <221> modified_base
   <222> (20)...(21)
   <223> 2' deoxythymidine
<400> 79
   ucagagagau ccucauaaat t 21
<210> 80
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-deoxy
<220>
   <221> modified_base
   <222> (20)...(21)
   <223> 2' deoxythymidine
<400> 80
   ucagagagau ccucauaaat t 21
<210> 81
   <211> 21
   <212> DNA
   <213> Artificial Sequence
<220>
   <223> RNA/DNA, synthetic, RNA with 2'deoxythymidines at 3' end

<220>
   <221> modified_base
   <222> 18
   <223> 2'-deoxy
<220>
   <221> modified_base
   <222> (20)...(21)
   <223> 2' deoxythymidine
<400> 81
   ucagagagau ccucauaaat t 21
<210> 82
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-deoxy
<220>
   <221> modified_base
   <222> (20)...(21)
   <223> 2' deoxythymidine
<400> 82
   ucagagagau ccucauaaat t 21
<210> 83
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-deoxy
<220>
   <221> modified_base
   <222> (20)...(21)
   <223> 2' deoxythymidine
<400> 83
   ucagagagau ccucauaaat t 21
<210> 84
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-deoxy
<220>
   <221> modified_base
   <222> (20)...(21)
   <223> 2' deoxythymidine
<400> 84
   ucagagagau ccucauaaat t 21
<210> 85
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-deoxy
<220>
   <221> modified_base
   <222> (20)...(21)
   <223> 2' deoxythymidine
<400> 85
   ucagagagau ccucauaaat t 21
<210> 86
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-deoxy
<220>
   <221> modified_base
   <222> (20)...(21)
   <223> 2' deoxythymidine
<400> 86 21
   ucagagagau ccucauaaat t 21
<210> 87
   <211> 21
   <212> DNA
   <213> Artificial Sequence
<220>
   <223> RNA/DNA, synthetic, RNA with 2'deoxythymidines at 3' end
<220>
   <221> modified_base
   <222> 12
   <223> 2'-deoxy
<220>
   <221> modified_base
   <222> (20)...(21)
   <223> 2' deoxythymidine
<400> 87 21
   ucagagagau ccucauaaat t 21
<210> 88
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-deoxy
<220>
   <221> modified_base
   <222> (20)...(21)
   <223> 2' deoxythymidine
<400> 88
   ucagagagau ccucauaaat t 21
<210> 89
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-deoxy
<220>
   <221> modified_base
   <222> (20)...(21)
   <223> 2' deoxythymidine
<400> 89
   ucagagagau ccucauaaat t 21
<210> 90
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-deoxy
<220>
   <221> modified_base
   <222> (20)...(21)
   <223> 2' deoxythymidine
<400> 90
   ucagagagau ccucauaaat t 21
<210> 91
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-deoxy
<220>
   <221> modified_base
   <222> (20)...(21)
   <223> 2' deoxythymidine
<400> 91
   ucagagagau ccucauaaat t 21
<210> 92
   <211> 21
   <212> DNA
   <213> Artificial Sequence
<220>
   <223> RNA/DNA, synthetic, RNA with 2'deoxythymidines at 3' end
<223> 2'-doxy
<220>
   <221> modified_base
   <222> (20)...(21)
   <223> 2' deoxythymidine
<400> 92
   ucagagagau ccucauaaat t 21
<210> 93
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-deoxy
<220>
   <221> modified_base
   <222> (20)...(21)
   <223> 2' deoxythymidine
<400> 93
   ucagagagau ccucauaaat t 21
<210> 94
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-deoxy
<220>
   <221> modified_base
   <222> (20)...(21)
   <223> 2' deoxythymidine
<400> 94
   ucagagagau ccucauaaat t 21
<210> 95
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-deoxy
<220>
   <221> modified_base
   <222> (20)...(21)
   <223> 2' deoxythymidine
<400> 95
   ucagagagau ccucauaaat t 21
<210> 96
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-deoxy
<220>
   <221> modified_base
   <222> (20)...(21)
   <223> 2' deoxythymidine
<400> 96
   ucagagagau ccucauaaat t 21
<210> 97
   <211> 21
   <212> DNA
   <213> Artificial Sequence
<220>
   <223> RNA/DNA, synthetic, RNA with 2'deoxythymidines at 3' end
<220>
   <221> modified base
   <222> 2
   <223> 2'-deoxy
<220>
   <221> modified base
   <222> (20)...(21)
   <223> 2' deoxythymidine
<400> 97
   ucagagagau ccucauaaat t 21
<210> 98
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-deoxy
<220>
   <221> modified_base
   <222> (20)...(21)
   <223> 2' deoxythymidine
<400> 98
   ucagagagau ccucauaaat t 21
<210> 99
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-O-methyl
<220>
   <221> modified_base
   <222> (20)...(21)
   <223> 2' deoxythymidine
<400> 99
   uuuaugagga ucucucugat t 21
<210> 100
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-O-methyl
<220>
   <221> modified_base
   <222> (20)...(21)
   <223> 2' deoxythymidine
<400> 100
   uuuaugagga ucucucugat t 21
<210> 101
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-O-methyl
<220>
   <221> modified_base
   <222> (20)...(21)
   <223> 2' deoxythymidine
<400> 101
   uuuaugagga ucucucugat t 21
<210> 102
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-0-methyl
<220>
   <221> modified_base
   <222> (20)...(21)
   <223> 2' deoxythymidine
<400> 102
   uuuaugagga ucucucugat t 21
<210> 103
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(9)
   <223> 2'-O-methyl
<220>
   <221> modified_base
   <222> (20)...(21)
   <223> 2' deoxythymidine
<400> 103
   uuuaugagga ucucucugat t 21
<210> 104
   <211> 21
   <212> DNA
   <213> Artificial Sequence
<220>
   <223> RNA/DNA, synthetic, RNA with 2'deoxythymidines at 3' end
<220>
   <221> modified_base
   <222> (4)...(6)
   <223> 2'-O-methyl
<220>
   <221> modified_base
   <222> (20)...(21)
   <223> 2' deoxythymidine
<400> 104
   uuuaugagga ucucucugat t 21
<210> 105
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-O-methyl
<220>
   <221> modified_base
   <222> (20)...(21)
   <223> 2' deoxythymidine
<400> 105
   uuuaugagga ucucucugat t 21
<210> 106
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-O-methyl
<220>
   <221> modified_base
   <222> (20)...(21)
   <223> 2' deoxythymidine
<400> 106
   uuuaugagga ucucucugat t 21
<210> 107
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(18)
   <223> 2'-O-methyl
<220>
   <221> modified_base
   <222> (20)...(21)
   <223> 2' deoxythymidine
<400> 107
   uuuaugagga ucucucugat t 21
<210> 108
   <211> 21
   <212> DNA
   <213> Artificial Sequence
<220>
   <223> RNA/DNA, synthetic, RNA with 2'deoxythymidines at 3' end
<220>
   <221> modified_base
   <222> (15)...(16)
   <223> 2'-O-methyl
<220>
   <221> modified_base
   <222> (20)...(21)
   <223> 2' deoxythymidine
<400> 108
   uuuaugagga ucucucugat t 21
<210> 109
   <211> 21
   <212> DNA
   <213> Artificial Sequence

<220>
   <223> RNA/DNA, synthetic, RNA with 2'deoxythymidines at 3' end
<220>
   <221> modified_base
   <222> (13)...(14)
   <223> 2'-O-methyl
<220>
   <221> modified_base
   <222> (20)...(21)
   <223> 2' deoxythymidine
<400> 109
   uuuaugagga ucucucugat t 21
<210> 110
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-O-methyl
<220>
   <221> modified_base
   <222> (20)...(21)
   <223> 2' deoxythymidine
<400> 110
   uuuaugagga ucucucugat t 21
<210> 111
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(10)
   <223> 2'-O-methyl
<220>
   <221> modified_base
   <222> (20)...(21)
   <223> 2' deoxythymidine
<400> 111
   uuuaugagga ucucucugat t 21
<210> 112
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-O-methyl
<220>
   <221> modified_base
   <222> (20)...(21)
   <223> 2' deoxythymidine
<400> 112
   uuuaugagga ucucucugat t 21
<210> 113
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-0-methyl
<220>
   <221> modified_base
   <222> (20)...(21)
   <223> 2' deoxythymidine
<400> 113
   uuuaugagga ucucucugat t 21
<210> 114
   <211> 21
   <212> DNA
   <213> Artificial Sequence
<220>
   <223> RNA/DNA, synthetic, RNA with 2'deoxythymidines at 3' end
<220>
   <221> modified_base
   <222> (3)...(4)
   <223> 2'-O-methyl
<220>
   <221> modified_base
   <222> (20)...(21)
   <223> 2' deoxythymidine
<400> 114
   uuuaugagga ucucucugat t 21
<210> 115
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-O-methyl
<220>
   <221> modified_base
   <222> (20)...(21)
   <223> 2' deoxythymidine
<400> 115
   uuuaugagga ucucucugat t 21
<210> 116
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-O-methyl
<220>
   <221> modified_base
   <222> (20)...(21)
   <223> 2' deoxythymidine
<400> 116
   uuuaugagga ucucucugat t 21
<210> 117
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-O-methyl
<220>
   <221> modified_base
   <222> (20)...(21)
   <223> 2' deoxythymidine
<400> 117
   uuuaugagga ucucucugat t 21
<210> 118
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-O-methyl
<220>
   <221> modified_base
   <222> (20)...(21)
   <223> 2' deoxythymidine
<400> 118
   uuuaugagga ucucucugat t 21
<210> 119
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-0-methyl
<220>
   <221> modified_base
   <222> (20)...(21)
   <223> 2' deoxythymidine
<400> 119
   uuuaugagga ucucucugat t 21
<210> 120
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-O-methyl
<220>
   <221> modified_base
   <222> (20)...(21)
   <223> 2' deoxythymidine
<400> 120
   uuuaugagga ucucucugat t 21
<210> 121
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-O-methyl
<220>
   <221> modified_base
   <222> (20)...(21)
   <223> 2' deoxythymidine
<400> 121
   uuuaugagga ucucucugat t 21
<210> 122
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-0-methyl
<220>
   <221> modified_base
   <222> (20)...(21)
   <223> 2' deoxythymidine
<400> 122
   uuuaugagga ucucucugat t 21
<210> 123
   <211> 21
   <212> DNA
   <213> Artificial Sequence
<220>
   <223> RNA/DNA, synthetic, RNA with 2'deoxythymidines at 3' end
<220>
   <221> modified_base
   <222> 12
   <223> 2'-O-methyl
<220>
   <221> modified_base
   <222> (20)...(21)
   <223> 2' deoxythymidine
<400> 123
   uuuaugagga ucucucugat t 21
<210> 124
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-O-methyl
<220>
   <221> modified_base
   <222> (20)...(21)
   <223> 2' deoxythymidine
<400> 124
   uuuaugagga ucucucugat t 21
<210> 125
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-O-methyl
<220>
   <221> modified_base
   <222> (20)...(21)
   <223> 2' deoxythymidine
<400> 125
   uuuaugagga ucucucugat t 21
<210> 126
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-O-methyl
<220>
   <221> modified_base
   <222> (20)...(21)
   <223> 2' deoxythymidine
<400> 126
   uuuaugagga ucucucugat t 21
<210> 127
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-O-methyl
<220>
   <221> modified_base
   <222> (20)...(21)
   <223> 2' deoxythymidine
<400> 127
   uuuaugagga ucucucugat t 21
<210> 128
   <211> 21
   <212> DNA
   <213> Artificial Sequence
<220>
   <223> RNA/DNA, synthetic, RNA with 2'deoxythymidines at 3' end
<220>
   <221> modified_base
   <222> 7
   <223> 2'-O-methyl

<220>
   <221> modified_base
   <222> (20)...(21)
   <223> 2' deoxythymidine
<400> 128
   uuuaugagga ucucucugat t 21
<210> 129
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-O-methyl
<220>
   <221> modified_base
   <222> (20)...(21)
   <223> 2' deoxythymidine
<400> 129
   uuuaugagga ucucucugat t 21
<210> 130
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-O-methyl
<220>
   <221> modified_base
   <222> (20)...(21)
   <223> 2' deoxythymidine
<400> 130
   uuuaugagga ucucucugat t 21
<210> 131
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-0-methyl
<220>
   <221> modified_base
   <222> (20)...(21)
   <223> 2' deoxythymidine
<400> 131
   uuuaugagga ucucucugat t 21
<210> 132
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-O-methyl
<220>
   <221> modified_base
   <222> (20)...(21)
   <223> 2' deoxythymidine
<400> 132
   uuuaugagga ucucucugat t 21
<210> 133
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-O-methyl
<220>
   <221> modified_base
   <222> (20)...(21)
   <223> 2' deoxythymidine
<400> 133
   uuuaugagga ucucucugat t 21
<210> 134
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-O-methyl
<220>
   <221> modified_base
   <222> (20)...(21)
   <223> 2' deoxythymidine
<400> 134
   uuuaugagga ucucucugat t 21
<210> 135
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-O-methyl
<220>
   <221> modified_base
   <222> (20)...(21)
   <223> 2' deoxythymidine
<400> 135
   ucagagagau ccucauaaat t 21
<210> 136
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-O-methyl
<220>
   <221> modified_base
   <222> (20)...(21)
   <223> 2' deoxythymidine
<400> 136
   ucagagagau ccucauaaat t 21
<210> 137
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-O-methyl
<220>
   <221> modified_base
   <222> (20)...(21)
   <223> 2' deoxythymidine
<400> 137
   ucagagagau ccucauaaat t 21
<210> 138
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-O-methyl
<220>
   <221> modified_base
   <222> (20)...(21)
   <223> 2' deoxythymidine
<400> 138
   ucagagagau ccucauaaat t 21
<210> 139
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(9)
   <223> 2'-O-methyl
<220>
   <221> modified_base
   <222> (20)...(21)
   <223> 2' deoxythymidine
<400> 139
   ucagagagau ccucauaaat t 21
<210> 140
   <211> 21
   <212> DNA
   <213> Artificial Sequence
<220>
   <223> RNA/DNA, synthetic, RNA with 2'deoxythymidines at 3' end
<220>
   <221> modified_base
   <222> (4)...(6)
   <223> 2'-O-methyl
<220>
   <221> modified_base
   <222> (20)...(21)
   <223> 2' deoxythymidine
<400> 140
   ucagagagau ccucauaaat t 21
<210> 141
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-0-methyl
<220>
   <221> modified_base
   <222> (20)...(21)
   <223> 2' deoxythymidine
<400> 141
   ucagagagau ccucauaaat t 21
<210> 142
   <211> 21
   <212> DNA
   <213> Artificial Sequence
<220>
   <223> RNA/DNA, synthetic, RNA with 2'deoxythymidines at 3' end
<220>
   <221> modified_base
   <222> (19)...(20)
   <223> 2'-O-methyl
<220>
   <221> modified_base
   <222> (20)...(21)
   <223> 2' deoxythymidine
<400> 142
   ucagagagau ccucauaaat t 21
<210> 143
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(17)
   <223> 2'-O-methyl
<220>
   <221> modified_base
   <222> (20)...(21)
   <223> 2' deoxythymidine
<400> 143
   ucagagagau ccucauaaat t 21
<210> 144
   <211> 21
   <212> DNA
   <213> Artificial Sequence
<220>
   <223> RNA/DNA, synthetic, RNA with 2'deoxythymidines at 3' end
<220>
   <221> modified_base
   <222> (14)...(15)
   <223> 2'-O-methyl
<220>
   <221> modified_base
   <222> (20)...(21)
   <223> 2' deoxythymidine
<400> 144
   ucagagagau ccucauaaat t 21
<210> 145
   <211> 21
   <212> DNA
   <213> Artificial Sequence
<220>
   <223> RNA/DNA, synthetic, RNA with 2'deoxythymidines at 3' end
<220>
   <221> modified_base
   <222> (12)...(13)
<220>
   <221> modified_base
   <222> (20)...(21)
   <223> 2' deoxythymidine
<400> 145
   ucagagagau ccucauaaat t 21
<210> 146
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(11)
   <223> 2'-O-methyl
<220>
   <221> modified_base
   <222> (20)...(21)
   <223> 2' deoxythymidine
<400> 146
   ucagagagau ccucauaaat t 21
<210> 147
   <211> 21
   <212> DNA
   <213> Artificial Sequence
<220>
   <223> RNA/DNA, synthetic, RNA with 2'deoxythymidines at 3' end
<220>
   <221> modified_base
   <222> (8)...(9)
   <223> 2'-O-methyl
<220>
   <221> modified_base
   <222> (20)...(21)
   <223> 2' deoxythymidine
<400> 147
   ucagagagau ccucauaaat t 21
<210> 148
   <211> 21
   <212> DNA
   <213> Artificial Sequence
<220>
   <223> RNA/DNA, synthetic, RNA with 2'deoxythymidines at 3' end
<220>
   <221> modified_base
   <222> (6)...(7)
   <223> 2'-0-methyl
<220>
   <221> modified_base
   <222> (20)...(21)
   <223> 2' deoxythymidine
<400> 148
   ucagagagau ccucauaaat t 21
<210> 149
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-O-methyl
<220>
   <221> modified_base
   <222> (20)...(21)
   <223> 2' deoxythymidine
<400> 149
   ucagagagau ccucauaaat t 21
<210> 150
   <211> 21
   <212> DNA
   <213> Artificial Sequence
<220>
   <223> RNA/DNA, synthetic, RNA with 2'deoxythymidines at 3' end
<220>
   <221> modified_base
   <222> (3)...(4)
   <223> 2'-O-methyl
<220>
   <221> modified_base
   <222> (20)...(21)
   <223> 2' deoxythymidine
<400> 150
   ucagagagau ccucauaaat t 21
<210> 151
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-O-methyl
<220>
   <221> modified_base
   <222> (20)...(21)
   <223> 2' deoxythymidine
<400> 151
   ucagagagau ccucauaaat t 21
<210> 152
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-O-methyl
<220>
   <221> modified_base
   <222> (20)...(21)
   <223> 2' deoxythymidine
<400> 152
   ucagagagau ccucauaaat t 21
<210> 153
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-O-methyl
<220>
   <221> modified_base
   <222> (20)...(21)
   <223> 2' deoxythymidine
<400> 153
   ucagagagau ccucauaaat t 21
<210> 154
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-O-methyl
<220>
   <221> modified_base
   <222> (20)...(21)
   <223> 2' deoxythymidine
<400> 154
   ucagagagau ccucauaaat t 21
<210> 155
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-O-methyl
<220>
   <221> modified_base
   <222> (20)...(21)
   <223> 2' deoxythymidine
<400> 155
   ucagagagau ccucauaaat t 21
<210> 156
   <211> 21
   <212> DNA
   <213> Artificial Sequence
<220>
   <223> RNA/DNA, synthetic, RNA with 2'deoxythymidines at 3' end

<220>
   <221> modified_base
   <222> 15
   <223> 2'-O-methyl
<220>
   <221> modified_base
   <222> (20)...(21)
   <223> 2' deoxythymidine
<400> 156
   ucagagagau ccucauaaat t 21
<210> 157
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-O-methyl
<220>
   <221> modified_base
   <222> (20)...(21)
   <223> 2' deoxythymidine
<400> 157,
   ucagagagau ccucauaaat t 21
<210> 158
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-O-methyl
<220>
   <221> modified_base
   <222> (20)...(21)
   <223> 2' deoxythymidine
<400> 158
   ucagagagau ccucauaaat t 21
<210> 159
   <211> 21
   <212> DNA
   <213> Artificial Sequence
<220>
   <223> RNA/DNA, synthetic, RNA with 2'deoxythymidines at 3' end '
<220>
   <221> modified_base
   <222> 12
   <223> 2'-O-methyl
<220>
   <221> modified_base
   <222> (20)...(21)
   <223> 2' deoxythymidine
<400> 159
   ucagagagau ccucauaaat t 21
<210> 160
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-O-methyl
<220>
   <221> modified_base
   <222> (20)...(21)
   <223> 2' deoxythymidine
<400> 160
   ucagagagau ccucauaaat t 21
<210> 161
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-O-methyl
<220>
   <221> modified_base
   <222> (20)...(21)
   <223> 2' deoxythymidine
<400> 161
   ucagagagau ccucauaaat t 21
<210> 162
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-O-methyl
<220>
   <221> modified_base
   <222> (20)...(21)
   <223> 2' deoxythymidine
<400> 162
   ucagagagau ccucauaaat t 21
<210> 163
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-O-methyl
<220>
   <221> modified_base
   <222> (20)...(21)
   <223> 2' deoxythymidine
<400> 163
   ucagagagau ccucauaaat t 21
<210> 164
   <211> 21
   <212> DNA
   <213> Artificial Sequence
<220>
   <223> RNA/DNA, synthetic, RNA with 2'deoxythymidines at 3' end
<220>
   <221> modified_base
   <222> 7
   <223> 2'-O-methyl
<220>
   <221> modified_base
   <222> (20)...(21)
   <223> 2' deoxythymidine
<400> 164
   ucagagagau ccucauaaat t 21
<210> 165
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-O-methyl
<220>
   <221> modified_base
   <222> (20)...(21)
   <223> 2' deoxythymidine
<400> 165
   ucagagagau ccucauaaat t 21
<210> 166
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-O-methyl
<220>
   <221> modified_base
   <222> (20)...(21)
   <223> 2' deoxythymidine
<400> 166
   ucagagagau ccucauaaat t 21
<210> 167
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-O-methyl
<220>
   <221> modified_base
   <222> (20)...(21)
   <223> 2' deoxythymidine
<400> 167
   ucagagagau ccucauaaat t 21
<210> 168
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-0-methyl
<220>
   <221> modified_base
   <222> (20)...(21)
   <223> 2' deoxythymidine
<400> 168
   ucagagagau ccucauaaat t 21
<210> 169
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-O-methyl
<220>
   <221> modified_base
   <222> (20)...(21)
   <223> 2' deoxythymidine
<400> 169
   ucagagagau ccucauaaat t 21
<210> 170
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-O-methyl
<220>
   <221> modified_base
   <222> (20)...(21)
   <223> 2' deoxythymidine
<400> 170
   ucagagagau ccucauaaat t 21
<210> 171
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-O-methyl
<220>
   <221> modified_base
   <222> (20)...(21)
   <223> 2' deoxythymidine
<400> 171
   gugauguaug ucagagagut t 21
<210> 172
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-deoxy
<220>
   <221> modified_base
   <222> (20)...(21)
   <223> 2' deoxythymidine
<400> 172
   acucucugac auacaucact t 21
<210> 173
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-deoxy
<220>
   <221> modified_base
   <222> (20)...(21)
   <223> 2' deoxythymidine
<400> 173
   acucucugac auacaucact t 21
<210> 174
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-deoxy
<220>
   <221> modified_base
   <222> (20)...(21)
   <223> 2' deoxythymidine
<400> 174
   acucucugac auacaucact t 21
<210> 175
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-deoxy
<220>
   <221> modified_base
   <222> (20)...(21)
   <223> 2' deoxythymidine
<400> 175
   acucucugac auacaucact t 21
<210> 176
   <211> 21
   <212> DNA
   <213> Artificial Sequence
<220>
   <223> RNA/DNA, synthetic, RNA with 2'deoxythymidines at 3' end
<220>
   <221> modified_base
   <222> (8)...(10)
   <223> 2'-deoxy
<220>
   <221> modified_base
   <222> (20)...(21)
   <223> 2' deoxythymidine
<400> 176
   acucucugac auacaucact t 21
<210> 177
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(6)
   <223> 2'-deoxy
<220>
   <221> modified_base
   <222> (20)...(21)
   <223> 2' deoxythymidine
<400> 177
   acucucugac auacaucact t 21
<210> 178
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-deoxy

<220>
   <221> modified_base
   <222> (20)...(21)
   <223> 2' deoxythymidine
<400> 178
   acucucugac auacaucact t 21
<210> 179
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-deoxy
<220>
   <221> modified_base
   <222> (20)...(21)
   <223> 2' deoxythymidine
<400> 179
   acucucugac auacaucact t 21
<210> 180
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(18)
   <223> 2'-deoxy
<220>
   <221> modified_base
   <222> (20)...(21)
   <223> 2' deoxythymidine
<400> 180
   acucucugac auacaucact t 21
<210> 181
   <211> 21
   <212> DNA
   <213> Artificial Sequence
<220>
   <223> RNA/DNA, synthetic, RNA with 2'deoxythymidines at 3' end
<220>
   <221> modified_base
   <222> (15)...(16)
   <223> 2'-deoxy
<220>
   <221> modified_base
   <222> (20)...(21)
   <223> 2' deoxythymidine
<400> 181
   acucucugac auacaucact t 21
<210> 182
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(14)
   <223> 2'-deoxy
<220>
   <221> modified_base
   <222> (20)...(21)
   <223> 2' deoxythymidine
<400> 182
   acucucugac auacaucact t 21
<210> 183
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-deoxy
<220>
   <221> modified_base
   <222> (20)...(21)
   <223> 2' deoxythymidine
<400> 183
   acucucugac auacaucact t 21
<210> 184
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(10)
   <223> 2'-deoxy
<220>
   <221> modified_base
   <222> (20)...(21)
   <223> 2' deoxythymidine
<400> 184
   acucucugac auacaucact t 21
<210> 185
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-deoxy
<220>
   <221> modified_base
   <222> (20)...(21)
   <223> 2' deoxythymidine
<400> 185
   acucucugac auacaucact t 21
<210> 186
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-deoxy
<220>
   <221> modified_base
   <222> (20)...(21)
   <223> 2' deoxythymidine
<400> 186
   acucucugac auacaucact t 21
<210> 187
   <211> 21
   <212> DNA
   <213> Artificial Sequence
<220>
   <223> RNA/DNA, synthetic, RNA with 2'deoxythymidines at 3' end
<220>
   <221> modified_base
   <222> (3)...(4)
   <223> 2'-deoxy
<220>
   <221> modified_base
   <222> (20)...(21)
   <223> 2' deoxythymidine
<400> 187
   acucucugac auacaucact t 21
<210> 188
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-deoxy
<220>
   <221> modified_base
   <222> (20)...(21)
   <223> 2' deoxythymidine
<400> 188
   acucucugac auacaucact t 21
<210> 189
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-O-methyl
<220>
   <221> modified_base
   <222> (20)...(21)
   <223> 2' deoxythymidine
<400> 189
   acucucugac auacaucact t 21
<210> 190
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-O-methyl
<220>
   <221> modified_base
   <222> (20)...(21)
   <223> 2' deoxythymidine
<400> 190
   acucucugac auacaucact t 21
<210> 191
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-O-methyl
<220>
   <221> modified_base
   <222> (20)...(21)
   <223> 2' deoxythymidine
<400> 191
   acucucugac auacaucact t 21
<210> 192
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-O-methyl
<220>
   <221> modified_base
   <222> (20)...(21)
   <223> 2' deoxythymidine
<400> 192
   acucucugac auacaucact t 21
<210> 193
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(9)
   <223> 2'-O-methyl
<220>
   <221> modified_base
   <222> (20)...(21)
   <223> 2' deoxythymidine
<400> 193
   acucucugac auacaucact t 21
<210> 194
   <211> 21
   <212> DNA
   <213> Artificial Sequence
<220>
   <223> RNA/DNA, synthetic, RNA with 2'deoxythymidines at 3' end
<220>
   <221> modified_base
   <222> (4)...(6)
   <223> 2'-O-methyl
<220>
   <221> modified_base
   <222> (20)...(21)
   <223> 2' deoxythymidine
<400> 194
   acucucugac auacaucact t 21
<210> 195
   <211> 21
   <212> DNA
   <213> Artificial Sequence

<220>
   <223> RNA/DNA, synthetic, RNA with 2'deoxythymidines at 3' end
<220>
   <221> modified_base
   <222> (1)...(3)
   <223> 2'-O-methyl
<220>
   <221> modified_base
   <222> (20)...(21)
   <223> 2' deoxythymidine
<400> 195
   acucucugac auacaucact t 21
<210> 196
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-O-methyl
<220>
   <221> modified_base
   <222> (20)...(21)
   <223> 2' deoxythymidine
<400> 196
   acucucugac auacaucact t 21
<210> 197
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(18)
   <223> 2'-O-methyl
<220>
   <221> modified_base
   <222> (20)...(21)
   <223> 2' deoxythymidine
<400> 197
   acucucugac auacaucact t 21
<210> 198
   <211> 21
   <212> DNA
   <213> Artificial Sequence
<220>
   <223> RNA/DNA, synthetic, RNA with 2'deoxythymidines at 3' end
<220>
   <221> modified_base
   <222> (15)...(16)
   <223> 2'-0-methyl
<220>
   <221> modified_base
   <222> (20)...(21)
   <223> 2' deoxythymidine
<400> 198
   acucucugac auacaucact t 21
<210> 199
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(14)
   <223> 2'-O-methyl
<220>
   <221> modified_base
   <222> (20)...(21)
   <223> 2' deoxythymidine
<400> 199
   acucucugac auacaucact t 21
<210> 200
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-O-methyl
<220>
   <221> modified_base
   <222> (20)...(21)
   <223> 2' deoxythymidine
<400> 200
   acucucugac auacaucact t 21
<210> 201
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(10)
   <223> 2'-O-methyl
<220>
   <221> modified_base
   <222> (20)...(21)
   <223> 2' deoxythymidine
<400> 201
   acucucugac auacaucact t 21
<210> 202
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-O-methyl
<220>
   <221> modified_base
   <222> (20)...(21)
   <223> 2' deoxythymidine
<400> 202
   acucucugac auacaucact t 21
<210> 203
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-O-methyl
<220>
   <221> modified_base
   <222> (20)...(21)
   <223> 2' deoxythymidine
<400> 203
   acucucugac auacaucact t 21
<210> 204
   <211> 21
   <212> DNA
   <213> Artificial Sequence
<220>
   <223> RNA/DNA, synthetic, RNA with 2'deoxythymidines at 3' end
<220>
   <221> modified_base
   <222> (3)...(4)
   <223> 2'-O-methyl
<220>
   <221> modified_base
   <222> (20)...(21)
   <223> 2' deoxythymidine
<400> 204
   acucucugac auacaucact t 21
<210> 205
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-O-methyl
<220>
   <221> modified_base
   <222> (20)...(21)
   <223> 2' deoxythymidine
<400> 205
   acucucugac auacaucact t 21
<210> 206
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-deoxy
<220>
   <221> modified_base
   <222> (20)...(21)
   <223> 2' deoxythymidine
<400> 206
   gugauguaug ucagagagut t 21
<210> 207
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-deoxy
<220>
   <221> modified_base
   <222> (20)...(21)
   <223> 2' deoxythymidine
<400> 207
   gugauguaug ucagagagut t 21
<210> 208
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-deoxy
<220>
   <221> modified_base
   <222> (20)...(21)
   <223> 2' deoxythymidine
<400> 208
   gugauguaug ucagagagut t 21
<210> 209
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-deoxy
<220>
   <221> modified_base
   <222> (20)...(21)
   <223> 2' deoxythymidine
<400> 209
   gugauguaug ucagagagut t 21
<210> 210
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(9)
   <223> 2'-deoxy
<220>
   <221> modified_base
   <222> (20)...(21)
   <223> 2' deoxythymidine
<400> 210
   gugauguaug ucagagagut t 21
<210> 211
   <211> 21
   <212> DNA
   <213> Artificial Sequence
<220>
   <223> RNA/DNA, synthetic, RNA with 2'deoxythymidines at 3' end
<220>
   <221> modified_base
   <222> (4)...(6)
   <223> 2'-deoxy
<220>
   <221> modified_base
   <222> (20)...(21)
   <223> 2' deoxythymidine
<400> 211
   gugauguaug ucagagagut t 21
<210> 212
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-deoxy
<220>
   <221> modified_base
   <222> (20)...(21)
   <223> 2' deoxythymidine
<400> 212
   gugauguaug ucagagagut t 21
<210> 213
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-deoxy
<220>
   <221> modified_base
   <222> (20)...(21)
   <223> 2' deoxythymidine
<400> 213
   gugauguaug ucagagagut t 21
<210> 214
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(18)
   <223> 2'-deoxy

<220>
   <221> modified_base
   <222> (20)...(21)
   <223> 2' deoxythymidine
<400> 214
   gugauguaug ucagagagut t 21
<210> 215
   <211> 21
   <212> DNA
   <213> Artificial Sequence
<220>
   <223> RNA/DNA, synthetic, RNA-with 2'deoxythymidines at 3' end
<220>
   <221> modified_base
   <222> (15)...(16)
   <223> 2'-deoxy
<220>
   <221> modified_base
   <222> (20)...(21)
   <223> 2' deoxythymidine
<400> 215
   gugauguaug ucagagagut t 21
<210> 216
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(14)
   <223> 2'-deoxy
<220>
   <221> modified_base
   <222> (20)...(21)
   <223> 2' deoxythymidine
<400> 216
   gugauguaug ucagagagut t 21
<210> 217
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-deoxy
<220>
   <221> modified_base
   <222> (20)...(21)
   <223> 2' deoxythymidine
<400> 217
   gugauguaug ucagagagut t 21
<210> 218
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(10)
   <223> 2'-deoxy
<220>
   <221> modified_base
   <222> (20)...(21)
   <223> 2' deoxythymidine
<400> 218
   gugauguaug ucagagagut t 21
<210> 219
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-deoxy
<220>
   <221> modified_base
   <222> (20)...(21)
   <223> 2' deoxythymidine
<400> 219
   gugauguaug ucagagagut t 21
<210> 220
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-deoxy
<220>
   <221> modified_base
   <222> (20)...(21)
   <223> 2' deoxythymidine
<400> 220
   gugauguaug ucagagagut t 21
<210> 221
   <211> 21
   <212> DNA
   <213> Artificial Sequence
<220>
   <223> RNA/DNA, synthetic, RNA with 2'deoxythymidines at 3' end
<220>
   <221> modified_base
   <222> (3)...(4)
   <223> 2'-deoxy
<220>
   <221> modified_base
   <222> (20)...(21)
   <223> 2' deoxythymidine
<400> 221
   gugauguaug ucagagagut t 21
<210> 222
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(2)
   <223> 2'-deoxy
<220>
   <221> modified_base
   <222> (20)...(21)
   <223> 2' deoxythymidine
<400> 222
   gugauguaug ucagagagut t 21
<210> 223
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(19)
   <223> 2'-O-methyl
<220>
   <221> modified_base
   <222> (20)...(21)
   <223> 2' deoxythymidine
<400> 223
   gugauguaug ucagagagut t 21
<210> 224
   <211> 21
   <212> DNA
   <213> Artificial Sequence
<220>
   <223> RNA/DNA, synthetic, RNA with 2'deoxythymidines at 3' end
<220>
   <221> modified_base
   <222> (16)...(18)
   <223> 2'-O-methyl
<220>
   <221> modified_base
   <222> (20)...(21)
   <223> 2' deoxythymidine
<400> 224
   gugauguaug ucagagagut t 21
<210> 225
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(15)
   <223> 2'-0-methyl
<220>
   <221> modified_base
   <222> (20)...(21)
   <223> 2' deoxythymidine
<400> 225
   gugauguaug ucagagagut t 21
<210> 226
   <211> 21
   <212> DNA
   <213> Artificial Sequence
<220>
   <223> RNA/DNA, synthetic, RNA with 2'deoxythymidines at 3' end
<220>
   <221> modified_base
   <222> (10)...(12)
   <223> 2'-O-methyl
<220>
   <221> modified_base
   <222> (20)...(21)
   <223> 2' deoxythymidine
<400> 226
   gugauguaug ucagagagut t 21
<210> 227
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(9)
   <223> 2'-O-methyl
<220>
   <221> modified_base
   <222> (20)...(21)
   <223> 2' deoxythymidine
<400> 227
   gugauguaug ucagagagut t 21
<210> 228
   <211> 21
   <212> DNA
   <213> Artificial Sequence
<220>
   <223> RNA/DNA, synthetic, RNA with 2'deoxythymidines at 3' end
<220>
   <221> modified_base
   <222> (4)...(6)
   <223> 2'-O-methyl
<220>
   <221> modified_base
   <222> (20)...(21)
   <223> 2' deoxythymidine
<400> 228
   gugauguaug ucagagagut t 21
<210> 229
   <211> 21
   <212> DNA
   <213> Artificial Sequence
<220>
   <223> RNA/DNA, synthetic, RNA with 2'deoxythymidines at 3' end
<220>
   <221> modified_base
   <222> (1)...(3)
   <223> 2'-O-methyl
<220>
   <221> modified_base
   <222> (20)...(21)
   <223> 2' deoxythymidine
<400> 229
   gugauguaug ucagagagut t 21
<210> 230
   <211> 21
   <212> DNA
   <213> Artificial Sequence
<220>
   <223> RNA/DNA, synthetic, RNA with 2'deoxythymidines at 3' end
<220>
   <221> modified_base
   <222> (18)...(19)
   <223> 2'-0-methyl
<220>
   <221> modified_base
   <222> (20)...(21)
   <223> 2' deoxythymidine
<400> 230
   gugauguaug ucagagagut t 21
<210> 231
   <211> 21
   <212> DNA
   <213> Artificial Sequence
<220>
   <223> RNA/DNA, synthetic, RNA with 2'deoxythymidines at 3' end
<220>
   <221> modified_base
   <222> (17)...(18)
   <223> 2'-0-methyl
<220>
   <221> modified_base
   <222> (20)...(21)
   <223> 2' deoxythymidine
<400> 231
   gugauguaug ucagagagut t 21
<210> 232
   <211> 21
   <212> DNA
   <213> Artificial Sequence
<220>
   <223> RNA/DNA, synthetic, RNA with 2'deoxythymidines at 3' end
<220>
   <221> modified_base
   <222> (15)...(16)
   <223> 2'-O-methyl
<220>
   <221> modified_base
   <222> (20)...(21)
   <223> 2' deoxythymidine
<400> 232
   gugauguaug ucagagagut t 21
<210> 233
   <211> 21
   <212> DNA
   <213> Artificial Sequence
<220>
   <223> RNA/DNA, synthetic, RNA with 2'deoxythymidines at 3' end
<220>
   <221> modified_base
   <222> (13)...(14)
   <223> 2'-0-methyl
<220>
   <221> modified_base
   <222> (20)...(21)
   <223> 2' deoxythymidine
<400> 233
   gugauguaug ucagagagut t 21
<210> 234
   <211> 21
   <212> DNA
   <213> Artificial Sequence
<220>
   <223> RNA/DNA, synthetic, RNA with 2'deoxythymidines at 3' end
<220>
   <221> modified_base
   <222> (11)...(12)
   <223> 2'-O-methyl
<220>
   <221> modified_base
   <222> (20)...(21)
   <223> 2' deoxythymidine
<400> 234
   gugauguaug ucagagagut t 21
<210> 235
   <211> 21
   <212> DNA
   <213> Artificial Sequence
<220>
   <223> RNA/DNA, synthetic, RNA with 2'deoxythymidines at 3' end
<220>
   <221> modified_base
   <222> (9)...(10)
   <223> 2'-O-methyl
<220>
   <221> modified_base
   <222> (20)...(21)
   <223> 2' deoxythymidine
<400> 235
   gugauguaug ucagagagut t 21
<210> 236
   <211> 21
   <212> DNA
   <213> Artificial Sequence
<220>
   <223> RNA/DNA, synthetic, RNA with 2'deoxythymidines at 3' end
<220>
   <221> modified_base
   <222> (7)...(8)
   <223> 2'-0-methyl

<220>
   <221> modified_base
   <222> (20)...(21)
   <223> 2' deoxythymidine
<400> 236
   gugauguaug ucagagagut t 21
<210> 237
   <211> 21
   <212> DNA
   <213> Artificial Sequence
<220>
   <223> RNA/DNA, synthetic, RNA with 2'deoxythymidines at 3' end
<220>
   <221> modified_base
   <222> (5)...(6)
   <223> 2'-O-methyl
<220>
   <221> modified_base
   <222> (20)...(21)
   <223> 2' deoxythymidine
<400> 237
   gugauguaug ucagagagut t 21
<210> 238
   <211> 21
   <212> DNA
   <213> Artificial Sequence
<220>
   <223> RNA/DNA, synthetic, RNA with 2'deoxythymidines at 3' end
<220>
   <221> modified_base
   <222> (3)...(4)
   <223> 2'-O-methyl
<220>
   <221> modified_base
   <222> (20)...(21)
   <223> 2' deoxythymidine
<400> 238
   gugauguaug ucagagagut t 21
<210> 239
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-O-methyl
<220>
   <221> modified_base
   <222> (20)...(21)
   <223> 2' deoxythymidine
<400> 239
   gugauguaug ucagagagut t 21
<210> 240
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-O-methyl
<220>
   <221> modified_base
   <222> (20)...(21)
   <223> 2' deoxythymidine
<400> 240
   gugauguaug ucagagagut t 21
<210> 241
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-O-methyl
<220>
   <221> modified_base
   <222> (20)...(21)
   <223> 2' deoxythymidine
<400> 241
   gugauguaug ucagagagut t 21
<210> 242
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-O-methyl
<220>
   <221> modified_base
   <222> (20)...(21)
   <223> 2' deoxythymidine
<400> 242
   gugauguaug ucagagagut t 21
<210> 243
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-0-methyl
<220>
   <221> modified_base
   <222> (20)...(21)
   <223> 2' deoxythymidine
<400> 243
   gugauguaug ucagagagut t 21
<210> 244
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-O-methyl
<220>
   <221> modified_base
   <222> (20)...(21)
   <223> 2' deoxythymidine
<400> 244
   gugauguaug ucagagagut t 21
<210> 245
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-O-methyl
<220>
   <221> modified_base
   <222> (20)...(21)
   <223> 2' deoxythymidine
<400> 245
   gugauguaug ucagagagut t 21
<210> 246
   <211> 21
   <212> DNA
   <213> Artificial Sequence
<220>
   <223> RNA/DNA, synthetic, RNA with 2'deoxythymidines at 3' end
<220>
   <221> modified_base
   <222> 12
   <223> 2'-O-methyl
<220>
   <221> modified_base
   <222> (20)...(21)
   <223> 2' deoxythymidine
<400> 246
   gugauguaug ucagagagut t 21
<210> 247
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-O-methyl
<220>
   <221> modified_base
   <222> (20)...(21)
   <223> 2' deoxythymidine
<400> 247
   gugauguaug ucagagagut t 21
<210> 248
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-O-methyl
<220>
   <221> modified_base
   <222> (20)...(21)
   <223> 2' deoxythymidine
<400> 248
   gugauguaug ucagagagut t 21
<210> 249
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-O-methyl
<220>
   <221> modified_base
   <222> (20)...(21)
   <223> 2' deoxythymidine
<400> 249
   gugauguaug ucagagagut t 21
<210> 250
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-O-methyl

<220>
   <221> modified_base
   <222> (20)...(21)
   <223> 2' deoxythymidine
<400> 250
   gugauguaug ucagagagut t 21
<210> 251
   <211> 21
   <212> DNA
   <213> Artificial Sequence
<220>
   <223> RNA/DNA, synthetic, RNA with 2'deoxythymidines at 3' end
<220>
   <221> modified_base
   <222> 7
   <223> 2'-O-methyl
<220>
   <221> modified_base
   <222> (20)...(21)
   <223> 2' deoxythymidine
<400> 251
   gugauguaug ucagagagut t 21
<210> 252
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-O-methyl
<220>
   <221> modified_base
   <222> (20)...(21)
   <223> 2' deoxythymidine
<400> 252
   gugauguaug ucagagagut t 21
<210> 253
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-O-methyl
<220>
   <221> modified_base
   <222> (20)...(21)
   <223> 2' deoxythymidine
<400> 253
   gugauguaug ucagagagut t 21
<210> 254
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-0-methyl
<220>
   <221> modified_base
   <222> (20)...(21)
   <223> 2' deoxythymidine
<400> 254
   gugauguaug ucagagagut t 21
<210> 255
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-O-methyl
<220>
   <221> modified_base
   <222> (20)...(21)
   <223> 2' deoxythymidine
<400> 255
   gugauguaug ucagagagut t 21
<210> 256
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-O-methyl
<220>
   <221> modified_base
   <222> (20)...(21)
   <223> 2' deoxythymidine
<400> 256
   gugauguaug ucagagagut t 21
<210> 257
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-O-methylcmnm5u
<220>
   <221> modified_base
   <222> (20)...(21)
   <223> 2' deoxythymidine
<400> 257
   gugauguaug ucagagagut t 21
<210> 258
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-O-methyl
<220>
   <221> modified_base
   <222> (20)...(21)
   <223> 2' deoxythymidine
<400> 258
   acucucugac auacaucact t 21
<210> 259
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-0-methyl
<220>
   <221> modified_base
   <222> (20)...(21)
   <223> 2' deoxythymidine
<400> 259
   acucucugac auacaucact t 21
<210> 260
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-O-methyl
<220>
   <221> modified_base
   <222> (20)...(21)
   <223> 2' deoxythymidine
<400> 260
   acucucugac auacaucact t 21
<210> 261
   <211> 21
   <212> DNA
   <213> Artificial Sequence
<220>
   <223> RNA/DNA, synthetic, RNA with 2'deoxythymidines at 3' end
<220>
   <221> modified_base
   <222> (0)...(0)
   <223> 2'-O-methyl
<220>
   <221> modified_base
   <222> (20)...(21)
   <223> 2' deoxythymidine
<400> 261
   acucucugac auacaucact t 21
<210> 262
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-O-methyl
<220>
   <221> modified_base
   <222> (20)...(21)
   <223> 2' deoxythymidine
<400> 262
   acucucugac auacaucact t 21
<210> 263
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-O-methyl
<220>
   <221> modified_base
   <222> (20)...(21)
   <223> 2' deoxythymidine
<400> 263
   acucucugac auacaucact t 21
<210> 264
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-O-methyl
<220>
   <221> modified_base
   <222> (20)...(21)
   <223> 2' deoxythymidine
<400> 264
   acucucugac auacaucact t 21
<210> 265
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-O-methyl
<220>
   <221> modified_base
   <222> (20)...(21)
   <223> 2' deoxythymidine
<400> 265
   acucucugac auacaucact t 21
<210> 266
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-O-methyl
<220>
   <221> modified_base
   <222> (20)...(21)
   <223> 2' deoxythymidine
<400> 266
   acucucugac auacaucact t 21
<210> 267
   <211> 21
   <212> DNA
   <213> Artificial Sequence
<220>
   <223> RNA/DNA, synthetic, RNA with 2'deoxythymidines at 3' end

<220>
   <221> modified_base
   <222> 10
   <223> 2'-O-methyl
<220>
   <221> modified_base
   <222> (20)...(21)
   <223> 2' deoxythymidine
<400> 267
   acucucugac auacaucact t 21
<210> 268
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-O-methyl
<220>
   <221> modified_base
   <222> (20)...(21)
   <223> 2' deoxythymidine
<400> 268
   acucucugac auacaucact t 21
<210> 269
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-O-methyl
<220>
   <221> modified_base
   <222> (20)...(21)
   <223> 2' deoxythymidine
<400> 269
   acucucugac auacaucact t 21
<210> 270
   <211> 21
   <212> DNA
   <213> Artificial Sequence
<220>
   <223> RNA/DNA, synthetic, RNA with 2'deoxythymidines at 3' end
<220>
   <221> modified_base
   <222> 7
   <223> 2'-O-methyl
<220>
   <221> modified_base
   <222> (20)...(21)
   <223> 2' deoxythymidine
<400> 270
   acucucugac auacaucact t 21
<210> 271
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-O-methyl
<220>
   <221> modified_base
   <222> (20)...(21)
   <223> 2' deoxythymidine
<400> 271
   acucucugac auacaucact t 21
<210> 272
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-O-methyl
<220>
   <221> modified_base
   <222> (20)...(21)
   <223> 2' deoxythymidine
<400> 272
   acucucugac auacaucact t 21
<210> 273
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-O-methyl
<220>
   <221> modified_base
   <222> (20)...(21)
   <223> 2' deoxythymidine
<400> 273
   acucucugac auacaucact t 21
<210> 274
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-0-methyl
<220>
   <221> modified_base
   <222> (20)...(21)
   <223> 2' deoxythymidine
<400> 274
   acucucugac auacaucact t 21
<210> 275
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-0-methyl
<220>
   <221> modified_base
   <222> (20)...(21)
   <223> 2' deoxythymidine
<400> 275
   acucucugac auacaucact t 21
<210> 276
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-O-methyl
<220>
   <221> modified_base
   <222> (20)...(21)
   <223> 2' deoxythymidine
<400> 276
   acucucugac auacaucact t 21
<210> 277
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-deoxy
<220>
   <221> modified_base
   <222> (20)...(21)
   <223> 2' deoxythymidine
<400> 277
   gugauguaug ucagagagut t 21
<210> 278
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-deoxy
<220>
   <221> modified_base
   <222> (20)...(21)
   <223> 2' deoxythymidine
<400> 278
   gugauguaug ucagagagut t 21
<210> 279
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-deoxy
<220>
   <221> modified_base
   <222> (20)...(21)
   <223> 2' deoxythymidine
<400> 279
   gugauguaug ucagagagut t 21
<210> 280
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-deoxy
<220>
   <221> modified_base
   <222> (20)...(21)
   <223> 2' deoxythymidine
<400> 280
   gugauguaug ucagagagut t 21
<210> 281
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-deoxy
<220>
   <221> modified_base
   <222> (20)...(21)
   <223> 2' deoxythymidine
<400> 281
   gugauguaug ucagagagut t 21
<210> 282
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-deoxy
<220>
   <221> modified_base
   <222> (20)...(21)
   <223> 2' deoxythymidine
<400> 282
   gugauguaug ucagagagut t 21
<210> 283
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-deoxy
<220>
   <221> modified_base
   <222> (20)...(21)
   <223> 2' deoxythymidine
<400> 283
   gugauguaug ucagagagut t 21
<210> 284
   <211> 21
   <212> DNA
   <213> Artificial Sequence
<220>
   <223> RNA/DNA, synthetic, RNA with 2'deoxythymidines at 3' end
<220>
   <221> modified_base
   <222> 12
   <223> 2'-deoxy
<220>
   <221> modified_base
   <222> (20)...(21)
   <223> 2' deoxythymidine
<400> 284
   gugauguaug ucagagagut t 21
<210> 285
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-deoxy
<220>
   <221> modified_base
   <222> (20)...(21)
   <223> 2' deoxythymidine
<400> 285
   gugauguaug ucagagagut t 21
<210> 286
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-deoxy

<220>
   <221> modified_base
   <222> (20)...(21)
   <223> 2' deoxythymidine
<400> 286
   gugauguaug ucagagagut t 21
<210> 287
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-deoxy
<220>
   <221> modified_base
   <222> (20)...(21)
   <223> 2' deoxythymidine
<400> 287
   gugauguaug ucagagagut t 21
<210> 288
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-deoxy
<220>
   <221> modified_base
   <222> (20)...(21)
   <223> 2' deoxythymidine
<400> 288
   gugauguaug ucagagagut t 21
<210> 289
   <211> 21
   <212> DNA
   <213> Artificial Sequence
<220>
   <223> RNA/DNA, synthetic, RNA with 2'deoxythymidines at 3' end
<220>
   <221> modified_base
   <222> 7
   <223> 2'-deoxy
<220>
   <221> modified_base
   <222> (20)...(21)
   <223> 2' deoxythymidine
<400> 289
   gugauguaug ucagagagut t 21
<210> 290
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-deoxy
<220>
   <221> modified_base
   <222> (20)...(21)
   <223> 2' deoxythymidine
<400> 290
   gugauguaug ucagagagut t 21
<210> 291
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-deoxy
<220>
   <221> modified_base
   <222> (20)...(21)
   <223> 2' deoxythymidine
<400> 291
   gugauguaug ucagagagut t 21
<210> 292
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-deoxy
<220>
   <221> modified_base
   <222> (20)....(21)
   <223> 2' deoxythymidine
<400> 292
   gugauguaug ucagagagut t 21
<210> 293
   <211> 21
   <212> DNA
   <213> Artificial Sequence
<220>
   <223> RNA/DNA, synthetic, RNA with 2'deoxythymidines at 3' end
<220>
   <221> modified_base
   <222> 3
   <223> 2'-deoxy
<220>
   <221> modified_base
   <222> (20)...(21)
   <223> 2' deoxythymidine
<400> 293
   gugauguaug ucagagagut t 21
<210> 294
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-deoxy
<220>
   <221> modified_base
   <222> (20)...(21)
   <223> 2' deoxythymidine
<400> 294
   gugauguaug ucagagagut t 21
<210> 295
   <211> 21
   <212> DNA
   <213> Artificial Sequence
<220>
   <223> RNA/DNA, synthetic, RNA with 2'deoxythymidines at 3' end
<220>
   <221> modified_base
   <222> 1
   <223> 2'-deoxy
<220>
   <221> modified_base
   <222> (20)...(21)
   <223> 2' deoxythymidine
<400> 295
   gugauguaug ucagagagut t 21
<210> 296
   <211> 21
   <212> DNA
   <213> Artificial Sequence
<220>
   <223> RNA/DNA, synthetic, RNA with 2'deoxythymidines at 3' end
<220>
   <221> modified_base
   <222> 19
   <223> 2'-deoxy
<220>
   <221> modified_base
   <222> (20)...(21)
   <223> 2' deoxythymidine
<400> 296
   acucucugac auacaucact t 21
<210> 297
   <211> 21
   <212> DNA
   <213> Artificial Sequence
<220>
   <223> RNA/DNA, synthetic, RNA with 2'deoxythymidines at 3' end
<220>
   <221> modified_base
   <222> 18
   <223> 2'-deoxy
<220>
   <221> modified_base
   <222> (20)...(21)
   <223> 2' deoxythymidine
<400> 297
   acucucugac auacaucact t 21
<210> 298
   <211> 21
   <212> DNA
   <213> Artificial Sequence
<220>
   <223> RNA/DNA, synthetic, RNA with 2'deoxythymidines at 3' end
<220>
   <221> modified_base
   <222> 17
   <223> 2'-deoxy
<220>
   <221> modified_base
   <222> (20)...(21)
   <223> 2' deoxythymidine
<400> 298
   acucucugac auacaucact t 21
<210> 299
   <211> 21
   <212> DNA
   <213> Artificial Sequence
<220>
   <223> RNA/DNA, synthetic, RNA with 2'deoxythymidines at 3' end
<220>
   <221> modified_base
   <222> 16
   <223> 2'-deoxy
<220>
   <221> modified_base
   <222> (20)...(21)
   <223> 2' deoxythymidine
<400> 299
   acucucugac auacaucact t 21
<210> 300
   <211> 21
   <212> DNA
   <213> Artificial Sequence
<220>
   <223> RNA/DNA, synthetic, RNA with 2'deoxythymidines at 3' end
<220>
   <221> modified_base
   <222> 15
   <223> 2'-deoxy
<220>
   <221> modified_base
   <222> (20)...(21)
   <223> 2' deoxythymidine
<400> 300
   acucucugac auacaucact t 21
<210> 301
   <211> 21
   <212> DNA
   <213> Artificial Sequence
<220>
   <223> RNA/DNA, synthetic, RNA with 2'deoxythymidines at 3' end
<220>
   <221> modified_base
   <222> 14
   <223> 2'-deoxy
<220>
   <221> modified_base
   <222> (20)...(21)
   <223> 2' deoxythymidine
<400> 301
   acucucugac auacaucact t 21
<210> 302
   <211> 21
   <212> DNA
   <213> Artificial Sequence
<220>
   <223> RNA/DNA, synthetic, RNA with 2'deoxythymidines at 3' end
<220>
   <221> modified_base
   <222> 13
   <223> 2'-deoxy
<220>
   <221> modified_base
   <222> (20)...(21)
   <223> 2' deoxythymidine
<400> 302
   acucucugac auacaucact t 21
<210> 303
   <211> 21
   <212> DNA
   <213> Artificial Sequence
<220>
   <223> RNA/DNA, synthetic, RNA with 2'deoxythymidines at 3' end

<220>
   <221> modified_base
   <222> 12
   <223> 2'-deoxy
<220>
   <221> modified_base
   <222> (20)...(21)
   <223> 2' deoxythymidine
<400> 303
   acucucugac auacaucact t 21
<210> 304
   <211> 21
   <212> DNA
   <213> Artificial Sequence
<220>
   <223> RNA/DNA, synthetic, RNA with 2'deoxythymidines at 3' end
<220>
   <221> modified_base
   <222> 11
   <223> 2'-deoxy
<220>
   <221> modified_base
   <222> (20)...(21)
   <223> 2' deoxythymidine
<400> 304
   acucucugac auacaucact t 21
<210> 305
   <211> 21
   <212> DNA
   <213> Artificial Sequence
<220>
   <223> RNA/DNA, synthetic, RNA with 2'deoxythymidines at 3' end
<220>
   <221> modified_base
   <222> 10
   <223> 2'-deoxy
<220>
   <221> modified_base
   <222> (20)...(21)
   <223> 2' deoxythymidine
<400> 305
   acucucugac auacaucact t 21
<210> 306
   <211> 21
   <212> DNA
   <213> Artificial Sequence
<220>
   <223> RNA/DNA, synthetic, RNA with 2'deoxythymidines at 3' end
<220>
   <221> modified_base
   <222> 9
   <223> 2'-deoxy
<220>
   <221> modified_base
   <222> (20)...(21)
   <223> 2' deoxythymidine
<400> 306
   acucucugac auacaucact t 21
<210> 307
   <211> 21
   <212> DNA
   <213> Artificial Sequence
<220>
   <223> RNA/DNA, synthetic, RNA with 2'deoxythymidines at 3' end
<220>
   <221> modified_base
   <222> 8
   <223> 2'-deoxy
<220>
   <221> modified_base
   <222> (20)...(21)
   <223> 2' deoxythymidine
<400> 307
   acucucugac auacaucact t 21
<210> 308
   <211> 21
   <212> DNA
   <213> Artificial Sequence
<220>
   <223> RNA/DNA, synthetic, RNA with 2'deoxythymidines at 3' end
<220>
   <221> modified_base
   <222> 7
   <223> 2'-deoxy
<220>
   <221> modified_base
   <222> (20)....(21)
   <223> 2' deoxythymidine
<400> 308
   acucucugac auacaucact t 21
<210> 309
   <211> 21
   <212> DNA
   <213> Artificial Sequence
<220>
   <223> RNA/DNA, synthetic, RNA with 2'deoxythymidines at 3' end
<220>
   <221> modified_base
   <222> 6
   <223> 2'-deoxy
<220>
   <221> modified_base
   <222> (20)...(21)
   <223> 2' deoxythymidine
<400> 309
   acucucugac auacaucact t 21
<210> 310
   <211> 21
   <212> DNA
   <213> Artificial Sequence
<220>
   <223> RNA/DNA, synthetic, RNA with 2'deoxythymidines at 3' end
<220>
   <221> modified_base
   <222> 5
   <223> 2'-deoxy
<220>
   <221> modified_base
   <222> (20)...(21)
   <223> 2' deoxythymidine
<400> 310
   acucucugac auacaucact t 21
<210> 311
   <211> 21
   <212> DNA
   <213> Artificial Sequence
<220>
   <223> RNA/DNA, synthetic, RNA with 2'deoxythymidines at 3' end
<220>
   <221> modified_base
   <222> 4
   <223> 2'-deoxy
<220>
   <221> modified_base
   <222> (20)...(21)
   <223> 2' deoxythymidine
<400> 311
   acucucugac auacaucact t 21
<210> 312
   <211> 21
   <212> DNA
   <213> Artificial Sequence
<220>
   <223> RNA/DNA, synthetic, RNA_with 2'deoxythymidines at 3' end
<220>
   <221> modified_base
   <222> 3
   <223> 2'-deoxy
<220>
   <221> modified_base
   <222> (20)...(21)
   <223> 2' deoxythymidine
<400> 312
   acucucugac auacaucact t 21
<210> 313
   <211> 21
   <212> DNA
   <213> Artificial Sequence
<220>
   <223> RNA/DNA, synthetic, RNA with 2'deoxythymidines at 3' end
<220>
   <221> modified_base
   <222> 2
   <223> 2'-deoxy
<220>
   <221> modified_base
   <222> (20)...(21)
   <223> 2' deoxythymidine
<400> 313
   acucucugac auacaucact t 21
<210> 314
   <211> 21
   <212> DNA
   <213> Artificial Sequence
<220>
   <223> RNA/DNA, synthetic, RNA with 2'deoxythymidines at 3' end

<220>
   <221> modified_base
   <222> 1
   <223> 2'-deoxy
<220>
   <221> modified_base
   <222> (20)...(21)
   <223> 2' deoxythymidine
<400> 314
   gaaagagcau cuacggugat t 21
<210> 315
   <211> 19
   <212> RNA
   <213> Artificial Sequence
<220>
   <221> modified_base
   <222> (1)...(2)
   <223> 2'-O-methyl
<220>
   <221> modified_base
   <222> 8,10,11,12,14,17
   <223> 2'-O-methyl
<400> 315
   gaaagagcau cuacgguga 19
<210> 316
   <211> 19
   <212> RNA
   <213> Artificial Sequence
<220>
   <221> modified_base
   <222> (1)...(2)
   <223> 2'-O-methyl
<220>
   <221> modified_base
   <222> 8,9,10,13,14,16
   <223> 2'-O-methyl
<400> 316
   gaaaggauuu ggcuacaaa 19
<210> 317
   <211> 19
   <212> RNA
   <213> Artificial Sequence
<220>
   <221> modified_base
   <222> (1)...(2)
   <223> 2'-O-methyl
<220>
   <221> modified_base
   <222> 5,9,10,11,12,14,15,17,19
   <223> 2'-O-Methyl
<400> 317
   acagcaaauu ccaucgugu 19
<210> 318
   <211> 19
   <212> RNA
   <213> Artificial Sequence
<220>
   <221> modified_base
   <222> (1)...(2)
   <223> 2'-O-methyl
<220>
   <221> modified_base
   <222> 8,9,11,12,13,14
   <223> 2'-O-Methyl
<400> 318
   ggaaagacug uuccaaaaa 19
<210> 319
   <211> 4
   <212> RNA
   <213> Artificial Sequence
<220>
   <223> nucleotide loop
<400> 319
   uucg 4
<210> 320
   <211> 9
   <212> RNA
   <213> Artificial Sequence
<220>
   <223> nucleotide loop
<400> 320
   uuuguguag 9
<210> 321
   <211> 10
   <212> RNA
   <213> Artificial Sequence
<220>
   <223> nucleotide loop
<400> 321
   cuuccuguca 10
<210> 322
   <211> 19
   <212> DNA
   <213> Artificial Sequence
<220>
   <223> 2'-O-Methyl
<220>
   <221> modified_base
   <222> 1
   <223> Cy3 label
<220>
   <221> modified_base
   <222> 3,4,9,12
   <223> 2'-O-Methyl
<400> 322
   ggccttagct acaggagag 19
<210> 323
   <211> 4
   <212> RNA
   <213> Artificial Sequence
<220>
   <223> nucleotide loop
<400> 323
   uucg 4
<210> 324
   <211> 9
   <212> RNA
   <213> Artificial Sequence
<220>
   <223> nucleotide loop
<400> 324
   uuuguguag 9
<210> 325
   <211> 10
   <212> RNA
   <213> Artificial Sequence
<220>
   <223> nucleotide loop
<400> 325
   cuuccuguca 10
<210> 326
   <211> 21
   <212> DNA
   <213> Artificial Sequence
<220>
   <223> RNA/DNA, synthetic, RNA with 2'deoxythymidines at 3'
<220>
   <221> modified_base
   <222> (20)...(21)
   <223> 2' deoxythymidine
<400> 326
   gugauguaug ucagagagut t 21
<210> 327
   <211> 19
   <212> RNA
   <213> Artificial Sequence
<220>
   <223> 2'-0-methyl
<400> 327
   gaaaaaucag agagauccu 19
<210> 328
   <211> 19
   <212> RNA
   <213> Artificial Sequence
<220>
   <223> 2'-O-methyl
<400> 328
   uaccggaaaa cucgacgca 19
<210> 329
   <211> 19
   <212> RNA
   <213> Artificial Sequence
<220>
   <223> 2'-O-methyl
<400> 329
   acgucgccag ucaaguaac 19
<210> 330
   <211> 19
   <212> RNA
   <213> Artificial Sequence
<220>
   <223> 2'-O-methyl
<400> 330
   gauuacgucg ccagucaag 19
<210> 331
   <211> 19
   <212> RNA
   <213> Artificial Sequence
<220>
   <223> 2'-O-methyl
<400> 331
   agagaucgug gauuacguc 19
<210> 332
   <211> 19
   <212> RNA
   <213> Artificial Sequence
<220>
   <223> 2'-O-methyl
<400> 332
   uguuguuuug gagcacgga 19
<210> 333
   <211> 19
   <212> RNA
   <213> Artificial Sequence
<220>
   <223> 2'-O-methyl
<220>
   <221> modified_base
   <222> 4,7,8,12,14,16,17
   <223> 2'-O-methyl
<400> 333
   uagcgacuaa acacaucaa 19
<210> 334
   <211> 17
   <212> RNA
   <213> Artificial Sequence
<220>
   <223> 2'-O-methyl
<220>
   <221> modified_base
   <222> 4,7,8,12,14,16,17
   <223> 2'-O-methyl
<400> 334
   uagcgacuaa acacauc 17
<210> 335
   <211> 19
   <212> RNA
   <213> Artificial Sequence
<220>
   <223> synthetic human cyclophilin siRNA sense strand
<400> 335
   ggccuuagcu acaggagag 19

## Description

### FIELD OF THE INVENTION

The present invention relates to the field of modified polynucleotides.

### BACKGROUND

RNA-induced gene silencing in mammalian cells is presently believed to implicate a minimum of three different levels of control: (i) transcription inactivation (siRNA-guided DNA and histone methylation); (ii) small interfering RNA (siRNA)-induced mRNA degradation; and (iii) mRNA-induced transcriptional attenuation. The RNA interference (RNAi) generated by siRNA can be long lasting and effective over multiple cell divisions. Consequently, the ability to assess gene function via siRNA mediated methods, as well as to develop therapies for over-expressed genes, represents an exciting and valuable tool that will accelerate gene function analysis, drug target validation, and genome-wide investigations. Moreover, RNAi has broad potential as a therapeutic tool.

Relatively recent discoveries in the field of RNA metabolism have revealed that the uptake of duplex RNA (dsRNA) can induce RNAi.

In these circumstances, a Type III RNase called Dicer processes the long ds RNA into siRNA that subsequently partner with the RNA Interfering Silencing Complex (RISC) to mediate the degradation of target transcripts in a sequence specific manner. This phenomenon has been observed in a diverse group of organisms. Unfortunately, in mammalian cells, the use of long dsRNA to induce RNAi has been met with only limited success. In large part, this ineffectiveness is due to induction of the interferon response, which results in a general, as opposed to targeted, inhibition of protein synthesis.

Recently, it has been shown that when synthetic siRNAs are introduced into mammalian cells in culture, sequence-specific inhibition of target mRNA can be realized without inducing an interferon response. These short duplexes, can act catalytically at sub-molar concentrations to cleave greater than 95% of the target mRNA in a cell. A description of the mechanisms for siRNA activity, as well as some of its applications is provided in Provost et al., Ribonuclease Activity and RNA Binding of Recombinant Human Dicer, E.M.B.O.J., 2002 Nov., 1, 21(21): 5864 -5874; Tabara et al., The dsRNA Binding Protein RDE-4 Interacts with RDE-1, DCR-1 and a DexH-box Helicase to Direct RNAi in C. elegans, Cell. 2002, June 28,109(7):861-71; Ketting *et al.,* Dicer Functions in RNA Interference and in Synthesis of Small RNA Involved in Developmental Timing in *C. elegans;* Martinez et al., Single-Stranded Antisense siRNAs Guide Target RNA Cleavage in RNAi, Cell 2002, Sept. 6, 110(5):563; Chiu and Rana, siRNA function in RNAi: chemical modification analysis, RNA 9 2003: 1034-1048; and Holen et al., Similar behaviour of single-strand and double-strand siRNAs suggests they act through a common RNAi pathway, Nucleic Acids Research, 31(9) 2003: 2401-2407.

There are four main issues that must be addressed when working with siRNA: (i) functionality; (ii) specificity; (iii) delivery methods; and (iv) stability. Functionality refers to the ability of a particular siRNA to silence the desired target. Methods for improving functionality are, for example, the subject of U.S. Patent Application Serial No. 10/714,333. Specificity refers to the ability of a particular siRNA to silence a desired target and only the desired target. Thus, specificity refers to minimizing off-target effects. Delivery methods are the means by which a user introduces a particular siRNA into a cell and may, for example, include using vectors or modifications of the siRNA itself Stabilization refers to the ability of an siRNA to resist degradation by enzymes and other harmful substances that exist in intra-cellular and extra-cellular environments. For example, when naked siRNA molecules are introduced into blood, serum, or serum-containing media, they are not stable and are almost immediately degraded, which reduces or eliminates their effectiveness.

The present invention addresses the second and fourth issues; specificity and stability, by providing modifications to siRNA that can either increase or decrease specificity and/or increase stability.

### SUMMARY OF THE INVENTION

The present invention is directed to compositions and methods for performing RNA interference. In general the siRNA chemical modifications described herein affect two critical properties of the molecules to which they are associated: stability and specificity. Those that affect stability are particularly advantageous for use in applications that require exposure to blood, serum, serum-containing media, and other biological material that contain nucleases or other factors that tend to degrade nucleic acids. Modifications that reduce the level of off-target effects induced by a siRNA directed against a specific target are particularly valuable in research and therapeutic settings. Multiple distinct combinations of modifications that substantially improve RNAi applications are disclosed and are applicable in the design of optimum silencing reagents, transfection controls, and exaequo reagents.

The present invention provides:
[1] An siRNA, comprising:
   (a) an antisense strand, wherein said antisense strand is comprised of a first 5' terminal antisense nucleotide and said first 5' terminal antisense nucleotide is phosphorylated at said first 5' terminal antisense nucleotide's 5' carbon position; and
   (b) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification;
   and wherein all nucleotides comprised in said antisense strand and sense strand other than said first 5' terminal sense nucleotide and second 5' terminal sense nucleotide are unmodified.
[2] An siRNA, comprising:
   (a) an antisense strand, wherein said antisense strand is comprised of a first 5' terminal antisense nucleotide and said first 5' terminal antisense nucleotide is phosphorylated at said first 5' terminal antisense nucleotide's 5' carbon position; and
   (b) a sense strand, wherein said sense strand is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a 2'-O-alkyl modification, wherein each of said first 2' carbon sense modification and said second 2' carbon sense modification is a 2'-O-alkyl modification;
   and wherein all nucleotides comprised in said antisense strand and sense strand other than said first 5' terminal sense nucleotide, second 5' terminal sense nucleotide and third 5' terminal sense nucleotide are unmodified.
[3] An siRNA which is a unimolecular siRNA capable of forming a hairpin, said unimolecular siRNA comprising:
   (a) an antisense region, wherein said antisense region is comprised of a first 5' terminal antisense nucleotide and said first 5' terminal antisense nucleotide is phosphorylated at said first 5' terminal antisense nucleotide's 5' carbon position;
   (b) a sense region, wherein said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
   (c) a loop region, wherein said loop region is located between said sense region and said antisense region;
   and wherein all nucleotides comprised in said antisense region and sense region other than said first 5' terminal sense nucleotide and second 5' terminal sense nucleotide are unmodified.
[4] An siRNA which is a unimolecular siRNA capable of forming a hairpin, said unimolecular siRNA comprising:
   (a) an antisense region, wherein said antisense region is comprised of a first 5' terminal antisense nucleotide and said first 5' terminal antisense nucleotide is phosphorylated at said first 5' terminal antisense nucleotide's 5' carbon position;
   (b) a sense region, wherein said sense region is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a 2'-O-alkyl modification; and
   (c) a loop region, wherein said loop region is located between said sense region and said antisense region;
   and wherein all nucleotides comprised in said antisense region and sense region other than said first 5' terminal sense nucleotide, second 5' terminal sense nucleotide and third 5' terminal sense nucleotide are unmodified.
[5] An siRNA, comprising:
   (a) an antisense strand, wherein said antisense strand is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification; and
   (b) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification;
   and wherein all nucleotides comprised in said antisense strand and sense strand other than said first 5' terminal antisense nucleotide, second 5' terminal antisense nucleotide, first 5' terminal sense nucleotide and second 5' terminal sense nucleotide are unmodified.
[6] An siRNA, comprising:
   (a) an antisense strand, wherein said antisense strand is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification; and
   (b) a sense strand, wherein said sense strand is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a third 2' carbon sense modification;
   and wherein all nucleotides comprised in said antisense strand and sense strand other than said first 5' terminal antisense nucleotide, second 5' terminal antisense nucleotide, first 5' terminal sense nucleotide, second 5' terminal sense nucleotide and third 5' terminal sense nucleotide are unmodified.
[7] An siRNA which is a unimolecular siRNA capable of forming a hairpin, said unimolecular siRNA comprising:
   (a) an antisense region, wherein said antisense region is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification;
   (b) a sense region, wherein said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
   (c) a loop region, wherein said loop region is located between said sense region and said antisense region;
   and wherein all nucleotides comprised in said antisense region and sense region other than said first 5' terminal antisense nucleotide, second 5' terminal antisense nucleotide, first 5' terminal sense nucleotide and second 5' terminal sense nucleotide are unmodified.
[8] An siRNA which is a unimolecular siRNA capable of forming a hairpin, said unimolecular siRNA comprising:
   (a) an antisense region, wherein said antisense region is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification;
   (b) a sense region, wherein said sense region is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a third 2' carbon sense modification; and
   (c) a loop region, wherein said loop region is located between said sense region and said antisense region;
   and wherein all nucleotides comprised in said antisense region and sense region other than said first 5' terminal antisense nucleotide, second 5' terminal antisense nucleotide, first 5' terminal sense nucleotide, second 5' terminal sense nucleotide and third 5' terminal sense nucleotide are unmodified.
[9] The siRNA of any one of [1] to [8], wherein each of said first 2' carbon sense modification and said second 2' carbon sense modification is a 2'-O-alkyl modification, and preferably wherein at least one and preferably each of the 2'-O-alkyl modifications is a 2'-O-methyl modification.
[10] The siRNA of any one of [1] to [9], wherein said siRNA is comprised of between 18 and 30 base pairs and preferably between 19 and 25 base pairs.
[11] The siRNA of any one of [1], [2], [5] and [6], wherein said siRNA is comprised of between 18 and 30 base pairs and preferably between 19 and 25 base pairs, and wherein said antisense strand and said sense strand are substantially complementary and form a duplex that does not contain overhangs.
[12] The siRNA of any one of [1], [2], [5] and [6], further comprising a conjugate attached to the 3' or 5' end of said sense strand, wherein the conjugate is preferably cholesterol or polyethylene glycol.
[13] A method for reducing off-target effects during RNAi, said method comprising exposing an siRNA of any one of [1] to [12] to a target nucleic acid or a cell that is expressing or is capable of expressing said target nucleic acid, wherein said method is not a method for treatment of the human or animal body by surgery or therapy or a diagnostic method practised on the human or animal body.
[14] A pharmaceutical composition comprising an siRNA as defined in any one of [1] to [12] and a pharmaceutically acceptable carrier or diluent.
[15] An siRNA as defined in any one of [1] to [12] for use in a method for treatment of the human or animal body by therapy or a diagnostic method practised on the human or animal body.

For a better understanding of the present invention together with other and further advantages and embodiments, reference is made to the following description taken in conjunction with the examples, the scope of the which is set forth in the appended claims.

### BRIEF DESCRIPTION OF THE FIGURES

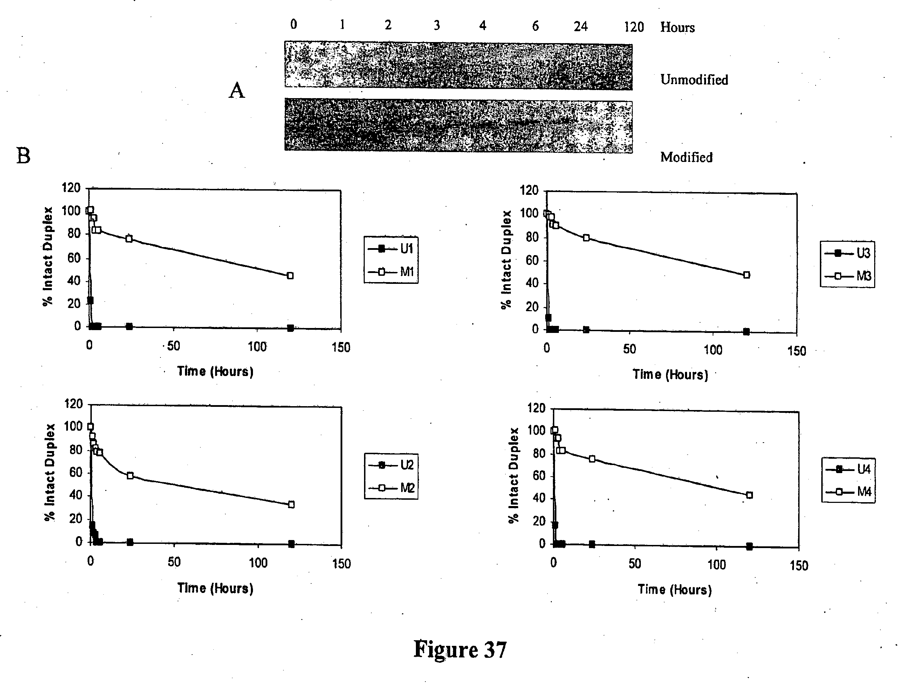

The preferred embodiments of the present invention have been chosen for purposes of illustration and description but are not intended to restrict the scope of the invention in any way. The benefits of the preferred embodiments of certain aspects of the invention are shown in the accompanying figures, wherein:
**Figure 1A** illustrates the functionality of orthoester modifications on sense and/or antisense strands as measured 24 hours post-transfection. See "Preferred Embodiments" for a description of modified duplexes. The degree of SEAP silencing induced by each siRNA was determined by comparing the level of SEAP expression in cells co-transfected with the siRNA + SEAP expression plasmid with cells transfected with the SEAP expression plasmid alone.
**Figure 1B** illustrates the functionality of orthoester modifications (on sense and/or antisense strands) as measured 48 hours post-transfection.
**Figure 2A** illustrates the functionality of orthoester modifications (on sense and/or antisense strands) in conjunction with other modifications, as measured 24 hours post-transfection.
**Figure 2B** illustrates the functionality of orthoester modifications on sense and/or antisense strands in conjunction with other modifications, as measured 72 hours post-transfection.
**Figure 2C** illustrates the functionality of orthoester modifications on sense and/or antisense strands in conjunction with other modifications as measured 144 hours post-transfection.
**Figure 3** illustrates the effects of sense and antisense strand modifications on siRNA functionality.
**Figure 4** illustrates the effects of sense and antisense strand modifications on siRNA functionality.
**Figure 5** illustrates the effects of thio-based modifications of an antisense strand in conjunction with various modifications on the sense strand.
**Figure 6** illustrates the effects of phosphorothioate modifications in both sense and antisense strands.
**Figure 7** illustrates the effects of 2'-O-methyl modifications (in both sense and antisense strands) in conjunction with a variety of other modifications.
**Figure 8** illustrates the effects of siRNAs that are 2'-deoxy-RNA hybrids (on either strand) in conjunction with other chemical modifications.
**Figure 9** illustrates the functionality of a cholesterol conjugate at the 5' end of a sense strand in conjunction with other chemical modifications.
**Figure 10** illustrates the functionality of a PEG conjugate at the 5' end of a sense strand in conjunction with other chemical modifications.
**Figure 11** illustrates the increased potency of modified siRNAs having a cholesterol conjugate at the 5' end of a sense strand.
**Figure 12** illustrates protected RNA nucleotide phosphoramidites that can be used for Dharmacon 2'-ACE RNA synthesis chemistry.
**Figure 13** illustrates an outline of a Dharmacon RNA synthesis cycle.
**Figure 14** illustrates the structure of a preferred 2'-ACE protected RNA.
**Figure 15A** illustrates functional consequences of a single 2'-deoxy modification (sense or antisense strand) on an otherwise naked siRNA.
**Figure 15B** illustrates the functional consequences of two tandem 2'-deoxy modifications (sense or antisense strand) on an otherwise naked siRNA.
**Figure 15C** illustrates the functional consequences of three tandem 2'-deoxy modifications (sense or antisense strand) on an otherwise naked siRNA.
**Figure 16A** illustrates the functionality consequences of a single 2'-O-methyl modification (sense or antisense strand) on an otherwise naked siRNA.
**Figure 16B** illustrates the functionality consequences of two tandem 2'-O-methyl modifications (sense or antisense strand) on an otherwise naked siRNA.
**Figure 16C** illustrates the functional consequences of three tandem 2'-O-methyl modifications (sense or antisense strand) on an otherwise naked siRNA.
**Figure 17** illustrates the consequences of modifications in the sense and the antisense strands on duplex stability.
**Figure 18** illustrates the effect of a conjugate comprising a 5' cholesterol moiety on passive uptake of naked and modified siRNA.
**Figure 19** illustrates the functional consequences of two tandem 2'-deoxy modifications at various positions in a sense strand.
**Figure 20** illustrates the functional consequences of three tandem 2'-deoxy modifications at various positions in a sense strand.
**Figure 21** illustrates the functional consequences of a single 2'-deoxy modification at various positions in an antisense strand.
**Figure 22** illustrates the functional consequences of two tandem 2'-deoxy modifications at various positions in an antisense strand.
**Figure 23** illustrates the functional consequences of three tandem 2'-deoxy modifications at various positions in an antisense strand.
**Figure 24** illustrates the functional consequences of two tandem 2'-O-methyl modifications at various positions in a sense strand.
**Figure 25** illustrates the functional consequences of three tandem 2'-O-methyl modifications at various positions in a sense strand.
**Figure 26** illustrates the functional consequences of a single 2'-O-methyl modification at various positions in an antisense strand.
**Figure 27** illustrates the functional consequences of two tandem 2'-O-methyl modifications at various positions in an antisense strand.
**Figure 28** illustrates the functional consequences of three tandem 2'-O-methyl modifications at various positions in an antisense strand.
**Figure 29** illustrates the functional consequences of two 2'-O-methyl modifications on positions 1 and 2 of (1) the 5' sense, (2) the 5' antisense, or (3) the 5' sense and antisense strands, using siRNAs directed against the human cyclophilin gene.
**Figure 30** illustrates the functional consequences of two 2'-O-methyl modifications on positions 1 and 2 of (1) the 5' sense, (2) the 5' antisense, and (3) the 5' sense and antisense strands using siRNAs directed against the firefly luciferase gene. Note: in this figure, "p" represents phosphorylation of the 5' end of the antisense strand.
**Figure 31** illustrates the functional consequences of two 2'-O-methyl modifications on positions 1 and 2 of (1) the 5' sense, (2) the 5' antisense, and (3) the 5' sense and antisense strands using siRNAs directed against the firefly luciferase gene. Note: in this figure, "p" represents phosphorylation of the 5' end of the antisense strand.
**Figure 32** illustrates the stability of modified siRNAs in human serum. Modification "a" = 2'-O-methyl modification of all Cs and Us (in combination with 3' idT capping) of the sense strand. Modification "b" = 2' F modification of all Cs and Us (in combination with 3' idT capping) of the sense strand.
**Figure 33** illustrates the affinity of modified siRNA-cholesterol conjugates for albumin and other serum proteins.
**Figure 34** illustrates the effects that small molecule conjugates have on siRNA potency
**Figure 35** illustrates the stability of siRNA conjugates in human serum. siRNAa carries (1) 2'-O-methyl groups on all Cs and Us, and (2) a 3' idT cap, on the sense strand. siRNAa-conjugate carries (1) 2'-O-methyl groups on all Cs and Us, (2) a 3' idT cap, and (3) a 5' cholesterol conjugate, on the sense strand.
**Figure 36** illustrates the effects that cholesterol conjugates have on passive siRNA uptake.
**Figure 37A** is a representation of results from a typical serum stability experiment that
shows an ethidium bromide stained gel containing siRNA (unmodified, top, and "molecule 1 modifications" modified, bottom) that have been exposed to serum. For
the purposes of this figure, the term "molecule 1 modifications" refers to molecules that contain 2'-O-methyl modifications on positions 1 and 2 of the sense strand, 2'-O-methyl modifications on all Cs and Us of the sense strand, 2'-fluoro (Fl) modification of all Cs and Us of the antisense strand, and a phosphate modification on the 5' terminus of the antisense strand. **Figure 37B** is a set of line graphs that plot the relative stability of four human cyclophilin B siRNAs in modified and unmodified forms (U1 = 5'GAA AGA GCA UCU ACG GUG A (SEQ. ID NO. 315), U2 = 5'GAA AGG
AUU UGG CUA CAA A (SEQ. ID NO. 316), U3 = 5'ACA GCA AAU UCC AUC GUG U (SEQ. ID NO. 317), and U4 = 5'GGA AAG ACU GUU CCA AAA
A, (SEQ. ID NO. 318), sense strands). The X-axis represents time in hours. The Y-axis represents percentage of duplexes that remain intact. Black squares represent unmodified sequences. White squares represent modified sequences.

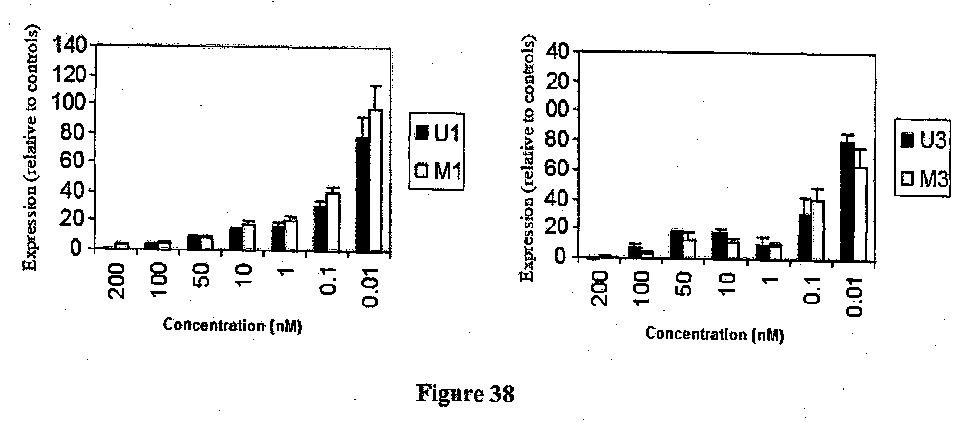

**Figure 38** compares the ability of siRNA (U1 and U3) that are naked or modified (molecule 1 modifications) to silence a given target at varying concentrations. The Y-axis represents the level of expression relative to controls (untransfected cells). The X-axis represents the concentration of siRNA during the transfection procedures. Black bars represent unmodified sequences. White bars represent modified sequences. For the purposes of this figure, the term "molecule 1 modifications" refers to molecules that contain 2'-O-methyl modifications on positions 1 and 2 of the sense strand, 2'-O-methyl modifications on all Cs and Us of the sense strand, 2'-Fluoro (F1) modification of all Cs and Us of the antisense strand, and a phosphate modification on the 5' terminus of the antisense strand.

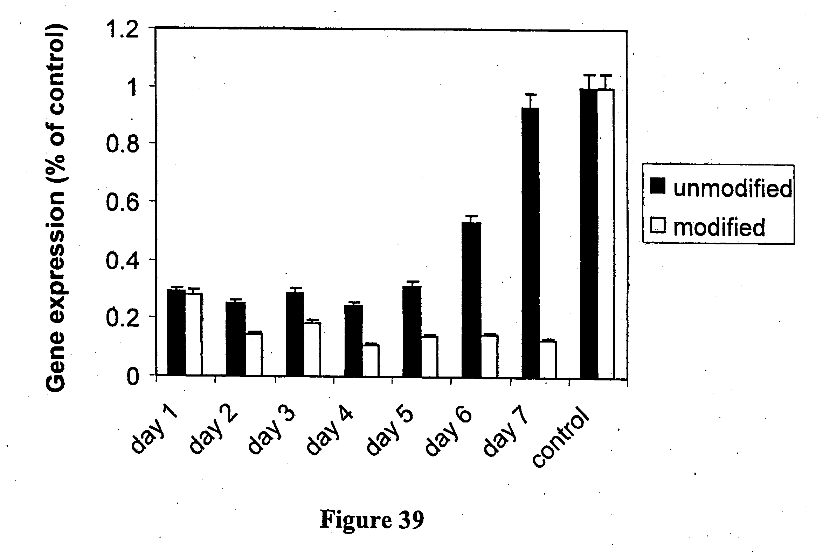

**Figure 39** assesses the level of silencing induced by modified (carrying "molecule 1 modifications) and unmodified siRNA over the course of seven days. The Y-axis represents the level of gene expression relative to controls (untransfected cells). The X-axis represents the number of days after transfection. Black bars represent unmodified sequences. White bars represent modified sequences. For the purposes of this figure, the term "molecule 1 modifications" refers to molecules that contain 2'-O-methyl modifications on positions 1 and 2 of the sense strand, 2'-O-methyl modifications on all Cs and Us of the sense strand, 2'-Fluoro (Fl) modification of all Cs and Us of the antisense strand, and a phosphate modification on the 5' terminus of the antisense strand.

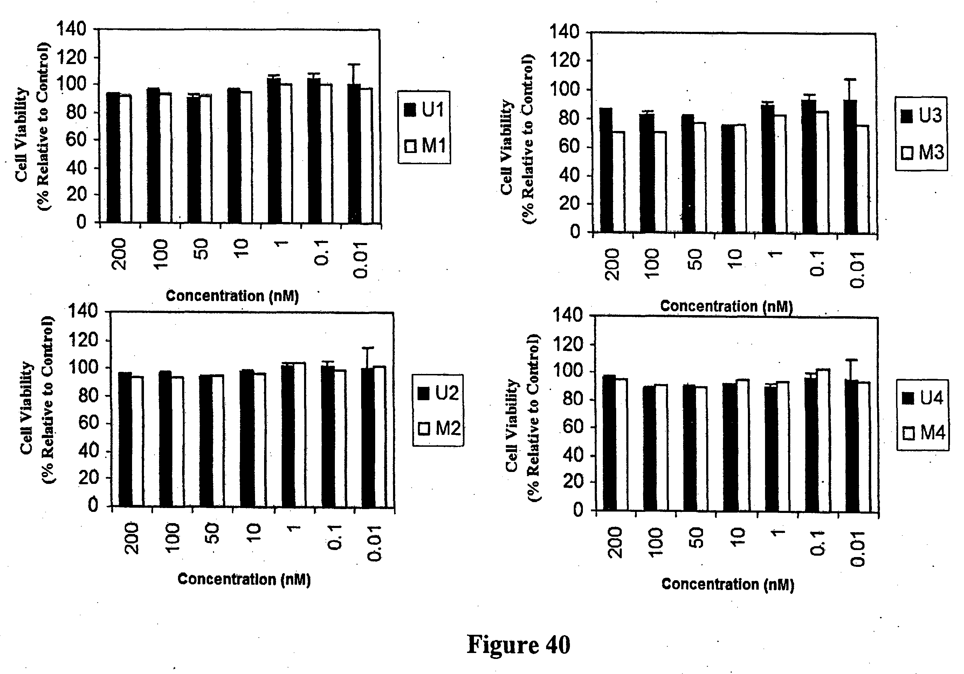

**Figure 40** compares the level of cell death induced by four separate modified (according to molecule 1 modifications) and unmodified siRNAs transfected into cells at varying concentrations. The Y-axis represents the relative level of cell viability (as compared to untransfected cells). The X-axis represents the concentration of the siRNA during the transfection procedures. Cultures were tested 24-48 hours after transfection using an Alamar Blue assay. Black bars represent unmodified sequences. White bars represent modified sequences. For the purposes of this figure, the term "molecule 1 modifications" refers to molecules that contain 2'-O-methyl modifications on positions 1 and 2 of the sense strand, 2'-O-methyl modifications on all Cs and Us of the sense strand, 2'-Fluoro (FI) modification of all Cs and Us of the antisense strand, and a phosphate modification on the 5' terminus of the antisense strand.

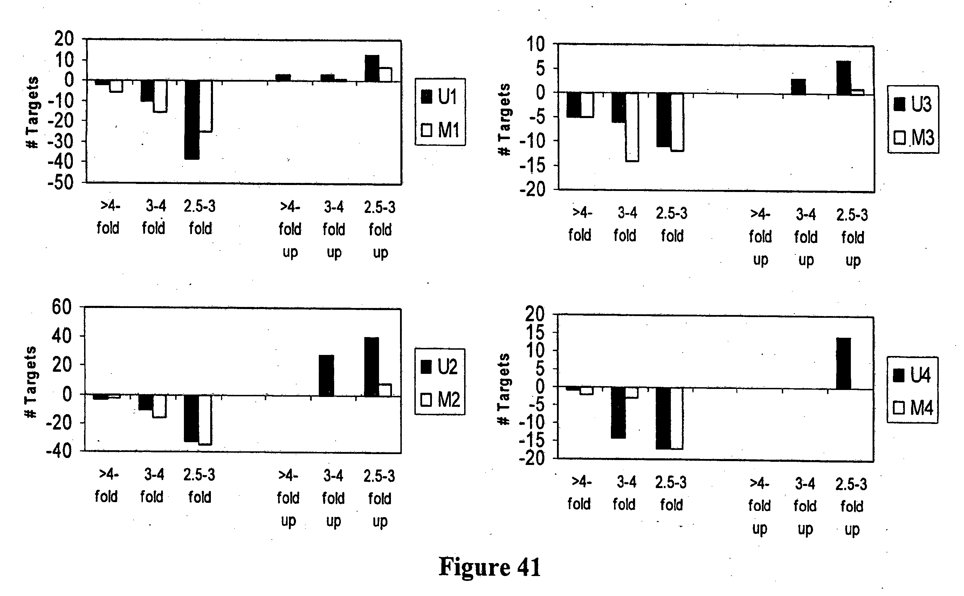

**Figure 41** compares the level of off-targeting by four separate human cyclophilin B siRNAs in modified (according to molecule 1 modifications) and unmodified forms. Data are divided into six separate groups: number of targets that show decreased expression by more than four fold (4x), number of targets that show decreased expression by three to four fold (3-4x), number of targets that show decreased expression by 2.5-3 fold (2.5-3x), number of targets that show increased expression by greater than four fold (>4x), number of targets that show increased expression by three to four fold (3-4x), and number of targets that show increased expression by 2.5-3 fold (2.5-3x). Black bars represent unmodified sequences. White bars represent modified sequences. For the purposes of this figure, the term "molecule 1 modifications" refers to molecules that contain 2'-O-methyl modifications on positions 1 and 2 of the sense strand, 2'-O-methyl modifications on all Cs and Us of the sense strand, 2'-Fluoro (Fl) modification of all Cs and Us of the antisense strand, and a phosphate modification on the 5' terminus of the antisense strand.

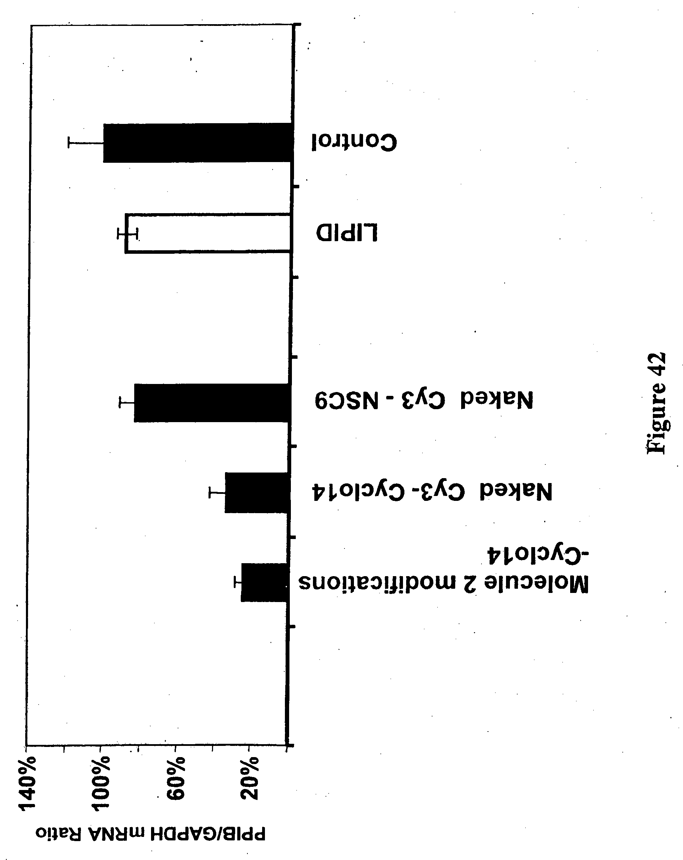

**Figure 42** shows a histogram comparing the gene silencing ability of Cyclo14 siRNA containing 1) molecule 2 modifications 2) naked molecules modified with Cy3, and 3) naked NSC9 molecules modified with Cy3. The Y-axis depicts the level of human cyclophilin B expression relative to a control gene (GAPDH). "Lipid" represents control cells treated with the transfection reagent, Lipofectamine 2000. "Control" represents cells that are untreated. NS9 is non-specific sequence #9. For the purposes of this figure, the term "molecule 2 modifications" refers to molecules that contain 2'-O-methyl modifications on positions 1 and 2 of the sense strand, 2'-O-methyl modifications on all Cs and Us of the sense strand, a Cy3 label on the 5' end of the sense strand, 2'-Fluoro (FI) modifications on all Cs and Us of the antisense strand, and a phosphate modification on the 5' terminus of the antisense strand.

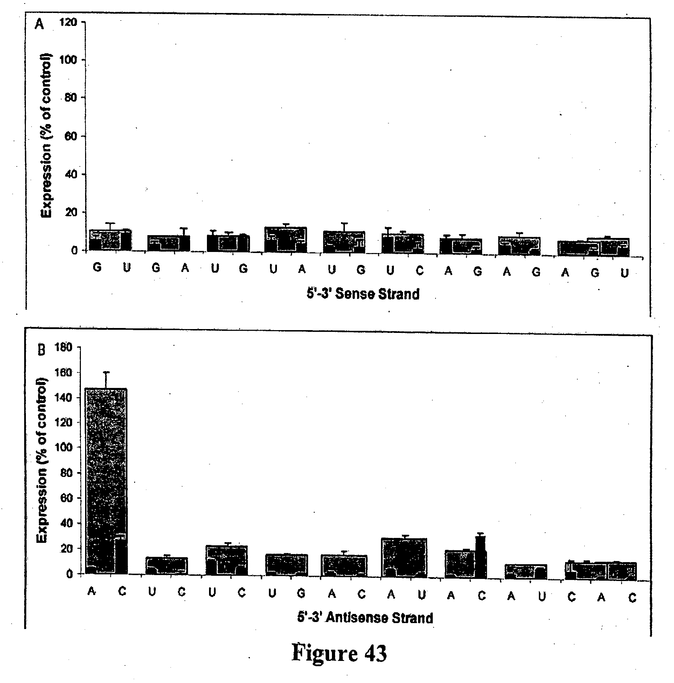

**Figures 43A and 43B** depict the relationship between modification and function for 2'-O-methylated SEAP-2217 siRNA. The figures demonstrate the effect of gene silencing of single base (black bars) and paired (gray) 2'-O-methyl modifications of the sense strand (**Figure 43A**) and antisense strand (**Figure 43B**) of SEAP-2217. The X-axis represents the relative position of the modification along the siRNA (5'→3'). The Y-axis represents the percent expression relative to controls.

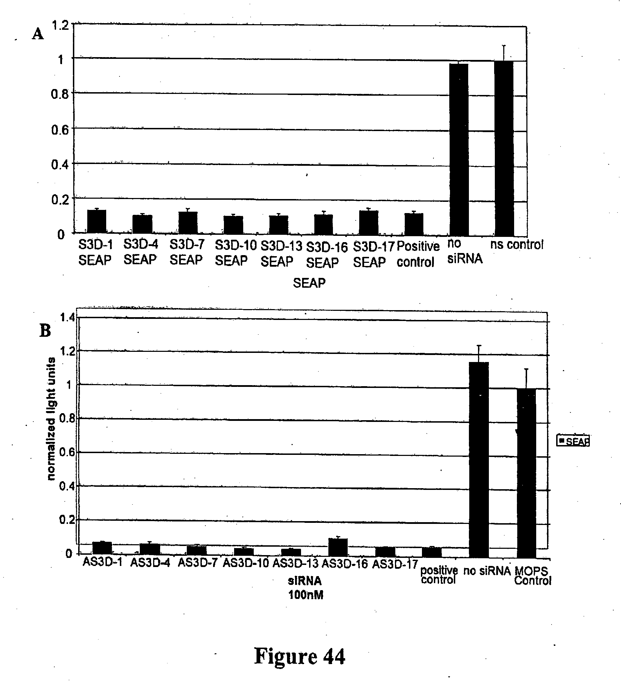

**Figures 44A and 44B** show the effects of the addition of 2'-deoxy groups on SEAP-2217 function. Specifically, three consecutive nucleotides (*e.g*., positions 1, 2, and 3) are modified with the 2'-deoxy group. The identification number associated with each group (*e.g*., S3D-16 is decoded as follows: "S"= sense strand modification, "3" = the number of nucleotides that are consecutively modified, "D"= 2' deoxy modification, "16" = the first position of the modification). **Figure 44A** demonstrates sense strand modifications. **Figure 44B** demonstrates antisense strand modifications. Controls include: no siRNA and non-specific (ns) RNA.

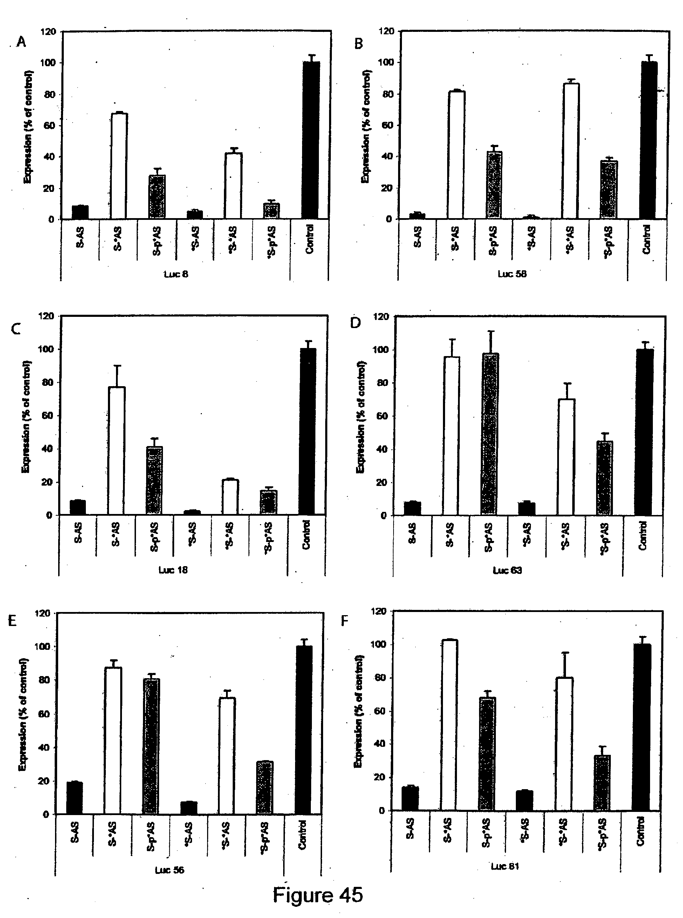

**Figures 45A -45F** show the effects of 2'-O-methylation with or without 5' phosphorylation on the antisense strands of six different luciferase-specific siRNA (luc 8,18, 56, 58, 63, and 81). "S" = sense strand. "AS" = antisense strand. "*" indicates 2'-O-methylation at positions 1 and 2 of the designated strand. "p" indicates 5' phosphorylation of the designated strand. The Y-axis represents the % expression of control (untransfected) cells. "Control" = mock transfected cells.

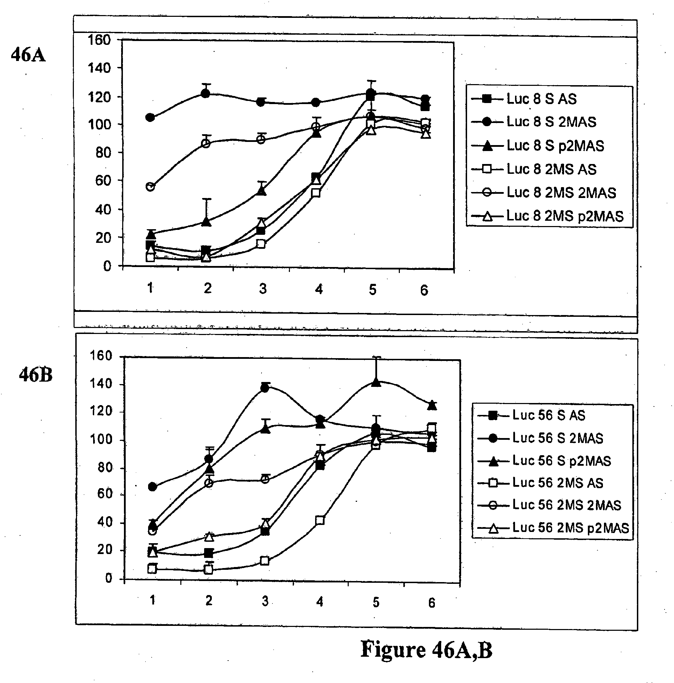

**Figures 46A-E** are representations of the dosage dependence of five luciferase-specific siRNAs (luc 8, 56, 58, 63, 81) produced according to a certain embodiment of the present invention. "S" = sense strand. "AS" = antisense strand. "M" indicates 2'-O-methylation at positions 1 and 2 of the designated strand. "p" indicates 5'phosphorylation of the designated strand. The Y- axis represents the % expression of control (untransfected) cells. The X-axis represents the concentration of the siRNA during the transfection with 1 = 200 nM, 2 = 100 nM, 3 = 10 nM, 4 = 1 nM, 5 = 0.1 nM, and 6 = 0.01 nM.

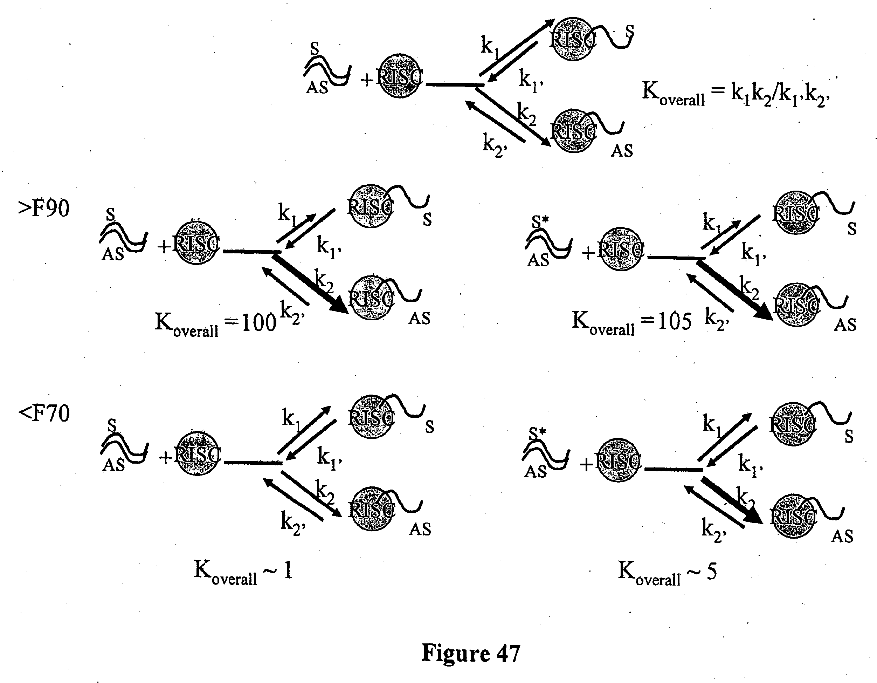

**Figure 47** is a representation of the possible kinetics of sense and antisense strand-RISC interactions, and the sensitivity of this interaction to: (1) 2'-O-methylation (*); and (2) the relative functionality of the molecule. In highly functional molecules (*e.g*. >F90, greater than 90% functionality), RISC shows a strong preference for association with the antisense strand (Kₒᵥₑᵣₐₗₗ = 100). Modification of the sense strand with 2'-O-methyl groups at nucleotide positions 1 and 2 further biases the AS strand preference, but the relative percent change is minor. Sense and antisense strands from poor or moderately functional duplexes exhibit a more balanced equilibrium with RISC (Kₒᵥₑᵣₐₗₗ ~ 1). In these cases, modification of S essentially eliminates this strand's ability to associate with RISC and strongly biases the preferences toward RISC-AS interactions.

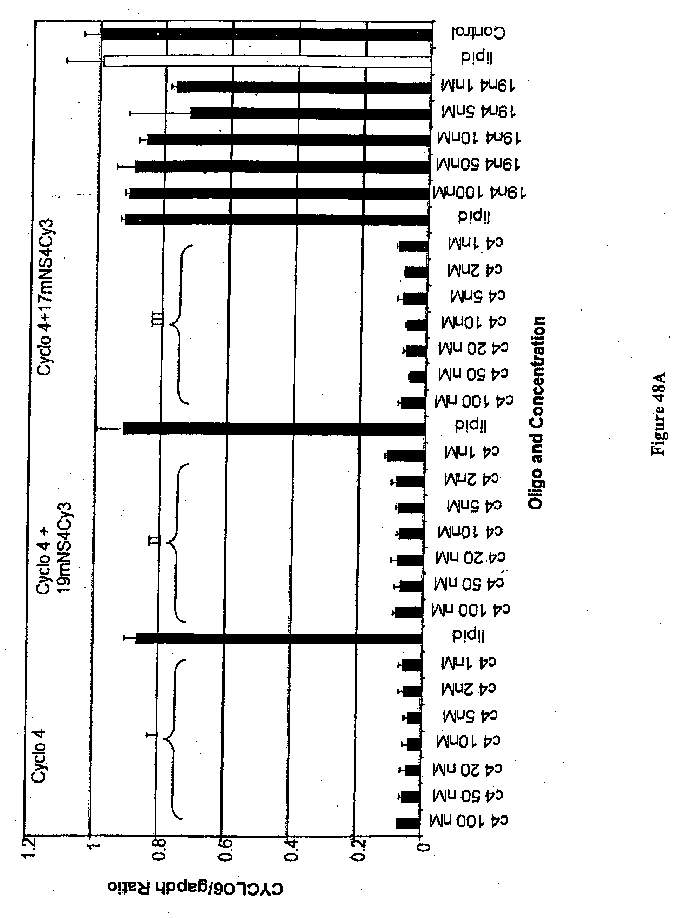

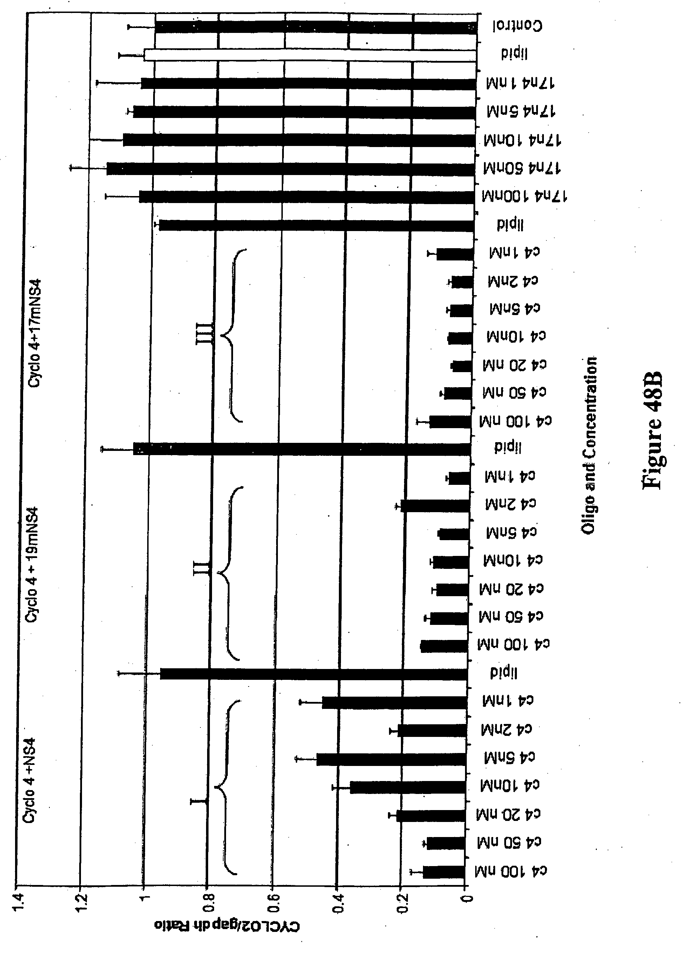

**Figures 48A** **and** **48B** are representations of competition studies between cyclo4 and NS4 siRNA. **A-I:** Cyclo4 siRNA was transfected into cells at varying concentrations, **A-II:** Cyclo 4 (at varying concentrations) plus a Cy3 conjugated 19 bp NS4 duplex modified with 2'-O-methyl groups on positions 1 and 2 of the sense and antisense strand, 2'-O-methyl groups on the Cs and Us of the sense strand, 2' Fl groups on the Cs and Us of the antisense strand, and a Cy3 group on the 5' end of the sense strand; A-**III:** Cyclo 4 (at varying concentrations) plus a Cy3 conjugated 17 bp NS4 duplex modified with 2'-O-methyl groups on positions 1 and 2 of the sense and antisense strand, 2'-O-methyl groups on the Cs and Us of the sense strand, and 2' Fl groups added to the Cs and Us of the antisense strand; **B-I** Cyclo 4 (at varying concentrations) plus a 19 bp NS4 duplex; **B-II:** Cyclo 4 (at varying concentrations) plus a 19 bp NS4 duplex modified with 2'-O-methyl groups on positions 1 and 2 of the sense and antisense strand, 2'-O-methyl groups on the Cs and Us of the sense strand, and 2' Fl groups on the Cs and Us of the antisense strand; **B-III:** Cyclo 4 (at varying concentrations) plus a 17 bp NS4 duplex modified with 2'-O-methyl groups on positions 1 and 2 of the sense and antisense strand, 2'-O-methyl groups on the Cs and Us of the sense strand, and 2' FI groups added to the Cs and Us of the antisense strand. Note: All transfections were performed using Lipofectamine 2000 (manufacturer's instructions) and total nucleic acid concentrations of 100nM. Data was normalized to internal GAPDH levels.

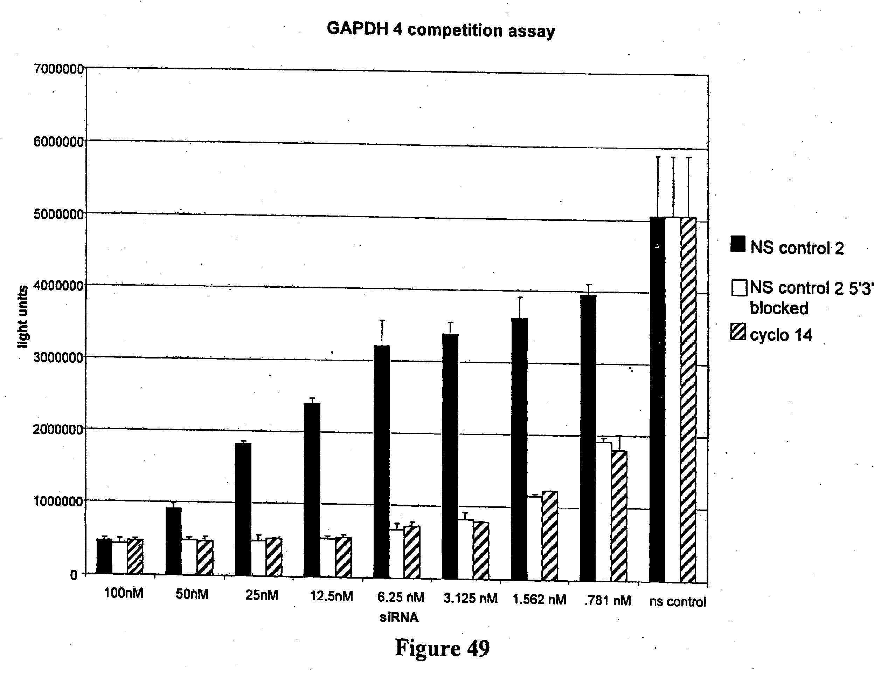

**Figure 49** is a representation of competition studies between GAPDH and Non-specific Control #2. GAPDH siRNA were introduced into HeLa cells at varying concentrations (0.781-100 nM) along with: (1) a non-competing siRNA, cyclo14; (2) a competing siRNA (non-specific sequence #2); or (3) the competing siRNA labeled with 2'-O-methyl groups on positions 1 and 2 of both the sense and antisense strand.

### DETAILED DESCRIPTION

The present invention will now be described in connection with preferred embodiments. These embodiments are presented to aid in an understanding of the present invention and are not intended, and should not be construed, to limit the invention in any way.

This disclosure is not a primer on compositions and methods for performing RNA interference. Basic concepts known to those skilled in the art have not been set forth in detail

The present invention is directed to compositions and methods for performing RNA interference, including siRNA-induced gene silencing. Through the use of the present invention, modified polynucleotides, and derivatives thereof, one may improve the efficiency of RNA interference applications.

Unless stated otherwise, the following terms and phrases have the meanings provided below:

### Alkyl

The term "alkyl" refers to a hydrocarbyl moiety that can be saturated or unsaturated, and substituted or unsubstituted. It may comprise moieties that are linear, branched, cyclic and/or heterocyclic, and contain functional groups such as ethers, ketones, aldehydes, carboxylates, etc.

Exemplary alkyl groups include but are not limited to substituted and unsubstituted groups of methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl and alkyl groups of higher number of carbons, as well as 2-methylpropyl, 2-methyl-4-ethylbutyl, 2,4-diethylpropyl, 3-propylbutyl, 2,8-dibutyldecyl, 6,6-dimethyloctyl, 6-propyl-6-butyloctyl, 2-methylbutyl, 2-methylpentyl, 3-methylpentyl, and 2-ethylhexyl. The term alkyl also encompasses alkenyl groups, such as vinyl, allyl, aralkyl and alkynyl groups.

Substitutions within an alkyl group can include any atom or group that can be tolerated in the alkyl moiety, including but not limited to halogens, sulfurs, thiols, thioethers, thioesters, amines (primary, secondary, or tertiary), amides, ethers, esters, alcohols and oxygen. The alkyl groups can by way of example also comprise modifications such as azo groups, keto groups, aldehyde groups, carboxyl groups, nitro, nitroso or nitrile groups, heterocycles such as imidazole, hydrazino or hydroxylamino groups, isocyanate or cyanate groups, and sulfur containing groups such as sulfoxide, sulfone, sulfide, and disulfide.

Further, alkyl groups may also contain hetero substitutions, which are substitutions of carbon atoms, by for example, nitrogen, oxygen or sulfur. Heterocyclic substitutions refer to alkyl rings having one or more heteroatoms. Examples of heterocyclic moieties include but are not limited to morpholino, imidazole, and pyrrolidino.

### 2'-O-alkyl modified nucleotide

The phrase "2'-O-alkyl modified nucleotide" refers to a nucleotide unit having a sugar moiety, for example a deoxyribosyl moiety that is modified at the 2' position such that an oxygen atom is attached both to the carbon atom located at the 2' position of the sugar and to an alkyl group.

### Amine and 2' amine modified nucleotide

The term "amine" refers to moieties that can be derived directly or indirectly from ammonia by replacing one, two, or three hydrogen atoms by other groups, such as, for example, alkyl groups. Primary amines have the general structures RNH₂ and secondary amines have the general structure R₂NH. The phrase "2' amine modified nucleotide" refers to a nucleotide unit having a sugar moiety that is modified with an amine or nitrogen-containing group attached to the 2' position of the sugar.

The term amine includes, but is not limited to methylamine, ethylamine, propylamine, isopropylamine, aniline, cyclohexylamine, benzylamine, polycyclic amines, heteroatom substituted aryl and alkylamines, dimethylamine, diethylamine, diisopropylamine, dibutylamine, methylpropylamine, methylhexylamine, methylcyclopropylamine, ethylcylohexylamine, methylbenzylamine, methycyclohexylmethylamine, butylcyclohexylamine, morpholine, thiomorpholine, pyrrolidine, piperidine, 2,6-dimethylpiperidine, piperazine, and heteroatom substituted alkyl or aryl secondary amines.

### Antisense strand

The phrase "antisense strand" as used herein, refers to a polynucleotide or region of a polynucleotide that is substantially or 100% complementary to a target nucleic acid of interest. An antisense strand may be comprised of a polynucleotide region that is RNA, DNA or chimeric RNA/DNA. For example, an antisense strand may be complementary, in whole or in part, to a molecule of messenger RNA, an RNA sequence that is not mRNA (*e.g*., tRNA, rRNA and hnRNA) or a sequence of DNA that is either coding or non-coding. The phrase "antisense strand" includes the antisense region of both polynucleotides that are formed from two separate strands, as well as unimolecular siRNAs that are capable of forming hairpin structures. The phrases "antisense strand" and "antisense region" are intended to be equivalent and are used interchangeably. The antisense strand can be modified with a diverse group of small molecules and/or conjugates.

### 2' carbon modification

The phrase "2' carbon modification" refers to a nucleotide unit having a sugar moiety, for example a deoxyribosyl moiety that is modified at the 2' position. A "2'-O-alkyl modified nucleotide" is modified at this position such that an oxygen atom is attached both to the carbon atom located at the 2' position of the sugar and to an alkyl group, *e.g*., 2'-O-methyl, 2'-O-ethyl, 2'-O-propyl, 2'-O-isopropyl, 2'-O-butyl, 2-O-isobutyl, 2'-O-ethyl-O-methyl (-OCH₂CH₂OCH₃), and 2'-O-ethyl-OH (-OCH₂CH₂OH). A "2' carbon sense modification" refers to a modification at the 2' carbon position of a nucleotide on the sense strand or within a sense region of polynucleotide. A "2' carbon antisense modification" refers to a modification at the 2' carbon position of a nucleotide on the antisense strand or within an antisense region of polynucleotide.

### Complementary

The term "complementary" refers to the ability of polynucleotides to form base pairs with one another. Base pairs are typically formed by hydrogen bonds between nucleotide units in antiparallel polynucleotide strands or regions. Complementary polynucleotide strands or regions can base pair in the Watson-Crick manner (*e.g*., A to T, A to U, C to G), or in any other manner that allows for the formation of stable duplexes.

Perfect complementarity or 100% complementarity refers to the situation in which each nucleotide unit of one polynucleotide strand or region can hydrogen bond with each nucleotide unit of a second polynucleotide strand or region. Less than perfect complementarity refers to the situation in which some, but not all, nucleotide units of two strands or two regions can hydrogen bond with each other. For example, for two 20-mers, if only two base pairs on each strand can hydrogen bond with each other, the polynucleotide strands or regions exhibit 10% complementarity. In the same example, if 18 base pairs on each strand or each region can hydrogen bond with each other, the polynucleotide strands exhibit 90% complementarity. Substantial complementarity refers to polynucleotide strands or regions exhibiting 90% or greater complementarity.

### Conjugate and terminal conjugate

The term "conjugate" refers to a molecule or moiety that alters the physical properties of an siRNA such as those that increase stability and/or facilitate uptake of siRNA by itself. A "terminal conjugate" may be attached directly or through a linker to the 3' and/or 5' end of an siRNA. An internal conjugate may be attached directly or indirectly through a linker to a base, to the 2' position of the ribose, or to another suitable position or positions, for example, 5-aminoallyl uridine.

In an siRNA having two separate strands that are not attached to one another (*i.e.,* an siRNA that is not a unimolecular or hairpin siRNA), one or both 5' ends of the strands can bear a conjugate, and/or one or both 3' ends of the strands comprising the siRNA can bear a conjugate.

Conjugates may, for example, be amino acids, peptides, polypeptides, proteins, antibodies, antigens, toxins, hormones, lipids, nucleotides, nucleosides, sugars, carbohydrates, polymers such as polyethylene glycol and polypropylene glycol, as well as analogs or derivatives of all of these classes of substances. Additional examples of conjugates also include steroids, such as cholesterol, phospholipids, di- and tri-acylglycerols, fatty acids, hydrocarbons that may or may not contain unsaturation or substitutions, enzyme substrates, biotin, digoxigenin, and polysaccharides. Still other examples include thioethers such as hexyl-S-tritylthiol, thiocholesterol, acyl chains such as dodecandiol or undecyl groups, phospholipids such as di-hexadecyl-rac-glycerol, triethylammonium 1,2-di-O-hexadecyl-rac-glycero-3-H-phosphonate, polyamines, polyethylene glycol, adamantane acetic acid, palmityl moieties, octadecylamine moieties, hexylaminocarbonyl-oxycholesterol, farnesyl, geranyl and geranylgeranyl moieties.

Conjugates can also be detectable labels. For example, conjugates can be fluorophores. Conjugates can include fluorophores such as TAMRA, BODIPY, Cyanine derivatives such as Cy3 or Cy5 Dabsyl, fluoroscein, or any other suitable fluorophore known in the art.

A conjugate may be attached to any position on the terminal nucleotide that is convenient and that does not substantially interfere with the desired activity of the siRNA(s) that bear it, for example the 3' or 5' position of a ribosyl sugar. A conjugate substantially interferes with the desired activity of an siRNA if it adversely affects its functionality such that the ability of the siRNA to mediate RNA interference is reduced by greater than 80% in an *in vitro* assay employing cultured cells, where the functionality is measured at 24 hours post transfection.

### Deoxynucleotide

The term "deoxynucleotide" refers to a nucleotide or polynucleotide lacking an OH group at the 2' or 3' position of a sugar moiety with appropriate bonding and/or 2',3' terminal dideoxy, instead having a hydrogen bonded to the 2' and/or 3' carbon.

### Deoxyribonucleotide

The terms "deoxyribonucleotide" and "DNA" refer to a nucleotide or polynucleotide comprising at least one ribosyl moiety that has an H at its 2' position of a ribosyl moiety.

### Downstream

One region of a strand of nucleotides is considered to be downstream of a second region, if the 5' most portion of the first region is the closest portion of that region to the 3' end of the second region.

### Exaequo agent

The phrase "exaequo agent" refers to a nucleic acid that is, from the perspective of its participation in the RNAi pathway, or ability to compete with other nucleic acids for the ability to participate in the RNAi pathway, inert or semi-inert. Molecules can be used as an exaequo agent whereby said agents are used to equalize or to make level the total amount of nucleic acid in a solution.

### First 5' terminal antisense nucleotide

The phrase "first 5' terminal antisense nucleotide" refers to the nucleotide of the antisense strand or region that is located at the 5' most position of that strand with respect to the bases of the antisense strand or region that have corresponding complementary bases on the sense strand or region. Thus, in an siRNA that is made of two separate strands (*i.e*., not a unimolecular or hairpin siRNA), it refers to the 5' most base other than bases that are part of any 5' overhang on the antisense strand. When the first 5' terminal antisense nucleotide is part of a hairpin molecule, the term "terminal" refers to the 5' most relative position within the antisense region and thus is the 5' most nucleotide of the antisense region.

### First 5' terminal sense nucleotide

The phrase "first 5' terminal sense nucleotide" is defined in reference to the antisense nucleotide. In molecules that are comprised of two separate strands (*i.e*., not a unimolecular or hairpin siRNA), it refers to the nucleotide of the sense strand that is located at the 5' most position of that strand with respect to the bases of the sense strand that have corresponding complementary bases on the antisense strand. Thus, in an siRNA that is made of two separate strands (*i.e*., not a unimolecular or hairpin siRNA), it is the 5' most base other than bases that are,part of any 5' overhang on the sense strand or region. When the first 5' terminal sense nucleotide is part of a unimolecular siRNA that is capable of forming a hairpin molecule, the term "terminal" refers to the relative position within the sense strand or region as measured by the distance from the base complementary to the first 5' terminal antisense nucleotide.

### Functional

SiRNA may be divided into five (5) groups (non-functional, semi-functional, functional, highly functional, and hyper-functional) based on the level or degree of silencing that they induce in cultured cell lines. As used herein, these definitions are based on a set of conditions where the siRNA is transfected into said cell line at a concentration of 100 nM and the level of silencing is tested at a time of roughly 24 hours after transfection, and not exceeding 72 hours after transfection. In this context, "non-functional siRNA" are defined as those siRNA that induce less than 50% (<50%) target silencing. "Semi-functional siRNA" induce 50-79% target silencing. "Functional siRNA" are molecules that induce 80-95% gene silencing. "Highly-functional siRNA" are molecules that induce greater than 95% gene silencing. "Hyperfunctional siRNA" are a special class of molecules. For purposes of this document, hyperfunctional siRNA are defined as those molecules that: (1) induce greater than 95% silencing of a specific target when they are transfected at subnanomolar concentrations (*i.e*., less than one nanomolar); and/or (2) induce functional (or better) levels of silencing for greater than 96 hours. These relative functionalities (though not intended to be absolutes) may be used to compare siRNA to a particular target for applications such as functional genomics, target identification and therapeutics.

### Functional Dose

A "functional dose" refers to a dose of siRNA that will be effective at causing a greater than or equal to 95% reduction in mRNA at levels of 100 nM at 24, 48, 72, and 96 hours following administration, while a "marginally functional dose" of siRNA will be effective at causing a greater than or equal to 50% reduction of mRNA at 100 nM at 24 hours following administration and a "non-functional dose" of RNA will cause a less than 50% reduction in mRNA levels at 100 nM at 24 hours following administration.

### Halogen

The term "halogen" refers to an atom of either fluorine, chlorine, bromine, iodine or astatine. The phrase "2' halogen modified nucleotide" refers to a nucleotide unit having a sugar moiety that is modified with a halogen at the 2' position, attached directly to the 2' carbon.

### 2' halogen modified pyrimidine

The phrase "2' halogen modified pyrimidine" refers to a pyrimidine (*e.g*. cytosine or uracil) that contains a halogen group attached to the 2' carbon of the sugar of a nucleotide.

### Internucleotide linkage_

The phrase "internucleotide linkage" refers to the type of bond or link that is present between two nucleotide units in an siRNA and may be modified or unmodified. The phrase "modified internucleotide linkage" includes all modified internucleotide linkages now known in the art or that come to be known and that, from reading this disclosure, one skilled in the art will conclude is useful in connection with the present invention. Internucleotide linkages may have associated counterions, and the term is meant to include such counterions and any coordination complexes that can form at the internucleotide linkages.

Modifications of internucleotide linkages include, but are not limited to, phosphorothioates, phosphorodithioates, methylphosphonates, 5'-alkylenephosphonates, 5'-methylphosphonate, 3'-alkylene phosphonates, borontrifluoridates, borano phosphate esters and selenophosphates of 3'-5' linkage or 2'-5' linkage, phosphotriesters, thionoalkylphosphotriesters, hydrogen phosphonate linkages, alkyl phosphonates, alkylphosphonothioates, arylphosphonothioates, phosphoroselenoates, phosphorodiselenoates, phosphinates, phosphoramidates, 3'-alkylphosphoramidates, aminoalkylphosphoramidates, thionophosphoramidates, phosphoropiperazidates, phosphoroanilothioates, phosphoroanilidates, ketones, sulfones, sulfonamides, carbonates, carbamates, methylenehydrazos, methylenedimethylhydrazos, formacetals, thioformacetals, oximes, methyleneiminos, methylenemethyliminos, thioamidates, linkages with riboacetyl groups, aminoethyl glycine, silyl or siloxane linkages, alkyl or cycloalkyl linkages with or without heteroatoms of, for example, 1 to 10 carbons that can be saturated or unsaturated and/or substituted and/or contain heteroatoms, linkages with morpholino structures, amides, polyamides wherein the bases can be attached to the aza nitrogens of the backbone directly or indirectly, and combinations of such modified internucleotide linkages within an siRNA.

### Linker

A "linker" is a moiety that attaches other moieties to each other such as a nucleotide and its conjugate. A linker may be distinguished from a conjugate in that while a conjugate increases the stability and/or ability of a molecule to be taken up by a cell, a linker merely attaches a conjugate to the molecule that is to be introduced into the cell.

By way of example, linkers can comprise modified or unmodified nucleotides, nucleosides, polymers, sugars and other carbohydrates, polyethers such as, for example, polyethylene glycols, polyalcohols, polypropylenes, propylene glycols, mixtures of ethylene and propylene glycols, polyalkylamines, polyamines such as spermidine, polyesters such as poly(ethyl acrylate), polyphosphodiesters, and alkylenes. An example of a conjugate and its linker is cholesterol-TEG-phosphoramidites, wherein the cholesterol is the conjugate and the tetraethylene glycol and phosphate serve as linkers.

### Nucleotide

The term "nucleotide" refers to a ribonucleotide or a deoxyribonucleotide or modified form thereof, as well as an analog thereof. Nucleotides include species that comprise purines, *e.g*., adenine, hypoxanthine, guanine, and their derivatives and analogs, as well as pyrimidines, *e.g*., cytosine, uracil, thymine, and their derivatives and analogs.

Nucleotide analogs include nucleotides having modifications in the chemical structure of the base, sugar and/or phosphate, including, but not limited to, 5-position pyrimidine modifications, 8-position purine modifications, modifications at cytosine exocyclic amines, and substitution of 5-bromo-uracil; and 2'-position sugar modifications, including but not limited to, sugar-modified ribonucleotides in which the 2'-OH is replaced by a group such as an H, OR, R, halo, SH, SR, NH₂, NHR, NR₂, or CN, wherein R is an alkyl moiety as defined herein. Nucleotide analogs are also meant to include nucleotides with bases such as inosine, queuosine, xanthine, sugars such as 2'-methyl ribose, non-natural phosphodiester linkages such as methylphosphonates, phosphorothioates and peptides.

Modified bases refer to nucleotide bases such as, for example, adenine, guanine, cytosine, thymine, and uracil, xanthine, inosine, and queuosine that have been modified by the replacement or addition of one or more atoms or groups. Some examples of types of modifications that can comprise nucleotides that are modified with respect to the base moieties, include but are not limited to, alkylated, halogenated, thiolated, aminated, amidated, or acetylated bases, in various combinations. More specific modified bases include, for example, 5-propynyluridine, 5-propynylcytidine, 6-methyladenine, 6-methylguanine, N,N,-dimethyladenine, 2-propyladenine, 2-propylguanine, 2-aminoadenine, 1-methylinosine, 3-methyluridine, 5-methylcytidine, 5-methyluridine and other nucleotides having a modification at the 5 position, 5-(2-amino)propyl uridine, 5-halocytidine, 5-halouridine, 4-acetylcytidine, 1-methyladenosine, 2-methyladenosine, 3-methylcytidine, 6-methyluridine, 2-methylguanosine, 7-methylguanosine, 2,2-dimethylguanosine, 5-methylaminoethyluridine, 5-methyloxyuridine, deazanucleotides such as 7-deaza-adenosine, 6-azouridine, 6-azocytidine, 6-azothymidine, 5-methyl-2-thiouridine, other thio bases such as 2-thiouridine and 4-thiouridine and 2-thiocytidine, dhydrouridine, pseudouridine, queuosine, archaeosine, naphthyl and substituted naphthyl groups, any O- and N-alkylated purines and pyrimidines such as N6-methyladenosine, 5-methylcarbonylmethyluridine, uridine 5-oxyacetic acid, pyridine-4-one, pyridine-2-one, phenyl and modified phenyl groups such as aminophenol or 2,4,6-trimethoxy benzene, modified cytosines that act as G-clamp nucleotides, 8-substituted adenines and guanines, 5-substituted uracils and thymines, azapyrimidines, carboxyhydroxyalkyl nucleotides, carboxyalkylaminoalkyl nucleotides, and alkylcarbonylalkylated nucleotides. Modified nucleotides also include those nucleotides that are modified with respect to the sugar moiety, as well as nucleotides having sugars or analogs thereof that are not ribosyl. For example, the sugar moieties may be, or be based on, mannoses, arabinoses, glucopyranoses, galactopyranoses, 4'-thioribose, and other sugars, heterocycles, or carbocycles. The term nucleotide is also meant to include what are known in the art as universal bases. By way of example, universal bases include but are not limited to 3-nitropyrrole, 5-nitroindole, or nebularine.

Further, the term nucleotide also includes those species that have a detectable label, such as for example a radioactive or fluorescent moiety, or mass label attached to the nucleotide.

### Nucleotide unit

The phrase "nucleotide unit" refers to a single nucleotide residue and is comprised of a modified or unmodified nitrogenous base, a modified or unmodified sugar, and a modified or unmodified moiety that allows for linking of two nucleotides together or a conjugate that precludes further linkage.

### Off-Target

The term "off-target" and the phrase "off-target effects" refer to any instance in which an siRNA or shRNA directed against a given target causes an unintended effect by interacting either directly or indirectly with another mRNA sequence, a DNA sequence or a cellular protein or other moiety. For example, an "off-target effect" may occur when there is a simultaneous degradation of other transcripts due to partial homology or complementarity between that other transcript and the sense and/or antisense strand of the siRNA or shRNA

### Orthoester

The term "orthoester protected" or "orthoester modified" refers to modification of a sugar moiety in a nucleotide unit with an orthoester. Preferably, the sugar moiety is a ribosyl moiety. In general, orthoesters have the structure RC(OR')₃ wherein R' can be the same or different, R can be an H, and wherein the underscored C is the central carbon of the orthoester. The orthoesters of the invention are comprised of orthoesters wherein a carbon of a sugar moiety in a nucleotide unit is bonded to an oxygen, which is in turn bonded to the central carbon of the orthoester. To the central carbon of the orthoester is, in turn, bonded two oxygens, such that in total three oxygens bond to the central carbon of the orthoester. These two oxygens bonded to the central carbon (neither of which is bonded to the carbon of the sugar moiety) in turn, bond to carbon atoms that comprise two moieties that can be the same or different. For example, one of the oxygens can be bound to an ethyl moiety, and the other to an isopropyl moiety. In one example, R can be an H, one R' can be a ribosyl moiety, and the other two R' can be two 2-ethyl-hydroxyl moieties. Orthoesters can be placed at any position on the sugar moiety, such as, for example, on the 2', 3' and/or 5' positions. Preferred orthoesters, and methods of making orthoester protected polynucleotides, are described in US Patent Nos. 5,889,136 and 6,008,400.

### Overhang

The term "overhang" refers to terminal non-base pairing nucleotide(s) resulting from one strand or region extending beyond the terminus of the complementary strand to which the first strand or region forms a duplex. One or both of two polynucleotides or polynucleotide regions that are capable of forming a duplex through hydrogen bonding of base pairs may have a 5' and/or 3' end that extends beyond the 3' and/or 5' end of complementarity shared by the two polynucleotides or regions. The single-stranded region extending beyond the 3' and/or 5' end of the duplex is referred to as an overhang.

### Pharmaceutically acceptable carrier

The phrase "pharmaceutically acceptable carrier" includes compositions that facilitate the introduction of dsRNA, dsDNA, or dsRNA/DNA hybrids into a cell and includes but is not limited to solvents or dispersants, coatings, anti-infective agents, isotonic agents, and agents that mediate absorption time or release of the inventive polynucleotides and siRNA.

### Polynucleotide

The term "polynucleotide" refers to polymers of nucleotides, and includes but is not limited to DNA, RNA, DNA/RNA hybrids including polynucleotide chains of regularly and irregularly alternating deoxyribosyl moieties and ribosyl moieties (i.e., wherein alternate nucleotide units have an -OH, then an -H, then an -OH, then an -H, and so on at the 2' position of a sugar moiety), and modifications of these kinds of polynucleotides wherein the attachment of various entities or moieties to the nucleotide units at any position are included. Unless otherwise specified, or clear from context, the term "polynucleotide" includes both unimolecular siRNAs and siRNAs comprised of two separate strands.

### Polyribonucleotide

The term "polyribonucleotide" refers to a polynucleotide comprising two or more modified or unmodified ribonucleotides and/or their analogs.

### Ribonucleotide and ribonucleic acid

The term "ribonucleotide" and the phrase "ribonucleic acid" (RNA), refer to a modified or unmodified nucleotide or polynucleotide comprising at least one ribonucleotide unit. A ribonucleotide unit comprises an oxygen attached to the 2' position of a ribosyl moiety having a nitrogenous base attached in N-glycosidic linkage at the 1' position of a ribosyl moiety, and a moiety that either allows for linkage to another nucleotide or precludes linkage.

### RNA interference and RNAi

The phrase "RNA interference" and the term "RNAi," are synonymous and refer to the process by which a polynucleotide or siRNA comprising at least one ribonucleotide unit exerts an effect on a biological process. The process includes, but is not limited to, gene silencing by degrading mRNA, attenuating translation, interactions with tRNA, rRNA, hnRNA, cDNA and genomic DNA, as well as methylation of DNA with ancillary proteins.

### Second 5' terminal antisense nucleotide

The phrase "second 5' terminal antisense nucleotide" refers to the nucleotide that is immediately adjacent to the first 5' terminal antisense nucleotide and attached to the 3' position of the first 5' terminal antisense nucleotide. Thus, it is the second most 5' nucleotide of the antisense strand or region within the set of nucleotides for which there are corresponding sense nucleotides.

### Second 5' terminal sense nucleotide

The phrase "second 5' terminal sense nucleotide" refers to the nucleotide that is immediately adjacent to the first 5' terminal sense nucleotide and attached to the 3' position of the first 5' terminal sense nucleotide. Thus, it is the second most 5' nucleotide of the sense strand or region within the set of nucleotides for which there are corresponding antisense nucleotides.

### Sense strand

The phrase "sense strand" refers to a polynucleotide or region that has the same nucleotide sequence, in whole or in part, as a target nucleic acid such as a messenger RNA or a sequence of DNA. The phrase "sense strand" includes the sense region of both polynucleotides that are formed from two separate strands, as well as unimolecular siRNAs that are capable of forming hairpin structures. When a sequence is provided, by convention, unless otherwise indicated, it is the sense strand (or region), and the presence of the complementary antisense strand (or region) is implicit. The phrases "sense strand" and "sense region" are intended to be equivalent and are used interchangeably.

### siRNA or short interfering DNA

The term "siRNA" and the phrase "short interfering RNA" refer to unimolecular nucleic acids and to nucleic acids comprised of two separate strands that are capable of performing RNAi and that have a duplex region that is between 18 and 30 base pairs in length. Additionally, the term siRNA and the phrase "short interfering RNA" include nucleic acids that also contain moieties other than ribonucleotide moieties, including, but not limited to, modified nucleotides, modified internucleotide linkages, non-nucleotides, deoxynucleotides and analogs of the aforementioned nucleotides.

siRNAs can be duplexes, and can also comprise short hairpin RNAs, RNAs with loops as long as, for example, 4 to 23 or more nucleotides, RNAs with stem loop bulges, micro-RNAs, and short temporal RNAs. RNAs having loops or hairpin loops can include structures where the loops are connected to the stem by linkers such as flexible linkers. Flexible linkers can be comprised of a wide variety of chemical structures, as long as they are of sufficient length and materials to enable effective intramolecular hybridization of the stem elements. Typically, the length to be spanned is at least about 10-24 atoms.

When the siRNAs are hairpins, the sense strand and antisense strand are part of one longer molecule.

### Stabilized

The term "stabilized" refers to the ability of the dsRNAs to resist degradation while maintaining functionality and can be measured in terms of its half life in the presence of, for example, biological materials such as serum. The half-life of an siRNA in, for example, serum refers to the time taken for the 50% of the siRNA to be degraded.

### Substantial complementarity

Substantial complementarity refers to polynucleotide strands exhibiting 90% or greater complementarity.

### Trackability

The term "trackability" refers to the ability to follow the movement or localization of a molecule after said molecule has been, e.g., introduced into a celL Molecules that are "trackable" are useful in monitoring the success or failure of, *e.g*., a cellular transfection procedure.

### Preferred Embodiments

The present invention will now be described in connection with preferred embodiments. These embodiments are presented in order to aid in an understanding of the present invention and are not intended, and should not be construed, to limit the invention in any way.

In a first embodiment that is related to the present invention, there is provided an siRNA that has a sense strand that comprises a polynucleotide comprised of at least one orthoester modified nucleotide, and an antisense strand that comprises a polynucleotide having at least one 2' modified nucleotide unit. Preferably, the modified nucleotides are ribonucleotides or their analogs. Orthoesters can be placed at any position on the sugar moiety, such as, for example, on the 2', 3' and/or 5' positions. Preferably, the orthoester moiety is at the 2' position of the sugar moiety. Preferred orthoesters, and methods of making orthoester protected polynucleotides, are described in U.S. Patent Nos. 5,889,136 and 6,008,400. Also, preferably, orthoesters are attached at the 2' position of a ribosyl moiety. Preferably the orthoester comprises two 2-ethyl-hydroxyl substituents. The most preferred orthoester is illustrated below, and is also referred to herein as a 2'-ACE or as a 2'-bis(hydroxy ethyl) orthoester moiety:

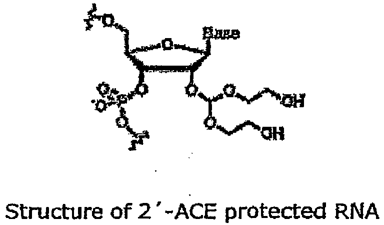

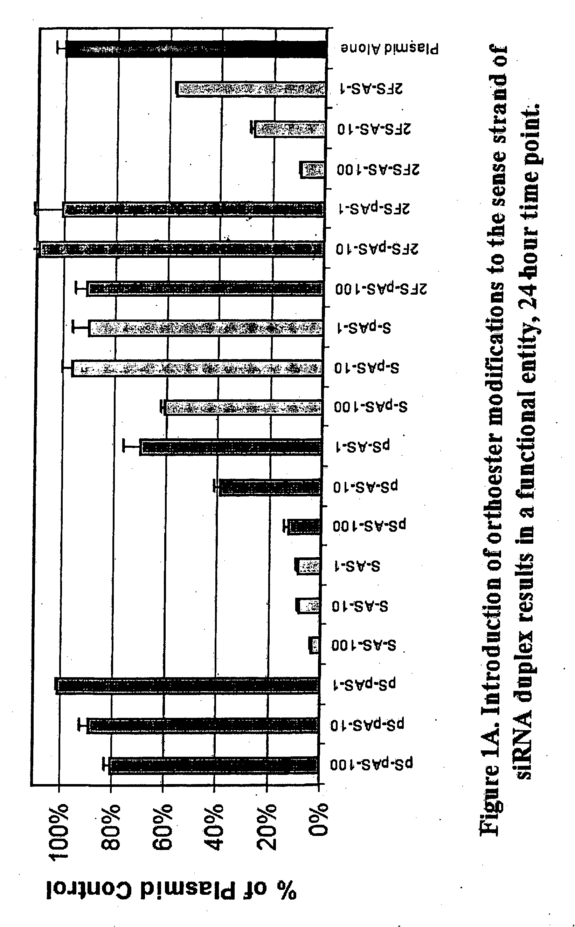

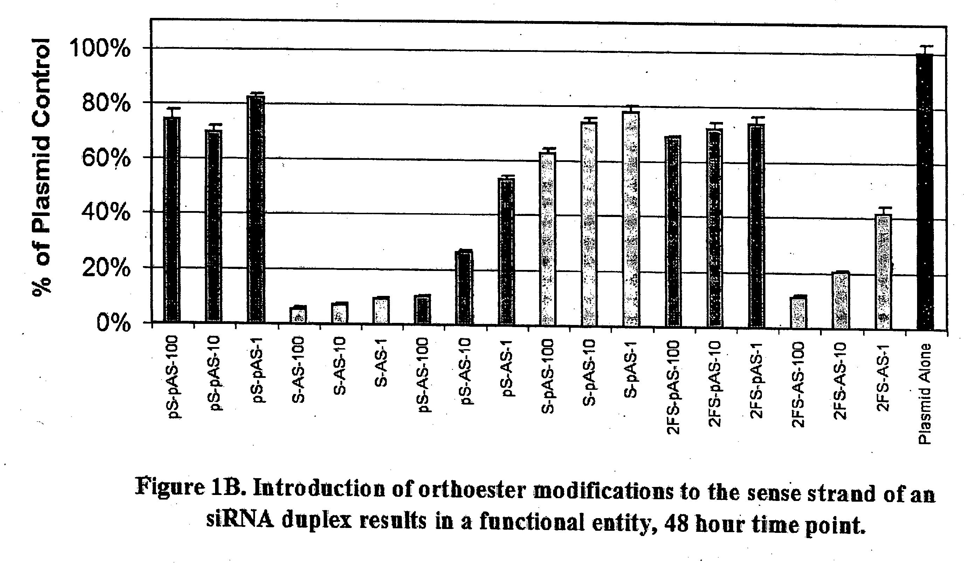

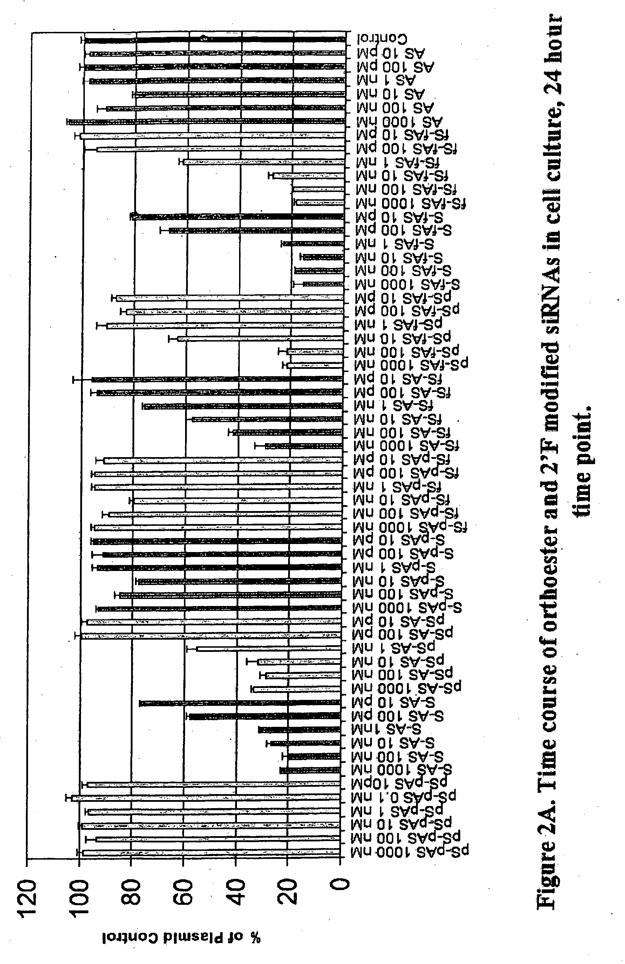

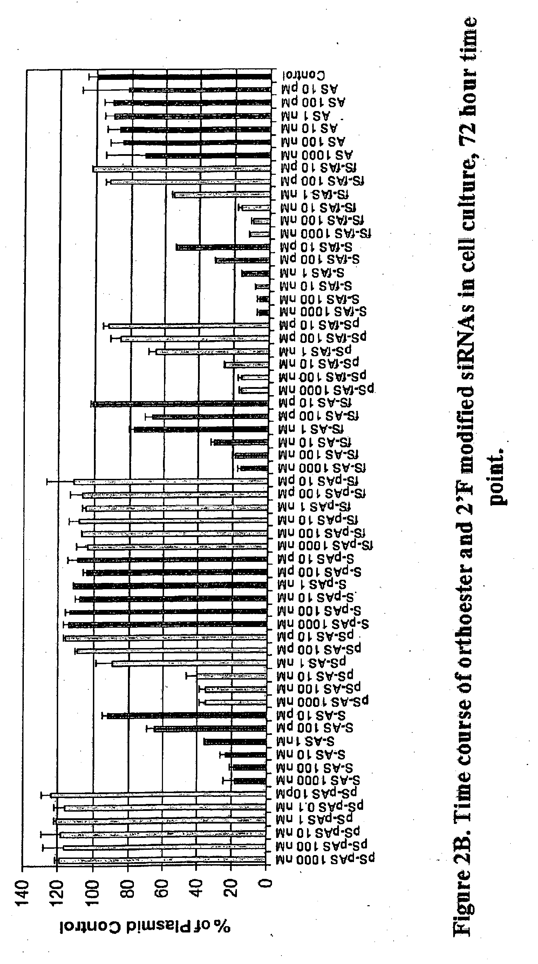

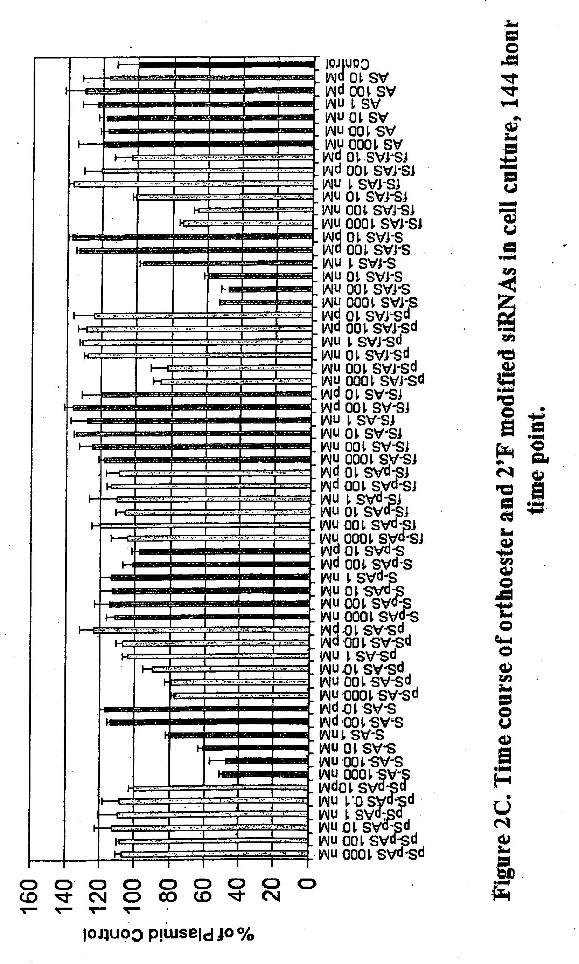

The benefits of including orthoester groups on the sense strand can be seen by reference to **Figures 1A****,** **1B****,** **2A****,** **2B****,** and **2C****.**

The data of **Figure 1** were generated using an siRNA duplex targeting SEAP (human secreted alkaline phosphatase) synthesized using Dharmacon, Inc.'s proprietary ACE chemistry in several variants. These variants include naked, or unmodified, RNA; ACE protected RNA, wherein every 2'-OH is modified with an orthoester, and 2' fluoro modified variants, wherein the fluorine is bonded to the 2' carbon of each and every C and U.

Duplexes of siRNA can be comprised of sense and antisense strands. An array of all possible combinations of sense and antisense strands was created. With reference to the figures, the following nomenclature was used:
S - naked sense strand in an siRNA duplex
AS - naked antisense strand in an siRNA duplex
pS - 2'ACE protected sense strand in an siRNA duplex
pAS - 2'ACE protected antisense strand in an siRNA duplex
2FS - sense strand in an siRNA duplex with all C and U's modified such that a fluorine atom is bound to the 2' carbon of each C- and/or U-bearing nucleotide unit.
2FAS - antisense strand in an siRNA duplex with all C and U's modified such that a fluorine atom is bound to the 2' carbon or each C- and/or U-bearing nucleotide unit.
S - AS, refers to duplex siRNA formed from naked sense and naked antisense strands. pS - AS, refers to duplex siRNA formed from an ACE modified sense strand and a naked antisense strand.
P2'F - 2'-ACE modification of all nucleotides except Cs and Us, which have a 2'F modification.

The duplexes were co-transfected with the pAAV6 plasmid (SEAP expression plasmid) plus the siRNA of interest, or, alternatively, the siRNA were transfected into HEK293 cells that stably expressed SEAP. Under both conditions, standard transfection protocols were used and results did not vary amongst Hela, MDA 75, or 3TELi (mouse) cells lines.

The level of siRNA induced SEAP silencing was determined at a different time points after transfection. (24, 48, 72, or 144 hours) using SEAP detection kits from Clontech according to the manufacturer's protocols. The protein reduction levels are in good correspondence with the mRNA reduction levels (the levels of mRNA were measured using QuantiGene kits (Bayer)). The level of siRNA induced toxicity was measured using AlmaBlue toxicity assay or the levels of expression of housekeeping gene (cyclophilin) or both. Unless specified, no significant toxicity was observed.

Each duplex was transfected into the cells at concentrations varying between 1 and 100 nanomolar **(****Figure 1****)** and 10 picomolar to 1 micromolar **(****Figure 2****).** In **Figures 1** and **2** the effects of introduction of the ACE modifications on the sense and antisense strands of the siRNA duplex in combination with naked and 2' fluoro modifications are shown.

The presence of the ACE modifications on the antisense strand of the oligos significantly interferes with the siRNA duplex functionality. The ACE modified sense oligos were potent in the SEAP silencing independent of whether they were used with naked or 2' F modified AS oligos.

The extent of silencing was comparable at 24, 48, 72 hours and a detectable reduction in the siRNA silencing was observed after 144 hours.

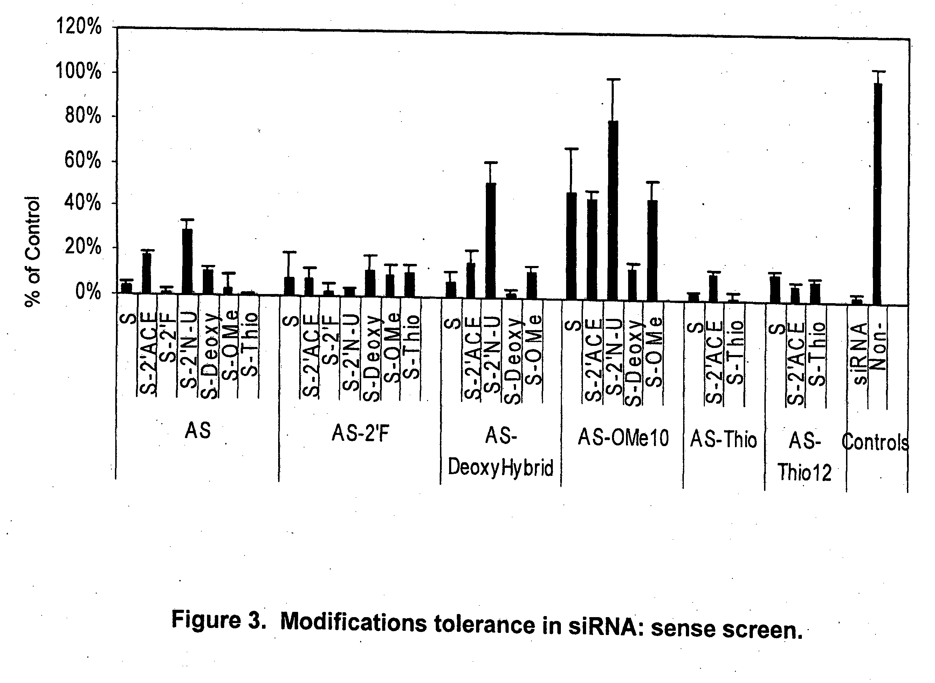

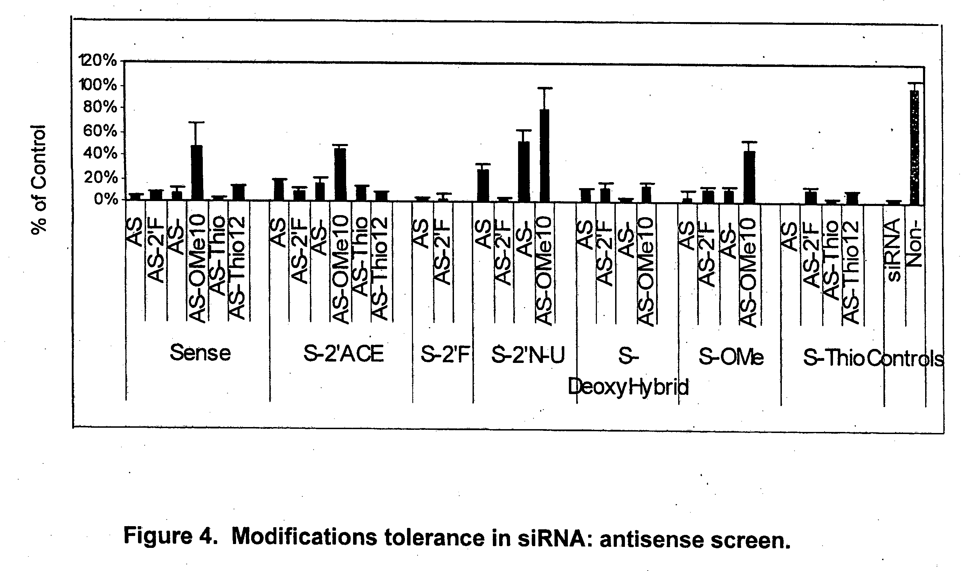

**Figures 3** and **4** summarize siRNA functionality screens when AS **(****Figure 3****)** or Sense **(****Figure 4****)** strands were kept constant and screened in combination with the variety of modifications on the opposite strand. Specifically, the AS strand was either (1) naked, (2) modified with 2'F groups at all Cs and Us, (3) modified with 2'-deoxy groups at position 2, 4, 6, 14, 16, and 18 (counting from the 5'end), (4) modified with 2'-O-methyl groups at all Us and Cs with the exception of those located at positions 67-10 (counting from the 5' end of the strand), (5) modified with phosphorothioates at all positions, or (6) modified with phosphorothioates at positions 1-6 and 14-19 (counting from the 5' end of the strand). These AS strands were then paired with sense strands that were (1) naked, (2) 2'-ACE modified at all positions, (3) 2' F modified on all Cs and Us, (4) 2' amine (NH₂) modified on all Us, (5) 2'-deoxy modified on all odd numbered nucleotides (e.g. 1,3,5...), (6) 2'-O-methyl modified at all Cs and Us, or (7) carrying phosphorothioates at all positions. The results of these studies demonstrate that while the described 2'-O-methyl modifications of the antisense strand are not tolerated, other modifications including phosphorothioate, 2' F, and 2' deoxy modifications are permitted on this strand in combination with a variety of sense strand modifications. Similarly, **Figure 4** shows that while amine modifications are generally not tolerated on the sense strand, all other modifications including 2'-ACE, 2' F, 2' deoxy, 2'-O-methyl, and phosphorothioate additions are permitted in combination with a variety of AS strand modifications without severally disrupting functionality.

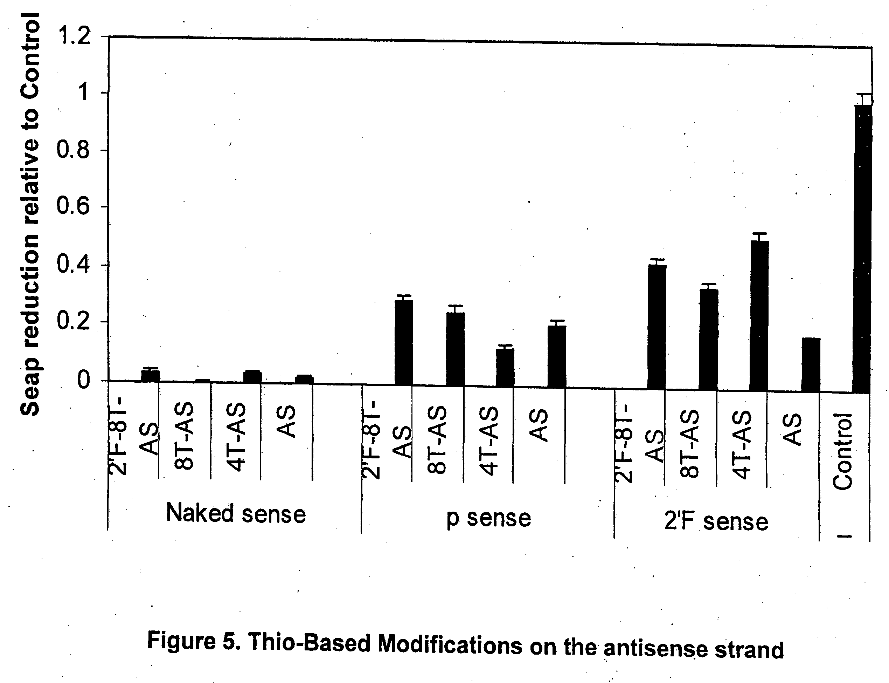

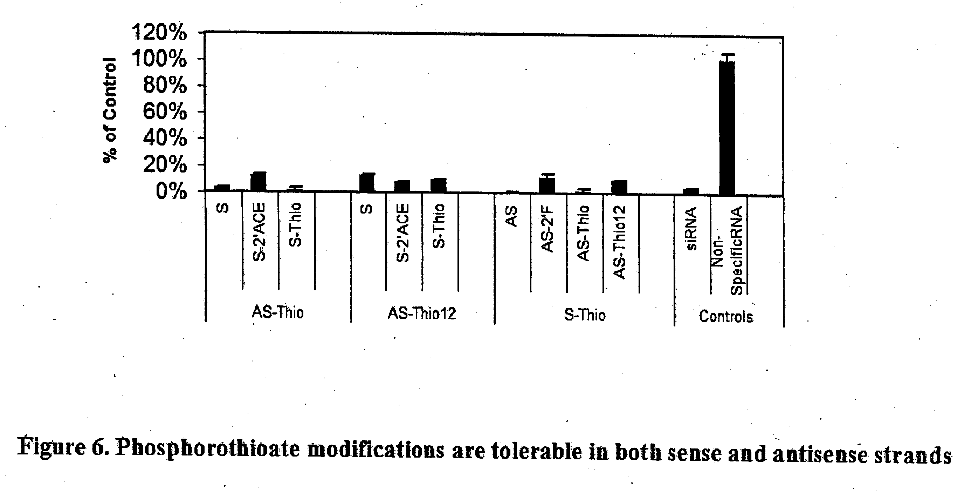

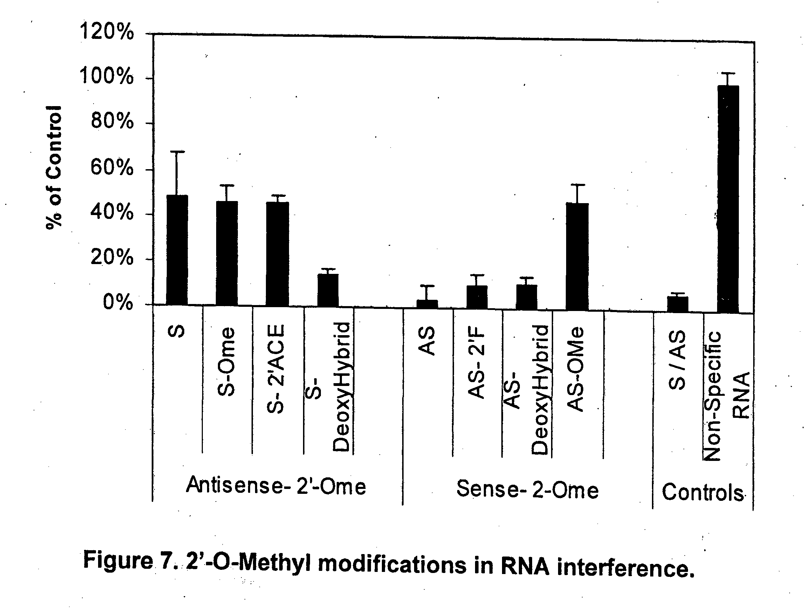

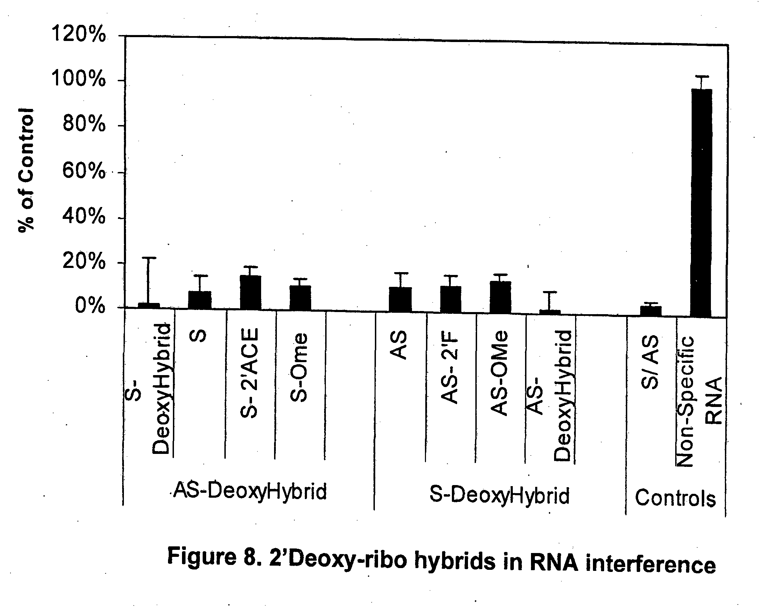

**Figures 5****,** **6****,** **7** and **8** present a more detailed data grouped based on the type of modification used. In Figure 5, naked, 2'O-ACE modified, or 2'F modified sense strands were paired with either: 2F-8T AS = four phosphorothioates on each end of the strand, starting on the second nucleotide in from each end, plus F groups on the Cs and Us; 8T-AS= four phosphorothioates on each end of the strand, starting on the second nucleotide in from each end; 4T-AS = Two phosphorothioates on each end of the strand starting on the second nucleotide in from each end. In **Figure 6** antisense strands containing phosphorothioate modifications at ever position, or at twelve positions (AS-Thio12, six phosphorothioate modifications on either end of the strand) were paired with complementary sense strands that were naked; 2'-ACE modified at all positions, or phosphorothioate modified at all positions. Alternatively, sense strands carrying phosphorothioate modifications at all positions were paired with antisense strands that were naked; carried 2' F groups on all Cs and Us, carried phosphorothioates at all positions, or carried 12 phosphorothioates (modification of six nucleotides on each end). For **Figure 7****:** Antisense strands carrying 2'-Ome = 2'-O-methyl modifications on all Us and Cs excluding those that occur between nucleotide positions 7-11 and S-Ome = 2'-O-methyl modifications on Cs and Us of sense; were matched with either naked, S-2'ACE = 2'ACE modifications on all nucleotides; S-Deoxy-hybrid = 2'deoxy modifications on all odd nucleotides across the strand (1, 3 5...) S-Ome = 2'-O-methyl modification of all Cs and Us of the sense strand; AS-2'F = 2'F modifications of all Cs and Us on the strand, AS-deoxyHybrid = 2' deoxy modification of nucleotides 2,4,6, 14, 16, 18, or As-Ome = 2'-O-methy modification of all Us and Cs excluding those that occur between nucleotide positions 7-11. **For** **Figure 8****:** AS strands carrying deoxy modifications (AS-deoxyHybrid = 2' deoxy modification of nucleotides 2,4,6, 14, 16, 18.) were combined with sense strands that were naked, S-2'ACE modified = 2' ACE modifications on all the nucleotides; S-Ome = 2'O-methyl modifications on all Us and Cs; or 2' deoxy (positions 1,3,5.*..etc.*). Similarly, sense- 2'deoxy hybrids (positions 1,3,5...*etc.*) were paired with antisense strands that were naked, 2'F modified (Cs and Us), 2'-O-methyl (Us and Cs excluding those between positions 7 and 11), and 2'deoxy (positions 2,3,6,14,16,18).

**Figure 5** in particular demonstrates that phosphorothioate modifications are well tolerated when placed in the antisense strand in combination with naked, 2'ACE modified and 2'F modified sense strands.

**Figure 6** further illustrates that phosphorothioate backbone modifications are acceptable both on the sense and antisense strands with the same limitation of nonspecifically induced toxicity.

**Figure 7** illustrates that presence of 2'-O-methyl modifications are well tolerated on sense and but not antisense strands of the siRNA duplex. It is worth mentioning that the functional siRNA duplex is formed by the combination of the 2'-O-methyl modified AS strand and deoxyribohybrid in the sense strand.

**Figure 8** demonstrates the suitability of the deoxyribohybrid type modification in RNA interference. Deoxyribohybrids are RNA/DNA hybrid oligos where deoxy and ribo entities are incorporated together in an oligo in, for example, a sequence of alternating deoxy- and ribonucleotides. It is important in the design of these kinds of oligos to keep the size of continuous DNA /RNA duplex stretches shorter than 5 nucleotides to avoid the induction of RNAse H activity. The deoxyribohybrids were functional both in sense and antisense strands in combination with 2' fluoro and 2'ACE modified oligos. Also the deoxyribohybrid sense strand was the only modification (under these conditions) that supported siRNA activity when the antisense strand was modified with 2'-O-methyl.

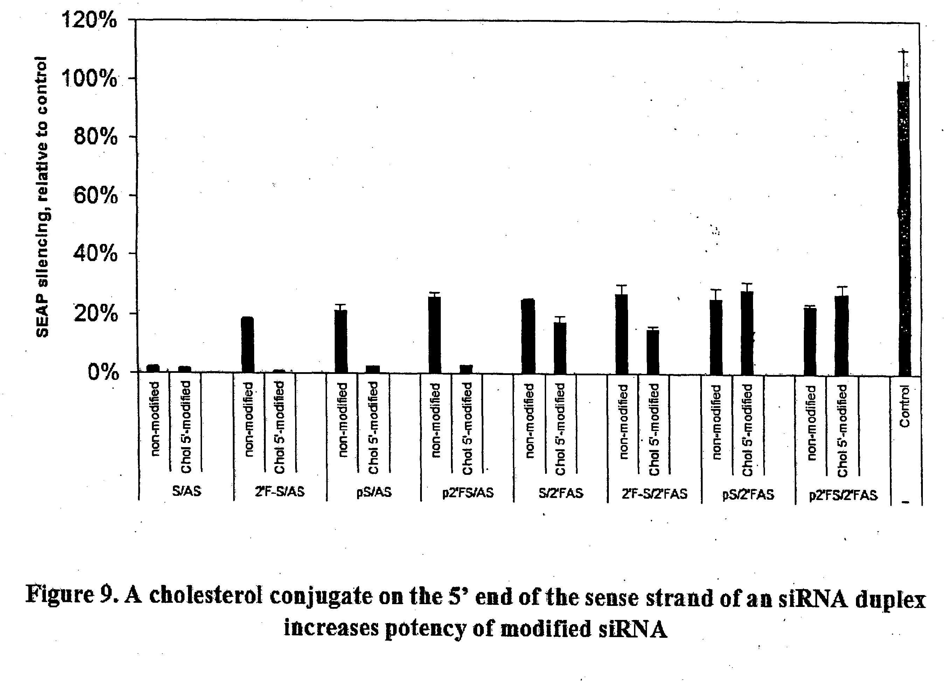

**Figure 9** demonstrates the utility of a conjugate comprising cholesterol for improvement of the potency of ACE and 2' fluoro modified siRNAs. Employing a conjugate comprising cholesterol on the sense strand alleviates negative effects due to modifications to the sense strand, but does not ameliorate negative effects due to modifications to the antisense strand.

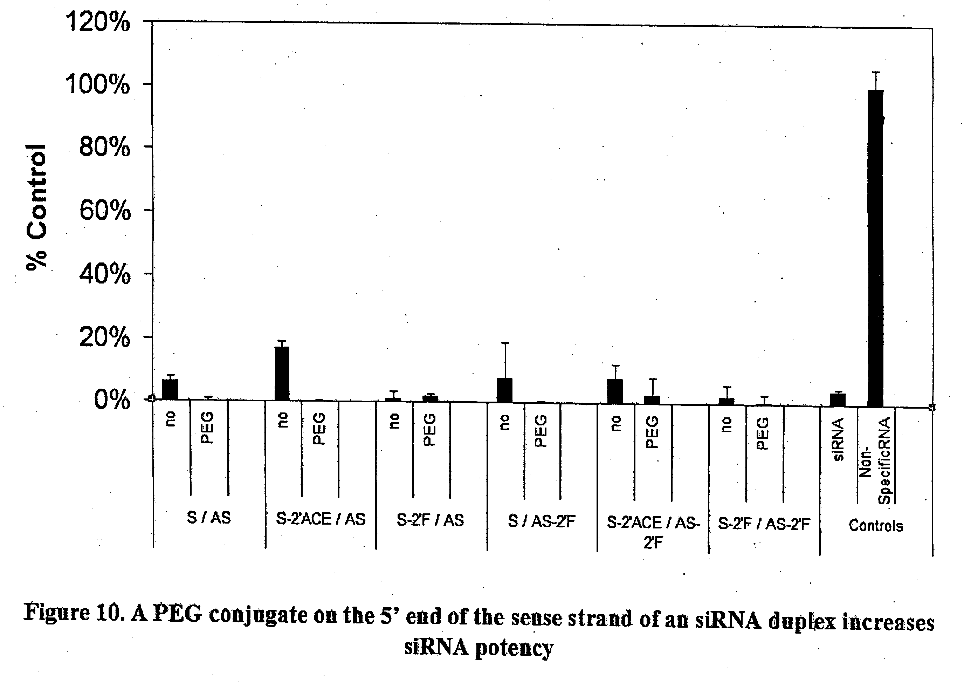

**Figure 10** shows equivalent data for a PEG conjugate on the sense strand.

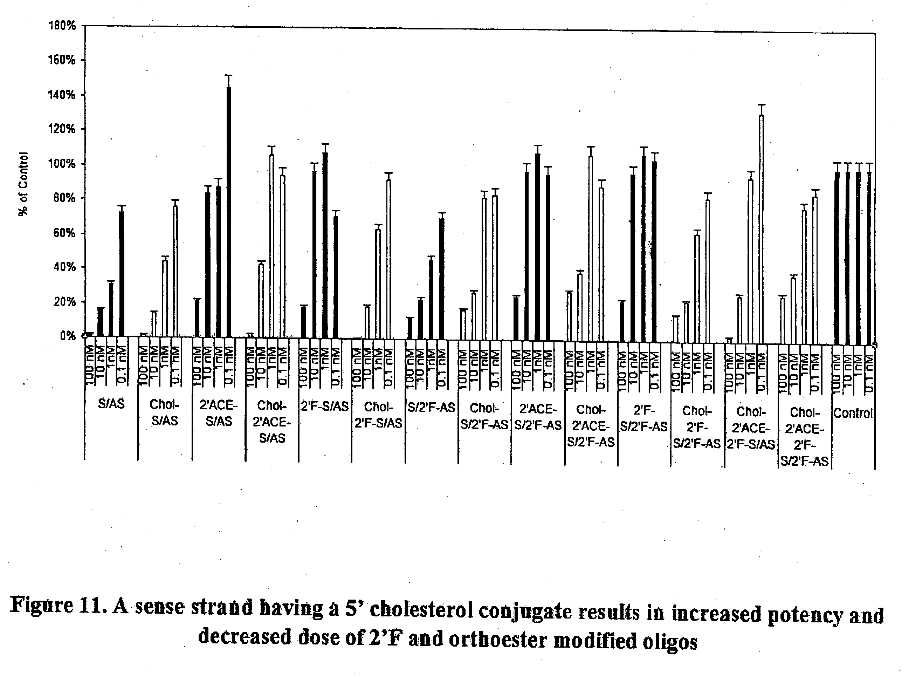

**Figure 11** demonstrates that the presence of a conjugate comprising cholesterol improves the potency of the modified siRNA oligos.

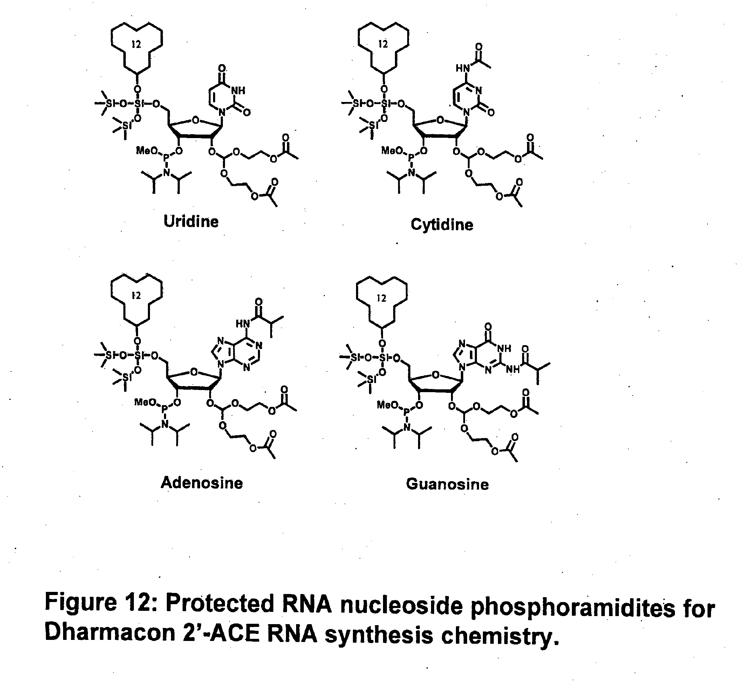

**Figure 12** shows the structures of protected RNA nucleotide phosphoramidites used in Dharmacon's 2'-ACE RNA synthesis chemistry.

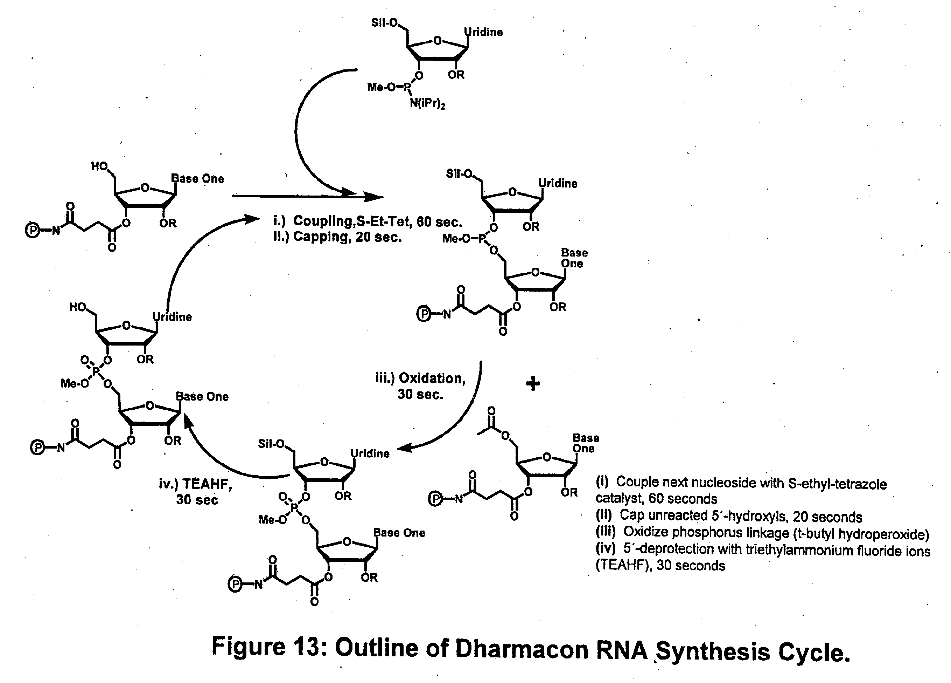

**Figure 13** outlines an RNA synthesis cycle. Preferably, the cycle is carried out in an automated fashion on a suitable synthesizing machine. In step (i), the incoming phosphoramidite (here, bearing a uridine as nitrogenous base), can bear any acceptable group on the phosphoramidite moiety at the 3' position in place of the methyl group shown. For example, an alkyl group or a cyanoethyl group can be employed at that position. This RNA synthesis cycle can be carried out, with certain changes, when synthesizing polynucleotides having modified internucleotide linkages, and/or when synthesizing polynucleotides having other modifications, such as at the 2' position, as described hereinafter.

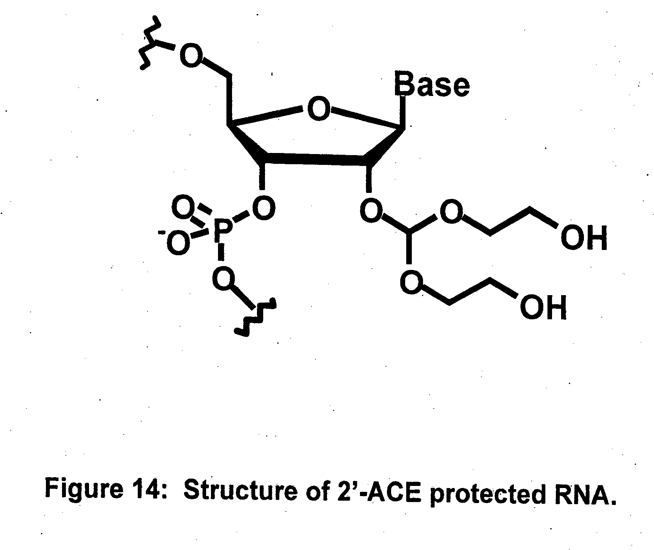

**Figure 14** illustrates the structure of a 2'-ACE protected RNA product immediately prior to 2' deprotection. If it is desired to retain the orthoester at the 2' position, this 2' deprotection step is not carried out.

For a 19-mer duplex having a di-dT overhang at the 3' end, G2'-N-UGA2'-N-UG2'-N-UA2'-N-UG2'-N-UCAGAGAG2'-NUdTdT with 2' amine modified nucleotide units at the second, fifth, seventh, ninth, eleventh, and nineteenth position of the sense strand, significant loss in functionality occurred whether the antisense strand was naked, 2' fluoro modified at all C's and U's, was a deoxyhybrid comprising alternating ribo and deoxyribonucleotide units, or had 2'-O-methyl modifications. Preferably, the sense strand does not comprise 2' amino modifications at the second, fifth, seventh, ninth, eleventh, and nineteenth positions.

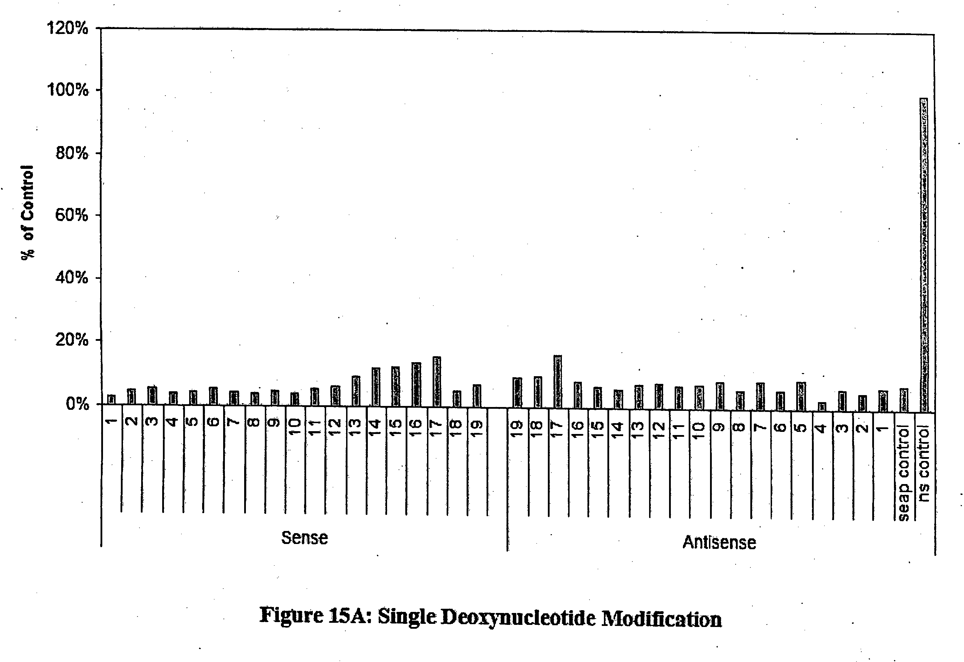

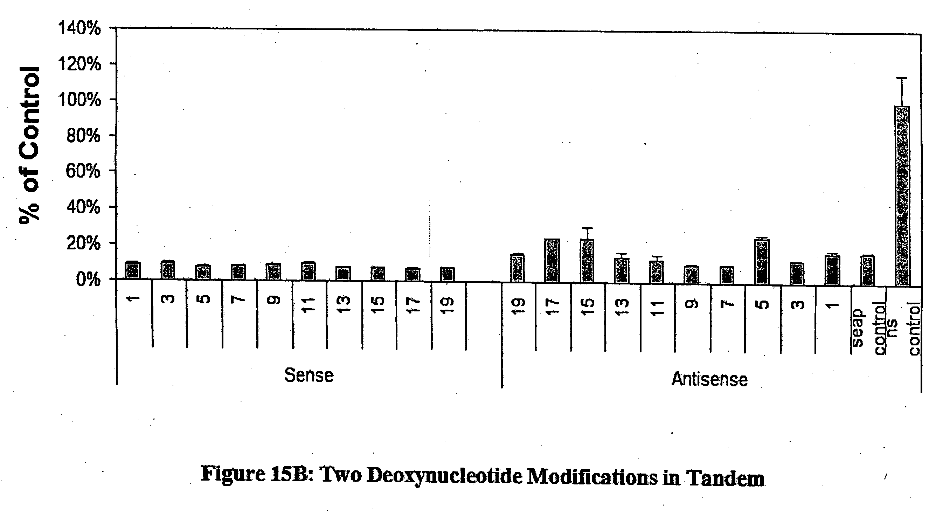

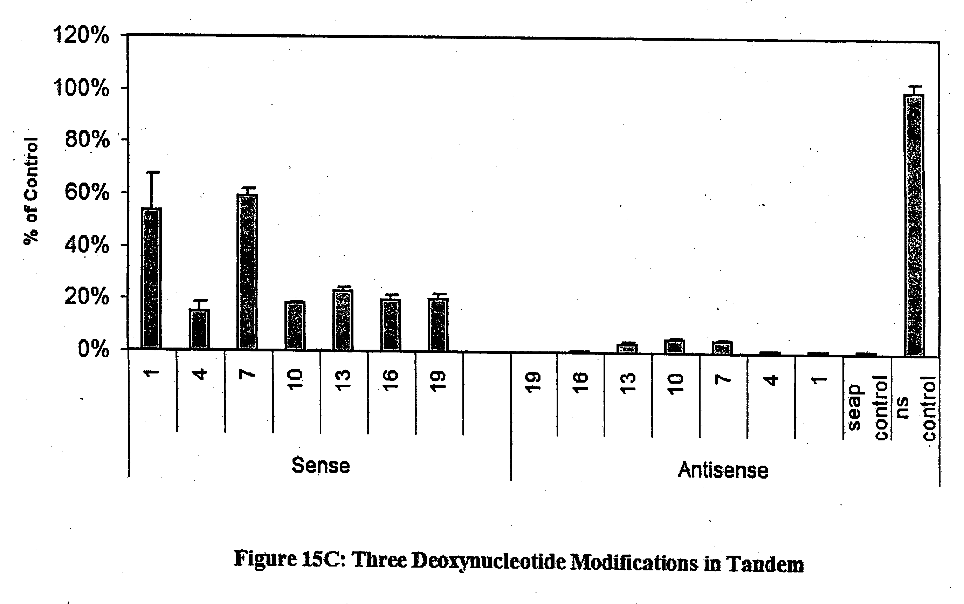

On an siRNA 19-mer with a 3' di-dT overhang (see SEQ. ID NOs. 171-314), replacement of any ribonucleotide unit with a deoxyribonucleotide unit does not significantly affect the functionality of the 19-mer in RNAi, whether the modification is on the sense or the antisense strand (see **Figure 15A****).** On the same siRNA 19-me, replacement of two adjacent ribonucleotide units with two deoxyribonucleotide units in tandem does not significantly affect the functionality of the 19-mer in RNAi. **Figure 15B** illustrates that when positions 1 and 2, 3 and 4, 5 and 6, and so on, are independently modified to be deoxyribonucleotides, functionality is not significantly affected when the modifications are borne on the sense strand and exhibit only a slight negative effect on functionality when the modifications are on the antisense strand. On the same siRNA 19-mer, replacement of three adjacent ribonucleotide units with three deoxyribonucleotide units in tandem does not significantly affect the functionality if the modifications are on the antisense strand, but can significantly affect functionality (in some instances) if the modified units are the first through third or seventh through ninth nucleotides of the sense strand. In this experiment, units 1 to 3, 4 to 6, 7 to 9, and so on of the polyribonucleotide were independently replaced with deoxyribonucleotide units (See **Figure 15C**).

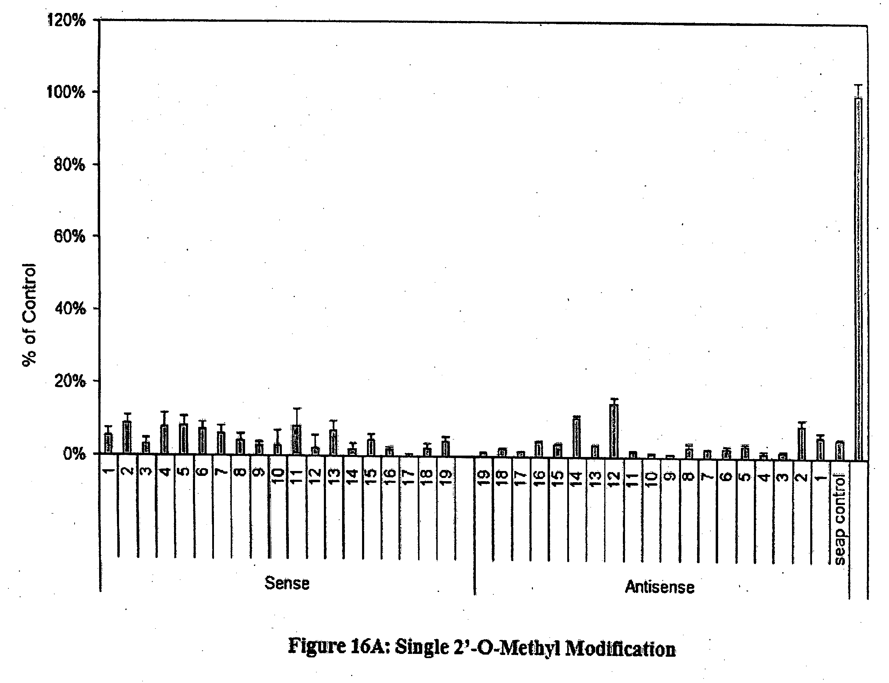

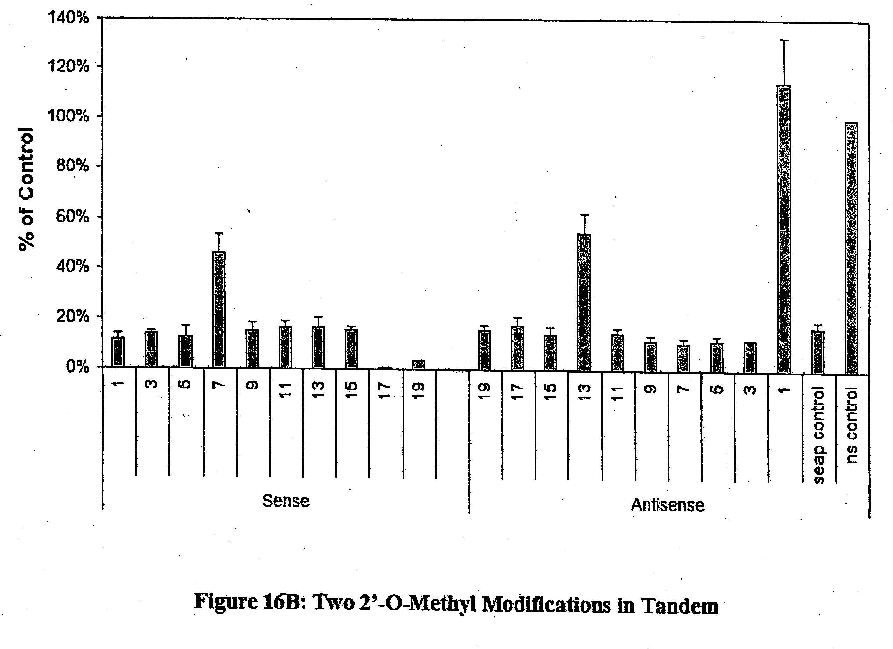

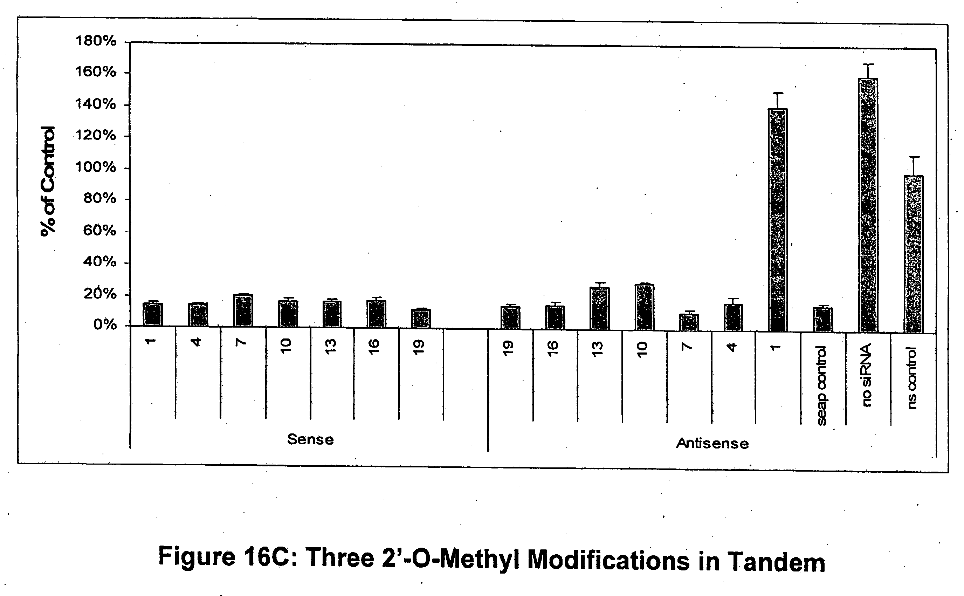

On the same siRNA 19-mer polyribonucleotide with 3' di-dT overhang, modification of any individual unit with a 2'-O-methyl moiety does not significantly affect the functionality of the 19-mer in RNAi, whether the modification is on the sense or the antisense strand (see **Figure 16A**). Using the same siRNA 19-mer, replacement of two adjacent ribonucleotide units with two 2'-O-methyl modifications in tandem does not significantly affect the functionality of the 19-mer in RNAi unless the modifications are placed at the first and second or thirteenth and fourteenth positions of the antisense strand, or the seventh and eighth position of the sense strand (see **Figure 16B**). Subsequent experiments have identified positions of thirteen and fourteen of the antisense strand and seven and eight of the sense strand to be less critical. Most notably, in the absence of additional modifications that compensate for changes in functionality, the first and second positions of the antisense strand should not bear 2'-O-methyl modifications if functionality is to be preserved. Using the same siRNA 19-mer, replacement of three adjacent ribonucleotide units with 2'-O-methyl modifications in tandem does not significantly affect the functionality if the modifications are on the antisense strand at positions other than the first through third positions (see **Figure 16C**). In this experiment, positions 1 to 3, 4 to 6,7 to 9, and so on of the polyribonucleotide were independently modified with 2'O-methyl moieties.

Modification of the same polyribonucleotide with either a single 2'-deoxy moiety or a single 2'O-methyl moiety has no significant affect on functionality. Modification of the first and second or first, second and third positions of the antisense strand with two or more tandem 2'-O-methyl moieties can significantly reduce functionality. Positions 7 through 9 on the sense strand and 13 through 15 on the antisense strand are sensitive to two or more tandem 2'-O-methyl modifications. Thus, preferably the antisense strand does not comprise 2'-O-methyl modifications at the first and second; the first, second and third; the thirteenth and fourteenth; and the thirteenth, fourteenth and fifteenth positions unless compensating modifications occur at other positions within the duplex. Subsequent experiments have shown that 2'-O-methyl modification of positions 1 and 2 (or 1 and 2, and 3) on the antisense strand are key to duplex functionality. Modifications on positions 7-9 on the sense strand and 13-15 on the antisense strand are less critical

As a matter of practicality it is more economical to synthesize a sense strand in which all of the nucleotides are modified by an orthoester group, rather than a sense strand in which only selected nucleotides are so modified. However, in theory, if a practical means were developed to synthesize sense strands in which only certain nucleotides were modified, then those polynucleotides could be used in the present invention.

Preferably, the 2' modified nucleotide is selected from the group consisting of a 2' halogen modified nucleotide, a 2' amine modified nucleotide, a 2'-4-alkyl modified nucleotide, and a 2' alkyl modified nucleotide. Where the modification is a halogen, the halogen is preferably fluorine. When the modification is fluorine, preferably it is attached to one or more nucleotides comprising a cytosine or a uracil base moiety. Where the 2' modified nucleotide is a 2' amine modified nucleotide, the amine is preferably -NH₂. Where the 2' modified nucleotide is a 2'-O-alkyl modification, preferably the modification is a 2'-O-methyl, ethyl, propyl, isopropyl, butyl, or isobutyl moiety and most preferably, the 2'-O-alkyl modification is a 2'-O-methyl moiety. Where the 2' modified nucleotide is a 2'-alkyl modification, preferably the modification is a 2' methyl modification, wherein the carbon of the methyl moiety is attached directly to the 2' carbon of the sugar moiety.

**Figure 2C** demonstrates that siRNA effects start to fade out 144 hours after transfection. The dose as well as potency of the modified oligos were comparable to the naked siRNA duplex.

According to a second embodiment that is related to the present invention, there is provided an siRNA comprising a sense strand where the sense strand comprises a polynucleotide having at least one orthoester modified nucleotide as provided for according to the first embodiment; an antisense strand comprising a polynucleotide that has at least one 2' modified nucleotide as provided for according to the first embodiment related to the present invention; and a conjugate.

The conjugate within this embodiment is preferably selected from the group consisting of amino acids, peptides, polypeptides, proteins, sugars, carbohydrates, lipids, polymers, nucleotides, polynucleotides, and combinations thereof. More preferably it is selected from the group consisting of cholesterol, polyethylene glycol, antigens, antibodies, and receptor ligands. Even more preferably, the conjugate comprises cholesterol or polyethylene glycol. Most preferably, the conjugate comprises cholesterol and is linked to the 5' terminal nucleotide unit of the sense strand at the 5' carbon position.

Introduction of a cholesterol-containing conjugate at the 5' terminus of the sense strand resulted in an increase in potency for orthoester modified and 2' antisense modified siRNAs that was comparable to or even superior to the naked, or unmodified, duplexes. See **Figure 9** and **11**. A 5' cholesterol modification of the sense strand resulted in a decrease in the functionally effective dose for orthoester modified and 2' fluorine modified siRNAs that were comparable or even superior to the corresponding naked duplexes.

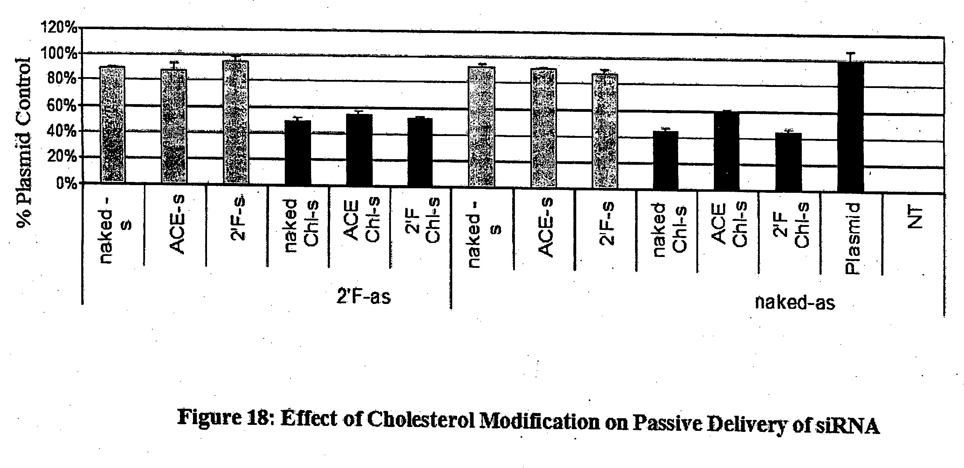

**Figure 9** demonstrates the utility of the cholesterol modification for improvement of the potency of ACE and 2' fluoro modified siRNAs. The positive cholesterol effect was observed with the modifications introduced mainly on the sense chloride. For example, attachment of a cholesterol moiety to the 5' end at the 5' position of the sense strand of SEQ. ID NOs. 1-16 results in RNAi in the absence of transfection agents (see **Figure 18**).

According to a third embodiment related to the present invention, there is provided an siRNA that has a sense strand comprised of at least one orthoester modified nucleotide, an antisense strand, and a conjugate. In this embodiment, the orthoester modification of the first embodiment may be used in combination with the conjugate of the second embodiment related to the present invention.

According to a fourth embodiment related to the present invention, there is provided an siRNA that has a sense strand, an antisense strand, and a conjugate, wherein the sense strand and/or the antisense strand has at least one 2' modified nucleotide. The 2' modified nucleotide of this embodiment is preferably selected according to the same parameters as the 2' modified nucleotide of the first embodiment related to the present invention. Similarly, the conjugate is preferably selected according to the same parameters by which the conjugate is selected in the above described second embodiment related to the present invention.

According to a fifth embodiment related to the present invention, there is provided an siRNA having a sense strand comprised of at least one orthoester modified nucleotide, an antisense strand comprised of at least one 2' modified nucleotide selected from the group consisting of a 2' halogen modified nucleotide, a 2' amine modified nucleotide, a 2'-O-alkyl modified nucleotide, and a 2' alkyl modified nucleotide, and a conjugate selected from the group consisting of amino acids, peptides, polypeptides, proteins, sugars, carbohydrates, lipids, polymers, nucleotides; polynucleotides, and combinations thereof, wherein the polyribonucleotide comprises between 18 and 30 nucleotide base pairs.

The orthoester of this embodiment is selected according to the criteria for selecting the orthoester of the first embodiment related to the present invention. Where the 2' modification is a halogen, preferably it is fluorine and is attached to at least one C- and/or U-containing nucleotide units of the antisense strand. Where the 2' modified nucleotide is a 2' amine modified nucleotide, the amine is preferably-NH₂. Where the 2' modified nucleotide is a 2'-O-alkyl modification, preferably it is a 2'-O-methyl ethyl, propyl, isopropyl, butyl, or isobutyl moiety and most preferably, the 2'-O-alkyl modification is a 2'-O-methyl moiety. Where the 2' modified nucleotide is a 2' alkyl modification, preferably it is a 2' methyl modification, wherein the carbon of the methyl moiety is attached directly to the 2' carbon of the sugar moiety.

According to a sixth embodiment related to the present invention there is provided a composition comprising the structures below: 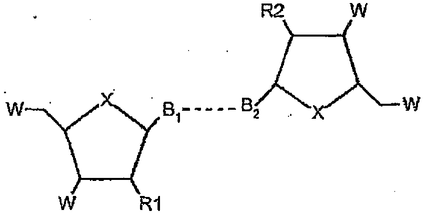 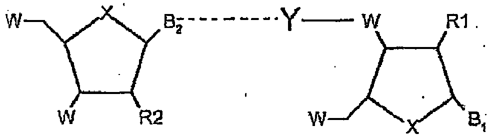 or 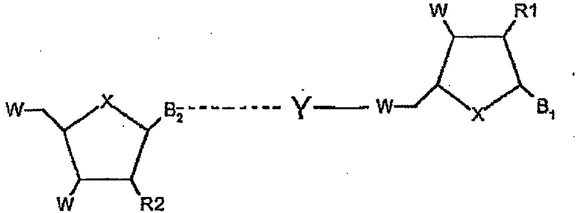 wherein each of B₁ and B₂ is a nitrogenous base, heterocycle or carbocycle; X is selected from the group consisting of O, S, C, and N; W is selected from the group consisting of an OH, a phosphate, a phosphate ester, a phosphodiester, a phosphotriester, a modified internucleotide linkage, a conjugate, a nucleotide, and a polynucleotide; R1 is an orthoester, R2 is selected from the group consisting of a 2'-O-alkyl group, an alkyl group, an amine, and a halogen; and Y is a nucleotide or polynucleotide. Where R2 is a halogen, the halogen is preferably a fluorine. Where R2 is a fluorine, the fluorine is preferably attached to one or more C- and/or U-containing nucleotide units. Where R2 is an amine, the amine is preferably-NH₂. Where R2 is a 2'-O-alkyl modification, preferably it is a 2'-O-methyl, ethyl, propyl, isopropyl butyl, or isobutyl moiety and most preferably a 2'-O-methyl moiety. Where R2 is a 2' alkyl modification, preferably it is a 2' methyl modification, wherein the carbon of the methyl moiety is attached directly to the 2' carbon of the sugar moiety.

R1, the orthoester, of this embodiment is selected according to the parameters for selecting the orthoester of the first embodiment related to the present invention.

The dashed lines in the formula indicate interaction by hydrogen bonding between nitrogenous bases. Preferably, B₁ and B₂ are naturally occurring nitrogenous bases such as, for example, adenine, thymine, guanine, cytosine, uracil, xanthine, hypoxanthine, and queuosine or analogs thereof Preferably, X is an O.

With respect to each of the above-described embodiments, the siRNAs can be of any length, but preferably are 18-30 nucleotide base pairs, more preferably 18-19 base pairs, excluding any overhang. By using siRNAs of less than about 30 base pairs in length one can avoid nonspecific processes, such as interferon-related responses, which can reduce the functionality of an siRNA application, while retaining a functional response in RNA interference applications. Additionally, preferably the nucleotides are ribonucleotides.

In the above-described embodiments, overhangs can be present on either or both strands, at either or both ends. Preferably, if an siRNA has an overhang, it is one to six nucleotide units in length, more preferably two to three, and most preferably two, and is located at the 3' end of each strand of the siRNA. However, siRNAs with blunt ends are functional. Overhangs of two nucleotides are most preferred.

Similarly in the above-described embodiments, either or both strands of the siRNA can have one or more modified internucleotide linkages. Preferably, the modified internucleotide linkages are selected from the group consisting of phosphorothioates and phosphorodithioates. Additionally, preferably, the polynucleotides comprises more than 4 modified internucleotide linkages. More preferably, the polynucleotides of the invention comprise more than 8 modified internucleotide linkages. Most preferably, about 10 modified internucleotide linkages are employed. For the greatest amount of stability, complete modification is preferred; however, a number of factors affect how many modified linkages can be employed in practice. These factors include the degree of stability conferred by the linkage, the degree to which the linkage affects functionality, the ability to introduce the linkage chemically, and the toxicity of the linkage. Preferably, modifications are localized on the 3' and 5' ends to protect against exonuclease activity.

The polynucleotides of the aforementioned embodiments are stabilized. The half-lives of these stabilized siRNA are from 20 seconds to 100 or more hours. Preferably, the stabilized siRNAs of the invention display half-lives of greater than one hour. More preferably, the stabilized siRNAs of the invention display half-lives of greater than 10, hours. Most preferably, the stabilized siRNAs of the invention display half-lives in excess of 100 hours. Additionally, preferably the effect of the siRNAs will survive cell division for at least one or more generations.

The polynucleotides of the invention exhibit enhanced stability in the presence of human serum. Preferably the half life of, for example, a 19-mer duplex of the present invention in human serum is from several minutes to 24 hours. More preferably, the half life of a 19-mer duplex in human serum is from 24 hours to 3 days. Most preferably, the half life of a 19-mer duplex in human serum if from 3 to 20 or more days.

For a 19-mer polyribonucleotide duplex comprising an antisense strand with deoxyribonucleic modifications at the second, fourth, sixth, fourteenth, sixteenth, and eighteenth positions, exposure to fetal bovine serum for half an hour at 37 degrees Centigrade resulted in protection of the fourth and sixth positions from degradation, presumably by serum nucleases. Similarly, for a 19-mer polyribonucleotide duplex comprising 2'-O-methyl modifications on the antisense strand at the second through sixth, twelfth, fourteenth, sixteenth and seventeenth, and nineteenth positions resulted in protection of these positions from degradation by serum nucleases. Introduction of phosphorothioate modifications in the antisense strand for a 19-mer polyribonucleotide duplex at between nucleotide units one through six and thirteen through nineteen rendered the modified internucleotide linkages resistant to serum nuclease degradation. However, a 19-mer modified with an ACE orthoester moiety at each 2'position of an antisense strand did not confer stability in human serum, presumably due to the action not of serum ribonucleases but of serum phosphodiesterases.

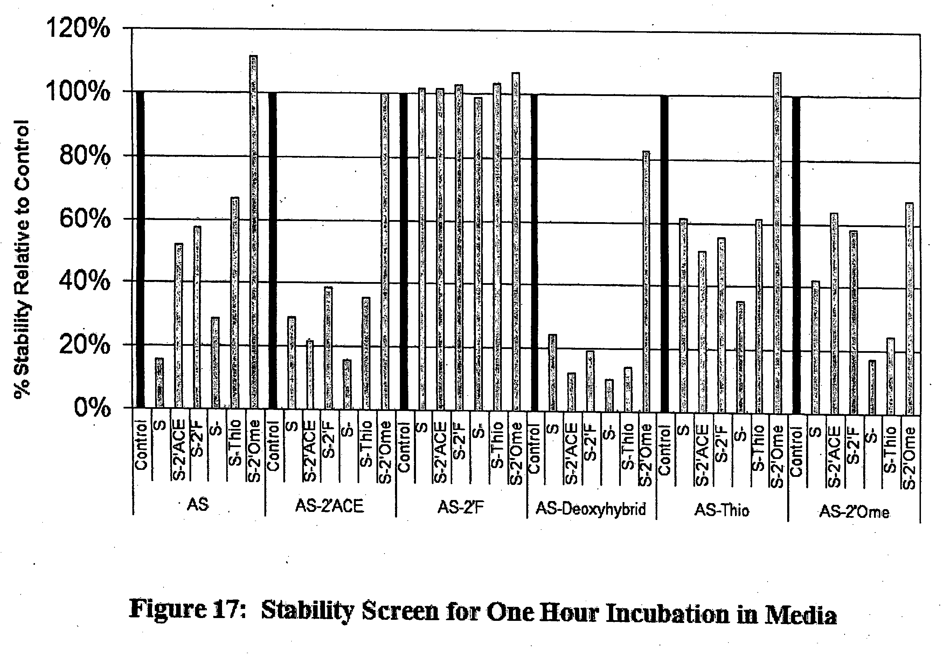

Modifications at the 2' position in the antisense strand of a polyribonucleotide duplex, at C and U nucleotide units, greatly enhance the stability of the polyribonucleotide duplex in serum. **Figure 17** illustrates stability as a function of type of modification at the 2' position on both the sense and antisense strands for 2'-O-methyl (SEQ. ID NO. 13), for 2'F (5'-2' G fU G A fU G fU A fU G fU fC A G A G A G fU dT dT-3') (SEQ. ID NO. 17); for phosphorothioate internucleatide linkages (SEQ. ID. NOs. 10 and 11) and for ACE-protected (SEQ. ID. NOs. 3 and 4). The vertical axis represents the percent ofnondegraded polynucleotide versus a control. Thus, the higher the percent stability relative to control, the less degradation observed. From **Figure 17** it is apparent that modifying the sense strand is sufficient to achieve stabilization.

Modification of each C and each U with either a 2'-O-methyl moiety or a 2' fluoro moiety results in complete stabilization of the sense and the antisense strand. Annealing a stable sense strand, such as one having 2' fluoro or 2'-O-methyl modifications, to a naked antisense strand results in improved stability.

The compositions of the invention can be made according to Dharmacon's RNA synthesis chemistry, which is based on a novel protecting group scheme. A class of silyl ethers is used to protect the 5'-hydroxyl (5'-SIL) in combination with an acid-labile orthoester protecting group on the 2'-hydroxyl (2'-ACE). This set of protecting groups is then used with standard phosphoramidite solid-phase synthesis technology. The structures of some protected and functionalized ribonucleotide phosphoramidites are as illustrated in **Figure 12**. As an alternative, compositions of the invention can be made using any other suitable RNA synthesis chemistry.

According to a seventh embodiment related to the present invention there is provided a method of performing RNA interference. This method is comprised of exposing an siRNA to a target nucleic acid in order to perform RNAi. Under this method, the siRNA is comprised of a sense strand and an antisense strand, and at least one of said sense strand and said antisense strand comprises at least one orthoester modified nucleotide.

Preferably, the polynucleotides of the antisense strand exhibit 90% or more complementarity to the target nucleic acid of interest More preferably, the polynucleotides antisense strand of the invention exhibit 99% or more complementarity to the target nucleic acid of interest. Most preferably, the polynucleotides of the invention are perfectly complementary to the target nucleic acid of interest over at least 18 to 19 contiguous bases.

Preferably, the at least one orthoester modified nucleotide is located on the sense strand, and the composition of the orthoester is defined by the parameters described above for the first embodiment related to the present invention.

In addition to the orthoester modification, any of the above described other modifications may also be present when using this method. For example, the antisense strand preferably comprises at least one modified nucleotide selected from the group consisting of a 2' halogen modified nucleotide, a 2' amine modified nucleotide, a 2'-O-alkyl modified nucleotide and a 2' alkyl modified nucleotide. Where the modified nucleotide is a 2' halogen modified nucleotide, the halogen is preferably a fluorine. Where the halogen is a fluorine, the fluorine is preferably attached to C- or U-containing nucleotide units. Where the 2' modification is an amine, preferably the amine is -NH₂. Where the 2' modification is a 2'-O-alkyl group, preferably the group is methoxy, -OCH₃. Where the 2' modification is an alkyl group, preferably the modification is a methyl groups, -CH₃. Further, preferably none of these modifications occur at nucleotides 8-11, and more preferably none of the occur at positions 7-12 of the antisense strand.

The method can also be carried out wherein the siRNA comprises a 5' conjugate. The conjugate can be selected according to the above-described criteria for selecting conjugates.

When using these methods, the siRNA can be of any number of base pairs, but is preferably is 18-30 base pairs, and more preferably is 19 base pairs. Additionally preferably the polynucleotide comprises an antisense strand and a sense strand of ribonuclcotides.

Overhangs of one or more base pairs at the 3' and/or 5' terminal nucleotide units on either or both strands can also be present according to the above-described parameters for overhangs.

According to an eighth embodiment related to the present invention, there is provided a method of performing RNA interference, comprised of exposing an siRNA to a target nucleic acid, wherein the siRNA is comprised of a sense strand, an antisense strand, and a conjugate, where either the sense strand or the antisense strand comprises a 2' modified nucleotide. Preferably, the siRNAs of this embodiment of the invention exhibit the same degree of complementarity as in the previous example.

According to this embodiment, the antisense strand preferably comprises at least one nucleotide selected from the group consisting of a 2' halogen modified nucleotide, a 2' amine modified nucleotide, a 2'-O-alkyl modified nucleotide and a 2' alkyl modified nucleotide. The modification may be on the antisense strand and/or on the sense strand. Where the modified nucleotide is a 2' halogen modified nucleotide, the halogen is preferably fluorine. Where the halogen is fluorine, the fluorine is preferably attached to at least one C- or U-containing nucleotides. The preferred 2' amine modification is -NH₂. The preferred 2'-O-alkyl modification is -OCH₃. The preferred 2' alkyl modification is -CH_{3.}

The method can also be carried out wherein the siRNA comprises a conjugate. The conjugate is selected according to the parameters for selecting the above-described conjugates. The siRNA can be of any number of base pairs, but as with the previous embodiment is preferably 18-30 base pairs, most preferably 18-19 base pairs. Similarly, overhangs of one or more base pairs on the 3' and/or 5' terminal nucleotide units on either or both strands can be present. Further, either the sense or antisense strand can comprise at least one modified internucleotide linkage, which preferably is selected from the group consisting of phosphorothioate linkages and phospharodithioate linkages. Preferably the sense and antisense strands are polyribonucleotides.

Each of the aforementioned embodiments permits the conducting of efficient RNAi interference because the polynucleotide is more stable than naked polynucleotides. Unlike naked polynucleotides, the polynucleotides of the present invention will resist degradation by nucleases and other substances that are present in blood, serum and other biological media.

An additional surprising benefit of the present invention is that it minimizes nonspecific RNA interference, also referred to as "off-targeting". Nonspecific RNA interference occurs when a sense strand silences or partially silences the function of untargeted genes. Orthoester modifications and the other modifications described herein, alone or in combination with one another, can be employed in the sense strand, the antisense strand, or both, to reduce or prevent such nonspecific RNA interference.

In reducing nonspecific RNA interference, preferably sense strand modifications are made at the 2' position at the 8^{th}, 9^{th}, 10^{th}, or 11 th nucleotide from the 5' terminus, with the 5' terminal nucleotide designated as the 1^{st}. More preferably, all of the 8^{th}, 9^{th}, 10^{th} and 11^{th} nucleotides are modified at the 2' position. Most preferably, the 8^{th}, 9^{th}, 10^{th} and 11^{th} nucleotides are all modified at the 2' position and the modification is an orthoester.

According to a ninth embodiment related to the invention, there is provided a method of performing RNA interference, said method comprising exposing an siRNA to a target nucleic acid, wherein said siRNA is comprised of a sense strand and an antisense strand, and wherein said sense strand is substantially nonfunctional. By "substantially nonfunctional" is meant that the sense strand is incapable of inhibiting expression of any non-targeted gene by 50% or more. Thus, a "substantially nonfunctional" sense strand is one that inhibits expression of non-tatget mRNAs by less than 50%. An added advantage is an enhanced stability in serum-containing media and serum.

According to this embodiment, the sense strand can comprise at least one 2'-O-alkyl modification, at least one cytosine- or uracil-containing nucleotide base, wherein the at least one cytosine- or uracil-containing nucleotide base has a 2'-O-methyl modification. Preferably, the 2'-O-alkyl modification is a 2'-O-methyl modification. More preferably, the 2'O-alkyl modification is a 2'-O-methyl modification is on the first, second, eighteenth and/or nineteenth nucleotide base.

The sense strand can further comprise a conjugate. Preferably, the conjugate is cholesterol. Preferably, the cholesterol is attached to the 5' and/or 3' end of the sense strand. Modification of an siRNA duplex with cholesterol drastically increases the duplex's affinity for albumin and other serum proteins, thus altering the biodistribution of the duplex without any significant toxicity.

The sense strand can comprise a cap on its 3' end. Preferably, the cap is an inverted deoxythymidine or two consecutive 2'O-methyl modified bases at the end positions (nucleotides 18 and 19).

The antisense strand can comprise at least one modified nucleotide. Preferably, the at least one modified nucleotide is a 2'-halogen modified nucleotide. Most preferably, the modified nucleotide is a 2'-fluorine modified nucleotide.

Where the sense strand comprises one or more cytosine- and/or uracil-containing nucleotide bases, each of the one or more cytosine- and/or uracil-containing nucleotide bases can be 2'-fluorine modified.

According to a tenth embodiment related to the invention there is provided a method of performing RNA interference, said method comprising exposing an siRNA to a target nucleic acid, wherein said siRNA comprises: (a) a conjugate; (b) a sense strand comprising at least one 2'-O-alkyl modification, wherein said sense strand is substantially nonfunctional; and (c) an antisense strand comprising at least one 2'-fluorine modification, wherein said sense and antisense strands form a duplex of 18-30 base pairs. Preferably, the least one 2'-O-alkyl modification is on the first, second, eighteenth and/or nineteenth nucleotide base. Preferably, the conjugate is cholesterol. Preferably, the cholesterol is attached to the 5' and/or 3' end of the sense strand.

The sense strand can further comprises a cap on its 3' end. Preferably, the cap is an inverted deoxythymidine (idT) or two consecutive 2'O-methyl modified bases at the end positions (nucleotides 18 and 19).

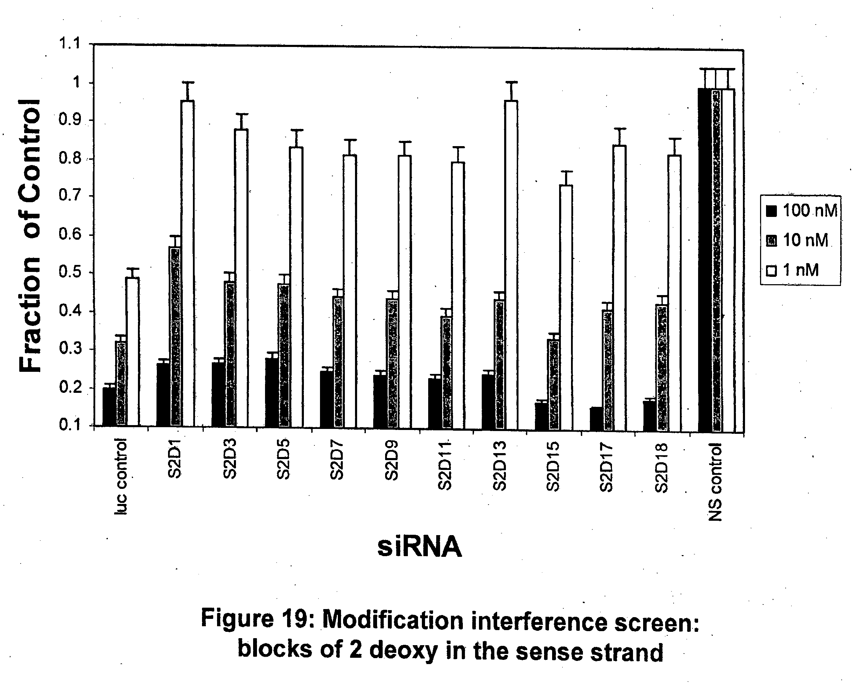

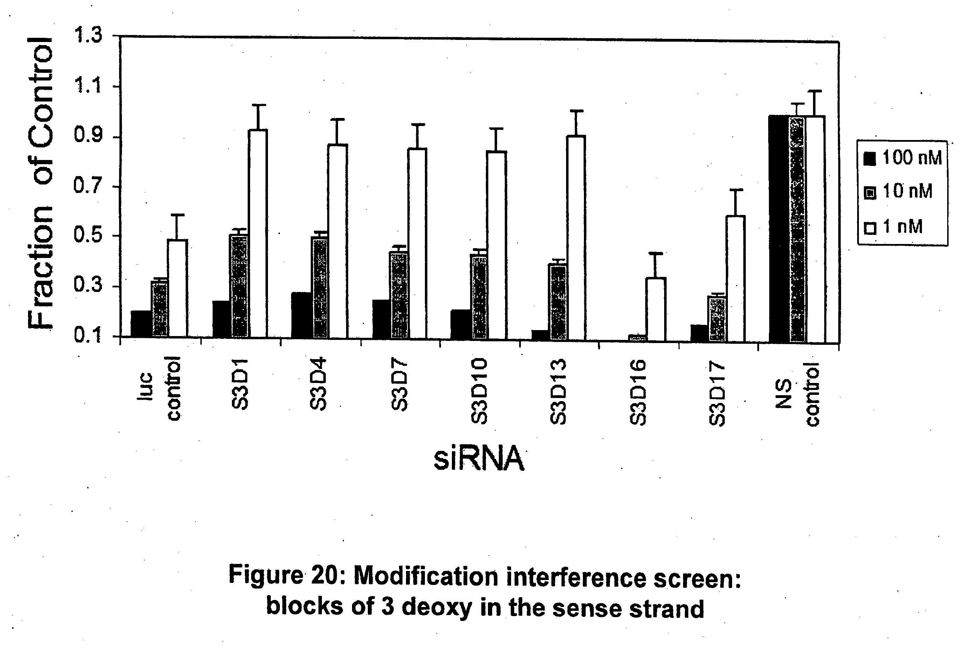

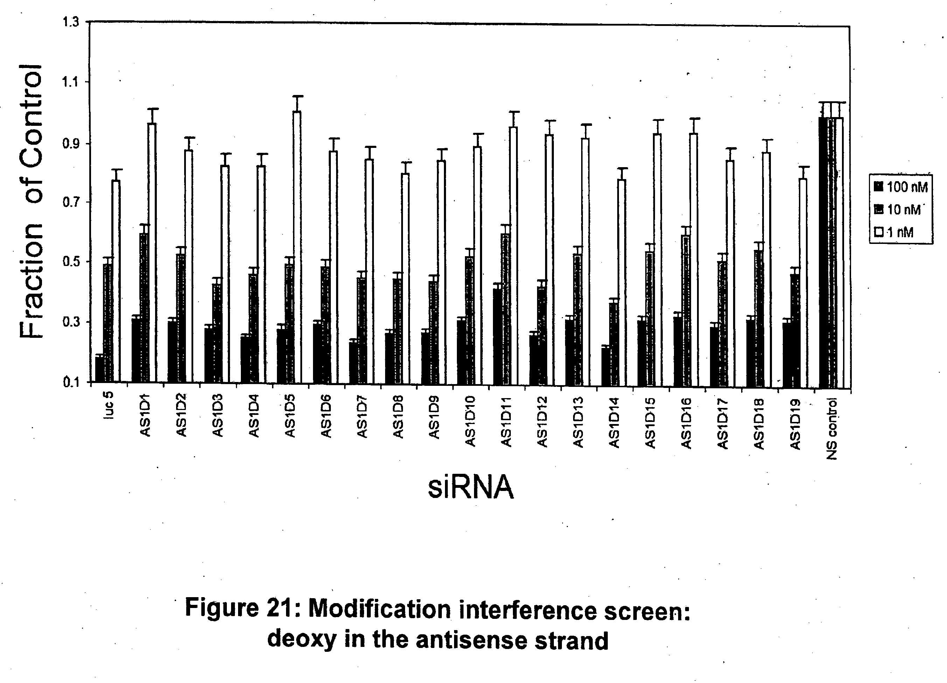

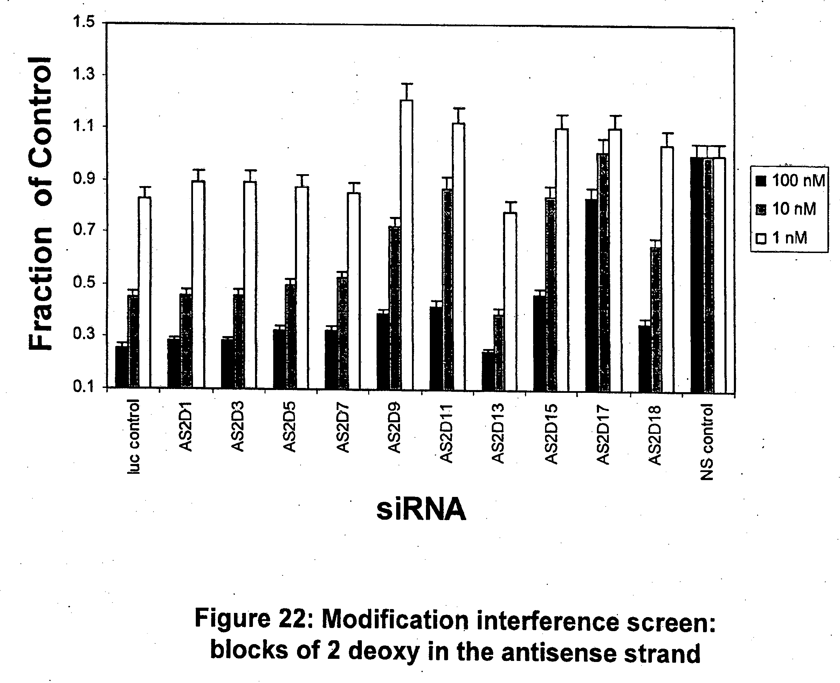

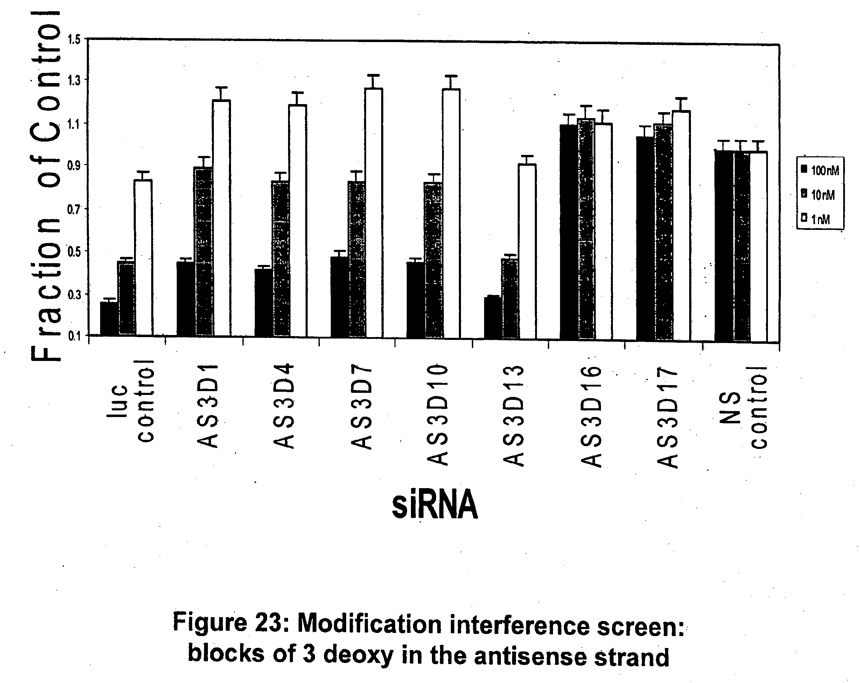

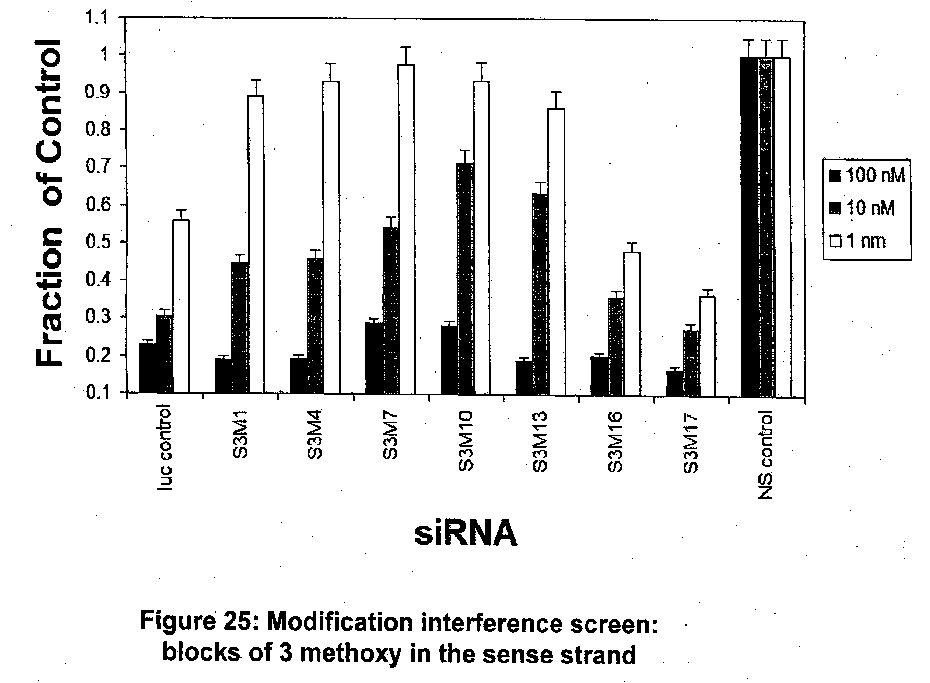

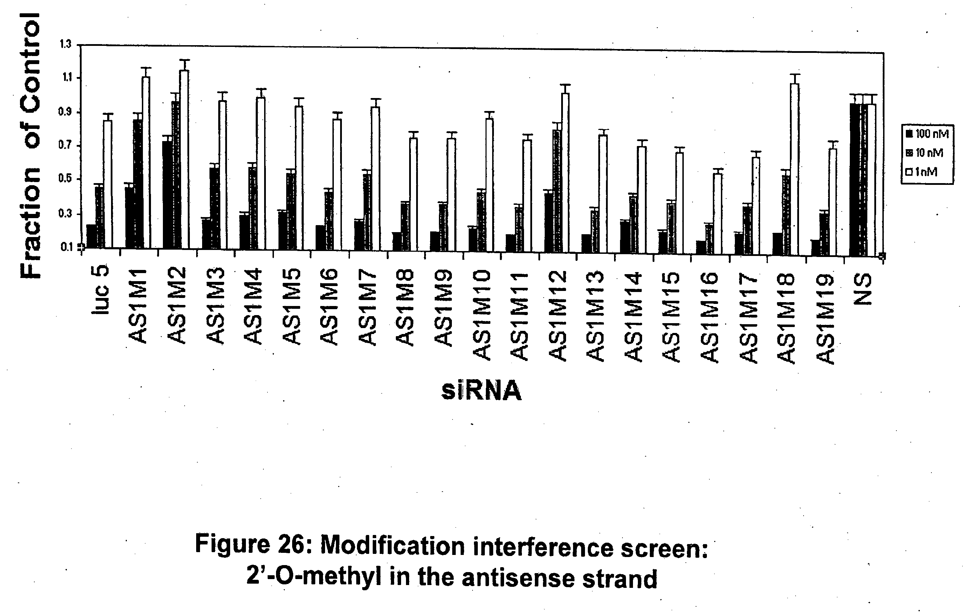

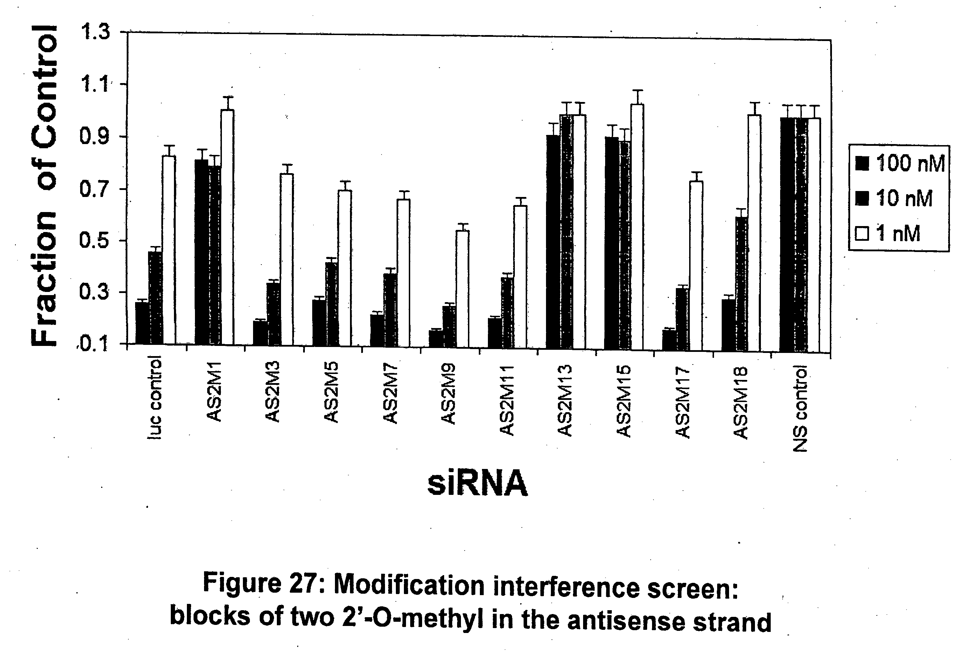

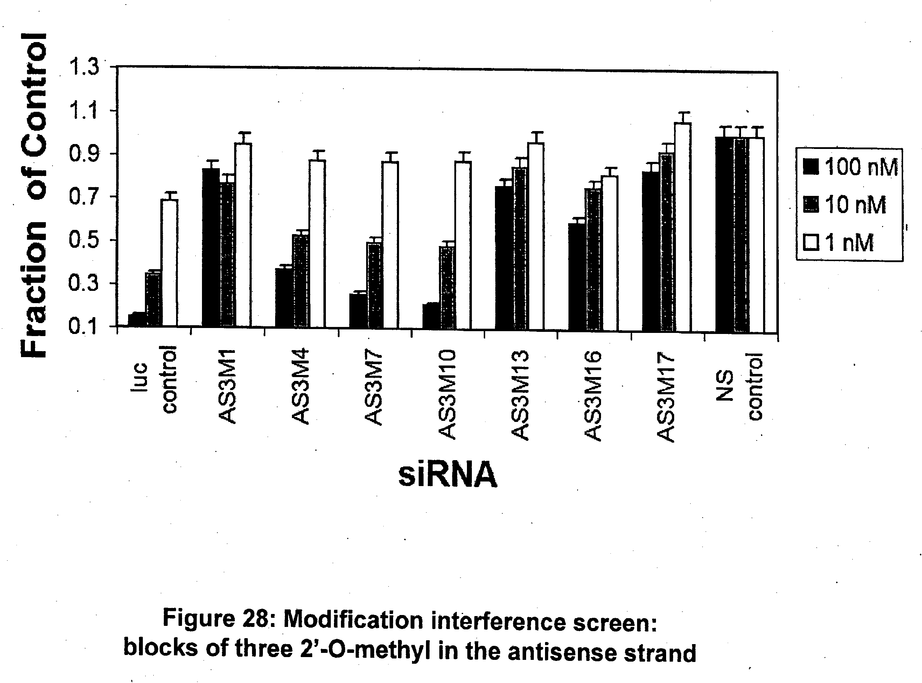

The advantages of the above described embodiments include allowing modifications of the sense strand of the siRNA duplex that promote the directionality of RISC complex assembly and prevent the sense strand from participating in gene silencing. The inventors have systematically studied the effects of using siRNAs having various modifications on the efficiency of siRNA-mediated silencing. The inventors have found that modification of each position on a sense and antisense strand with a 2'-deoxy or a 2'-O-methyl modification did not interfere with siRNA function. Where tandem blocks of 2 or 3 such modifications were used, patterns of well-tolerated modifications are different between the sense and antisense strands, siRNA duplexes having positions 1 and 2 of the sense strand modified with 2-O-methyl were fully functional. But modification of the same positions in the antisense strand resulted in completely nonfunctional siRNAs. See **Figures 19-31**. Phosphorylation of the antisense strand at its 5' end partially recovered antisense strand functionality.

The modifications described herein are an inexpensive, reliable, and non-toxic method of modifying siRNA duplexes such that a sense strand will be substantially unable to function as an antisense strand. The practical effect of this is that siRNA specificity and potency will be increased. Recent microarray analysis has suggested that the presence of 11 nucleotides is sufficient to induce nonspecific silencing, and that the homology between the sense strand of an siRNA duplex and other mRNA transcripts can, in some cases, generate at least half of non-specific activity (Jackson, A. L., et al. (2003). Expression profiling reveals off-target gene regulation by RNAi. Nature Biotechnology 21, 635-637). Thus, if the nonspecific activity of the sense strand is blocked, the duplex specificity can be increased. This would also have the effect of shifting the equilibrium toward a functional RISC formation lowering the siRNA concentration required as well.

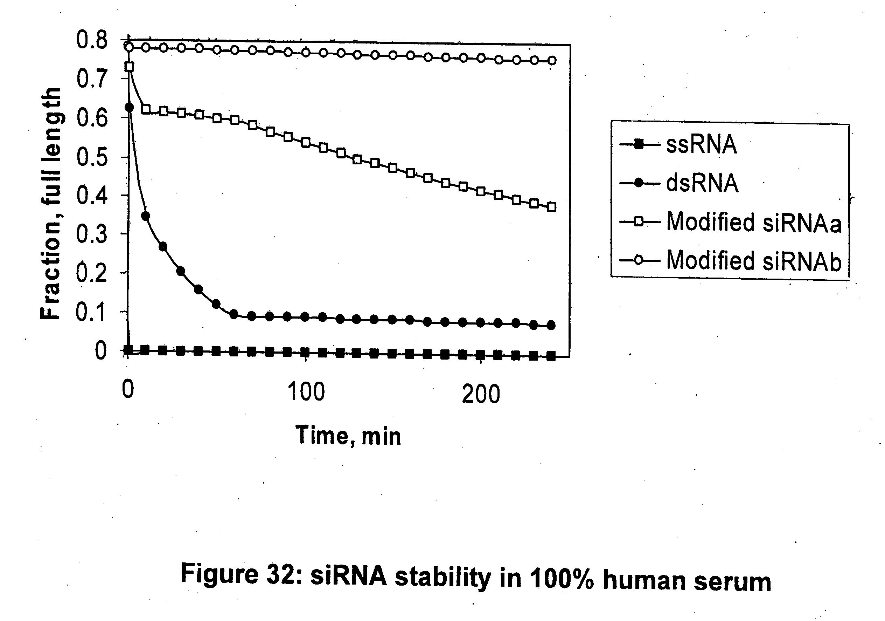

The inventors provide modifications that are well tolerated and increase the stability of an siRNA duplex in the presence of serum, such as human serum. Stabilizing modification of the sense strand of an siRNA duplex, alone, can confer some stability to a non-modified, or naked, antisense strand. Modification of every C or U of a sense strand with a 2'-O-alkyl modification, such as a 2'-O-methyl moiety, is very effective for stabilization of some sequences but not for others. Furthermore, the inventors discovered that 2'-O-methyl modification of the 5' terminal nucleotides of the sense strand is important. Modification in this manner is also expected to result in a low level of non-specific effects compared to fully modified siRNAs. As the data herein describe, modification at positions 1, 2, 18 and 19 of the sense strand does not interfere with duplex performance. One example of duplex stabilization that takes place with sense strand modification is shown in **Figure 32**. This figure demonstrates that the half-life of the anti SEAP siRNA 2217 was increased from 10 minutes to 4 hours when the sense strand of the duplex was modified with O-methyls on all Us and Cs. Modification of the 3' end by idT is important because the dTdT version of the antisense strand was two-fold less stable. This mode of modification can be applied to any sequence, because the antisense strand is left naked.

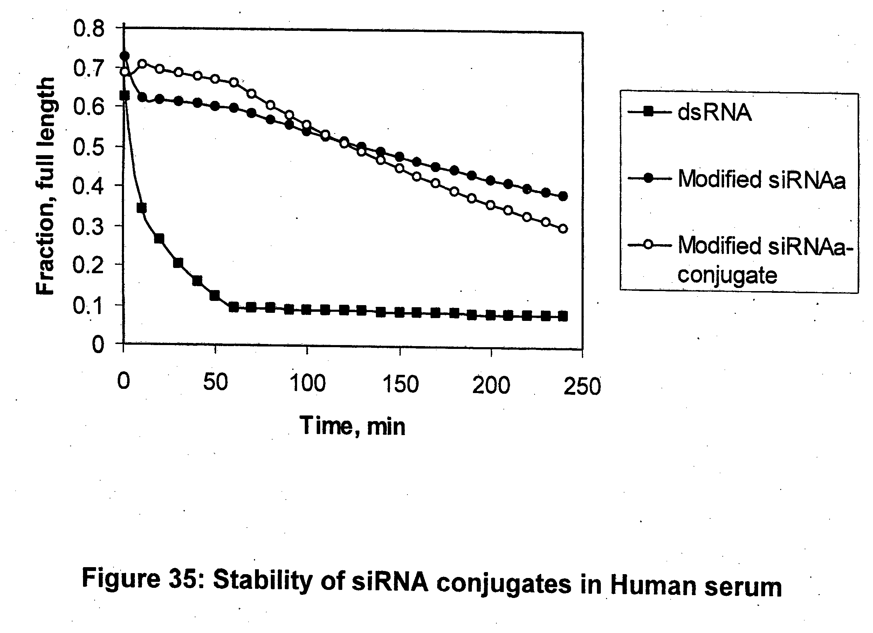

A half-life of several hours in serum should be sufficient to insure effective delivery of an siRNA, since intracellular siRNA is stabilized by the RISC complex. **Figure 35** shows the stability of an siRNA duplex when the Cs and Us of the sense strand are modified with 2'-O-methyls and an idT cap is placed on the 3' end. The functionality of this type of formulation is sequence dependent, but is significantly improved by the presence of cholesterol on the 5' end of the sense strand. Additional modifications (*e.g.*, 2'-O-methyl modifications of sense strand Cs and Us, plus 2'-O-methyl modification of positions 1 and 2 of the sense strand, 2' F modifications of Cs and Us of the antisense strand, and a phosphate group on the 5' end of the antisense strand, see **Figure 37** can further increase the serum stability of such molecules to 5 days without appreciably altering silencing functionality.

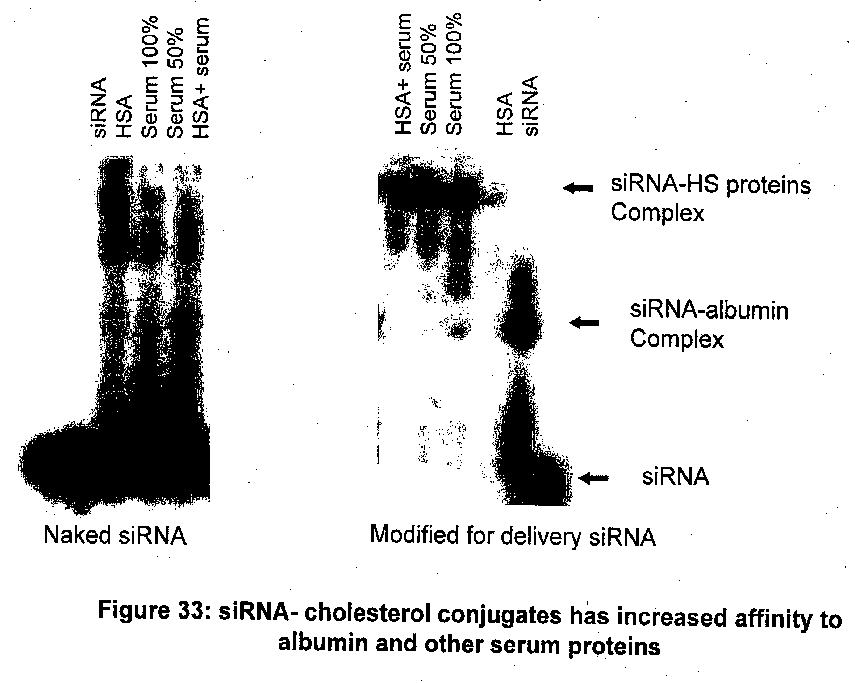

Modification of an siRNA with a cholesterol conjugate has another unexpected feature siRNAs modified with cholesterol display very high affinity for albumin and other serum proteins. See **Figure 33**. Serum protein affinity has proven useful in previous studies of antisense biodistribution in the mouse. The presence of phosphothio modifications is responsible for the majority of nonspecific antisense binding activity, but was proven beneficial for *in vivo* antisense applications, mainly because of high affinity to serum proteins and thus altered pharmacokinetic behavior. Cholesterol modified siRNAs display the advantage of serum protein affinity without the disadvantage of increased nonspecificity of phosphothio modifications.

According to the eleventh embodiment related to the present invention, there is provided an siRNA comprised of a sense strand and an antisense strand. The sense strand has a first 5' terminal sense nucleotide and a second 5' terminal sense nucleotide, both of which have 2' carbon modifications, preferably 2'-O-alkyl modifications. In some embodiments, the siRNA also comprises a label, which if present is preferably located on the first 5' terminal sense nucleotide. Further, at least one and preferably each of the pyrimidines (*e.g.*, cytosine or uracil) on the sense strand has a 2' carbon modifications, preferably 2'-O-alkyl modifications. These modifications are in addition to any modification of the first or second 5' terminal sense nucleotide, which may, depending on the sequence of the siRNA, be a pyrimidine.

The antisense strand of the eleventh embodiment related to the present invention comprises at least one 2'-halogen modified pyrimidine, preferably, a fluoro modified pyrimidine, and a first 5' terminal antisense nucleotide that has been phosphorylated. Preferably all of the pyrimidines on the antisense strand have 2'-fluoro modifications.

Preferably, the siRNA comprises from 18-30 base pairs, more preferably from 19 - 25 base pairs, and most preferably from 19 - 23 base pairs, exclusive of overhangs or loop or stem structures when present in unimolecular siRNAs that are capable of forming hairpins. Preferably, the sense strand and antisense strand are, exclusive of overhangs, loop or stem structures at least 79% complementary, more preferably at least 90% complementary over the range of base pairs, and most preferably 100% complementary over this range. Similarly preferably, the antisense region is preferably at least 79%, more preferably at least 90%, and most preferably at least 100% complementary to the target region. Preferably, the polynucleotide is RNA.

The siRNA may also contain overhangs at either the 5' or 3' end of either the sense strand or the antisense strand. However, in the case of polynucleotides comprised of two separate strands preferably, if there are any overhangs, they are only on the 3' end of the sense strand and/or the antisense strand. Additionally, preferably any overhangs are six or fewer bases in length, more preferably two or fewer bases in length. Most preferably, there are either no overhangs, or overhangs of two bases on one or both of the sense strand and antisense strand at the 3' end. According to the eleventh embodiment it is preferable not to have overhangs on the 5' end of the antisense strand. Overhanging nucleotides are frequently removed by one or more intracellular enzymatic processes or events, which may leave an unphosphorylated 5'-nucleotide. Therefore, it is preferable not to have overhangs on the 5' end of the antisense strand.

The phosphorylation of the first 5' terminal antisense nucleotide refers to the presence of one or more phosphate groups attached to the 5' carbon of the sugar moiety of the nucleotide. Preferably, there is only one phosphate group.

The 2'-O-alkyl modifications, regardless of on which bases they appear are preferably selected from the group consisting of 2'-O-methyl, 2'-O-ethyl, 2'-O-propyl, 2'-O-isopropyl, 2'-O-butyl, 2-O-isobutyl, 2'-O-ethyl-O-methyl (-CH₂CH₂OCH₃), and 2'-O-ethyl-OH (-OCH₂CH₂OH). Most preferably, the 2'-O-alkyl modification is a 2'-O-methyl moiety. Further, there is no requirement that the modification be the same on each of the first 5' terminal sense nucleotide and the second 5' terminal sense nucleotide. However, as a matter of practicality with respect to synthesizing the molecules of the present invention, it may be desirable to use the same modification throughout.

With respect to the 2'-O-alkyl, pyrimidine modifications, at least one pyrimidine other than any pyrimidine within the first two 5' terminal sense nucleotides is modified with a 2'-O-alkyl group. Preferably, all pyrimidines on the sense strand within the duplex forming region are modified. Further, preferably, the 2'-O-alkyl modifications are 2'-O-methyl groups. As with the 2'-O-alkyl modifications, the same alkyl modification need not be used on each nucleotide that has a 2'-O-alkyl group. When overhangs, loops or stems are present, the pyrimidines of those regions may or may not contain 2'-O-alkyl groups. However, at least one of the 2'-O-alkyl groups is preferably in the sense region.

With respect to the 2' halogen modified nucleotides, the modification appears on at least one of the pyrimidines of the antisense strand and more preferably on all of the pyrimidines of the antisense strand. Further, preferably the halogen is fluorine. When overhangs, stems or loops are presented, the pyrimidines of those regions may or may not contain halogen groups but preferably any stem or loop structure would not contain this modification, and the at least one halogen group is within the sense region. It should be noted that 2' halogen modifications, including 2' fluoro modifications may be used to increase the stability of the siRNA independent of the other modifications described herein. Thus, they may be used independently, as well as in connection with these other modifications in siRNA applications.

Other types of nucleotide modifications of the sense and/or antisense strands may be included if they do not greatly negate the benefits of the relevant siRNAs, including stability and with respect to the eleventh embodiment related to the present invention, functionality. For example, the use of pyrimidine modified nucleotides such a halogen, preferably fluorine, modified and additional 2'-O-alkyl modified pyrimidines provide a certain level of nuclease resistance.

In addition to chemical modifications, it is postulated that base pair mismatches or bulges can be added to the sense and/or antisense strands that alter the ability of these strands to participate in RISC-mediated association with targets that share less than 100% homology. Examples of such mismatches include (but are not limited to) purine-pyrimidine mismatches (*e.g*., G-U, C-A) and purine-purine or pyrimidine-pyrimidine mismatches (*e.g*., G-A, U-C). The introduction of these types of modification maybe combined with the above-described modifications, and evaluated to determine whether they alter other attributes of functionality (*e.g*., off-target effects) without detracting from the benefits of the relevant siRNAs.

In some applications, it may be desirable to use a label, for example, a fluorescent labeL When the label is fluorescent, preferably, the fluorescent label is Cy3^{™}, Cy5^{™}, or the Alexa dyes (Molecular Probes, Eugene, OR). These labels are preferably added to the 5' end of the sense strand, more preferably at the 5' terminal sense nucleotide. They may be used to enable users to visualize the distribution of the labeled siRNA within, *e.g*., a transfected cell, and allow one to assess the success of any given transfection. The use of labeled nucleotides is well known to persons of ordinary skill, and labels other than fluorescent labels, *e.g*., mass or radioactive labels, may be used in applications in which such types of labels would be advantageous.

The fluorescent modifications can be used to segregate transfected cells from untransfected cells. Specifically, a population of cells can be transfected with the molecules of the eleventh embodiment and subsequently sorted by FACS to segregate transfected cells from untransfected cells. This enables one to obtain a purer population (rather than a mixed one), which in turn improves ones ability to clearly identify phenotypes that result from gene silencing.

The molecules of the eleventh embodiment related to the present invention have a variety of uses including, for example, the molecules may be used as transfection control reagents or stable silencing reagents. When these molecules contain labels, transfection of these molecules into cells allows the user to visualize and to determine what fraction of the cells have been successfully transfected. In addition, these modifications do not appreciably alter siRNA function; thus, the molecules of the eleventh embodiment can simultaneously be transfection controls and silencing reagents. Further, because the above-described modifications do not place limitations on the sequences that may be used, they may be used in diverse siRNA and RNAi applications and are not target sequence dependent.

Thus, molecules of the eleventh embodiment, with their unique set of modifications, provide stability and "trackability" without altering functionality. They can also be used to isolate a pure population of cells that have been transfected. If 2'-O-alkyl groups are added to the first two or first three nucleotides of the antisense strand, the additional benefit of reducing off-target effects can be realized. Further if 2'-O-alkyl groups are added to the first two or three nucleotides of the sense strand, they can have the additional benefit of reducing sense strand off-target effects.

According to a twelfth embodiment related to the present invention there is provided another siRNA comprised of a sense strand and an antisense strand. The sense strand is defined according to the same parameters as the sense strand for the eleventh embodiment related to the present invention, including the 2' carbon modification, preferably 2'-O-alkyl modifications of the first and second 5' terminal sense nucleotide and at the at least one 2' carbon modification, preferably 2'-O-alkyl modification of pyrimidines. However, instead of the above-described modifications to the antisense strand, the antisense strand of the twelfth embodiment comprises first and second, or first, second and third, 5' terminal antisense nucleotides, each of which have 2'-O-alkyl modifications. Further, there is at least one 2'-O-alkyl modified pyrimidine on the antisense strand other than the modification on the first and second 5' terminal antisense nucleotides. Still further, preferably there are no 2' Fl modifications and there is no phosphorylation of the first 5' terminal antisense nucleotide. The absence of phosphorylation renders the molecule of limited functionality as compared to corresponding functional polynucleotides. As another variation to the above description, molecules of the twelfth embodiment can, in addition to all of the above attributes, contain 5'-blocking groups on the first nucleotide of the sense, antisense, or both sense and antisense strands. Such blocking groups will further eliminate the possibility that this molecule will be phosphorylated and enter RISC, and can consist of 5'-alkyl, 5'-O-alkyl, azide (N3), amines, and other moieties.

The other preferred parameters with respect to size, overhangs and other modifications are the same as for the eleventh embodiment However, because these compositions are not used for silencing of genes, they could be 16 - 28 base pairs in length, thought are preferably 18 - 30 base pairs in length.

The molecules of the twelfth embodiment may, in addition to being a transfection control, also act as a potential "filler" or exaequo agent, which is particularly useful in dosage experiments or as a negative control in microarray studies. Many experiments test the effects of a given siRNA at different concentrations (*e.g*., 100 nM, 50 nM, 25 nM, and 1 nM). Optimally, when transfection experiments are performed, one wants to have a constant concentration of "total siRNA" to avoid any anomalies that result from transfection of different levels of nucleic acids. Unfortunately, addition of any unmodified control siRNA (*e.g*., a non-specific control) has the potential downside of competing with the siRNA under study for interaction with RISC. Molecules of the twelfth embodiment alleviate this problem. The addition of 2'-O-methyl modifications on positions 1 and 2 (or 1, 2 and 3) of both the sense and antisense strands in the absence of a phosphorylated 5' terminal antisense nucleotide, limits the ability of this molecule to interact with RISC. Thus, this molecule can be transfected, but it cannot compete with other siRNA for RISC. As was the case with the molecules of the eleventh embodiment, the addition of 2'-O-methyl groups on the pyrimidines (Cs and Us) minimizes the possibility of nuclease digestion.

The molecules of the twelfth embodiment, unlike other "non-specific" sequences or controls, interact poorly with RISC. Thus, these molecules should generate only limited off-targeting side effects, and have the ability of being trackable without competing for sites on RISC.

The above-described embodiments related to the present invention (both the eleventh and the twelfth) may apply to siRNA that do not have contiguous sense and antisense strands (*i.e*., two separate strands), as well as to unimolecular siRNAs that are capable of forming hairpins (shRNA). The term "siRNA" includes both types of RNA. For example, the hairpin may comprise a loop structure, which preferably comprises from four to ten bases; and a sense region, wherein the sense region and antisense regions are independently 19-23 base pairs in length and substantially complementary to each other. Preferable sequences of the loop structure include, for example, 5'-UUCG (SEQ. ID NO. 319), 5'-UUUGUGUAG (SEQ. ID NO. 320), and 5'-CUUCCUGUCA (SEQ. ID NO. 321).

The hairpin is preferably constructed with the loop region downstream of the antisense region. This construction is desirable particularly with respect to the eleventh embodiment, because it is easier to phosphorylate the terminal antisense nucleotide. Thus, when designing the unimolecular siRNAs, it is preferable that there are no overhangs upstream of the 5' terminal antisense nucleotide.

When designing a unimolecular siRNA, specifically a left-handed unimolecular structure (*e.g*., 5' -AS-Loop-S) according to the present invention, preferably, the first 5' terminal sense nucleotide is defined as the nucleotide that is the 18^{th}, 19^{th} or 20^{th} base of the sense region counting from the base that is complementary to the first 5' terminal antisense nucleotide (*i.e*. counting from the 3' end of the sense region). The first 5' terminal sense nucleotide is defined in this manner because when unimolecular siRNA that are capable of forming hairpins enter a cell, typically, Dicer will process hairpin siRNAs that contain lengthier duplex regions, into molecules that are comprised of two separate strands (siRNA) of approximately 18 -20 base pairs, and it is desirable for these molecules to have the sense strand modifications associated with the end of this processed molecule. Most preferably, the first 5'terminal sense nucleotide is defined as the nucleotide that is the 19^{th} base of the sense region from the 3' end of the sense region. Further, preferably, the polynucleotide is capable of forming a left-handed hairpin.

The siRNA can further comprise a stem region, wherein the stem region comprises one or more nucleotides or modified nucleotides immediately adjacent to the 5' end and the 3' end of the loop structure, and wherein the one or more nucleotides or modified nucleotides of the stem region are or are not target-specific. Preferably, the entire length of the unimolecular siRNA contains fewer than 100 bases, more preferably fewer than 85 bases.

The unimolecular siRNAs of the present invention may ultimately be processed by cellular machinery such that they are converted into two separate strands. Alternatively, the molecules may bypass one or more steps in the RNAi pathway (*e.g*., Dicer processing) and enter RISC as unimolecular hairpin molecules. Further, these unimolecular siRNAs may be introduced into the cell with less than all modifications, and modified in the cell itself through the use of natural processes or processing molecules that have been introduced (*e.g*., with respect to the eleventh embodiment, phosphorylation in the cell by native kinases). However, preferably the polynucleotide is introduced with all modifications already present. (Similarly, when the siRNA is comprised of two separate strands, preferably those strands contain all modifications when introduced into the cell with all modification, though the antisense strand could *e.g*., be modified with a phosphorylation group after introduction.)

Although the above-described embodiments related to the present invention (eleventh and twelfth) are directed to increased stability, it is important to note that these and other types of modifications may also affect other parameters, such as specificity. Further, these stability and functionality modifications maybe combined (*e.g*., 2' carbon modifications (preferably O-methyl modifications) of the first and second (or first, second and third) 5' terminal sense and antisense nucleotides in conjunction with an additional 2' carbon modifications (preferably O-methyl modifications) of at least one sense pyrimidine, preferably all sense pyrimidine(s) other than the first two (or three) that have 2' carbon modifications (preferably-O-methyl modifications), at least one, preferably all, 2'-F1 modifications of antisense pyrimidine(s) other than the first two (or three that have 2' carbon modifications (preferably-O-methyl modifications) and phosphorylation of the 5' terminal antisense nucleotide.) Further, the above described modifications may be combined with siRNA that contain sequences that were selected at random, or according to rationale design as described in, for example, U.S. patent Application Serial No. 10/714,333.

Additionally stabilization modifications that are addressed to the phosphate backbone maybe included in the polynucleotides for some applications of the present invention. For example, at least one phosphorothioate and/or methylphosphonate may be substituted for the phosphate group at some or all 3' positions of any or all pyrimidines in the sense and/or antisense strands of the oligonucleotide backbone, as well as in any overhangs, loop structures or stem structures that may be present. Phosphorothioate (and methylphosphonate) analogues arise from modification of the phosphate groups in the oligonucleotide backbone. In the phosphorothioate, the phosphate O⁻ is replaced by a sulfur atom. In methylphosphonates, the oxygen is replaced with a methyl group. In one embodiment the phosphorothioate modification or methylphosphonate is located at the 3' positions of all antisense strand nucleotides that also contains 2' fluoro (or other halogen) modified nucleotides. Additionally, phosphorothioate 3' modifications maybe used instead of and independent of 2' fluoro modifications to increase stability of an siRNA molecule. These modifications may be used in combination with the other modifications disclosed herein, or independent of those modifications in siRNA applications.

Nucleases typically use both the oxygen groups on the phosphate moiety and the 2'OH position of the ribose ring to mediate attack on RNA. Substitution of a sulfur group for one of the oxygens eliminates the ability of the phosphate to participate in this, reaction, thus limiting the sensitivity of this site to nuclease digestion. However, it should be noted that phosphorothioates are typically toxic, thus, they would be beneficial primarily when any toxic effects are negated, which it is postulated might be accomplished by limiting the use of this modification to *e.g*., every other nucleotide, every third nucleotide, or every fourth nucleotide.

The molecules of the twelfth embodiment related to the present invention are of limited functionality, but as noted above, may be used in negative control studies, and as exaequo agents. The set of modifications associated with the molecules make them poor substrates for intracellular kinases that typically add an essential phosphate group to the 5' end of the antisense strand of siRNA. While the modifications strongly inhibit phosphorylation, it is possible that some small fraction of molecules carrying these modifications might still be phosphorylated at C5 of the 5'-antisense position and generate a small amount of silencing. In these cases, it may be desirable to modify the 5' carbon position of the 5' end of the sense and/or the antisense strand of these molecules with a blocking group that prevents kinase phosphorylation. The blocking group may for example be any group that prevents phosphorylation of the 5' carbon position of the terminal nucleoside, including, but not limited to 5'-alkyl , 5'-O-methyl, and 5'- amine groups.

Another example of a control or exaequo agent contain only the following 2' carbon modifications: (i) a first and second, or first, second and third, 5' terminal sense nucleotides that each comprises 2'modifications, such as the 2'modifications described above, for example 2'-O-methyl modifications; and (ii) a first and second, or first, second and third, 5' terminal antisense nucleotides that each comprises 2'modifications, such as the 2'modifications described above, for example 2'-O-methyl modifications, and the 5' terminal antisense nucleotide has not been phosphorylated.

The polynucleotides of the present invention may be synthesized by any method that is now known or that comes to be known and that from reading this disclosure a person of ordinary skill in the art would appreciate would be useful to synthesize the molecules of the present invention. siRNA duplexes containing the specified modifications may be chemically synthesized using compositions of matter and methods described in Scaringe, S.A (2000) "Advanced 5'-silyl-2'-orthoester approach to RNA oligonucleotide synthesis," Methods Enzymol. 317, 3-18;Scaringe, S.A. (2001) "RNA oligonucleotide synthesis via 5'-silyl-2'-orthoester chemistry," Methods 23, 206-217; Scaringe, S. and Caruthers, M.H. (1999) U.S. Patent No. 5,889,136; Scaringe, S. and Caruthers, M.H. (1999) U.S. Patent No. 6,008,400; Scaringe, S. (2000) U.S. Patent No. 6111086; Scaringe, S. (2003) U.S. Patent No. 6,590,093. The synthesis method utilizes nucleoside base-protected 5'-O-silyl-2'-O-orthoester-3'-O-phosphoramidites to assemble the desired unmodified siRNA sequence on a solid support in the 3' to 5' direction. Briefly, synthesis of the required phosphoramidites begins from standard base-protected ribonucleosides (uridine, N⁴-acetylcytidine, N²-isobutyrylguanosine and N⁶-isobutyryladenosine). Introduction of the 5'-O-silyl and 2'-O-orthoester protecting groups, as well as the reactive 3'-O-phosphoramidite moiety is then accomplished in five steps, including:
1. Simultaneous transient blocking of the 5'- and 3'-hydroxyl groups of the nucleoside sugar with Markiewicz reagent (1,3-dichloro-1,1,3,3,-tetraisopropyldisiloxane [TIPS-Cl₂]) in pyridine solution {Markiewicz, W.T. (1979) "Tetraisopropyldisiloxane-1,3-diyl, a Group for Simultaneous Protection of 3'- and 5'-Hydroxy Functions of Nucleosides," J. Chem. Research(S), 24-25}, followed by chromatographic purification;
2. Regiospecific conversion of the 2'-hydroxyl of the TIPS-nucleoside sugar to the bis(acetoxyethyl)orthoester [ACE derivative] using tris(acetoxyethyl)-orthoformate in dichloromethane with pyridinium p-toluenesulfonate as catalyst, followed by chromatographic purification;
3. Liberation of the 5'- and 3'-hydroxy groups of the nucleoside sugar by specific removal of the TIPS-protecting group using hydrogen fluoride and N,N,N"N'-tetramethylethylene diamine in acetonitrile, followed chromatographic purification;
4. Protection of the 5'-hydroxyl as a 5'-O-silyl ether using benzhydroxy-*bis*(trimethylsilyloxy)silyl chloride [BzH-Cl] in dichloromethane, followed by chromatographic purification; and
5. Conversion to the 3'-O-phosphoramidite derivative using *bis*(N,N-diisopropylamino)methoxyphosphine and 5-ethylthio-1H-tetrazole in dichloromethane/acetonitrile, followed by chromatographic purification.

The phosphoramidite derivatives are typically thick, colorless to pale yellow syrups. For compatibility with automated RNA synthesis instrumentation, each of the products is dissolved in a pre-determined volume of anhydrous acetonitrile, and this solution is aliquoted into the appropriate number of serum vials to yield a 1.0-mmole quantity of phosphoramidite in each vial. The vials are then placed in a suitable vacuum desiccator and the solvent removed under high vacuum overnight. The atmosphere is then replaced with dry argon, the vials are capped with rubber septa, and the packaged phosphoramidites are stored at -20°C until needed. Each phosphoramidite is dissolved in sufficient anhydrous acetonitrile to give the desired concentration prior to installation on the synthesis instrument.

The synthesis of the desired oligoribonucleotide is carried out using automated synthesis instrumentation. It begins with the 3'-terminal nucleoside covalently bound via its 3'-hydroxyl to a solid beaded polystyrene support through a cleavable linkage. The appropriate quantity of support for the desired synthesis scale is measured into a reaction cartridge, which is then affixed to synthesis instrument. The bound nucleoside is protected with a 5'-O-dimethoxytrityl moiety, which is removed with anhydrous acid (3% [v/v] dichloroacetic acid in dichloromethane) in order to free the 5'-hydroxyl for chain assembly.

Subsequent nucleosides in the sequence to be assembled are sequentially added to the growing chain on the solid support using a four-step cycle, consisting of the following general reactions:
1. Coupling: the appropriate phosphoramidite is activated with 5-ethylthio-1H-tetrazole and allowed to react with the free 5'-hydroxyl of the support bound nucleoside or oligonucleotide. Optimization of the concentrations and molar excesses of these two reagents, as well as of the reaction time, results in coupling yields generally in excess of 98% per cycle.
2. Oxidation: the internucleotide linkage formed in the coupling step leaves the phosphorous atom in its P(III) [phosphite] oxidation state. The biologically-relevant oxidation state is P(V) phosphate]. The phosphorous is therefore oxidized from P(III) to P(V) using a solution of *tert-*butylhydroperoxide in toluene.
3. Capping: the small quantity of residual un-reacted 5'-hydroxyl groups must be blocked from participation in subsequent coupling cycles in order to prevent the formation of deletion-containing sequences. This is accomplished by treating the supports with a large excess of acetic anhydride and 1-methylimidazole in acetonitrile, which efficiently blocks residual 5'-hydroxyl groups as acetate esters.
4. De-silylation: the silyl-protected 5'-hydtoxyl must be deprotected prior to the next coupling reaction. This is accomplished through treatment with triethylamine trihydrogen fluoride in N,N-dimethylformamide, which rapidly and specifically liberates the 5'-hydroxyl without concomitant removal of other protecting groups (2'-O-ACE, N-acyl base-protecting groups, or phosphate methyl).

It should be noted that in between the above four reaction steps are several washes with acetonitrile, which are employed to remove the excess of reagents and solvents prior to the next reaction step. The above cycle is repeated the necessary number of times until the unmodified portion of the oligoribonucleotide has been assembled. The above synthesis method is only exemplary and should not be construed as limited the means by which the molecules may be made. Any method that is now known or that comes to be known for synthesizing siRNA and that from reading this disclosure one skilled in the art would conclude would be useful in connection with the present invention may be employed.

The siRNA duplexes of certain embodiments of the eleventh and twelfth embodiments related to the present invention include two modified nucleosides (*e.g*., 2'-O-methyl derivatives) at the 5'-end of each strand. The 5'-O-silyl-2'-O-methyl-3'-O-phosphoramidite derivatives required for the introduction of these modified nucleosides are prepared using procedures similar to those described previously (steps 4 and 5 above), starting from base-protected 2'-O-methyl nucleosides (2'-O-methyl-uridine, 2'-O-methyl- N⁴-acetylcytidine, 2'-O-methyl-N²-isobutyrylguanosine and 2'-O-methyl-N⁶-isobutyryladenosine). The absence of the 2'-hydroxyl in these modified nucleosides eliminates the need for ACE protection of these compounds. As such, introduction of the 5'-O-silyl and the reactive 3'-O-phosphoramidite moiety is accomplished in two steps, including:
1. Protection of the 5'-hydroxy as a 5'-O-silyl ether using benzhydroxy-*bis*(trimethylsityloxy)silyl chloride (BzH-Cl) in N,N-dimethylformamide, followed by chromatographic purification; and
2. Conversion to the 3'-O-phosphoramidite derivative using *bis*(N,N-diisopropylamino)methoxyphosphine and 5-ethylthio-1H-tetrazole in dichloromethane/acetonitrile, allowed by chromatographic purification.

Post-purification packaging of the phosphoramidites is carried out using the procedures described previously for the standard nucleoside phosphoramidites. Similarly, the incorporation of the two 5'-O-silyl-2'-O-methyl nucleosides via their phosphoramidite derivatives is accomplished by twice applying the same four-step cycle described previously for the standard nucleoside phosphoramidites.

The siRNA duplexes of certain embodiments of the eleventh embodiment related to the present invention include a phosphate moiety at the 5'-end of the antisense strand This phosphate is introduced chemically as the final coupling to the antisense sequence. The required phosphoramidite derivative (*bis*(eyanoethyl)-N,N-diisopropylamino phosphoramidite) is synthesized as follows in brief phosphorous trichloride is treated one equivalent of N,N-diisopropylamine in anhydrous tetrahydrofuran in the presence of excess triethylamine. Then, two equivalents of 3-hydroxypropionitrile are added and allowed to react completely. Finally, the product is purified by chromatography. Post purification packaging of the phosphoramidite is carried out using the procedures described previously for the standard nucleoside phosphoramidites. Similarly, the incorporation of the phosphoramidite at the 5'-end of the antisense strand is accomplished by applying the same four-step cycle described previously for the standard nucleoside phosphoramidites.

The modified, protected oligoribonucleotide remains linked to the solid support at the finish of chain assembly. A two-step rapid cleavage/deprotection procedure is used to remove the phosphate methyl protecting groups, cleave the oligoribonucleotide from the solid support, and remove the N-acyl base-protecting groups. It should be noted that this procedure also removes the cyanoethyl protecting groups from the 5'-phosphate on the antisense strand Additionally, the procedure removes the acetyl functionalities from the ACE orthoester, converting the 2'-O-ACE protecting group into the bis(2-hydroxyethyl)orthoester. This new orthoester is significantly more labile to mild acid as well as more hydrophilic than the parent ACE group. The two-step procedure is briefly as follows:
1. The support-bound oligonbonucleotide is treated with a solution of disodium 2-carbamoyl-2-cyanoethylene-1,1 -dithiolate trihydrate in N,N-dimethylformamide. This reagent rapidly and efficiently removes the methyl protecting groups from the internucleotide phosphate linkages without cleaving the oligoribonucleotide from the solid support. The support is then washed with water to remove excess dithiolate.
2. The oligoribonucleotide is cleaved from the solid support with 40% (wlv) aqueous methylamine at room temperature. The methylamine solution containing the crude oligoribonucleotide is then heated to 55°C to remove the protecting groups from the nucleoside bases. The crude orthoester-protected oligoribonucleotide is obtained following solvent removal *in vacuo.*

Removal of the 2'-orthoesters is the final step in the synthesis process. This is accomplished by treating the crude oligoribonucleotide with an aqueous solution of acetic acid and N,N,N',N'-tetramethylethylene diamine, pH 3.8, at 55°C for 35 minutes. The completely deprotected oligoribonucleotide is then desalted by ethanol precipitation and isolated by centrifugation.

In addition, incorporation of fluorescent labels at the 5'-terminus of a polynucleotide is a common and well-understood manipulation for those skilled in the art. In general, there are two methods that are employed to accomplish this incorporation, and the necessary materials are available from several commercial sources (*e.g*., Glen Research Inc., Sterling, Virginia, USA; Molecular Probes Inc., Eugene, Oregon, USA; TriLink BioTechnologies Inc., San Diego, California, USA; and others). The first method utilizes a fluorescent molecule that has been derivatized with a phosphoramidite moiety similar to the phosphoramidite derivatives of the nucleosides described previously. In such case, the fluorescent dye is appended to the support-bound polynucleotide in the final cycle of chain assembly. The fluorophore-modified polynucleotide is then cleaved from the solid support and deprotected using the standard procedures described above. This method has been termed "direct labeling." Alternatively, the second method utilizes a linker molecule derivatized with a phosphoramidite moiety that contains a protected reactive functional group (*e.g*., amino, sulfhydryl, carbonyl, carboxyl, and others). This linker molecule is appended to the support-bound polynucleotide in the final cycle of chain assembly. The linker-modified polynucleotide is then cleaved from the solid support and deprotected using the standard procedures described above. The functional group on the linker is deprotected either during the standard deprotection procedure, or by utilizing a subsequent group-specific treatment The crude linker-modified polynucleotide is then reacted with an appropriate fluorophore derivative that will result in formation of a covalent bond between a site on the fluorophore and the functional group of the linker. This method has been termed "indirect labeling."

Once synthesized, the polynucleotides of the present invention may immediately used or be stored for future use. Preferably, the polynucleotides of the invention are stored as duplexes in a suitable buffer. Many buffers are known in the art suitable for storing siRNAs. For example, the buffer may be comprised of 100 mM KCl, 30 mM HEPES-pH 7.5, and 1mM MgCl₂. Preferably, the siRNAs of the present invention retain 30% to 100% of their activity when stored in such a buffer at 4°C for one year. More preferably,-they retain 80% to 100% of their biological activity when stored in such a buffer at 4°C for one year. Alternatively, the compositions can be stored at -20°C in such a buffer for at least a year or more. Preferably, storage for a year or more at -20°C results in less than a 50% decrease in biological activity. More preferably, storage for a year or more at -20°C results in less than a 20% decrease in biological activity after a year or more. Most preferably, storage for a year or more at-20°C results in less than a 10% decrease in biological activity.

In order to ensure stability of the siRNA pools prior to usage, they may be retained in dried-down form at -20°C until they are ready for use. Prior to usage, they should be resuspended; however, once resuspended, for example, in the aforementioned buffer, they should be kept at -20°C until used. The aforementioned buffer, prior to use, may be stored at approximately 4°C or room temperature. Effective temperatures at which to conduct transfection are well known to persons skilled in the art, and include for example, room temperature.

In developing the present invention, two or more different modifications were added to a duplex to increase stability. Applicants appreciate that other modifications and combinations may be discovered in the future that assist in improving stability. Additionally, the modifications of the present invention could be combined with modifications that are desired for other purposes. For example, in some instances, one modification could stabilize the molecule against one particular set of conditions (*e.g*., one type of nuclease) while a second modification could stabilize the molecule against a second set of conditions (*e.g*., a different family of nucleases). Alternatively, two separate modifications could act additively or synergistically to stabilize a molecule towards a certain set of conditions. In still other instances, one modification could stabilize the molecule, but have detrimental consequences on other desirable properties, *e.g*., the potency or toxicity of the siRNA. In cases such as these, additional modifications could be added that restore these aspects of functionality of the molecule.

A variety of approaches can be used to identify both the type of molecule and the key position needed to enhance stability. In one non-limiting example, a modification-function walk is performed. In this procedure, a single type of modification is added to one or more nucleotides across the sense and/or antisense strand. Subsequently, modified and unmodified molecules are tested for (1) functionality and (2) stability, by one of several methods. Thus, for example, 2'-O-Me groups can be added to positions 1 and 2, 3 and 4, 5 and 6, 7 and 8, 9 and 10, 11 and 12, 13 and 14, 15 and 16, 17 and 18, or 18 and 19 of either the sense and/or antisense strand and tested for functionality (*e.g*., by measuring the ability of these molecules to silence specific targets) and stabilize the molecule against actions by, *e.g*., nucleases. If key positions are identified that enhance stability, but result in a loss of duplex functionality, then a second round of modification walks, whereby additional chemical groups (*e.g*., 5' phosphate on the 5' end of the antisense strand), mismatches, or bulges that are suspected to increase duplex functionality can be added to molecules that already contain the modification that enhance stability.

In one aspect, herein labelled a "thirteenth embodiment", the present invention is directed to an siRNA, comprising:
(a) an antisense strand, wherein said antisense strand is comprised of a first 5' terminal antisense nucleotide and said first 5' terminal antisense nucleotide is phosphorylated at said first 5' terminal antisense nucleotide's 5' carbon position; and
(b) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification.

Preferably, the siRNA comprises from 18 - 30 base pairs, more preferably from 19 - 25 base pairs, and most preferably from 19-23 base pairs, exclusive of overhangs. Preferably, the sense strand and antisense strand are at least substantially complementary over the range of base pairs, and more preferably 100 % complementary over this range. Preferably the polynucleotide is RNA.

The siRNA may also contain overhangs at either the 5' or 3' end of either the sense strand or the antisense strand. However, preferably if there are any overhangs, they are on the 3' end of the sense strand and/or the antisense strand. Additionally, preferably any overhangs are six or fewer bases in length, more preferably two or fewer bases in length. Most preferably, there are either no overhangs, or overhangs of two bases on one or both of the sense strand and antisense strand at the 3' end. Because overhanging nucleotides are frequently removed by one or more intracellular enzymatic processes or events, thus leaving an unphosphorylated 5'-nucleotide, it is preferable not to have overhangs on the 5' end of the antisense strand.

The phosphorylation of the first 5' terminal antisense nucleotide refers to the presence of one or more phosphate groups attached to the 5' carbon of the sugar moiety of the nucleotide. Preferably there is only one phosphate group.

The 2' carbon modifications refer to modifications on the 2' carbon of the nucleotide's sugar moiety. The "first 2' carbon sense modification" refers to the modification that is on the first 5' terminal sense nucleotide. The "second 2' carbon sense modification" refers to the modification that is on the second 5' terminal sense nucleotide.

It is known that addition of chemical modifications to key positions along an RNA-RNA, RNA-DNA, or DNA-DNA duplex can significantly alter the chemical and functional properties of these molecules. Applicants appreciate that modifications can be added to the sense strand of a siRNA to prevent that strand from entering RISC and inducing sense strand specific off-target effects. Such modifications can include addition of any number of chemical moieties. Preferably, the moiety is attached to the 2' position of the nucleotide's ribose ring (i.e., the 2' carbon). According to the present embodiment, preferably the modification is a 2'-O-alkyl group. However, it may be any other modification that when used in the context of the present invention minimizes off-target effects by this strand. For example, the 2' modified nucleotide may be selected from the group consisting of a 2' halogen modified nucleotide, a 2' amine modified nucleotide and a 2' alkyl modified nucleotide if such modifications are included under conditions that minimize off-target effects. Where the modification is a halogen, the halogen is preferably fluorine. Where the 2' modified nucleotide is a 2' amine modified nucleotide, the amine is preferably -NH₂. Where the 2' modified nucleotide is a 2'-alkyl modification, preferably the modification is a 2' methyl modification, wherein the carbon of the methyl moiety is attached directly to the 2' carbon of the sugar moiety.

As noted above, preferably the modification is a 2'-O-alkyl group. More preferably the modification is selected from the group consisting of 2'-O-methyl, 2'-O-ethyl, 2'-O-propyl, 2'-O-isopropyl, 2'-O-butyl, 2-O-isobutyl, 2'-O-ethyl-O-methyl (-OCH₂CH₂OCH₃), and 2'-O-ethyl-OH (-OCH₂CH₂OH). Most preferably, the 2'-O-alkyl modification is a 2'-O-methyl moiety. Further, there is no requirement that the modification be the same on each of the first 5' terminal sense nucleotide and the second 5' terminal sense nucleotide. However, as a matter of practicality with respect to synthesizing the molecules of the present invention, it may be desirable to use the same modification throughout.

In order to determine what modifications are permissible, several non-limiting assays can be performed to identify modifications that limit off-target effects. In one non-limiting example, the sense strand (carrying the modification being tested) can be labeled with one of many labeled nucleotides. Subsequently, a binding assay can be performed whereby the affinity of RISC for the modified sense strand can be compared with that of the unmodified form.

In another aspect, herein labelled "a fourteenth embodiment", the present invention is directed to a unimolecular siRNA that is capable of forming a hairpin siRNA, said unimolecular siRNA comprising:
(a) an antisense region, wherein said antisense region is comprised of a first 5' terminal antisense nucleotide and wherein said first 5' terminal antisense nucleotide is phosphorylated at said first 5' terminal antisense nucleotide's 5' carbon position;
(b) a sense region, wherein said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
(c) a loop region, wherein said loop region is located between said sense region and said antisense region.

According to this embodiment, the range of modifications is the same as those for the thirteenth embodiment. However, because the polynucleotide is unimolecular and is capable of forming a hairpin, and not two separate strands, there is one contiguous molecule that comprises both a sense region and an antisense region. Preferably, the sense region and the antisense region are at least substantially complementary, more preferably 100% complementary. As with the thirteenth embodiment, preferably the sense region and the antisense region comprise 18 - 30 base pairs, more preferably from 19 - 25 base pairs, and most preferably from 19 -23 base pairs. Preferably the nucleotide is RNA.

When designing a unimolecular siRNA, specifically a left-handed unimolecular structure (*e.g*., 5'-AS-Loop-S) according to the present invention, preferably, the first 5' terminal sense nucleotide is defined as the nucleotide that is the 18^{th}, 19^{th} or 20^{th} base of the sense region counting from the base that is complementary to the first 5' terminal antisense nucleotide (*i.e*., counting from the 3' end of the sense region). The first 5' terminal sense nucleotide is defined in this manner because when unimolecular siRNAs that are capable of forming hairpins enter a cell, typically, Dicer will process hairpin siRNAs that contain lengthier duplex regions, into molecules that are comprised of two separate strands (siRNA) of approximately 18 -20 base pairs, and it is desirable for these molecules to retain the sense strand modifications associated with the 5' end of the sense strand of this processed molecule. Most preferably, the first 5' terminal sense nucleotide is defined as the nucleotide that is the 19^{th} base of the sense region from the 3' end of the sense region. Further, preferably, the polynucleotide is capable of forming a left-handed hairpin.

The hairpin may comprise a loop structure, which preferably comprises from four to ten bases, and a sense region, wherein the sense region and antisense region are independently 19-23 base pairs in length and substantially complementary to each other. Preferable sequences of the loop structure include, for example, 5'-UUCG (SEQ. ID NO. 323), 5'-UUUGUGUAG (SEQ. ID NO. 324), and 5'-CUUCCUGUCA (SEQ. ID NO. 325).

The hairpin is preferably constructed with the loop region downstream of the antisense region. This construction is desirable because it is easier to phosphorylate the terminal antisense nucleotide. Thus, when designing the unimolecular siRNA, it is preferable that there are no overhangs upstream of the 5' terminal antisense nucleotide. At the 3' end of the sense region in some embodiments, it may be desirable to have an overhang of, *e.g*., one, two or three nucleotides. Additionally, preferably the unimolecular siRNA is capable of forming a left handed hairpin.

The shRNA can further comprise a stem region, wherein the stem region comprises one or more nucleotides or modified nucleotides immediately adjacent to the 5' end and the 3' end of the loop structure, and wherein the one or more nucleotides or modified nucleotides of the stem region are (or are not) target-specific. Preferably, the entire length of the unimolecular siRNA contains fewer than 100 bases, more preferably fewer than 85 bases.

The unimolecular siRNA of the present invention may ultimately be processed by cellular machinery such that they are converted into two separate strands. Further, these unimolecular siRNA maybe introduced into the cell with less than all modifications, and modified in the cell itself through the use of natural processes or processing molecules that have been introduced (*e.g*., phosphorylation in the cell). However, preferably the siRNA is introduced with all modifications already present. (Similarly, the strands of the first embodiment are preferably introduced into the cell with all modifications, though the antisense strand could, e.g., be modified after introduction.)

In a further aspect, herein labelled "a fifteenth embodiment", the present invention is directed to a method for minimizing off-target effects, said method comprising exposing a modified siRNA to a target nucleic acid, or a cell or organism that is either expressing the target nucleic acid or capable of expressing the target nucleic acid, wherein said siRNA comprises an antisense strand and a sense strand, wherein:
(a) said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
(b) said antisense strand is comprised of a first 5' terminal antisense nucleotide and said first 5' terminal antisense nucleotide is phosphorylated at said first 5' terminal antisense nucleotide's 5' position.
The modified siRNAs of the fifteenth embodiment are preferably the siRNAs of the thirteenth embodiment.

In another aspect, herein labelled "a sixteenth embodiment", the present invention is directed to a method for minimizing off-target effects, said method comprising exposing a unimolecular siRNA capable of forming a hairpin to a target nucleic acid, or a cell or organism that is either expressing the target nucleic acid or capable of expressing the target nucleic acid, wherein said siRNA comprises an antisense region and a sense region, wherein:
(a) said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification;
(b) said antisense region is comprised of a first 5' terminal antisense nucleotide and said first 5' terminal antisense nucleotide is phosphorylated at said first 5' terminal antisense nucleotide's 5' carbon position; and
(c) a loop region, wherein said loop region is located between said sense region and said antisense region.
The modified siRNAs of the sixteenth embodiment are preferably the siRNA of the fourteenth embodiment

In other embodiments there can still be additional modifications such that the combination of the modifications can be described as follows: (1) positions 1 and 2 (or 1, 2, and 3) of the sense strand (or sense region in an shRNA), counting from the 5' end of that strand are modified with 2' modifying groups (carbon 2 on the ribose sugar); (2) positions 1 and 2 of the antisense strand (or antisense region in an shRNA), counting from the 5' end of that strand are modified with 2' modifying groups (carbon 2 on the ribose sugar); and (3) the 5' end of the antisense strand is phosphorylated (carbon 5 on the ribose sugar). A combination of all three types of modifications may be beneficial in substantially reducing both sense and antisense strand induced off-target effects.

In another aspect, herein labelled "a seventeenth embodiment", the present invention provides an siRNA comprising:
(a) an antisense strand, wherein said antisense strand is comprised of a first 5' terminal antisense nucleotide and a second 5' terminal antisense nucleotide, wherein said first 5' terminal, antisense nucleotide comprises a first 2' carbon antisense modification and said second 5' terminal antisense nucleotide comprises a second 2' carbon antisense modification and said first 5' terminal antisense nucleotide is phosphorylated at said first 5' terminal antisense nucleotide's 5' carbon position; and
(b) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification.

According to this seventeenth embodiment, the modifications are defined similarly to those of the thirteenth embodiment with the addition of 2' carbon modifications on the first 5' terminal antisense nucleotide and the second 5' terminal antisense nucleotide. Alternatively, the modifications are defined similarly to those of the thirteenth embodiment, with the addition of 2' carbon modifications on the second terminal antisense nucleotide. These 2' carbon modifications are defined in the same way as the modifications for the first 5' terminal sense nucleotide and the second 5' terminal sense nucleotide. Additionally, there may be a further 2' carbon modification on the third 5' terminal antisense nucleotide.

In a further aspect, herein labelled "an eighteenth embodiment", the present invention provides a unimolecular siRNA that is capable of forming a hairpin molecule. The siRNA is comprised of:
(a) an antisense region, wherein said antisense region is comprised of a first 5' terminal antisense nucleotide and a second 5' terminal antisense nucleotide, wherein said first 5' terminal antisense nucleotide comprises a first 2' carbon antisense modification and said second 5' terminal antisense nucleotide comprises a second 2' carbon antisense modification and said first 5' terminal antisense nucleotide is phosphorylated at said first 5' terminal antisense nucleotide's 5' carbon position;
(b) a sense region, wherein said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
(c) a loop region, wherein said loop region is located between said sense region and said antisense region.

According to this eighteenth embodiment, the modifications are defined similarly to those of the seventeenth embodiment with the addition of 2' carbon modifications on the first 5' terminal antisense nucleotide and the second 5' terminal antisense nucleotide. These 2' carbon modifications are defined in the same way as the modifications for the first 5' terminal sense nucleotide and the second 5' terminal sense nucleotide.

According to "a nineteenth embodiment", the present invention provides a method for reducing off-target effects during gene silencing. This method comprises administering the siRNA of the seventeenth embodiment to a target nucleic acid or to a cell or organism that is expressing or is capable of expressing the target nucleic acid.

According to "a twentieth embodiment", the present invention provides a method for reducing off-target effects during gene silencing. This method comprises administering the siRNA of the eighteenth embodiment to a target nucleic acid or to a cell or organism that is expressing or is capable of expressing the target nucleic acid.

Although the above-described embodiments are directed to increased specificity, it is important to note that siRNA may be modified with other types and combinations of modifications that may be designed to affect other parameters, such as those described above that affect stability.

However, some stability directed modifications render siRNA of limited functionality and should not be used except for example, as a control or exaequo agent. (*E.g*. a sense strand, wherein said sense strand is comprised of a first 5' terminal sense nucleotide and a second 5' terminal sense nucleotide, wherein said first 5' terminal sense nucleotide comprises a first 2'-O-alkyl (preferably methyl) sense modification and said second 5' terminal sense nucleotide comprises a second 2'-O-alkyl (preferably methyl) sense modification; at least one (preferably all) 2'-O-alkyl (preferably methyl) pyrimidine modified sense nucleotide(s), wherein said at least one 2'-O-alkyl (preferably methyl) pyrimidine modified sense nucleotide(s) is a nucleotide other than said first 5' terminal sense nucleotide or said second 5' terminal sense nucleotide; and an antisense strand, wherein said antisense strand is comprised of a first 5' terminal antisense nucleotide and a second 5' terminal antisense nucleotide, wherein said first 5' terminal antisense nucleotide comprises and a first 2'-O-alkyl (preferably methyl) antisense modification and said second 5' terminal sense nucleotide comprises a second 2'-O-alkyl (preferably methyl) antisense modification; and 2' labeled pyrimidines on the antisense strand, preferably, at least one 2'-O-alkyl (preferably methyl) pyrimidine modified antisense nucleotide, wherein said at least one (preferably all) 2'-O-alkyl (preferably methyl) modified antisense nucleotide is a nucleotide other than said first 5' terminal antisense nucleotide or said second 5' terminal antisense nucleotide, and optionally a label such as a fluorescent label on the 5' terminal sense nucleotide ).

Further, certain other related combinations of modifications can be used to develop siRNA that may be used as controls or exaequo agents. For example, an siRNA or shRNA may be developed similar to the seventeenth and eighteenth embodiments described in this disclosure, without the first 5' terminal antisense nucleotide's having been phosphorylated. Thus, under one embodiment there are 2' modifications that are limited to: (i) a first and second, or first, second and third, 5' terminal sense nucleotides that each comprises 2'modifications, such as the 2'modifications described above, for example 2'-O-methyl modifications; and (ii) a first and second, or first, second and third, 5' terminal antisense nucleotides that each comprises 2'modifications, such as the 2'modifications described above, for example 2'-O-methyl modifications.

When using exaequo agents or controls, it may be desirable to modify the 5' carbon position of the 5' end of the sense and/or the antisense strand with a blocking group. The blocking group may for example be an alkyl group or any other group that prevents phosphorylation of the 5' carbon position of the nucleotide. Phosphorylation may occur in a cell due to the activity of kinases that are present in cells. Exemplary blocking groups include but are not limited to methyl, O-methyl, and amine groups.

These molecules are not functional, but may be used in negative control studies, and as exaequo agents. Further, if a label such as the labels described above are used, these agents can be useful as tracking agents, which would assist in detection of transfection, as well as detection of where in the cell the molecule is present. Because these agents are not used for silencing, they can contain from 16 - 28 base pairs, though preferably contain 18 - 30 base pairs.

Additionally stabilization modifications that are addressed to the phosphate backbone may be included in the siRNAs for some applications of the present invention. For example, at least one phosphorothioate and/or methylphosphonate may be substituted for the phosphate group at some or all 3' positions of any or all pyrimidines in the sense and/or antisense strands of the oligonucleotide backbone, as well as in any overhangs, loop structures or stem structures that may be present Phosphorothioate (and methylphosphonate) analogues arise from modification of the phosphate groups in the oligonucleotide backbone. In the phosphorothioate, the phosphate O⁻ is replaced by a sulfur atom. In methylphosphonates, the oxygen is replaced with a methyl group. In one embodiment the phosphorothioate modification or methylphosphonate is located at the 3' positions of all antisense strand nucleotides that also contains 2' fluoro (or other halogen) modified nucleotides. Additionally, phosphorothioate 3' modifications may be used instead of and independent of 2' fluoro modifications to increase stability of an siRNA molecule. These modifications may be used in combination with the other modifications disclosed herein, or independent of those modifications in siRNA applications.

Nucleases typically use both the oxygen groups on the phosphate moiety and the 2'OH position of the ribose ring to mediate attack on RNA. Substitution of a sulfur group for one of the oxygens eliminates the ability of the phosphate to participate in this reaction, thus limiting the sensitivity of this site to nuclease digestion. However, it should be noted that phosphorothioates are typically toxic, thus, they would be beneficial primarily when any toxic effects are negated, which it is postulated might be accomplished by limiting the use of this modification to, *e.g*., every other nucleotide, every third nucleotide, or every fourth nucleotide.

Furthermore, for some applications it may be desirable to incorporate a label into the nucleotides of the present invention, *e.g*., a fluorescent label, a radioactive label or a mass label.

The above described modifications of the present invention may be combined with siRNA that contains sequences that were selected at random, or according to rationale design as described in, for example, U.S. patent Application Serial No. 10/714,333. Additionally, it may be desirable to select sequences in whole or in part based on internal thermal stability, which may facilitate processing by cellular machinery.

In developing the thirteenth through twentieth embodiments of the present invention, two or more different modifications were added to a duplex to minimize off-target effects. As noted above, Applicants appreciate that other modifications and combinations that assist in minimizing off-target effects may be discovered in the future. Additionally, the modifications of the present invention could be combined with modifications that are desired for other purposes. For example, in some instances, one modification could affect one particular step of off-target silencing (*e.g*. sense strand association with RISC) while a second modification could affect a completely different step *e.g*. altering the ability of sense/antisense strands to associate with targets that have less than 100% homology. Alternatively, two separate modifications could affect the same step. In some cases, two or more modifications could act additively or synergistically, limiting off-target effects by minimizing undesirable interactions or processes at one or more steps. In still other instances, one modification could eliminate off-target effects, but have detrimental consequences on more desirable properties e.g. the potency or stability of the siRNA. In cases such as these, additional modifications could be added that restore functionality of the molecule.

A variety of approaches can be used to identify both the type of molecule and the key position needed to eliminate sense and/or antisense strand off-targeting effects. In one non-limiting example, a modification-function walk is performed. In this procedure, a single type of modification is added to one or more nucleotides across the sense and/or antisense strand. Subsequently, modified and unmodified molecules are tested for: (1) functionality; and (2) off-targeting effects, by one of several methods. Thus, for example, 2'-O-Me groups can be added to positions 1 and 2, 3 and 4, 5 and 6, 7 and 8, 9 and 10, 11 and 12, 13 and 14,15 and 16, 17 and 18, or 18 and 19 of either the sense and/or antisense strand and tested for functionality (*e.g*., by measuring the ability of these molecules to silence specific targets) and off-target effects (*e.g*., by microarray analysis). If key positions are identified that eliminate some or all off-targeting, but result in a loss of duplex functionality, then a second round of modification walks, whereby additional chemical groups (*e.g*., 5' phosphate on the 5' end of the antisense strand), mismatches, or bulges that are suspected to increase duplex functionality can be added to molecules that already contain the modification that eliminates off-targeting.

It should be noted that the modifications of the thirteenth through twentieth embodiment of the present invention may have different effects depending on the functionality of the siRNA that are employed. Thus, in highly functional siRNA, the modifications of the present invention may cause a molecule to lose a certain amount of functionality, but would nonetheless be desirable because off-target effects are reduced. By contrast when moderately or poorly functional siRNA are used, there is very little functionality decrease and in some cases, functionality can increase.

Because the ability of the dsRNA of the present invention to retain functionality and to resist degradation is not dependent on the sequence of the bases, the cell type, or the species into which it is introduced, the present invention is applicable across a broad range of organisms, including but not limited plants, animals, protozoa, bacteria, viruses and fungi. The present invention is particularly advantageous for use in mammals such as cattle, horse, goats, pigs, sheep, canines, rodents such as hamsters, mice, and rats, and primates such as, gorillas, chimpanzees, and humans.

The present invention may be used advantageously with diverse cell types, including but not limited to primary cells, germ cell lines and somatic cells. The cells may be, for example, stem cells or differentiated cells. For example, the cell types may be embryonic cells, oocytes, sperm cells, adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes and cells of the endocrine or exocrine glands.

The present invention is applicable for use for employing RNA interference (and/or using as a control) directed against a broad range of genes, including but not limited to the 45,000 genes of a human genome, such as those implicated in diseases such as diabetes, Alzheimer's and cancer, as well as all genes in the genomes of the humans, mice, hamsters, chimpanzees, goats, sheep, horses, camels, pigs, dogs, cats, nematodes (*e.g*., *C. elegans*), flies (*e.g.*, *D. melanogaster*), and other vertebrates and invertebrates.

The siRNAs of the present invention may be administered to a cell by any method that is now known or that comes to be known and that from reading this disclosure, one skilled in the art would conclude would be useful with the present invention. For example, the siRNAs may be passively delivered to cells.

Passive uptake of modified siRNAs can be modulated, for example, by the presence of a conjugate such as a polyethylene glycol moiety or a cholesterol moiety at the 5' terminal of the sense strand and/or, in appropriate circumstances, a pharmaceutically acceptable carrier.

Other methods for delivery include, but are not limited to, transfection techniques employing DEAE-Dextran, calcium phosphate, cationic lipids/liposomes, microinjection, electroporation, immunoporation, and coupling of the siRNAs to specific conjugates or ligands such as antibodies, antigens, or receptors.

Preferably, the siRNAs comprise duplexes when they are administered.

Further, the method of assessing the level of gene silencing is not limited. Thus, the silencing ability of any given siRNA can be studied by one of any number of art tested procedures including but not limited to Northern analysis, Western Analysis, RT PCR, expression profiling, and others.

Further, the siRNA of the present invention may be used in a diverse set of applications, including but not limited to basic research, drug discovery and development, diagnostics and therapeutics. For example, the present invention may be used to validate whether a gene product is a target for drug discovery or development. In this application, the mRNA that corresponds to a target nucleic acid sequence of interest is identified for targeted degradation. Inventive siRNAs that are specific for targeting the particular gene are introduced into a cell or organism, preferably in duplex form. The cell or organism is maintained under conditions allowing for the degradation of the targeted mRNA, resulting in decreased activity or expression of the gene. The extent of any decreased expression or activity of the gene is then measured, along with the effect of such decreased expression or activity, and a determination is made that if expression or activity is decreased, then the nucleic acid sequence of interest is an agent for drug discovery or development. In this manner, phenotypically desirable effects can be associated with RNA interference of particular target nucleic acids of interest, and in appropriate cases toxicity and pharmacokinetic studies can be undertaken and therapeutic preparations developed.

The present invention may also be used in RNA interference applications that induce transient or permanent states of disease or disorder in an organism by, for example, attenuating the activity of a target nucleic acid of interest believed to be a cause or factor in the disease or disorder of interest. Increased activity of the target nucleic acid of interest may render the disease or disorder worse, or tend to ameliorate or to cure the disease or disorder of interest, as the case may be. Likewise, decreased activity of the target nucleic acid of interest may cause the disease or disorder, render it worse, or tend to ameliorate or cure it, as the case may be. Target nucleic acids of interest can comprise genomic or chromosomal nucleic acids or extrachromosomal nucleic acids, such as viral nucleic acids.

Further, the present invention may be used in RNA interference applications that determine the function of a target nucleic acid or target nucleic acid sequence of interest. For example, knockdown experiments that.reduce or eliminate the activity of a certain target nucleic acid of interest. This can be achieved by performing RNA, interference with one or more siRNAs targeting a particular target nucleic acid of interest. Observing the effects of such a knockdown can lead to inferences as to the function of the target nucleic acid of interest. RNA interference can also be used to examine the effects of polymorphisms, such as biallelic polymorphisms, by attenuating the activity of a target nucleic acid of interest having one or the other allele, and observing the effect on the organism or system studied. Therapeutically, one allele or the other, or both, may be selectively silenced using RNA interference where selective allele silencing is desirable.

Still further, the present invention may be used in RNA interference applications, such as diagnostics, prophylactics, and therapeutics including use of the compositions in the manufacture of a medicament in animals, preferably mammals, more preferably humans in the treatment of diseases, or over or under expression of a target. Preferably, the disease or disorder is one that arises from the malfunction of one or more proteins; the disease or disorder of which is related to the expression of the gene product of the one or more proteins. For example, it is widely recognized that certain cancers of the human breast are related to the malfunction of a protein expressed from a gene commonly known as the "*bcl-2*" gene. A medicament can be manufactured in accordance with the compositions and teachings of the present invention, employing one or more siRNAs directed against the *bcl-2* gene, and optionally combined with a pharmaceutically acceptable carrier, diluent and/or adjuvant, which medicament can be used for the treatment of breast cancer. Applicants have established the utility of the methods and compositions in cellular models. Methods of delivery of polynucleotides, such as siRNAs, to cells within animals, including humans, are well known in the art. Any delivery vehicle now known in the art, or that comes to be known, and has utility for introducing polynucleotides, such as siRNAs, to animals, including humans, is expected to be useful in the manufacture of a medicament in accordance with the present invention, so long as the delivery vehicle is not incompatible with any modifications that may be present a composition made according to the present invention. A delivery vehicle that is not compatible with a composition made according to the present invention is one that reduces the efficacy of the composition by greater than 95% as measured against efficacy in cell culture.

Animal models exist for many, many disorders, including, for example, cancers, diseases of the vascular system, inborn errors or metabolism, and the like. It is within ordinary skill in the art to administer nucleic acids to animals in dosing regimens to arrive at an optimal dosing regimen for particular disease or disorder in an animal such as a mammal, for example, a mouse, rat or non-human primate. Once efficacy is established in the mammal by routine experimentation by one of ordinary skill, dosing regimens for the commencement of human trials can be arrived at based on data arrived at in such studies.

Dosages of medicaments manufactured in accordance with the present invention may vary from micrograms per kilogram to hundreds of milligrams per kilogram of a subject. As is known in the art, dosage will vary according to the mass of the mammal receiving the dose, the nature of the mammal receiving the dose, the severity of the disease or disorder, and the stability of the medicament in the serum of the subject, among other factors well known to persons of ordinary skill in the art.

For these applications, an organism suspected of having a disease or disorder that is amenable to modulation by manipulation of a particular target nucleic acid of interest is treated by administering siRNA. Results of the siRNA treatment may be ameliorative, palliative, prophylactic, and/or diagnostic of a particular disease or disorder. Preferably, the siRNA is administered in a pharmaceutically acceptable manner with a pharmaceutically acceptable carrier or diluent.

Therapeutic applications of the present invention can be performed with a variety of therapeutic compositions and methods of administration. Pharmaceutically acceptable carriers and diluents are known to persons skilled in the art. Methods of administration to cells and organisms are also known to persons skilled in the art. Dosing regimens, for example, are known to depend on the severity and degree of responsiveness of the disease or disorder to be treated, with a course of treatment spanning from days to months, or until the desired effect on the disorder or disease state is achieved. Chronic administration of siRNAs may be required for lasting desired effects with some diseases or disorders. Suitable dosing regimens can be determined by, for example, administering varying amounts of one or more siRNAs in a pharmaceutically acceptable carrier or diluent, by a pharmaceutically acceptable delivery route, and amount of drug accumulated in the body of the recipient organism can be determined at various times following administration. Similarly, the desired effect (for example, degree of suppression of expression of a gene product or gene activity) can be measured at various times following administration of the siRNA, and this data can be correlated with other pharmacokinetic data, such as body or organ accumulation. Those of ordinary skill can determine optimum dosages, dosing regimens, and the like. Those of ordinary skill may employ EC₅₀ data from *in vivo* and *in vitro* animal models as guides for human studies.

Still further, the present invention may be used in RNA interference applications, such as diagnostics, prophylactics, and therapeutics. For these applications, an organism suspected of having a disease or disorder that is amenable to modulation by manipulation of a particular target nucleic acid of interest is treated by administering siRNA. Results of the siRNA treatment maybe ameliorative, palliative, prophylactic, and/or diagnostic of a particular disease or disorder. Preferably, the siRNA is administered in a pharmaceutically acceptable manner with a pharmaceutically acceptable carrier or diluent.

Further, the siRNAs can be administered in a cream or ointment topically, an oral preparation such as a capsule or tablet or suspension or solution, and the like. The route of administration may be intravenous, intramuscular, dermal, subdermal, cutaneous, subcutaneous, intranasal, oral, rectal, by eye drops, by tissue implantation of a device that releases the siRNA at an advantageous location, such as near an organ or tissue or cell type harboring a target nucleic acid of interest.

According to another embodiment related to the present invention there is provided an siRNA, comprising a sense strand and an antisense strand, wherein the sense strand and the antisense strand each comprises at least one orthoester modification at a 2' position.

According to another embodiment related to the present invention there is provided an siRNA, comprising a sense strand and an antisense strand, wherein said antisense strand comprises at least one orthoester modification and/or at least one modification selected from the group consisting of a 2'-halogen modification, a 2'-alkyl modification, a 2'-O-alkyl modification, a 2'-amine modification, and a 2'-deoxy modification.

According to another embodiment related to the present invention there is provided an siRNA, comprising a sense strand and an antisense strand, wherein the sense strand and/or the antisense strand comprises at least one orthoester modification, and wherein the sense strand and/or the antisense strand comprises at least one 2' modification selected from the group consisting of a 2'-halogen modification, a 2'-alkyl modification, a 2'-O-alkyl modification, a 2'-amine modification, and a 2'-deoxy modification.

In yet another embodiment related to the present invention there is provided an siRNA, comprising: (a) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and (b) an antisense strand, wherein said antisense strand is comprised of a first 5' terminal antisense nucleotide and a second 5' terminal antisense nucleotide, wherein said first 5' terminal antisense nucleotide comprises a first 2' carbon antisense modification and a 5' modification, and said second 5' terminal antisense nucleotide comprises a second 2' carbon antisense modification.

In yet another embodiment related to the present invention there is provided an siRNA, comprising a sense strand and an antisense strand, wherein the antisense strand comprises at least one 2'-orthoester modification, and wherein the antisense strand further modified with one or more modifications selected from the group consisting of a 2' orthoester modification, a 2'-alkyl modification, a 2'-halogen modification, a 2'-O-alkyl modification, a 2'-amine modification, and a 2'-deoxy modification, wherein the 2'-alkyl modification, the 2'-O-methyl modification, and the 2' halogen modification is on one or more pyrimidines of the antisense strand, and wherein the siRNA further comprises a 3' cap. The 2' carbon modification can be a 2'-O-alkyl modification such as, for example, a 2'-O-methyl modification. The first 5' terminal sense nucleotide and/or the first 5' terminal antisense nucleotide can be further modified with a 5' blocking group. The 5' blocking group can be selected from the group consisting of a 5'-methyl modification, a 5'-O-methyl modification, and a 5' azide modification.

In another embodiment related to the present invention the first and second 2'-O-alkyl modifications of the 5' terminal sense nucleotide and/or the 5' terminal antisense nucleotide is a 2'-O-methyl modification, and the first 5' terminal sense and/or antisense nucleotide can further comprise a 5' blocking group. The 5' blocking group is selected from the group consisting of a 5'-methyl modification, a 5'-O-methyl modification, or a 5' azide modification.

In other embodiments related to the invention there is provided siRNA, comprising an antisense strand and a sense strand, wherein the sense strand is comprised of a first 5' terminal sense nucleotide and a second 5' terminal sense nucleotide, wherein the first 5' terminal sense nucleotide comprises a first 2' carbon sense modification and the second 5' terminal sense nucleotide comprises a second 2' carbon sense modification.

In other embodiments related to the present invention there is provided an siRNA comprising (a) a sense strand, wherein the sense strand is comprised of a first 5' terminal sense nucleotide and a second 5' terminal sense nucleotide, wherein the first 5' terminal sense nucleotide comprises a first 2' carbon sense modification and the second 5' terminal sense nucleotide comprises a second 2' carbon sense modification; and (b) an antisense strand, wherein the antisense strand is comprised of a first 5' terminal antisense nucleotide and a second 5' terminal antisense nucleotide, wherein the first 5' terminal antisense nucleotide comprises a 5' phosphate modification and the second 5' terminal antisense nucleotide comprises a 2' carbon antisense modification.

In other embodiments, any of the compositions of the present invention can further comprise a 3' cap. The 3' cap can be, for example, an inverted deoxythymidine.

In other embodiments, the compositions of the present invention can comprise at least one of a 2'-deoxy modification and/or a methylphosphonate internucleotide linkage. The compositions of the present invention can have one or more modified internucleotide linkages, including, for example, one or more phosphorothioate links, phosphorodithioate links, methylphosphonate links, and combinations thereof.

In other embodiments of the present invention, any of the compositions can comprise a conjugate. The conjugate can be selected from the group consisting of amino acids, peptides, polypeptides, proteins, sugars, carbohydrates, lipids, polymers, nucleotides, polynucleotides, and combinations thereof. The conjugate can be cholesterol or PEG. The conjugate can further comprise a label, such as, for example, a fluorescent label. The fluorescent label can be selected from the group consisting of of TAMRA, BODIPY, Cy3, Cy5, fluoroscein, and Dabsyl. Alternatively, the fluorescent label can be any fluorescent label known in the art.

In other embodiments, the compositions of the present invention can comprise at least one 2'-orthoester modification, wherein the 2'-orthoester modification is preferably a 2'-bis(hydroxy ethyl) orthoester modification.

In other embodiments, any of the compositions of the present invention can be used in a method of performing RNA interference, comprising administering one or more of the compositions of the invention to a cell. Preferably, where an orthoester modification is present on an siRNA, the orthoester modification is a 2'-bis(hydroxy ethyl) orthoester.

Having described the invention with a degree of particularity, examples will now be provided. These examples are not intended to and should not be construed to limit the scope of the claims in any way. Although the invention may be more readily understood through reference to the following examples, they are provided by way of illustration and are not intended to limit the present invention unless specified.

### EXAMPLES

### Example 1

### Synthesizing siRNAs

RNA oligonucleotides were synthesized in a stepwise fashion using the nucleotide addition reaction cycle illustrated in **Figure 13****.** The synthesis is preferably carried out as an automated process on an appropriate machine. Several such synthesizing machines are known to those of skill in the art. Each nucleotide is added sequentially (3'- to 5'-direction) to a solid support-bound oligonucleotide. Although polystyrene supports are preferred, any suitable support can be used. The first nucleoside at the 3'-end of the chain is covalently attached to a solid support. The nucleotide precursor, an activated ribonucleodde such as a phosphoramidite or H-phosphonate, and an activator such as a tetrazole, for example, S-ethyl-tetrazole (although any other suitable activator can be used) are added (step **i** in **Figure 13**), coupling the second base onto the 5'-end of the first nucleoside. The support is washed and any unreacted 5'-hydroxyl groups are capped with an acetylating reagent such as but not limited to acetic anhydride or phenoxyacetic anhydride to yield unreactive 5'-acetyl moieties (step **ii**). The P(III) linkage is then oxidized to the more stable and ultimately desired P(V) linkage (step **iii**), using a suitable oxidizing agent such as, for example, t-butyl hydroperoxide or iodine and water. At the end of the nucleotide addition cycle, the 5'-silyl group is cleaved with fluoride ion (step **iv**), for example, using triethylammonium fluoride or *t*-butyl ammonium fluoride. The cycle is repeated for each subsequent nucleotide. It should be emphasized that although **Figure 13** illustrates a phosphoramidite having a methyl protecting group, any other suitable group may be used to protect or replace the oxygen of the phosphoramidite moiety. For example, alkyl groups, cyanoethyl groups, or thio derivatives can be employed at this position. Further, the incoming activated nucleoside in step (i) can be a different kind of activated nucleoside, for example, an H-phosphonate, methyl phosphonamidite or a thiophosphoramidite. It should be noted that the initial, or 3', nucleoside attached to the support can have a different 5' protecting group such as a dimethoxytrityl group, rather than a silyl group. Cleavage of the dimethoxytrityl group require acid hydrolysis, as employed in standard DNA synthesis chemistry. Thus, an acid such as dichloroacetic acid (DCA) or trichloroacetic acid (TCA) is employed for this step alone. Apart from the DCA cleavage step, the cycle is repeated as many times as necessary to synthesize the polynucleotide desired.

Following synthesis, the protecting groups on the phosphates, which are depicted as methyl groups in **Figure 13****,** but need not be limited to methyl groups, are cleaved in 30 minutes utilizing 1 M disodium-2-carbamoyl-2-cyanoethylene-1,1-dithiolate trihydrate (dithiolate) in DMF (dimethylformamide). The deprotection solution is washed from the solid support bound oligonucleotide using water. The support is then treated with 40% methylamine for 20 minutes at 55°C. This releases the RNA oligonucleotides into solution, deprotects the exocyclic amines and removes the acetyl protection on the 2'-ACE groups. The oligonucleotides can be analyzed by anion exchange HPLC at this stage.

The 2'-orthoester groups are the last protecting groups to be removed, if removal is desired. The structure of the 2'-ACE protected RNA immediately prior to 2'-deprotection is as represented in **Figure 14****.**

For automated procedures, solid supports having the initial nucleoside are installed in the synthesizing instrument. The instrument will contain all the necessary ancillary reagents and monomers needed for synthesis. Reagents are maintained under argon, since some monomers, if not maintained under an inert gas, can hydrolyze. The instrument is primed so as to fill all lines with reagent A synthesis cycle is designed that defines the delivery of the reagents in the proper order according to the synthesis cycle, delivering the reagents in the order specified in **Figure 13****.** Once a cycle is defined, the amount of each reagent to be added is defined, the time between steps is defined, and washing steps are defined, synthesis is ready to proceed once the solid support having the initial nucleoside is added.

For the RNA analogs described herein, modification is achieved through three different general methods. The first, which is implemented for carbohydrate and base modifications, as well as for introduction of certain linkers and conjugates, employs modified phosphoramidites in which the modification is pre-existing. An example of such a modification would be the carbohydrate 2'- modified species (2'-F, 2'-NH₂, 2'-O-alkyl, *etc.)* wherein the 2' orthoester is replaced with the desired modification 3' or 5' terminal modifications could also be introduced such as fluoroscein derivatives, Dabsyl, cholesterol, cyanine derivatives or polyethylene glycol. Certain internucleotide bond modifications would also be introduced via the incoming reactive nucleoside intermediate. Examples of the resultant internucleotide bond modification include but are not limited to methylphosphonates, phosphoramidates, phosphorothioates or phoshorodithioates.

Many modifiers can be employed using the same or similar cycles. Examples in this class would include, for example, 2-aminopurine, 5-methyl cytidine, 5-aminoallyl uridine, diaminopurine, 2-O-alkyl, multi-atom spacers, single monomer spacers, 2'-aminonucleosides, 2'-fluoro nucleosides, 5-iodouridine, 4-thiouridine, acridines, 5-bromouridine, 5-fluorocytidine, 5-fluorouridine, 5-iodouridine, 5-iodocytidine, 5-biotin-thymidine, 5-fluoroscein -thymidine, inosine, pseudouridine, abasic monomer, nebularane, deazanucleoside, pyrene nucleoside, azanucleoside, etc. Often the rest of the steps in the synthesis would remain the same with the exception of modifications that introduce substituents that are labile to standard deprotection conditions. Here modified conditions would be employed that do not effect the substituent. Second, certain internucleotide bond modifications require an alteration of the oxidation step to allow for their introduction. Examples in this class include phosphorothioates and phosphorodithioates wherein oxidation with elemental sulfur or another suitable sulfur transfer agent is required. Third, certain conjugates and modifications are introduced by "post-synthesis" process, wherein the desired molecule is added to the biopolymer after solid phase synthesis is complete. An example of this would be the addition of polyethylene glycol to a pre-synthesized oligonucleotide that contains a primary amine attached to a hydrocarbon linker. Attachment in this case can be achieved by using a N-hydroxy- succinimidyl ester of polyethylene glycol in a solution phase reaction.

While this outlines the most preferred method for synthesis of synthetic RNA, and its analogs, any nucleic acid synthesis method which is capable of assembling these molecules could be employed in their assembly. Examples of alternative methods include 5'-DMT-2'-TBDMS and 5'-DMT-2'-TOM synthesis approaches. Some 2'-O-methyl, 2'-F and backbone modifications can be introduced in transcription reactions using modified and wild type T7 and SP6 polymerases, for example.

### Synthesizing Modified RNA

The following guidelines are provided for synthesis of modified RNAs, and can readily be adapted to use on any of the automated synthesizers known in the art.

### 3' Terminal Modifications

There are several methods for incorporating 3' modifications. The 3' modification can be anchored or "loaded" onto a solid support of choice using methods known in the art. Alternatively, the 3' modification may be available as a phosphoramidite. The phosphoramidite is coupled to a universal support using standard synthesis methods where the universal support provides a hydroxyl at which the 3' terminal modification is created by introduction of the activated phosphoramidite of the desired terminal modification. Alternatively, the 3' modification could be introduced post synthetically after the polynucleotide is removed from the solid support. The free polynucleotide initially has a 3' terminal hydroxyl, amino; thiol, or halogen that reacts with an appropriately activated form of the modification of choice. Examples include but are not limited to N-hydroxy succinimidyl ester, thioether, disulfide, maliemido, or haloalkyl reactions. This modification now becomes the 3' terminus of the polynucleotides. Examples of modifications that can be conjugated post synthetically can be but are not limited to fluorosceins, acridines, TAMRA, dabsyl, cholesterol, polyethylene glycols, multi-atom spacers, cyanines, lipids, carbohydrates, fatty acids, steroids, peptides, or polypeptides.

### 5' Terminal Modifications

There are a number of ways to introduce a 5' modification into a polynucleotide. For example, a nucleoside having then 5' modification can be purchased and subsequently activated to a phosphoramidite. The phosphoramidite having the 5' modification may also be commercially available. Then, the activated nucleoside having the 5' modification is employed in the cycle just as any other activated nucleoside may be used. However, not all 5' modifications are available as phosphoramidites. In such an event, the 5' modification can be introduced in an analogous way to that described for 3' modifications above.

### Thioates

Polynucleotides having one or more thioate moieties, such as phosphorothioate linkages, were made in accordance with the synthesis cycle described above and illustrated in Figure 13. However, in place of the t-butyl hydroperoxide oxidation step, elemental sulfur or another sulfurizing agent was used.

### 5'-Thio Modifications

Monomers having 5' thiols can be purchased as phosphoramidites from commercial suppliers such as Glen Research. These 5' thiol modified monomers generally bear trityl protecting groups. Following synthesis, the trityl group can be removed by any method known in the art.

### Other Modifications

For certain modifications, the steps of the synthesis cycle will vary somewhat. For example, where the 3' end has an inverse dT (wherein the first base is attached to the solid support through the 5'-hydroxyl and the first coupling is a 3'-3' linkage) detritylation and coupling occurs more slowly, so extra detritylating reagent, such as dichloroactetic acid (DCA), should be used and coupling time should be increased to 300 seconds. Some 5' modifications may require extended coupling time. Examples include cholesterol, fluorophores such as Cy3 or Cy5 biotin, dabsyl, amino linkers, thio linkers, spacers, polyethylene glycol, phosphorylating reagent, BODIPY, or photocleavable linkers.

It should be noted that if a polynucleotide is to have only a single modification, that modification can be most efficiently carried out manually by removing the support having the partially built polynucleotide on it, manually coupling the monomer having the modification, and then replacing the support in the automated synthesizer and resuming automated synthesis.

### Example 2

### Deprotection and Cleavage of Synthesized Oligos from the Support

Cleaving can be done manually or in an automated process on a machine. Cleaving of the protecting moiety from the internucleotide linkage, for example a methyl group, can be achieved by using any suitable cleaving agent known in the art, for example, dithiolate or thiophenol. One molar dithiolate in DMF is added to the solid support at room temperature for 10 to 20 minutes. The support is then thoroughly washed with, for example, DMF, then water, then acetonitrile. Alternatively a water wash followed by a thorough acetonitrile will suffice to remove any residual dithioate.

Cleavage of the polynucleotide from the support and removal of exocyclic base protection can be done with 40% aqueous N-methylamine (NMA), followed by heating to 55 degrees Centigrade for twenty minutes. Once the polynucleotide is in solution, the NMA is carefully removed from the solid support. The solution containing the polynucleotide is then dried down to remove the NMA under vacuum. Further processing, including duplexing, desalting, gel purifying, quality control, and the like can be carried out by any method known in the art.

For some modifications, the NMA step may vary. For example, for a 3' amino modification, the treatment with NMA should be for forty minutes at 55 degrees Centigrade. Puromycin, 5' terminal amino linker modifications, and 2' amino nucleoside modifications are heated for 1 hour after addition of 40% NMA. Oligonucleotides modified with Cy5 are treated with ammonium hydroxide for 24 hours while protected from light.

### Preparation of Cleave Reagents

HPLC grade water and synthesis grade acetonitrile are used. The dithiolate is pre-prepared as crystals. Add 4.5 grams of dithiolate crystals to 90 mL of DMF. Forty percent NMA can be purchased, ready to use, from a supplier such as Sigma Aldrich Corporation.

### Annealing Single Stranded Polynucleotides

Single stranded polynucleotides can be annealed by any method known in the art, employing any suitable buffer. For example, equal amounts of each strand can be mixed in a suitable buffer, such as, for example, 50 mM HEPES pH 7.5,100 mM potassium chloride, 1 mM magnesium chloride. The mixture is heated for one minute at 90 degrees Centigrade, and allowed to cool to room temperature. In another example, each polynucleotide is separately prepared such that each is at 50 micromolar concentration. Thirty microliters of each polynucleotide solution is then added to a tube with 15 microliters of 5X annealing buffer, wherein the annealing buffer final concentration is 100 mM potassium chloride, 30 mM HEPES-KOH pH 7.4 and 2 mM magnesium chloride. Final volume is 75 microliters. The solution is then incubated for one minute at 90 degrees Centigrade, spun in a centrifuge for 15 seconds, and allowed to incubate at 37 degrees Centigrade for one hour, then allowed to come to room temperature. This solution can then be stored frozen at minus 20 degrees Centigrade and freeze thawed up to five times. The final concentration of the duplex is 20 micromolar. An example of a buffer suitable for storage of the polynucleotides is 20 mM KCl, 6 mM HEPES pH 7.5, 0.2 mM MgCl₂. All buffers used should be RNase free.

### Removal of the Orthoester Moiety

If desired, the orthoester moiety or moieties may be removed from the polynucleotide by any suitable method known in the art. One such method employs a volatile acetic acid-tetramethylenediamine (TEMED) pH 3.8 buffer system that can be removed by lyophilization following removal of the orthoester moiety or moieties. Deprotection at a pH higher than 3.0 helps minimize the potential for acid-catalyzed cleavage of the phosphodiester backbone. For example, deprotection can be achieved using 100 mM acetic acid adjusted to pH 3.8 with TEMED by suspending the orthoester protected polynucleotide and incubating it for 30 minutes at 60 degrees Centigrade. The solution is then lyophilized or subjected to a SpeedVac to dryness prior to use. If necessary, desalting following deprotection can be performed by any method known in the art, for example, ethanol precipitation or desalting on a reversed phase cartridge.

### Example 3

### siRNA Synthesized for Use in RNA Interference

The following is a list of 19-mer siRNAs having a di-dT overhang that were synthesized using Dharmacon, Inc.'s proprietary ACE chemistry, and were designed and used in accordance with the invention described herein. "SEAP" refers to human secreted alkaline phosphatase; "human cycle" refers to human cyclophilin; an asterisk between nucleotide units refers to a modified internucleotide linkage that is a phosphorothioate linkage; the structure 2'-F-C or 2'-F-U refers to a nucleotide unit having a fluorine atom attached to the 2' carbon of a ribosyl moiety; the structure 2'-N-C or 2'-N-U refers to a nucleotide unit having an -NH₂ group attached to the 2' carbon of a ribosyl moiety; the structure 2'-OME-C or 2'-OME-U refers to a nucleotide unit having a 2'-O-methyl modification at the 2' carbon of a ribosyl moiety of either Cs or Us, respectively; dG, dU, dA, dC, and dT refer to a nucleotide unit that is deoxy with respect to the 2' position, and instead has a hydrogen attached to the 2' carbon of the ribosyl moiety. Unless otherwise indicated, all nucleotide units in the list below are ribosyl with an -OH at the 2' carbon.

| **Table 1: SEAP Constructs** | | |
|---|---|---|
| **Identifier** | **Sequence** | **SEQ. ID NO.** |
| SP-2217-s | gugauguaugucagagagudtdt | 1 |
| SP-2217-as | acucucugacauacaucacdtdt | 2 |
| SP-2217-s-p | gugauguaugucagagagudtdt (ace on) | 3 |
| SP-2217-as-p | acucucugacauacaucacdtdt (ace on) | 4 |
| SP-2217-as4 | ac*u*cucugacauacau*c*acdtdt | 5 |
| SP-2217-as8 | ac*u*c*u*cugacauac*a*u*c*acdtdt | 6 |
| SP-2217-as8F | a2'-F-c*2'-F-u*2'-F-c*2'-F-u*2'-F- c2'-F-uga2'-F-ca2'-F-ua2'-F- c*a*2'-F-u*2'-F-c*a2'-F-cdtdt | 7 |
| SP-s-N | g2'-N-uga2'-N-ug2'-N-ua2'-N-ug2'- N-u2'-N-cagagag2'-N-udtdt | 8 |
| SP-as-N-12 | a2'-N-c2'-N-u2'-N-c2'-N-u2'-N-cug aca2'-N-ua2'-N-ca2'-N-u2'-N-ca2'- N-cdtdt | 9 |
| SP-s-thio | g*u*g*a*u*g*u*a*u*g*u*c*a*g*a*g*a* g*udtdt | 10 |
| SP-as-thio | a*c*u*c*u*c*u*g*a*c*a*u*a*c*a*u*c* a*cdtdt | 11 |
| SP-as-thio12 | a*c*u*c*u*c*ugacaua*c*a*u*c*a*cdtd t | 12 |
| SP-s-M | g2′-OMe-uga2′-OMe-ug2′-OMe-ua2′- OMe-ug2′-OMe-u2′-OMe-cagagag2′- OMe-udtdt | 13 |
| SP-as-M10 | a 2′-OMe-c 2′-OMe-u 2′-OMe-c 2′- OMe-u 2′-OMe-c u g a c a 2′-OMe-u a 2′-OMe-c a 2′-OMe-u 2′-OMe-c a 2′-OMe-c dt dt | 14 |
| SP-2217-s | dgudgadugduadugducdagdagdagdudtdt | 15 |
| SP-2217-as | adcudcudcugacauadcaducdacdtdt | 16 |
| SP-2217-sF | g2'-F-uga2'-F-ug2'-F-ua2'-F-ug2'- F-u2'-F-cagagag2'-F-udtdt | 17 |

| **Table 2: Human Cyclophylin Constructs** | | |
|---|---|---|
| **Identifier** | **Sequence** | **SEQ. ID NO.** |
| H-cyclo-476-s | ugguguuuggcaaaguucudtdt | 18 |
| H-cyclo-476-as | agaacuuugccaaacaccadtdt | 19 |
| H-cyc-F-s | (2'-F-u)gg(2'-F-u)g(2'-F- u)(2'-F-u)(2'-F-u)gg(2'-F- c)aaag(2'-F-u)(2'-F-u)(2'-F- c)(2'-F-u)dtdt | 20 |
| H-cyc-F-as9 | agaa(2'-F-c)(2'-F-u)(2'-F- u)(2'-F-u)g(2'-F-c)(2'-F- c)aaa(2'-F-c)a(2'-F-c)(2'-F- c)adtdt | 21 |
| H-cyc-F-as8 | agaa(2'-F-c)(2'-F-u)(2'-F- u)ug(2'-F-c)(2'-F-c)aaa(2'-F- c)a(2'-F-c)(2'-F-c).adtdt | 22 |
| H-cyclo-476-as6 | agaa(2'-F-c)(2'-F-u)(2'-F- u)ugccaaa(2'-F-c)a(2'-F- c)(2'-F-c)adtdt | 23 |
| H-cyclo-476-as1 | agaacuu(2'-Fu)gccaaacaccadtdt | 24 |

| **Table 3: Firefly Luciferase Constructs** | | |
|---|---|---|
| **Identifier** | **Sequence** | **SEQ. ID NO.** |
| Luc -1188-2'F-s | ga2'F-u2'F-ua2'F-ug2'F-u2'F- c2'F-cgg2'F-u2'F-ua2'F-ug2'F- uadtdt | 25 |
| Luc -1188-2'F-as | 2′F-ua2′F-ca2′F-uaa2′F-c2′F- cgga2′F-ca2′F-uaa2′F-u2′F- cdtdt | 26 |

### Example 4

### Performing RNA Interference

### Transfection

SiRNA duplexes were annealed using standard buffer (50 millimolar HEPES pH 7.5, 100 millimolar KCl, 1 mM MgCl₂). The transfections are done according to the standard protocol described below.

### Standard Transfection protocol for 96 well and 6 well plates: siRNAs

1. Protocols for 293 and Calu6, HeLa, MDA 75 are identical.
2. Cell are plated to be 95% confluent on the day of transfection.
3. SuperRNAsin (Ambion) is added to transfection mixture for protection against RNAses.
4. All solutions and handling have to be carried out in RNAse free conditions.
Plate 1 0.5 -1 ml in 25 ml of media in a small flask or 1 ml in 50 ml in a big flask.

### 96 well plate

1. Add 3 ml of 0.05 % trypsin-EDTA in a medium flask (6 in a big) incubate 5 min at 37 degrees C.
2. Add 7 ml (14 ml big) of regular media and pipet 10 times back and forth to resuspend cells.
3. Take 25 microliters of the cell suspension from step 2 and 75 microliters of trypan blue stain (1:4) and place 10 microliters in a cell counter.
4. Count number of cells in a standard hemocytometer.
5. Average number of cells x 4 x 10000 is number of cells per ml.
6. Dilute with regular media to have 350 000/ml.
7. Plate 100 microliters (35,000 cell for HEK293) in a 96 well plate.

### Transfection for 2 x 96 well plates (60 well format)

1. OPTI-MEM 2 ml + 80 microliters Lipofectamine 2000 (1:25) + 15 microliters of SuperRNAsin (AMBION).
2. Transfer siRNA aliquots (0.8 microliters of 100 micromolar to screen (total dilution factor is 1:750, 0.8 microliters of 100 micromolar solution will give 100 nanomolar final) to the deepdish in a desired order (Usually 3 columns x 6 for 60 well format or four columns by 8 for 96 well).
3. Transfer 100 microliters of OPTI-MEM.
4. Transfer 100 microliters of OPTI-MEM with Lipofectamine 2000 and SuperRNAsin to each well.
5. Leave for 20-30 min RT.
6. Add 0.55 ml of regular media to each well. Cover plate with film and mix.
7. Array out 100 x 3 x 2 directly to the cells (sufficient for two plates).

### Transfection for 2 x 6 well plates

8. 8 ml OPTI-MEM + 160 microliters Lipofectamine 2000 (1:25).30 microliters of SuperRNAsin (AMBION).
9. Transfer siRNA aliquots (total dilution factor is 1:750, 5 microliters of 100 micromolar solution will give 100 nanomolar final) to polystyrene tubes.
10. Transfer 1,300 microliters of OPTI-MEM with Lipofectamine 2000 and SuperRNAsin (AMNION).
11. Leave for 20-30 min RT.
12. Add 0.55 ml of regular media to each well. Cover plate with film and mix.
13. Transfer 2 ml to each well (sufficient for two wells).
The mRNA or protein levels are measured 24, 48, 72, and 96 hours post.transfection with standard kits or Custom B-DNA sets and Quantigene kits (Bayer).

### Example 5

### Measurement of Activity/Detection

The level of siRNA-induced RNA interference, or gene silencing, was estimated by assaying the reduction in target mRNA levels or reduction in the corresponding protein levels. Assays of mRNA levels were carried out using B-DNA™ technology (Quantagene Corp.). Protein levels for fLUC and rLUC were assayed by STEADY GLO™ kits (Promega Corp.). Human alkaline phosphatase levels were assayed by Great EscAPe SEAP Fluorescence Detection Kits (#K2043-1), BD Biosciences, Clontech.

### Example 6

### 2'-Deoxy Modifications/Firefly Luciferase Gene

The functional effect on an siRNA of having two tandem 2'-deoxy modifications, and three tandem 2'deoxy modifications in a sense strand and in an antisense strand were systematically examined by introducing the modifications into a 21-mer siRNA having a 19-mer region of complementarity and a di-dT overhang on the 3' ends of the duplex. The siRNAs were directed against the firefly luciferase gene (fLUC5) transfected into HEK293 cells. siRNA functionality was measured as described above. Toxicity was measured by ALMAR blue, and appeared unaffected. Functionality was assessed at three concentrations: 1,10 and 100 nM final. The sequences of the siRNAs used, and the placement of the 2'-deoxy modifications, are indicated in Table 4. The results of these experiments are shown in Figures 19-23.

| **Table 4: Constructs for 2'-Deoxy Modifications/fLUC** | | |
|---|---|---|
| **Identifier** | **Sequence** | **SEQ. ID NO.** |
| fLUC5-AS 3D19 | uuuaugaggaucucucdudgdadtdt | 27 |
| fLUC5-AS 3D16 | uuuaugaggaucucudcdudgadtdt | 28 |
| fLUC5-AS 3D13 | uuuaugaggaucdudcducugadtdt | 29 |
| fLUC5-AS 3D10 | uuuaugaggdadudcucucugadtdt | 30 |
| fLUC5-AS 3D7 | uuuaugdadgdgaucucucugadtdt | 31 |
| fLUC5-AS 3D4 | uuudadudgaggaucucucugadtdt | 32 |
| fLUC5-AS 3D1 | dududuaugaggaucucucugadtdt | 33 |
| fLUC5-AS 2D19 | uuuaugaggaucucucudgdadtdt | 34 |
| fLUC5-AS 2D17 | uuuaugaggaucucucdudgadtdt | 35 |
| fLUC5-AS 2D15 | uuuaugaggaucucdudcugadtdt | 36 |
| fLUC5-AS 2D13 | uuuaugaggaucdudcucugadtdt | 37 |
| fLUC5-AS 2D11 | uuuaugaggadudcucucugadtdt | 38 |
| fLUC5-AS 2D9 | uuuaugagdgdaucucucugadtdt | 39 |
| fLUC5-AS 2D7 | uuuaugdadggaucucucugadtdt | 40 |
| fLUC5-AS 2D5 | uuuadudgaggaucucucugadtdt | 41 |
| fLUC5-AS 2D3 | uududaugaggaucucucugadtdt | 42 |
| fLUC5-AS 2D1 | duduuaugaggaucucucugadtdt | 43 |
| fLUC5-AS 1D19 | uuuaugaggaucucucugdadtdt | 44 |
| fLUC5-AS 1D18 | uuuaugaggaucucucudgadtdt | 45 |
| fLUC5-AS 1D17 | uuuaugaggaucucucdugadtdt | 46 |
| fLUC5-AS 1D16 | uuuaugaggaucucudcugadtdt | 47 |
| fLUC5-AS 1D15 | uuuaugaggaucucducugadtdt | 48 |
| fLUC5-AS 1D14 | uuuaugaggaucudcucugadtdt | 49 |
| fLUC5-AS 1D13 | uuuaugaggaucducucugadtdt | 50 |
| fLUC5-AS 1D12 | uuuaugaggaudcucucugadtdt | 51 |
| fLUC5-AS 1D11 | uuuaugaggaducucucugadtdt | 52 |
| fLUC5-AS 1D10 | uuuaugaggdaucucucugadtdt | 53 |
| fLUC5-AS 1D9 | uuuaugagdgaucucucugadtdt | 54 |
| fLUC5-AS 1D8 | uuuaugadggaucucucugadtdt | 55 |
| fLUC5-AS 1D7 | uuuaugdaggaucucucugadtdt | 56 |
| fLUC5-AS 1D6 | uuuaudgaggaucucucugadtdt | 57 |
| fLUC5-AS 1D5 | uuuadugaggaucucucugadtdt | 58 |
| fLUC5-AS 1D4 | uuudaugaggaucucucugadtdt | 59 |
| fLUC5-AS 1D3 | uuduaugaggaucucucugadtdt | 60 |
| fLUC5-AS 1D2 | uduuaugaggaucucucugadtdt | 61 |
| fLUC5-AS 1D1 | duuuaugaggaucucucugadtdt | 62 |
| fLUC5-S 3D19 | ucagagagauccucaudadadadtdt | 63 |
| fLUC5-S 3D16 | ucagagagauccucadudadaadtdt | 64 |
| fLUC5-S 3D13 | ucagagagauccdudcdauaaadtdt | 65 |
| fLUC5-S 3D10 | ucagagagadudcdcucauaaadtdt | 66 |
| fLUC5-S 3D7 | ucagagdadgdauccucauaaadtdt | 67 |
| fLUC5-S 3D4 | ucadgdadgagauccucauaaadtdt | 68 |
| fLUC5-S 3D1 | dudcdagagagauccucauaaadtdt | 69 |
| fLUC5-S 2D19 | ucagagagauccucauadadadtdt | 70 |
| fLUC5-S 2D17 | ucagagagauccucaudadaadtdt | 71 |
| fLUC5-S 2D15 | ucagagagauccucdaduaaadtdt | 72 |
| fLUC5-S 2D13 | ucagagagauccdudcauaaadtdt | 73 |
| fLUC5-S 2D11 | ucagagagaudcdcucauaaadtdt | 74 |
| fLUC5-S 2D9 | ucagagagdaduccucauaaadtdt | 75 |
| fLUC5-S 2D7 | ucagagdadgauccucauaaadtdt | 76 |
| fLUC5-S 2D5 | ucagdadgagauccucauaaadtdt | 77 |
| fLUC5-S 2D3 | ucdadgagagauccucauaaadtdt | 78 |
| fLUC5-S 2D1 | dudcagagagauccucauaaadtdt | 79 |
| fLUC5-S 1D19 | ucagagagauccucauaadadtdt | 80 |
| fLUC5-S 1D18 | ucagagagauccucauadaadtdt | 81 |
| fLUC5-S 1D17 | ucagagagauccucaudaaadtdt | 82 |
| fLUC5-S 1D16 | ucagagagauccucaduaaadtdt | 83 |
| fLUC5-S 1D15 | ucagagagauccucdauaaadtdt | 84 |
| fLUC5-S 1D14 | ucagagagauccudcauaaadtdt | 85 |
| fLUC5-S 1D13 | ucagagagauccducauaaadtdt | 86 |
| fLUC5-S 1D12 | ucagagagaucdcucauaaadtdt | 87 |
| fLUC5-S 1D11 | ucagagagaudccucauaaadtdt | 88 |
| fLUC5-S 1D10 | ucagagagaduccucauaaadtdt | 89 |
| fLUC5-S 1D9 | ucagagagdauccucauaaadtdt | 90 |
| fLUC5-S 1D8 | ucagagadgauccucauaaadtdt | 91 |
| fLUC5-S 1D7 | ucagagdagauccucauaaadtdt | 92 |
| fLUC5-S 1D6 | ucagadgagauccucauaaadtdt | 93 |
| fLUC5-S 1D5 | ucagdagagauccucauaaadtdt | 94 |
| fLUC5-S 1D4 | ucadgagagauccucauaaadtdt | 95 |
| fLUC5-S 1D3 | ucdagagagauccucauaaadtdt | 96 |
| fLUC5-S 1D2 | udcagagagauccucauaaadtdt | 97 |
| fLUC5-S 1D1 | ducagagagauccucauaaadtdt | 98 |
| A "d" indicates that the nucleotide following the "d" is deoxy at the 2'. position. | | |

### Example 7

### 2'-O-Methyl Modifications/Firefly Luciferase Gene

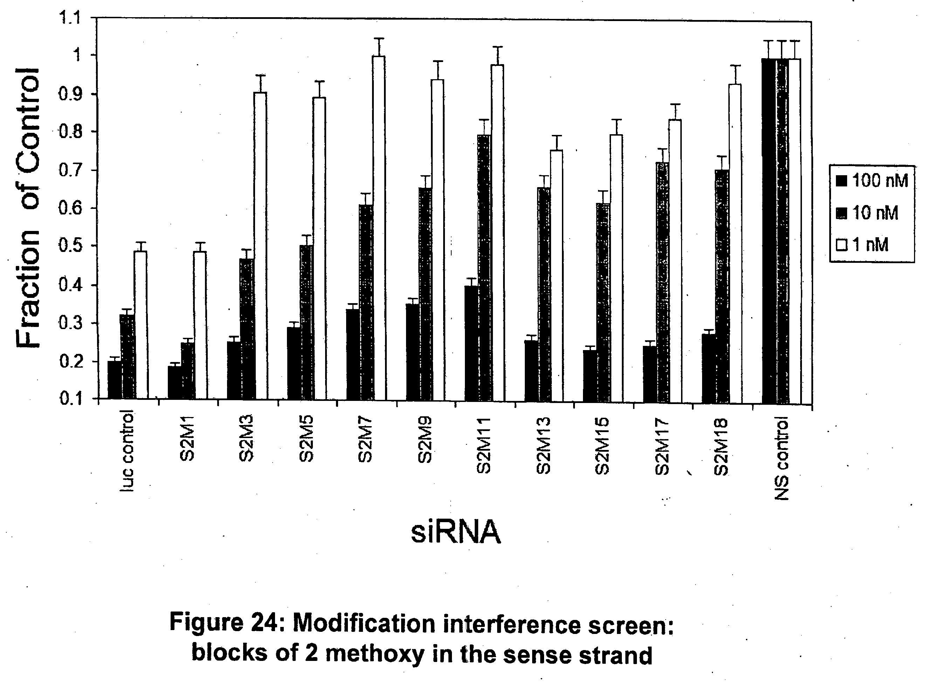

The functional effect on an siRNA of having a single 2'-O-methyl modification, two tandem 2'-O-methyl modifications, and three tandem 2'-O-methyl modifications in a sense strand and in an antisense strand were systematically examined by introducing the modifications into a 21-mer siRNA having a 19-mer region of complementarity and a di-dT overhang on the 3' ends of the duplex. The siRNAs were directed against the firefly luciferase gene (fLUC5) transfected into HEK293 cells. siRNA functionality was measured as described above. Functionality was assessed at three concentrations 1,10 and 100 nM final. Toxicity was measured by ALMAR blue, and appeared unaffected. The sequences of the siRNAs used, and the placement of the 2'-O-methyl modifications, are indicated in Table 5. The results of these experiments are shown in **Figures 24-28****.**

| **Table 5: Constructs for 2'-O-Methyl Modifications/fLUC** | | |
|---|---|---|
| **Identifier** | **Sequence** | **SEQ. ID NO.** |
| fLUC5-AS 3M19 | uuuaugaggaucucucmumgmadtdt | 99 |
| fLUC5-AS 3M16 | uuuaugaggaucucumcmumgadtdt | 100 |
| fLUC5-AS 3M13 | uuuaugaggaucmumcmucugadtdt | 101 |
| fLUC5-AS 3M10 | uuuaugaggmamumcucucugadtdt | 102 |
| fLUC5-AS 3M7 | uuuaugmamgmgaucucucugadtdt | 103 |
| fLUC5-AS 3M4 | uuumamumgaggaucucucugadtdt | 104 |
| fLUC5-AS 3M1 | mumumuaugaggaucucucugadtdt | 105 |
| fLUC5-AS 2M19 | uuuaugaggaucucucumgmadtdt | 106 |
| fLUC5-AS 2M17 | uuuaugaggaucucucmumgadtdt | 107 |
| fLUCS-AS 2M15 | uuuaugaggaucucmumcugadtdt | 108 |
| fLUC5-AS 2M13 | uuuaugaggaucmumcucugadtdt | 109 |
| fLUC5-AS 2M11 | uuuaugaggamumcucucugadtdt | 110 |
| fLUC5-AS 2M9 | uuuaugagmgmaucucucugadtdt | 111 |
| fLUC5-AS 2M7 | uuuaugmamggaucucucugadtdt | 112 |
| fLUC5-AS 2M5 | uuuamumgaggaucucucugadtdt | 113 |
| fLUC5-AS 2M3 | uumumaugaggaucucucugadtdt | 114 |
| fLUC5-AS 2M1 | mumuuaugaggaucucucugadtdt | 115 |
| fLUC5-AS 1M19 | uuuaugaggaucucucugmadtdt | 116 |
| fLUC5-AS 1M18 | uuuaugaggaucucucumgadtdt | 117 |
| fLUC5-AS 1M17 | uuuaugaggaucucucmugadtdt | 118 |
| fLUC5-AS 1M16 | uuuaugaggaucucumcugadtdt | 119 |
| fLUC5-AS 1M15 | uuuaugaggaucucmucugadtdt | 120 |
| fLUC5-AS 1M14 | uuuaugaggaucumcucugadtdt | 121 |
| fLUCS-AS 1M13 | uuuaugaggaucmucucugadtdt | 122 |
| fLUC5-AS 1M12 | uuuaugaggaumcucucugadtdt | 123 |
| fLUC5-AS 1M11 | uuuaugaggamucucucugadtdt | 124 |
| fLUG5-AS 1M10 | uuuaugaggmaucucucugadtdt | 125 |
| fLUC5-AS 1M9 | uuuaugagmgaucucucugadtdt | 126 |
| fLUC5-AS 1M8 | uuuaugamggaucucucugadtdt | 127 |
| fLUC5-AS 1M7 | uuuaugmaggaucucucugadtdt | 128 |
| fLUC5-AS 1M6 | uuuaumgaggaucucucugadtdt | 129 |
| fLUC5-AS 1M5 | uuuamugaggaucucucugadtdt | 130 |
| fLUC5-AS 1M4 | uuumaugaggaucucucugadtdt | 131 |
| fLUC5-AS 1M3 | uumuaugaggaucucucugadtdt | 132 |
| fLUC5-AS 1M2 | umuuaugaggaucucucugadtdt | 133 |
| fLUC5-AS 1M1 | muuuaugaggaucucucugadtdt | 134 |
| fLUC5-S 3M19 | ucagagagauccucaumamamadtdt | 135 |
| fLUC5-S 3M16 | ucagagagauccucamumamaadtdt | 136 |
| fLUC5-S 3M13 | ucagagagauccmumcmauaaadtdt | 137 |
| fLUC5-S 3M10 | ucagagagamumcmcucauaaadtdt | 138 |
| fLUC5-S 3M7 | ucagagmamgmauccucauaaadtdt | 139 |
| fLUC5-S 3M4 | ucamgmamgagauccucauaaadtdt | 140 |
| fLUC5-S 3M1 | mumcmagagagauccucauaaadtdt | 141 |
| fLUC5-S 2M19 | ucagagagauccucauamamadtdt | 142 |
| fLUC5-S 2M17 | ucagagagauccucamumaaadtdt | 143 |
| fLUC5-S 2M15 | ucagagagauccumcmauaaadtdt | 144 |
| fLUC5-S 2M13 | ucagagagaucmcmucauaaadtdt | 145 |
| fLUC5-S 2M11 | ucagagagamumccucauaaadtdt | 146 |
| fLUC5-S 2M9 | ucagagamgmauccucauaaadtdt | 147 |
| fLOC5-S 2M7 | ucagamgmagauccucauaaadtdt | 148 |
| fLUC5-S 2M5 | ucagmamgagauccucauaaadtdt | 149 |
| fLUC5-s 2M3 | ucmamgagagauccucauaaadtdt | 150 |
| fLUC5-S 2M1 | mumcagagagauccucauaaadtdt | 151 |
| fLUC5-S 1M19 | ucagagagauccucauaamadtdt | 152 |
| fLUC5-S 1M18 | ucagagagauccucauamaadtdt | 153 |
| fLUC5-S 1M17 | ucagagagauccucaumaaadtdt | 154 |
| fLUC5-S 1M16 | ucagagagauccucamuaaadtdt | 155 |
| fLUC5-S 1M15 | ucagagagauccucmauaaadtdt | 156 |
| fLUC5-S 1M14 | ucagagagauccumcauaaadtdt | 157 |
| fLUC5-S 1M13 | ucagagagauccmucauaaadtdt | 158 |
| fLUC5-S 1M12 | ucagagagaucmcucauaaadtdt | 159 |
| fLUC5-S 1M11 | ucagagagaumccucauaaadtdt | 160 |
| fLUC5-S 1M10 | ucagagagamuccucauaaadtdt | 161 |
| fLUC5-S 1M9 | ucagagagmauccucauaaadtdt | 162 |
| fLUC5-S 1M8 | ucagagamgauccucauaaadtdt | 163 |
| fLUC5-S 1M7 | ucagagmagauccucauaaadtdt | 164 |
| fLUC5-S 1M6 | ucagamgagauccucauaaadtdt | 165 |
| fLUC5-S 1M5 | ucagmagagauccucauaaadtdt | 166 |
| fLUC5-S 1M4 | ucamgagagauccucauaaadtdt | 167 |
| fLUC5-S 1M3 | ucmagagagauccucauaaadtdt | 168 |
| fLUC5-S 1M2 | umcagagagauccucauaaadtdt | 169 |
| fLUC5-S 1M1 | mucagagagauccucauaaadtdt | 170 |
| The letter "m" indicates that the nucleotide following the "m" is modified with a 2'-O-methyl moiety. | | |

### Example 8

### 2'-Deoxy and 2'-O-Methyl Modifications/Sense vs. Antisense Strands

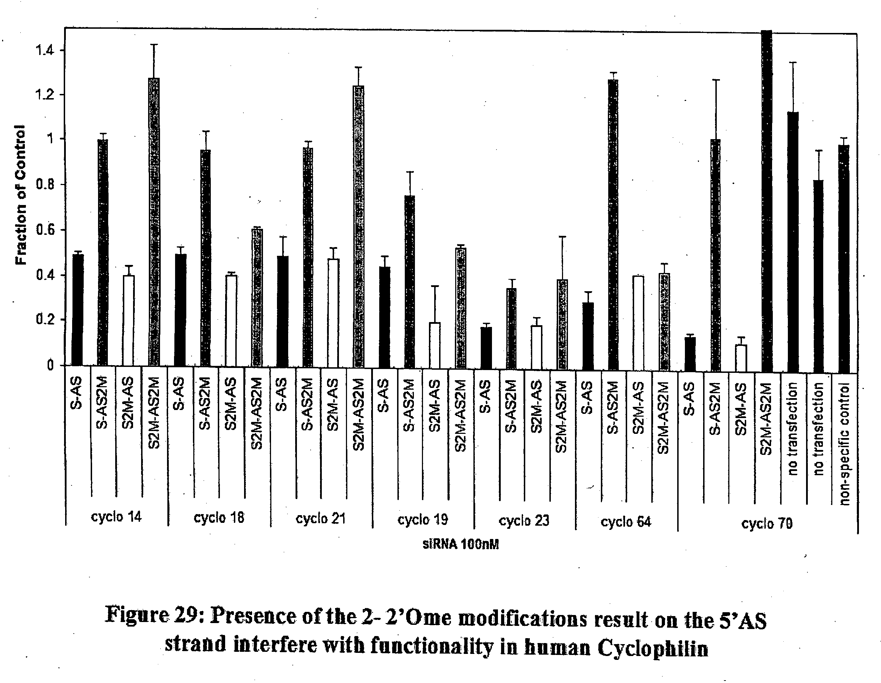

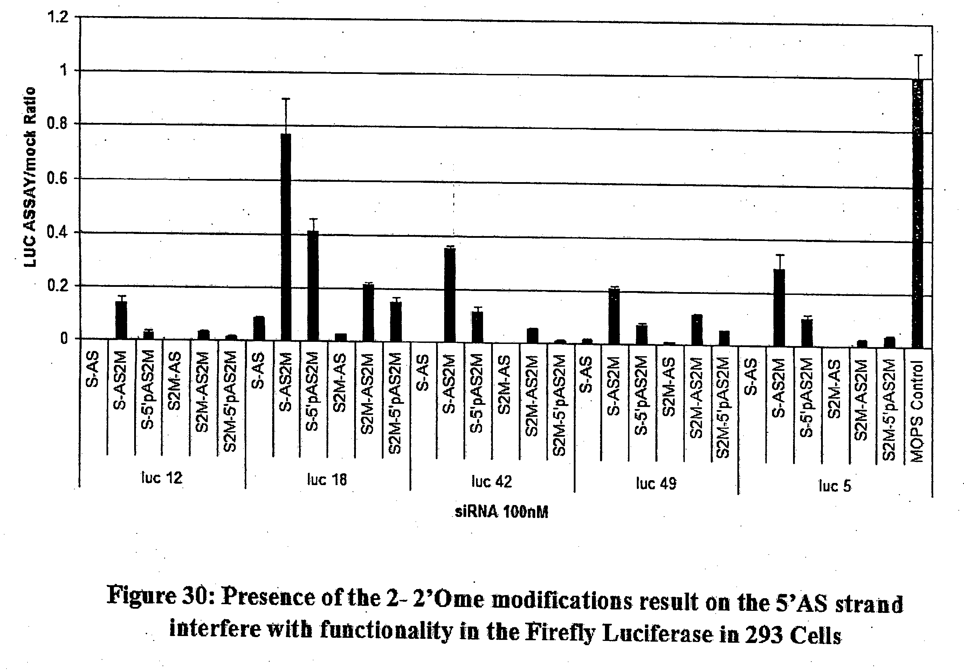

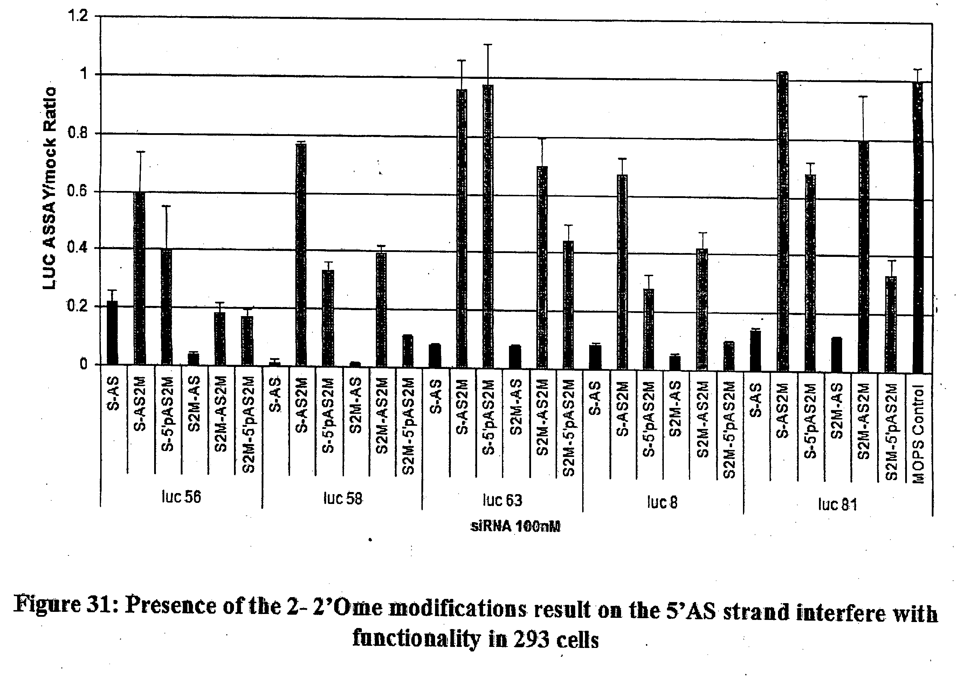

Fifteen duplexes, five directed against the human cyclophylin gene, and 10 were directed against the firefly luciferase gene were tested with various modifications (see **Figures 29-31**). For human cyclophilin B, duplexes tested included: (1) unmodified, (2) 2'-O-methyl modifications at the first and second positions of the sense strand, and (3) 2'-O-methyl modifications at the first and second positions of the antisense strand, and (4) 2'-O-methyl modifications on the sense and antisense strands. For luciferase, all the modifications described for human cyclophilin B plus: (1) 2'-O-methyl modifications on the AS strand in conjunction with 5' phosphorylation of the AS strand, and (2) 2'-O-methyl modification of the sense and antisense strands in conjunction with 5' phosphorylation of the antisense strand, were tested. For all 15 duplexes, modifications at positions 1 and 2 of the sense strand with 2'0-methyl moieties did not interfere with functionality. The same modifications of the antisense strand limited the functionality of the duplexes. This decrease in functionality was partially reduced where the antisense strand was phosphorylated at its 5' end (see **Figures 30** and **31**). Taking the above data together, 2'-O-methylaltion of positions 1 and 2 of the sense strand in combination with 5' phosphorylation of the antisense strand is an inexpensive, reliable, and non-toxic method of modifying an siRNA duplex to limit sense strand off-targeting without altering duplex functionality. This information is of commercial value because it helps increase siRNAs specificity and potency. Recent microarray data indicates that the presence of just 11 nucleotides is sufficient to induce nonspecific silencing. The homology present within a sense strand of an siRNA duplex typically constitutes at least half nonspecific functionality. If the inherent nonspecific functionality is blocked, the sense strand will not be able to contribute to off-targeting and the siRNA's specificity should increase. Shifling of the equilibrium toward a functional RISC-antisense strand complex will also lower the effective concentration of siRNA.

### Example 9

### Modified siRNAs with 5' Conjugates

The effects of various modifications on duplex stability, functionality, and passive uptake were assessed. **Figure 33** demonstrates the effects of cholesterol conjugation and (2'O-methyl modification on positions 1 and 2 of the sense and antisense strand plus 2'-O-methyl modification of all Cs and Us of the sense strand, plus 2' F modification of all Cs and Us of the antisense strand, plus 5' phosphorylation of the antisense strand) on duplex stability. Naked or modified siRNA were ³²P-labeled (AS strand, T4 Kinase, Promega), incubated with serum (or serum albumin), and run in gel shift assays (denaturing, 15% PAGE). Results of these studies show that while unmodified duplexes are quickly degraded, siRNA carrying cholesterol and the before described modifications are stable in the presence of serum or serum albumin. Similarly, siRNA carrying 2'-O-methyl modification of Cs and Us (sense) in combination with 3' idT capping and CHO (5' sense) conjugation, also exhibit enhanced stability (**Figure 35**).

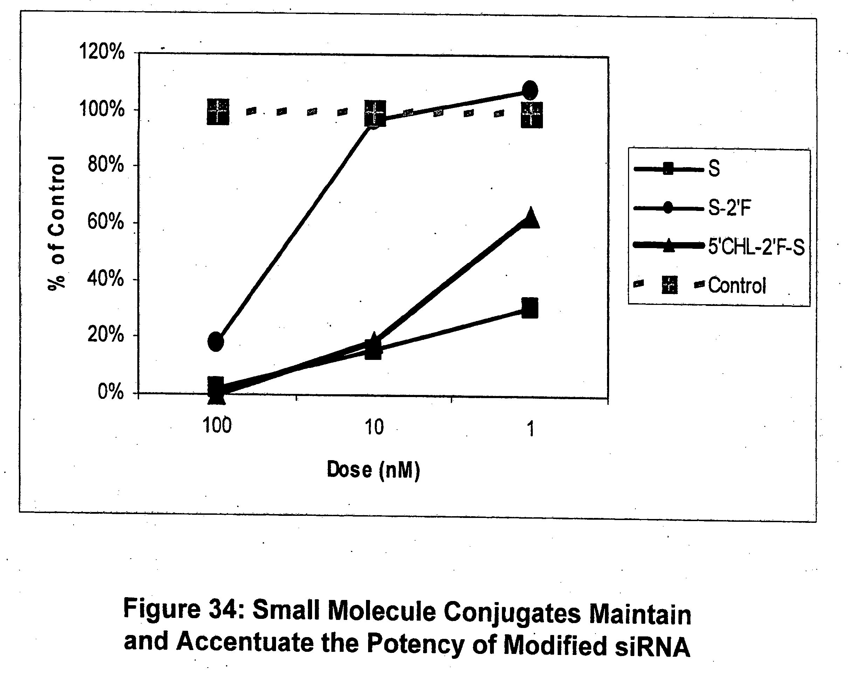

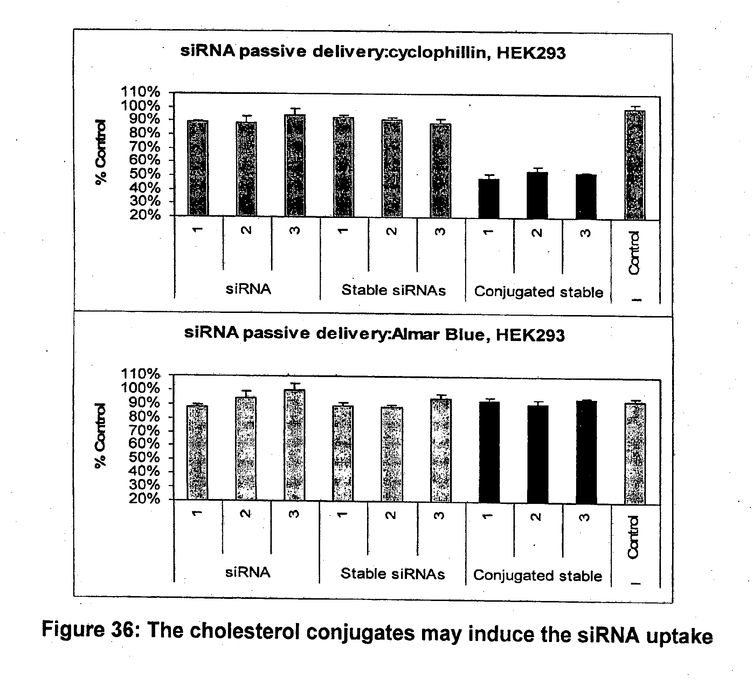

Studies on similarly modified molecules (*e.g*., cholesterol modification of the 5' end of the sense strand in conjugation with 2' F1 modification of the sense strand) reveal that such modifications can accentuate functionality (**Figure 34**) and passive uptake (**Figure 36**, modification = 2' F on Cs and Us of both the sense and antisense with cholesterol).

### Example 10

### 2'Deoxy and 2'-O-Methyl Modification Walks on SEAP 2217 Target

The constructs used for the 2'-deoxy and 2'-O-ethyl walks using siRNAs targeted against the SEAP construct (see **Figures 31** and **32**) are listed in Table 6.

| **Table 6** | | |
|---|---|---|
| **Constructs for 2'-Deoxy and 2'-O-Methyl Walks** | | |
| **Identifier** | **Sequence** | **SEQ ID NO.** |
| 2217-S 2M1 | mgmugauguaugucagagagudtdt | 171 |
| 2217-AS 3D19 | acucucugacauacaudcdadcdtdt | 172 |
| 2217-AS 3D16 | acucucugacauacadudcdacdtdt | 173 |
| 2217-AS 3D13 | acucucugacaudadcdaucacdtdt | 174 |
| 2217-AS 3D10 | acucucugadcdaduacaucacdtdt | 175 |
| 2217-AS 3D7 | acucucdudgdacauacaucacdtdt | 176 |
| 2217-AS 3D4 | acudcdudcugacauacaucacdtdt | 177 |
| 2217-AS 3D1 | dadcducucugacauacaucacdtdt | 178 |
| 2217-AS 2D19 | acucucugacauacaucdadcdtdt | 179 |
| 2217-AS 2D17 | acucucugacauacaudcdacdtdt | 180 |
| 2217-AS 2D15 | acucucugacauacdaducacdtdt | 181 |
| 2217-AS 2D13 | acucucugacaudadcaucacdtdt | 182 |
| 2217-AS 2D11 | acucucugacdaduacaucacdtdt | 183 |
| 2217-AS 2D9 | acucucugdadcauacaucacdtdt | 184 |
| 2217-AS 2D7 | acucucdudgacauacaucacdtdt | 185 |
| 2217-AS 2D5 | acucdudcugacauacaucacdtdt | 186 |
| 2217-AS 2D3 | acdudcucugacauacaucacdtdt | 187 |
| 2217-AS 2D1 | dadcucucugacauacaucacdtdt | 188 |
| 2217-AS 3M19 | acucucugacauacaumcmamcdtdt | 189 |
| 2217-AS 3M16 | acucucugacauacamumcmacdtdt | 190 |
| 2217-AS 3M13 | acucucugacaumamcmaucacdtdt | 191 |
| 2217-AS 3M10 | acucucugamcmamuacaucacdtdt | 192 |
| 2217-AS 3M7 | acucucmumgmacauacaucacdtdt | 193 |
| 2217-AS 3M4 | acumcmumcugacauacaucacdtdt | 194 |
| 2217-AS 3M1 | mamcmucucugacauacaucacdtdt | 195 |
| 2217-AS 2M19 | acucucugacauacaucmamcdtdt | 196 |
| 2217-AS 2M17 | acucucugacauacaumcmacdtdt | 197 |
| 2217-AS 2M15 | acucucugacauacmamucacdtdt | 198 |
| 2217-AS 2M13 | acucucugacaumamcaucacdtdt | 199 |
| 2217-AS 2M11 | acucucugacmamuacaucacdtdt | 200 |
| 2217-AS 2M9 | acucucugmamcauacaucacdtdt | 201 |
| 2217-AS 2M7 | acucucmumgacauacaucacdtdt | 202 |
| 2217-AS 2M5 | acucmumcugacauacaucacdtdt | 203 |
| 2217-AS 2M3 | acmumcucugacauacaucacdtdt | 204 |
| 2217-AS 2M1 | mamcucucugacauacaucacdtdt | 205 |
| 2217-S 3D19 | gugauguaugucagagdadgdudtdt | 206 |
| 2217-S 3D16 | gugauguaugucagadgdadgudtdt | 207 |
| 2217-S 3D13 | gugauguaugucdadgdagagudtdt | 208 |
| 2217-S 3D10 | gugauguaudgdudcagagagudtdt | 209 |
| 2217-S 3D7 | gugaugdudadugucagagagudtdt | 210 |
| 2217-S 3D4 | gugdadudguaugucagagagudtdt | 211 |
| 2217-S 3D1 | dgdudgauguaugucagagagudtdt | 212 |
| 2217-S 2D19 | gugauguaugucagagadgdudtdt | 213 |
| 2217-S 2017 | gugauguaugucagagdadgudtdt | 214 |
| 2217-S 2D15 | gugauguaugucagdadgagudtdt | 215 |
| 2217-S 2D13 | gugauguaugucdadgagagudtdt | 216 |
| 2217-S 2D11 | gugauguaugdudcagagagudtdt | 217 |
| 2217-S 2D9 | gugauguadudgucagagagudtdt | 218 |
| 2217-S 2D7 | gugaugdudaugucagagagudtdt | 219 |
| 2217-S 2D5 | gugadudguaugucagagagudtdt | 220 |
| 2217-S 2D3 | gudgdauguaugucagagagudtdt | 221 |
| 2217-S 2D1 | dgdugauguaugucagagagudtdt | 222 |
| 2217-S 3M19 | gugauguaugucagagmamgmudtdt | 223 |
| 2217-S 3M16 | gugauguaugucagamgmamgudtdt | 224 |
| 2217-S 3M13 | gugauguaugucmamgmagagudtdt | 225 |
| 2217-S 3M10 | gugauguaumgmumcagagagudtdt | 226 |
| 2217-S 3M7 | gugaugmumamugucagagagudtdt | 227 |
| 2217-S 3M4 | gugmamumguaugucagagagudtdt | 228 |
| 2217-S 3M1 | mgmumgauguaugucagagagudtdt | 229 |
| 2217-S 2M19 | gugauguaugucagagamgmudtdt | 230 |
| 2217-S 2M17 | gugauguaugucagagmamgudtdt | 231 |
| 2217-S 2M15 | gugauguaugucagmamgagudtdt | 232 |
| 2217-S 2M13 | gugauguaugucmamgagagudtdt | 233 |
| 2217-S 2M11 | gugauguaugmumcagagagudtdt | 234 |
| 2217-S 2M9 | gugauguamumgucagagagudtdt | 235 |
| 2217-S 2M7 | gugaugmumaugucagagagudtdt | 236 |
| 2217-S 2M5 | gugamumguaugucagagagudtdt | 237 |
| 2217-s 2M3 | gumgmauguaugucagagagudtdt | 238 |
| 2217-S 1M19 | gugauguaugucagagagmudtdt | 239 |
| 2217-S 1M18 | gugauguaugucagagamgudtdt | 240 |
| 2217-S 1M17 | gugauguaugucagagmagudtdt | 241 |
| 2217-S 1M16 | gugauguaugucagamgagudtdt | 242 |
| 2217-S 1M15 | gugauguaugucagmagagudtdt | 243 |
| 2217-S 1M14 | gugauguaugucamgagagudtdt | 244 |
| 2217-S 1M13 | gugauguaugucmagagagudtdt | 245 |
| 2217-S 1M12 | gugauguaugumcagagagudtdt | 246 |
| 2217-S 1M11 | gugauguaugmucagagagudtdt | 247 |
| 2217-S 1M10 | gugauguaumgucagagagudtdt | 248 |
| 2217-S 1M9 | gugauguamugucagagagudtdt | 249 |
| 2217-S 1M8 | gugaugumaugucagagagudtdt | 250 |
| 2217-S 1M7 | gugaugmuaugucagagagudtdt | 251 |
| 2217-S 1M6 | gugaumguaugucagagagudtdt | 252 |
| 2217-S 1M5 | gugamuguaugucagagagudtdt | 253 |
| 2217-S 1M4 | gugmauguaugucagagagudtdt | 254 |
| 2217-S 1M3 | gumgauguaugucagagagudtdt | 255 |
| 2217-S 1M2 | gmugauguaugucagagagudtdt | 256 |
| 2217-S 1M1 | mgugauguaugucagagagudtdt | 257 |
| 2217-AS 1M19 | acucucugacauacaucamcdtdt | 258 |
| 2217-AS 1M18 | acucucugacauacaucmacdtdt | 259 |
| 2217-AS 1M17 | acucucugacauacaumcacdtdt | 260 |
| 2217-AS 1M16 | acucucugacauacamucacdtdt | 261 |
| 2217-AS 1M15 | acucucugacauacmaucacdtdt | 262 |
| 2217-AS 1M14 | acucucugacauamcaucacdtdt | 263 |
| 2217-AS 1M13 | acucucugacaumacaucacdtdt | 264 |
| 2217-AS 1M12 | acucucugacamuacaucacdtdt | 265 |
| 2217-AS 1M11 | acucucugacmauacaucacdtdt | 266 |
| 2217-AS 1M10 | acucucugamcauacaucacdtdt | 267 |
| 2217-AS 1M9 | acucucugmacauacaucacdtdt | 268 |
| 2217-AS 1M8 | acucucumgacauacaucacdtdt | 269 |
| 2217-AS 1M7 | acucucmugacauacaucacdtdt | 270 |
| 2217-AS 1M6 | acucumcugacauacaucacdtdt | 271 |
| 2217-AS 1M5 | acucmucugacauacaucacdtdt | 272 |
| 2217-AS 1M4 | acumcucugacauacaucacdtdt | 273 |
| 2217-AS 1M3 | acmucucugacauacaucacdtdt | 274 |
| 2217-AS 1M2 | amcucucugacauacaucacdtdt | 275 |
| 2217-AS 1M1 | macucucugacauacaucacdtdt | 276 |
| 2217-S 1D19 | gugauguaugucagagagdudtdt | 277 |
| 2217-S 1D18 | gugauguaugucagagadgudtdt | 278 |
| 2217-S 1D17 | gugauguaugucagagdagudtdt | 279 |
| 2217-S 1D16 | gugauguaugucagadgagudtdt | 280 |
| 2217-S 1D15 | gugauguaugucagdagagudtdt | 281 |
| 2217-S 1D14 | gugauguaugucadgagagudtdt | 282 |
| 2217-S 1D13 | gugauguaugucdagagagudtdt | 283 |
| 2217-S 1D12 | gugauguaugudcagagagudtdt | 284 |
| 2217-S 1D11 | gugauguaugducagagagudtdt | 285 |
| 2217-S 1D10 | gugauguaudgucagagagudtdt | 286 |
| 2217-S 1D9 | gugauguadugucagagagudtdt | 287 |
| 2217-S 1D8 | gugaugudaugucagagagudtdt | 288 |
| 2217-S 1D7 | gugaugduaugucagagagudtdt | 289 |
| 2217-S 1D6 | gugaudguaugucagagagudtdt | 290 |
| 2217-S 1D5 | gugaduguaugucagagagudtdt | 291 |
| 2217-S 1D4 | gugdauguaugucagagagudtdt | 292 |
| 2217-S 1D3 | gudgauguaugucagagagudtdt | 293 |
| 2217-S 1D2 | gdugauguaugucagagagudtdt | 294 |
| 2217-S 1D1 | dgugauguaugucagagagudtdt | 295 |
| 2217-AS 1D19 | acucucugacauacaucadcdtdt | 296 |
| 2217-AS 1D18 | acucucugacauacaucdacdtdt | 297 |
| 2217-AS 1D17 | acucucugacauacaudcacdtdt | 298 |
| 2217-AS 1D16 | acucucugacauacaducacdtdt | 299 |
| 2217-AS 1D15 | acucucugacauacdaucacdtdt | 300 |
| 2217-AS 1D14 | acucucugacauadcaucacdtdt | 301 |
| 2217-AS 1D13 | acucucugacaudacaucacdtdt | 302 |
| 2217-AS 1D12 | acucucugacaduacaucacdtdt | 303 |
| 2217-AS 1D11 | acucucugacdauacaucacdtdt | 304 |
| 2217-AS 1D10 | acucucugadcauacaucacdtdt | 305 |
| 2217-AS 1D9 | acucucugdacauacaucacdtdt | 306 |
| 2217-AS 1D8 | acucucudgacauacaucacdtdt | 307 |
| 2217-AS 1D7 | acucucdugacauacaucacdtdt | 308 |
| 2217-AS 1D6 | acucudcugacauacaucacdtdt | 309 |
| 2217-AS 1D5 | acucducugacauacaucacdtdt | 310 |
| 2217-AS 1D4 | acudcucugacauacaucacdtdt | 311 |
| 2217-AS 1D3 | acducucugacauacaucacdtdt | 312 |
| 2217-AS 1D2 | adcucucugacauacaucacdtdt | 313 |
| 2217-AS 1D1 | dacucucugacauacaucacdtdt | 314 |
| The letter "d" indicates that the nucleotide following the letter "d" is deoxy at the 2' position. | | |
| The letter "m" indicates that the nucleotide following the letter "m" is modified with a 2'-O-methyl moiety. | | |

### Example 11

### Molecule 1 Modifications and Stability

For the purposes of Examples 11-18, the term "molecule 1 modifications" refers to molecules that contain 2'-O-methyl modifications on positions 1 and 2 of the sense strand, 2'-O-methyl modifications on all Cs and Us of the sense strand, 2'-fluoro (F1) modification of all Cs and Us of the antisense strand, and a phosphate modification on the 5' terminus of the antisense strand. Similarly, the term "molecule 2 modifications" refers to molecules that contain 2'-O-methyl modifications on positions 1 and 2 of the sense strand, 2'-O-methyl modifications on all Cs and Us of the sense strand, a Cy3 label on the 5' end of the sense strand, 2'-Fluoro (F1) modifications on all Cs and Us of the antisense strand, and a phosphate modification on the 5' terminus of the antisense strand.

To assess the effects of molecule 1 modifications on siRNA stability, four unique siRNA were synthesized in modified and unmodified forms. Subsequently, these molecules were incubated in 100% serum at 37°C for varying periods of time and then analyzed by PAGE to assess the intactness of the duplexes. Visualization of sequences was accomplished by ethidium bromide staining.

The results of these experiments are illustrated in **Figure 37** and show that duplexes carrying molecule 1 modifications are drastically more stable than unmodified equivalents. Unmodified molecules typically exhibit 50% degradation or greater within two minutes of being exposed to serum at room temperature. In contrast, the half-life for sequences carrying molecule 1 modifications typically ran between 125 and 135 hours. Thus molecule 1 modifications significantly enhanced stability by approximately 500-fold.

### Example 12

### Molecule 1 Modifications and siRNA Silencing Potency

To assess the effects of molecule 1 modifications on siRNA potency, two unique siRNA directed against human Cyclophilin B (U1 and U3) were synthesized in modified and unmodified forms using 2'-O-ACE chemistry and tested.for functionality in a whole cell assay. Briefly, modified and unmodified siRNA were transfected (Lipofectamine 2000) into HeLa cells (10,000 cells/well, 96 well plate) at concentrations between 0.01-200 nM and cultured for 24-48 hours. Subsequently, the level of expression of the intended target was assessed using a branched DNA assay (Genospectra, Fremont, CA).

Results of these experiments are illustrated in **Figure 38** and show that duplexes carrying molecule 1 modifications perform comparably with unmodified siRNA at all concentrations tested. Thus, molecule 1 modifications do not appear to alter the potency of siRNA.

### Example 13

### Molecule 1 Modifications and Silencing Longevity

To determine the effects of molecule 1 modifications on siRNA silencing longevity, siRNA directed against the human cyclophilin B gene were synthesized in the modified and unmodified forms and transfected into HeLa cells (100nM) as previously described. Subsequently, the level of silencing was monitored over the course of 7 days using a branched DNA assay.

An example of the results of these experiments are presented in Figure 39 and demonstrate that while the level of silencing induced by unmodified molecules depreciates from roughly 70% to 0% over the course of the seven day period, modified duplexes induce >80% functionality throughout the course of the experiment. Thus, siRNA modified with molecule 1 modifications enhance the longevity of silencing induced by these duplexes.

### Example 14

### Molecule 1 Modifications and Toxicity

To determine the effects of molecule 1 modifications on siRNA toxicity, 4 siRNA (U1-U4) directed against human cyclophilin B were synthesized in the modified and unmodified forms and transfected into HeLa cells at concentrations that ranged between 0.01-200nM. At t = 48 hours after transfection, Alamar Blue viability assays were performed to assess the level of cell death within the population. A side-by-side comparison between the modified and unmodified duplexes showed no difference in the level of cell death induced at any of the concentrations tested (**Figure 40**).

### Example 15

### Molecule 1 Modifications and Off-Target Effects

To assess the effects of molecule 1 modifications on off-target effects, four separate siRNA targeting human Cyclophilin B (U1-U4) were synthesized in both the modified and unmodified forms and transfected into HeLa cells at 100nM concentrations. Subsequently, total RNA from (1) mock-transfected, (2) transfected (unmodified), and (3) transfected (modified) cells was purified (Qiagen), converted into cDNA and labeled with Cy3 (mock-transfected) or Cy5 (transfected-unmodified, transfected-modified) using Agilent's Low RNA Input Linear Amp Kit. Labeled cDNA from mock-transfected and untransfected cells were then mixed and hybridized to an Agilent Human 1A (V2) Oligo Microarray containing over 21,000 probes. The number of off-targets associated with modified and unmodified samples was assessed using Agilent's Feature Extraction, Spotfire DecisionSite, and Spotfire Functional Genomics, software (versions 7.2, 7.2, and 7.1, respectively).

A summary of these off-target studies are shown in **Figure 41** and illustrate that modified and unmodified siRNA perform similarly in terms of the numbers of off-targeted genes. Specifically, when off-targeted genes were segregated based on the level of induction or repression (compared to wild type gene expression), modified siRNA performed similarly (or better than )unmodified counterparts.

### Example 16

### Molecule 2 Modifications and Silencing Potential

To test the functionality of siRNA containing molecule 2 modifications, Cyclo14 (5'GGCCTTAGCTACAGGAGAG, sense strand SEQ. ID NO. 322), an siRNA directed against human cyclophilin B, was synthesized with molecule 2 modifications and tested for the ability to silence the intended target. Briefly, Cyclo14 was synthesized with the appropriate modifications using 2'-O-ACE chemistry. Subsequently, T482 HeLa cells (10,000 cells per well, 96 well plates) were plated, cultured overnight, and transfected (Lipofectamine 2000) With the cyclo14 duplex at 100nM concentrations. Three days after transfection, the level of mRNA silencing was assessed using a branched DNA assay (Genospectra, Fremont, CA).

Results of these studies are presented in **Figure 42****.** Unmodified Cyclo 14 duplexes typically induce 80-95% silencing. Cyclo 14 duplexes modified with the Cy3 label alone induced roughly 70-80% silencing, and Cyclo14 siRNA carrying molecule 2 modifications induced 80% or better silencing. As similar modification of nonspecific sequences induced little or no silencing, addition of molecule 2 modifications has little or no effect on duplex functionality.

### Example 17

### The Effects of Molecule 2 Modifications on Trackability

To test the usefulness of molecule 2 modifications as a means of assessing transfection efficiencies, Cyclo 14 siRNA were prepared with the aforementioned modifications and visualized by fluorescence microscopy. Specifically Cyclo14 was synthesized with the appropriate modifications using 2'-O-ACE chemistry. Subsequently, T482 HeLa cells (10,000 cells per well, 96 well plates) were plated, cultured overnight, and transfected (Lipofectamine 2000) with the appropriate duplex at 100 nM concentrations. Forty-eight hours after transfection, cultures were incubated with Hoechst 33342 (2µg/ml, 20 minutes, 37°C) and then visualized on a Leica DM1L fluorescence microscope using Dapi and Rhodamine filters.

A fluorescence micrograph (figure not included herein) of HeLa cells transfected with Cyclo14 siRNA carrying molecule 2 modifications shows a strong perinuclear and nuclear stain. Cells transfected with unmodified Cyclo14 siRNA show no equivalent staining (data not shown), thus molecule 2 modifications provide an excellent means of assessing the intracellular position of any given siRNA and the success of transfection.

### Example 18

### The Effects of Molecule 2 Modifications on Stability

To test the whether molecule 2 modifications enhanced the intracellular stability of duplexes, Cyclo 14 siRNA carrying molecule 2 modifications were transfected into HeLa cells and compared with Cy3 labeled Cyclo14 transfected cells over the course of seven days. Addition of the aforementioned modifications significantly enhanced siRNA stability over duplexes modified with Cy3 alone. While both samples exhibit strong staining patterns on Day 2, the Cy3-Cyclo14 transfected cells lose their stain by day 7. In contrast, cells containing Cyclo14 siRNA carrying molecule 2 modifications retain a strong pattern of staining on day 7. Moreover, unlike Cy3-labeled duplexes, siRNA carrying molecule 2 modifications also promote nuclear access to the duplex.

### Example 19

### Identification of Chemical Modifications that Modify Silencing Activity

Using 2'-O- ACE chemistry as a platform for RNA synthesis, a modification walk consisting of one, two, or three consecutively modified nucleotides in sense (S) and antisense (AS) strands was performed on SEAP-2217, an siRNA directed against human secreted alkaline phosphatase (SEAP, SEAP-2217-sense strand 5'-G U G A U G U A U G U C A G A G A G U dT dT (SEQ. ID NO.326)). Subsequently, the silencing efficiency of these modified siRNAs was evaluated by cotransfecting each duplex with a SEAP expression vector (Clontech) into HEK293 cells (100nM siRNA, 50 ng/well SEAP expression vector, Lipofectamine 2000) and assaying for a decrease in target protein activity twenty-four hours after transfection. **Figure 43A** and **Figure 43B** show the relationship between modification and function for 2'-O-methylated SEAP-2217 siRNA. Unmodified duplexes targeting SEAP induce >90% silencing of the SEAP gene. Single base modifications of both S and AS Strands induced little or no effect on siRNA activity, suggesting that no single 2'-hydroxyl group on either strand plays an indispensable role in RNAi. In contrast, a walk of dual, side-by-side, modifications identified several key positions where the introduction of modified bases interfered significantly with silencing activity. The most profound interference with function was observed when two consecutive bases (positions 1 and 2) or three consecutive bases (positions 1, 2, and 3) of the 5' end of the AS strand were modified, thus hinting of a cooperative effect between adjacent modified groups. As similar modifications of the S strand failed to alter duplex functionality, paired 2'-O-methyl modified bases enable a distinction of S and AS strands.

An identical set of experiments was performed using 2'-deoxy modifications. Studies shown in **Figure 44A** and **Figure 44B** demonstrate that addition of three consecutive 2'-deoxy groups at different positions (*e.g*., 1 + 2 + 3, 4 + 5 + 6, *etc*...) on either strand of the duplex had no effect on duplex silencing activity. Similar results were observed for walks that examined the effects of two and one consecutive 2'-deoxy groups (data not shown).

To further test the effects of 2'-O-methyl modifications on duplex functionality, a series of siRNA directed against the luciferase gene (luc 8,18, 56, 58, 63, and 81) were synthesized using 2'-O-ACE chemistry and modified to contain O-methyl groups at the 2' position of the ribose ring.
Luc 8 5'-GAAAAAUCAGAGAGAUCCU (SEQ. ID NO. 327)
Luc 18 5'-UACCGGAAAACUCGACGCA (SEQ. ID NO. 328)
Luc 56 5'-ACGUCGCCAGUCAAGUAAC (SEQ. ID NO. 329)
Luc 58 5'-GAUUACGUCGCCAGUCAAG (SEQ. ID NO. 330)
Luc 63 5'-AGAGAUCGUGGAUUACGUC (SEQ. ID NO. 331)
Luc 81 5'-UGUUGUUUUGGAGCACGGA (SEQ. ID NO. 332)

### (Sequences listed above are the sense strand.)

Specifically, siRNA containing 2'-O-methyl modifications on the two 5'-most nucleotides of (1) the sense strand, (2) the antisense strand, or (3) both strands, were co-transfected along with a Luc-expression plasmid (pCMVLuc, 50ng/well) into HEK293 cells. Subsequently, a side-by-side comparison of the silencing ability of each duplex was performed to determine the effects of this modification on target transcript degradation.

Results of these studies showed that addition of the 2'-O-methyl groups only to the AS strand dramatically diminished the ability of the duplex to silence the target mRNA (see **Figure 45A-F**). In contrast, duplexes carrying this modification on the sense strand performed as well (luc 58, 63, 81) or better (luc 56, 8, 18) than equivalent, unmodified siRNA, suggesting that modification of the sense strand biased strand selection by RISC and (in some cases) increased the effective antisense strand concentration. Enhanced silencing could be the result of a decrease in the binding affinity of RISC to the 5' sense end of the molecule (and therefore an increase in the availability of free RISC for association to the opposing end), decreased ability of native kinases to phosphorylate the sense strand (thus decreasing competition between the sense and antisense strand for access to RISC), or a decline in the ability of RISC to unravel the duplex from the 5'-sense end. siRNA containing 2'-O methyl modifications on both strands exhibited decreased silencing abilities that were between the values observed for molecules that contained modifications on either single strand. One interpretation of these results is that 2'-O-methyl modifications lowers the binding affinity that RISC has for the modified strand. In cases where both strands are modified, neither strand receives an advantage over its complement, and a new equilibrium representing an average of the functionality of both modified molecules is established.

To test whether the diminished level of silencing observed in cells containing 2'-O-methylated S/AS siRNA was the result of a debilitated capacity of cellular kinases to phosphorylate the duplexes, siRNAs carrying the 2'-O-methyl modifications were modified to carry a phosphate group on the 5' end of the AS strand. Specifically, Luc siRNAs carrying 2'-O-methyl groups on either: (1) positions 1 and 2 of the 5' end of the antisense strand; or (2) positions 1 and 2 of the 5' end of both antisense and sense strands, were 5'-phosphorylated on the AS strand during synthesis. These duplexes were then introduced into HEK293 cells using previously described procedures and tested for the ability to silence the desired target. Results showed that in 83% of the cases tested (10/12), 5' phosphorylation of the antisense strand improved the silencing efficiency of the duplex over the equivalent unphosphorylated molecule (**Figure 45A**-**F**). In the remaining two cases, silencing remained unchanged or was improved only marginally.

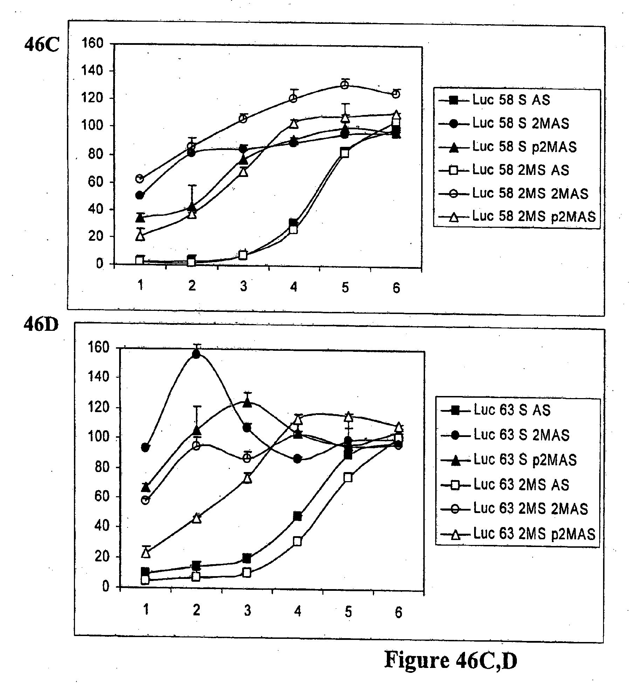

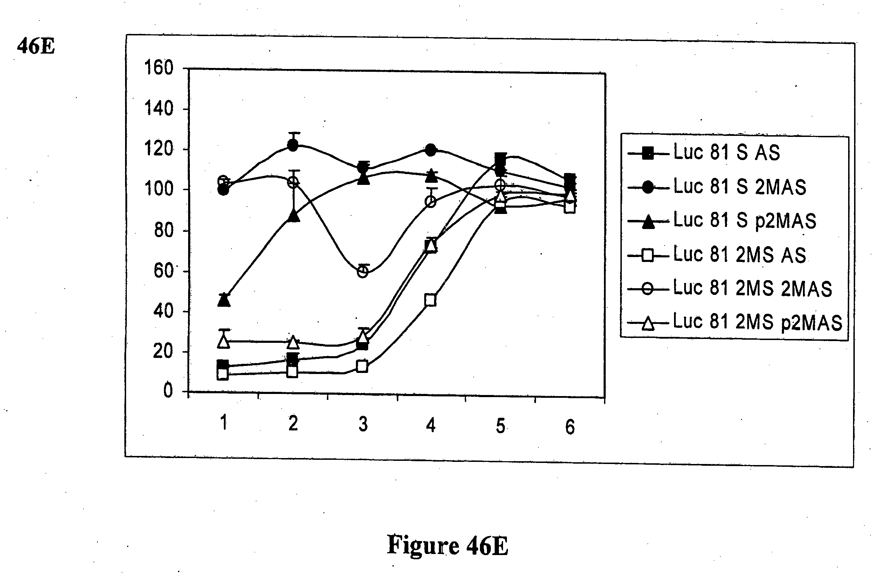

A more detailed examination of the effects of chemical modifications on silencing using dose response curves support the previous data. Five different siRNAs directed against the luciferase gene (luc 8, 56, 58, 63, and 81) were synthesized using the 2'-O-ACE chemistry in both 2'-O-ethyl modified and unmodified forms. These duplexes were then transfected into HeLa cells (along with a luciferase expression vector) at concentrations that varied between 0.01-200 nM and assayed (t = 24-48 hours) for the level of silencing of the luciferase protein. The results from these experiments are summarized in **Figure 46** and lead to the following conclusions: (1) addition of 2'-O-methyl groups to positions 1 and 2 of the 5' end of the antisense strand consistently disrupts silencing activity; (2) addition of 2'-O-methyl groups to positions 1 and 2 of the 5' end of the sense strand of siRNA either improves or has little or no effect on target specific silencing; (3) addition of 2'-O-methyl groups to positions 1 and 2 of the 5' end of both strands (sense and antisense) generates an intermediary silencing effect that is between that observed with unmodified duplexes and duplexes carrying a 2'-O-methyl modification on the 5' end of the antisense strand; and (4) addition of a phosphate group to the 5' end of the antisense strand of molecules that have 2'-O-methyl modifications on positions 1 and 2 of the 5' end of both the sense and antisense strands, improved the silencing activity of the molecule over duplexes that had 2'-O-methyl modifications on the 5' end of both strands.

These results suggest that addition of the 2'-O-methyl modification of positions 1 and 2 of the sense and antisense strands of siRNA in addition to 5' phosphorylation of the first terminal nucleotide of the antisense strand enhances the potency of the duplex at a variety of concentrations. Moreover, the effects of various modifications lead to the following conclusions: (1) addition of 2'-O-methyl groups to the 5' end of the sense or antisense strand severely reduces silencing by that strand; and (2) addition of a 5' phosphate group restores, nearly completely, silencing activity even in the presence of the 2'-O-methyl group. Thus, while some fraction of the inhibitory effects of AS-2'-O-methylation are likely the result of inhibition of duplex phosphorylation, some portion of the effects are most probably due to the effects of this form of modification on other steps in the RNAi pathway such as RISC binding, siRNA unwinding, RISC mediated siRNA-target association, or RISC mediated target cleavage. Importantly, the potential effect of 2'-O-methylation on these other steps led the authors to consider that the possibility that said modifications might also alter the ability of RISC to distinguish between intended targets that have 100% homology with the antisense strand and off-targets that have lesser amounts of homology.

### Example 20

### The Effects of 2'-O-methylation and 5' Phosphorylation on Off-Targeting

The observation that 2'-O-methylation of the antisense strand disrupts silencing while equivalent sense strand labeling has no effect on target specific silencing suggests a strategy for eliminating off-target silencing caused by the sense strand. Furthermore, the observation that steps downstream of duplex phosphorylation may be affected by 2'-O-methylation leads to the prospect that siRNA containing this combination of modifications (2'-O-methylation of positions 1 and 2 of the 5' end of the sense and antisense strands, plus phosphorylation of the 5' end of the AS strand) may have high potency and low sense/antisense off-targeting effects.

To test the effects of 5'-phosphorylation of the antisense strand in combination with 2'-O-methylation of: (1) the sense strand; (2) the antisense strand; and (3) the sense and antisense strands on off-targeting, two siRNA directed against four different genes (8 siRNA total: MAPK14-193 and -153, MPHOSPH1-202 and -203, IGF1R-73 and -74, and PTEN-213 and -214) were synthesized using the 2'-O-ACE chemistry and modified at the appropriate sites. When experiments were conducted under conditions where MAPK14-153 contains only a phosphate group on the 5' end of the antisense strand, extensive off-targeting is observed. When the sense strand is further modified with 2'-O-methyl.groups at positions 1 and 2, off-targeting due to that strand is eliminated, but a novel set of anti-sense strand off-targets are generated. This increase in antisense strand off-targeting is likely due to increased AS strand-RISC interactions in the absence of competition by the sense strand. When the antisense strand contains a phosphate group on the 5' end and is modified with 2'-O-methyl groups at positions 1 and 2, off-targets due to the antisense strand are lost, but sense strand off-targets are once again observed. Finally, when both the sense strand and the antisense strand are modified with 2'-O-methyl groups (on positions 1 and 2 of each strand) and a phosphate group is attached to the 5' end of the antisense strand, a drastic reduction in the level of off-targeting by both strands is observed.

Similar results to those described above were obtained for the remaining seven siRNAs directed against IGF1R, PTEN, MAPK14, and MPHOSPH1. Thus, from these experiments it is possible to include that: (1) different siRNA directed against the same target induced varying levels of off-target effects; (2) 2'-O-methyl modification of positions 1 and 2 of the 5'-end of the antisense strand (in combination with 5' phosphorylation of the antisense strand) eliminates off-target effects due to that strand but frequently leads to additional sense strand off-targets; (3) 2'-O-methyl modification of positions 1 and 2 of the 5'-end of the sense strand (in combination with 5' phosphorylation of the antisense strand) eliminates off-target Effects due to that strand but frequently leads to additional antisense strand off-targets; and (4) 2'-O-methyl modification of positions 1 and 2 of both sense and antisense strands (in combination with 5' phosphorylation of the antisense strand) drastically eliminates off-target effects from both strands. Thus, the combination of three separate modifications (5' phosphorylation of the antisense strand, 2'-O-methylation of positions 1 and 2 of the sense strand, and 2'-O-methylation of positions 1 and 2 of the antisense strand) leads to drastic reduction in off-target effects that are generated by both strands. In addition, in the three cases where specific silencing activity was tested (MAPK14, PTEN, and MPHOSPH1), the fully modified molecule (*e.g*., those that contain 5' phosphate on the antisense strand, 2'-O-methylation of positions 1 and 2 of the sense strand, and 2'-O-methylation of positions 1 and 2 of the antisense strand) that has minimal off-target effects, silenced the intended target as well or better than siRNA that contain only a 5'-phosphate group on the 5' end of the antisense strand.

### The Effects of 2'-O-Methxlation

The data described above clearly show that the addition of 2'-O-methyl groups to positions 1 and 2 of the 5' end of the AS strand disrupts siRNA activity. How this modification disrupts duplex function can be broken down into two distinct steps in the RNAi pathway. In the first, 2'-O-methylation clearly disrupts the ability of resident kinases to phosphorylate duplexes. Addition of the 5' phosphate group synthetically alleviates the need for cellular enzymes to fulfill this function and improves duplex functionality. Thus, one of the effects of this chemical modification is well-defined. While synthetic addition of phosphate groups to the 5' end of the antisense strand of siRNA modified with 2'-O-methyl groups at nucleotide positions 1 and 2 on both strands increases activity, full functionality is not recovered. This result suggests that steps downstream of the phosphorylation event (*e.g*., RISC binding, RISC mediated siRNA-target/off-target interactions, and/or RISC mediated siRNA-target/off-target cleavage) are also affected by 2'-O-methylation.

### 2'-O-Methviation and Enhanced Duplex Functionality

The binding of siRNA to RISC and subsequent duplex unwinding are critical parameters of functionality. Studies presented here show that in some cases 2'-O-Me modification of the sense strand alters the dose response curves in a fashion that is inversely proportional to the original functionality of the molecule. One interpretation of these findings is that RISC association with the sense and antisense strands exists in an equilibrium that is defined by "on" and "off"-coefficients (*e.g*., k₁, k_{1'}', k₂, and k_{2'}, **Figure 47**). Highly functional molecules exhibit a Kₒᵥₑᵣₐₗₗ (k₁ k₂/k_{1'} k_{2'}) that reflects a bias toward association with the antisense strand (*e.g*., Kₒᵥₑᵣₐₗₗ= 100). In contrast, non-or semi-functional molecules (<F70), exhibit a Kₒᵥₑᵣₐₗₗ that is demonstrative of a more impartial or balanced strand interaction (*e.g.*, for <F70 siRNA, Kₒᵥₑᵣₐₗₗ~1). Chemical modification of the sense strand of non- or semi- functional molecules drives the equilibrium toward RISC-AS interactions, thus dramatically changing the RISC-AS: RISC-S ratio (Kₒᵥₑᵣₐₗₗ ~ 5) and overall duplex functionality. In contrast, in the case of highly functional molecules, RISC-AS interactions are already preferred, thus chemical modification does less to further enhance RISC-AS association and functionality.

### 2'-O-Methylation and Off-Target Silencing

While synthetic addition of phosphate groups to the 5' end of the antisense strand of duplexes containing 2'-O-methyl modifications at positions 1 and 2 on both strands leads to partial rescue of the silencing phenotype, the absence of full recovery suggests that this modification affects additional steps that are downstream of duplex phosphorylation (*e.g*., RISC binding, RISC mediated siRNA-target/off-target interactions, and/or RISC mediated siRNA-target/off-target cleavage). This fact opens the door to the possibility that these same modifications might also place greater demands on the degree of siRNA-target complementarity necessary for silencing. It is possible that in cases where the position of the 2'-O-methyl group overlaps the site of homology between an off-target and a given siRNA (sense or antisense strand), the addition of the chemical modification destabilizes siRNA-off-target interactions, thus preventing a critical step in RNAi mediated down regulation. Alternatively, in cases where the position of the 2'-O-methyl group overlaps the site of homology between an off-target and a given siRNA, the additional chemical modification may alter duplex flexibility in that region and thus inhibit RISC's ability to cleave the target molecule. In yet another scenario, the site of the 2'-O-methyl modification may not overlap the position of homology with the off-target In this case, it is possible that the destabilizing effects of the modifications are transmitted down the molecule, thus eliminating the ability of RISC to pair and/or cleave targets that have less than 100% homology to the siRNA. To that end, 2'-O-methyl modifications at positions other than the 5' end of the S or AS sense strand may be used to eliminate off-target effects.

### Example 21

### Modified Duplexes as Exaequo Agents

In the course of running dosage or microarray experiments with siRNA, it is desirable to maintain the total siRNA concentration at a constant during transfection. Unfortunately, addition of a second siRNA, even one that is non-specific, can lessen the degree of silencing induced by the target specific siRNA, possibly due to competition between the two sequences for access to RISC. For that reason it is important to develop duplexes that can act as exaequo agents that do not disrupt gene targeting.

To access the ability of modified duplexes to act as non-competitive exaequo agents in, *e.g*., dosage experiments, the effect of modified and unmodified non-specific siRNA on specific siRNA silencing was tested. To accomplish this, an siRNA duplex directed against human cyclophilin B gene (*e.g*., cyclo4) was first transfected into cells at a variety of concentrations (*i.e*., a dose response). The target specific silencing induced by cyclo4 alone was then compared with the silencing induced by this molecule in the presence of a) un-modified non specific sequence #4 (NS4), or b) modified NS4. In this case, the modified version of NS4 was either 19 or 17 base pairs in length, (SEQ. ID NO. 333: UAGCGACUAAACACAUCAA and SEQ. ID NO. 334: UAGCGACUAAACACAUC, respectively) and contained 2'-O-methyl groups on positions 1 and 2 of the sense and antisense strand, 2'-O-methyl groups on the Cs and Us of the sense strand, and 2' F1 groups on the Cs and Us of the antisense strand. No phosphate group is attached to the 5' end of the sense or antisense strand.

Specifically, HeLa cells were plated into 96 well plates and allowed to adhere overnight. Subsequently, cells were transfected with one of the following:
a) cyclo4 siRNA (1-100nM), (SEQ. ID NO. 318: GGA AAG ACU GUU CCA AAA A)
b) cyclo4 siRNA (1-100nM) plus NS4 (19 base pairs),
c) cyclo4 siRNA (1-100nM), plus modified NS4 (17 or 19 base pairs),
d) cyclo4 siRNA (1-100nM) plus a modified NS4 (17 or 19 base pairs) that also contained a Cy3 label on the 5' end of the sense strand,
using Lipofectamine 2000. The concentrations of the second siRNA were matched with that of cyclo4 such that the total siRNA concentration during the transfection remained constant at 100nM. Subsequently target specific silencing was measured (t= 24 hours) using a branched DNA assay (Genospectra, Fremont, CA).

The results of these experiments are illustrated in **Figure 48** and demonstrate the following:
1. Cyclo4 is a potent duplex. Decreasing the concentration of cyclo4 from 100nM .to 1 nM does not alter the silencing ability of this duplex (F95, See **Figure 48A**-**I**).
2. Addition of a 19 base pair unmodified NS4 siRNA to cyclo4 causes an appreciable concentration dependent decrease in cyclo4 induced gene silencing, suggesting that NS4 competes with cyclo4 for, *e.g*., access to the RISC complex (see **Figures 48B-I**).
3. NS4 duplexes (17 or 19 base pairs) containing 2'-O-methyl groups on positions 1 and 2 of the sense and antisense strand, 2'-O-methyl groups on the Cs and Us of the sense strand, and 2' F1 groups on the Cs and Us of the antisense strand, fail to diminish target specific silencing by cyclo4, suggesting these duplexes do not compete with cyclo4 for, *e.g*., RISC (see **Figure 43** **BII** and **BIII**).
4. NS4 duplexes (17 or 19 base pairs) containing 2'-O-methyl groups on positions 1 and 2 of the sense and antisense strand, 2'-O-methyl groups on the Cs and Us of the sense strand, 2' F1 groups on the Cs and Us of the antisense strand, and a Cy3 group on the 5' end of the sense strand fail to diminish silencing by cyclo4, suggesting these duplexes do not compete with cyclo4 for, *e.g*., RISC (see **Figure 48** **AII** and **AIII**).
These results demonstrate that siRNAs containing the before mentioned modifications fail to compete with functional siRNA and can be used as exaequo agents in siRNA studies, including microarray studies.

It is conceivable that the reason why siRNAs carrying the above modifications fail to compete with the target specific siRNAs is that the modified duplexes fail to enter the cell. To test this, an siRNA carrying 2'-O-methyl groups on positions 1 and 2 of the sense and antisense strand, 2'-O-methyl groups on the Cs and Us of the sense strand, and 2' Fl groups on the Cs and Us of the antisense strand (no 5' phosphate group on the antisense strand) was labeled with a Cy3 moiety on the 5' end of the sense strand and transfected into HeLa cells. Forty-eight hours post transfection, cells were analyzed by fluorescence microscopy. Results of these experiments show that aforementioned modifications do not alter the ability of these duplexes to enter the cell by Lipofectamine 2000 transfection. Micrographs (not reproduced herein) of: (A) HeLa cells stained with cyclo14 siRNA duplex modified with 2'-O-methyl groups on positions 1 and 2 of the sense and antisense strand, 2'-O-methyl groups on the Cs and Us of the sense strand, 2' F1 groups added to the Cs and Us of the antisense strand and a Cy3 group on the 5' end of the sense strand; and (B) stained as in (A) and counterstained with Hoechst 33342 to identify the position of the nucleus demonstrated nuclear and perinuclear stains of siRNA containing these modifications.

To test whether addition of 2'-O-methyl groups on positions 1 and 2 of the sense and antisense strand was sufficient to eliminate the ability of a duplex to compete with other, co-transfected siRNA, an siRNA directed against GAPDH (GAPDH4) was transfected into HeLa cells at varying concentrations (0.781- 100nM) along with (1) non-specific control #2 (NS2 - UAAGGCUAUGAAGAGAUAC), (2) non-specific control #2 carrying 2'-O-methyl modifications on positions 1 and 2 of both the sense and antisense strand, or (3) cyclo14 (SEQ. ID. NO. 335: GGCCUUAGCUACAGGAGAG, a cyclophilin siRNA that does not compete with GAPDH 4). The concentrations of the second siRNA were matched with that of GAPDH4 such that the total siRNA concentration during the transfection was 100nM. Cells were then cultured for an additional 24 hours before branched DNA assays were performed to assess the level of GAPDH transcripts. Results of these experiments are illustrated in **Figure 49** and show the following:
1. GAPDH4 in the presence of Cyclo14 (or by itself, data not shown) induces roughly 90% silencing of the native transcript.
2. Addition of unmodified NS2 led to a dramatic decrease in GAPDH silencing,
   and
3. Modification of NS2 with 2'-O-methyl groups at positions 1 and 2 on both the sense and antisense strands eliminated interference effects observed with unmodified molecules.

These results demonstrate that siRNA containing the before mentioned modifications fail to compete with functional siRNA and can be used as exaequo agents in a variety of siRNA applications including microarray studies.

## Claims

1. An siRNA, comprising:
(a) an antisense strand, wherein said antisense strand is comprised of a first 5' terminal antisense nucleotide and said first 5' terminal antisense nucleotide is phosphorylated at said first 5' terminal antisense nucleotide's 5' carbon position; and
(b) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification;
and wherein all nucleotides comprised in said antisense strand and sense strand other than said first 5' terminal sense nucleotide and second 5' terminal sense nucleotide are unmodified.

2. An siRNA, comprising:
(a) an antisense strand, wherein said antisense strand is comprised of a first 5' terminal antisense nucleotide and said first 5' terminal antisense nucleotide is phosphorylated at said first 5' terminal antisense nucleotide's 5' carbon position; and
(b) a sense strand, wherein said sense strand is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a 2'-O-alkyl modification, wherein each of said first 2' carbon sense modification and said second 2' carbon sense modification is a 2'-O-alkyl modification;
and wherein all nucleotides comprised in said antisense strand and sense strand other than said first 5' terminal sense nucleotide, second 5' terminal sense nucleotide and third 5' terminal sense nucleotide are unmodified.

3. An siRNA which is a unimolecular siRNA capable of forming a hairpin, said unimolecular siRNA comprising:
(a) an antisense region, wherein said antisense region is comprised of a first 5' terminal antisense nucleotide and said first 5' terminal antisense nucleotide is phosphorylated at said first 5' terminal antisense nucleotide's 5' carbon position;
(b) a sense region, wherein said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
(c) a loop region, wherein said loop region is located between said sense region and said antisense region;
and wherein all nucleotides comprised in said antisense region and sense region other than said first 5' terminal sense nucleotide and second 5' terminal sense nucleotide are unmodified.

4. An siRNA which is a unimolecular siRNA capable of forming a hairpin, said unimolecular siRNA comprising:
(a) an antisense region, wherein said antisense region is comprised of a first 5' terminal antisense nucleotide and said first 5' terminal antisense nucleotide is phosphorylated at said first 5' terminal antisense nucleotide's 5' carbon position;
(b) a sense region, wherein said sense region is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a 2'-O-alkyl modification; and
(c) a loop region, wherein said loop region is located between said sense region and said antisense region;
and wherein all nucleotides comprised in said antisense region and sense region other than said first 5' terminal sense nucleotide, second 5' terminal sense nucleotide and third 5' terminal sense nucleotide are unmodified.

5. An siRNA, comprising:
(a) an antisense strand, wherein said antisense strand is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification; and
(b) a sense strand, wherein said sense strand is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification;
and wherein all nucleotides comprised in said antisense strand and sense strand other than said first 5' terminal antisense nucleotide, second 5' terminal antisense nucleotide, first 5' terminal sense nucleotide and second 5' terminal sense nucleotide are unmodified.

6. An siRNA, comprising:
(a) an antisense strand, wherein said antisense strand is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification; and
(b) a sense strand, wherein said sense strand is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a third 2' carbon sense modification;
and wherein all nucleotides comprised in said antisense strand and sense strand other than said first 5' terminal antisense nucleotide, second 5' terminal antisense nucleotide, first 5' terminal sense nucleotide, second 5' terminal sense nucleotide and third 5' terminal sense nucleotide are unmodified.

7. An siRNA which is a unimolecular siRNA capable of forming a hairpin, said unimolecular siRNA comprising:
(a) an antisense region, wherein said antisense region is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification;
(b) a sense region, wherein said sense region is comprised of a first 5' terminal sense nucleotide and a second 5' terminal sense nucleotide, wherein said first 5' terminal sense nucleotide comprises a first 2' carbon sense modification and said second 5' terminal sense nucleotide comprises a second 2' carbon sense modification; and
(c) a loop region, wherein said loop region is located between said sense region and said antisense region;
and wherein all nucleotides comprised in said antisense region and sense region other than said first 5' terminal antisense nucleotide, second 5' terminal antisense nucleotide, first 5' terminal sense nucleotide and second 5' terminal sense nucleotide are unmodified.

8. An siRNA which is a unimolecular siRNA capable of forming a hairpin, said unimolecular siRNA comprising:
(a) an antisense region, wherein said antisense region is comprised of a first 5' terminal antisense nucleotide and a second 5' terminal antisense nucleotide, said first 5' terminal antisense nucleotide is phosphorylated at said first 5' terminal antisense nucleotide's 5' carbon position, said first 5' terminal antisense nucleotide further comprises a first 2' carbon terminal antisense modification and said second 5' terminal antisense nucleotide comprises a second 2' carbon terminal antisense modification;
(b) a sense region, wherein said sense region is comprised of a first 5' terminal sense nucleotide, a second 5' terminal sense nucleotide and a third 5' terminal sense nucleotide, wherein said first 5' terminal sense nucleotide comprises a first 2' carbon sense modification, said second 5' terminal sense nucleotide comprises a second 2' carbon sense modification and said third 5' terminal sense nucleotide comprises a third 2' carbon sense modification; and
(c) a loop region, wherein said loop region is located between said sense region and said antisense region;
and wherein all nucleotides comprised in said antisense region and sense region other than said first 5' terminal antisense nucleotide, second 5' terminal antisense nucleotide, first 5' terminal sense nucleotide, second 5' terminal sense nucleotide and third 5' terminal sense nucleotide are unmodified.

9. The siRNA of any one of claims 1 to 8, wherein each of said first 2' carbon sense modification and said second 2' carbon sense modification is a 2'-O-alkyl modification, and preferably wherein at least one and preferably each of the 2'-O-alkyl modifications is a 2'-O-methyl modification.

10. The siRNA of any one of claims 1 to 9, wherein said siRNA is comprised of between 18 and 30 base pairs and preferably between 19 and 25 base pairs.

11. The siRNA of any one of claims 1, 2, 5 and 6, wherein said siRNA is comprised of between 18 and 30 base pairs and preferably between 19 and 25 base pairs, and wherein said antisense strand and said sense strand are substantially complementary and form a duplex that does not contain overhangs.

12. The siRNA of any one of claims 1, 2, 5 and 6, further comprising a conjugate attached to the 3' or 5' end of said sense strand, wherein the conjugate is preferably cholesterol or polyethylene glycol.

13. A method for reducing off-target effects during RNAi, said method comprising exposing an siRNA of any one of claims 1 to 12 to a target nucleic acid or a cell that is expressing or is capable of expressing said target nucleic acid, wherein said method is not a method for treatment of the human or animal body by surgery or therapy or a diagnostic method practised on the human or animal body.

14. A pharmaceutical composition comprising an siRNA as defined in any one of claims 1 to 12 and a pharmaceutically acceptable carrier or diluent.

15. An siRNA as defined in any one of claims 1 to 12 for use in a method for treatment of the human or animal body by therapy or a diagnostic method practised on the human or animal body.

## Patentansprüche

1. siRNA umfassend:
(a) einen Antisense-Strang, wobei der Antisense-Strang ein erstes 5'-terminales Antisense-Nukleotid umfasst, und das erste 5'-terminale Antisense-Nukleotid an der 5'-Kohlenstoffposition des ersten 5'-terminalen Antisense-Nukleotids phosphoryliert ist und
(b) einen Sense-Strang, wobei der Sense-Strang ein erstes 5'-terminales Sense-Nukleotid und ein zweites 5'-terminales Sense-Nukleotid umfasst, wobei das erste 5'-terminale Sense-Nukleotid eine erste 2'-Kohlenstoff-Sense-Modifikation umfasst, und das zweite 5'-terminale Sense-Nukleotid eine zweite 2'-Kohlenstoff-Sense-Modifikation umfasst, und wobei alle Nukleotide, die von dem Antisense-Strang und dem Sense-Strang umfasst sind, ausgenommen das erste 5'-terminale Sense-Nukleotid und das zweite 5'-terminale Sense-Nukleotid, nicht modifiziert sind.

2. siRNA umfassend:
(a) einen Antisense-Strang, wobei der Antisense-Strang ein erstes 5'-terminales Antisense-Nukleotid umfasst, und das erste 5'-terminale Antisense-Nukleotid an der 5'-Kohlenstoffposition des ersten 5'-terminalen Antisense-Nukleotids phosphoryliert ist und
(b) einen Sense-Strang, wobei der Sense-Strang ein erstes 5'-terminales Sense-Nukleotid, ein zweites 5'-terminales Sense-Nukleotid und ein drittes 5'-terminales Sense-Nukleotid umfasst, wobei das erste 5'-terminale Sense-Nukleotid eine erste 2'-Kohlenstoff-Sense-Modifikation umfasst, das zweite 5'-terminale Sense-Nukleotid eine zweite 2'-Kohlenstoff-Sense-Modifikation umfasst, und das dritte 5'-terminale Sense-Nukleotid eine 2'-O-Alkylmodifikation umfasst, wobei jede der ersten 2'-Kohlenstoff-Sense-Modifikation und der zweiten 2'-Kohlenstoff-Sense-Modifikation eine 2'-O-Alkylmodifikation ist, und worin alle Nukleotide, die von dem Antisense-Strang und dem Sense-Strang umfasst sind, ausgenommen das erste 5'-terminale Sense-Nukleotid, das zweite 5'-terminale Sense-Nukleotid und das dritte 5'-terminale Sense-Nukleotid, nicht modifiziert sind.

3. siRNA, die eine unimolekulare siRNA mit der Fähigkeit, eine Haarnadel zu bilden, ist, wobei die unimolare siRNA umfasst:
(a) eine Antisense-Region, worin die Antisense-Region ein erstes 5'-terminales Antisense-Nukleotid umfasst, und das erste 5'-terminale Antisense-Nukleotid an der 5'-Kohlenstoffposition des ersten 5'-terminalen Antisense-Nukleotids phosphoryliert ist;
(b) eine Sense-Region, worin die Sense-Region ein erstes 5'-terminales Sense-Nukleotid und ein zweites 5'-terminales Sense-Nukleotid umfasst, worin das erste 5'-terminale Sense-Nukleotid eine erste 2'-Kohlenstoff-Sense-Modifikation umfasst, und das zweite 5'-terinale Sense-Nukleotid eine zweite 2'-Kohlenstoff-Sense-Modifikation umfasst, und
(c) eine Loop-Region, worin die Loop-Region zwischen der Sense-Region und der Antisense-Region lokalisiert ist und worin alle Nukleotide, die von der Antisense-Region und der Sense-Region umfasst sind, ausgenommen das erste 5'-terminale Sense-Nukleotid und das zweite 5'-terminale Sense-Nukleotid, nicht modifiziert sind.

4. siRNA, die eine unimolekulare siRNA mit der Fähigkeit zur Bildung einer Haarnadel ist, wobei die unimolekulare siRNA umfasst:
(a) eine Antisense-Region, worin die Antisense-Region ein erstes 5'-terminales Antisense-Nukleotid umfasst, und das erste 5'-terminale Antisense-Nukleotid an der 5'-Kohlenstoffposition des ersten 5'-terminalen Antisense-Nukleotids phosphoryliert ist;
(b) eine Sense-Region, worin die Sense-Region ein erstes 5'-terminales Sense-Nukleotid, ein zweites 5'-terminales Sense-Nukleotid und ein drittes 5'-terminales Sense-Nukleotid umfasst, wobei das erste 5'-terminale Sense-Nukleotid eine erste 2'-Kohlenstoff-Sense-Modifikation umfasst, das zweite 5'-terminale Sense-Nukleotid eine zweite 2'-Kohlenstoff-Sense-Modifikation umfasst, und das dritte 5'-terminale Sense-Nukleotid eine 2'-O-Alkylmodifikation umfasst, und
(c) eine Loop-Region, worin die Loop-Region zwischen der Sense-Region und der Antisense-Region lokalisiert ist, und worin alle Nukleotide, die von der Antisense-Region und der Sense-Region umfasst sind, ausgenommen das erste 5'-terminale Sense-Nukleotid, das zweite 5'-terminale Sense-Nukleotid und das dritte 5'-terminale Sense-Nukleotid, nicht modifiziert sind.

5. siRNA, umfassend:
(a) einen Antisense-Strang, wobei der Antisense-Strang ein erstes 5'-terminales Antisense-Nukleotid und ein zweites 5'-terminales Antisense-Nukleotid umfasst, das erste 5'-terminale Antisense-Nukleotid an der 5'-Kohlenstoffposition des ersten 5'-terminalen Antisense-Nukleotids phosphoryliert ist, das erste 5'-terminale Antisense-Nukleotid weiter eine erste 2'-Kohlenstoff-terminale-Antisense-Modifikation umfasst, und das zweite 5'-terminale Antisense-Nukleotid eine zweite 2'-Kohlenstoff-terminale-Antisense-Modifikation umfasst, und
(b) einen Sense-Strang, wobei der Sense-Strang ein erstes 5'-terminales Sense-Nukleotid und ein zweites 5'-terminales Sense-Nukleotid umfasst, worin das erste 5'-terminale Sense-Nukleotid eine erste 2'-Kohlenstoff-Sense-Modifikation umfasst, und das zweite 5'-terminale Sense-Nukleotid eine zweite 2'-Kohlenstoff-Sense-Modifikation umfasst, und worin alle Nukleotide, die von dem Antisense-Strang und dem Sense-Strang umfasst sind, ausgenommen das erste 5'-terminale Antisense-Nukleotid, das zweite 5'-terminale Antisense-Nukleotid, das erste 5'-terminale Sense-Nukleotid und das zweite 5'-terminale Sense-Nukleotid nicht modifiziert sind.

6. siRNA, umfassend:
(a) einen Antisense-Strang, wobei der Antisense-Strang ein erstes 5'-terminales Antisense-Nukleotid und ein zweites 5'-terminales Antisense-Nukleotid umfasst, das erste 5'-terminale Antisense-Nukleotid an der 5'-Kohlenstoffposition des ersten 5'-terminalen Antisense-Nukleotids phosphoryliert ist, das erste 5'-terminale Antisense-Nukleotid weiter eine erste 2'-Kohlenstoff-terminale-Antisense-Modifikation umfasst, und das zweite 5'-terminale Antisense-Nukleotid eine zweite 2'-Kohlenstoff-terminale-Antisense-Modifikation umfasst, und
(b) einen Sense-Strang, wobei der Sense-Strang ein erstes 5'-terminales Sense-Nukleotid, ein zweites 5'-terminales Sense-Nukleotid und ein drittes 5'-terminales Sense-Nukleotid umfasst, worin das erste 5'-terminale Sense-Nukleotid eine erste 2'-Kohlenstoff-Sense-Modifikation umfasst, das zweite 5'-terminale Sense-Nukleotid eine zweite 2'-Kohlenstoff-Sense-Modifikation umfasst, und das dritte 5'-terminale Sense-Nukleotid eine dritte 2'-Kohlenstoff-Sense-Modifikation umfasst, und worin alle Nukleotide, die von dem Antisense-Strang und dem Sense-Strang umfasst sind, ausgenommen das erste 5'-terminale Antisense-Nukleotid, das zweite 5'-terminale Antisense-Nukleotid, das erste 5'-terminale Sense-Nukleotid, das zweite 5'-terminale Sense-Nukleotid und das dritte 5'-terminale Sense-Nukleotid nicht modifiziert sind.

7. siRNA, die eine unimolekulare siRNA mit der Fähigkeit zur Bildung einer Haarnadel ist, wobei die unimolekulare siRNA umfasst:
(a) eine Antisense-Region, worin die Antisense-Region ein erstes 5'-terminales Antisense-Nukleotid und ein zweites 5'-terminales Antisense-Nukleotid umfasst, das erste 5'-terminale Antisense-Nukleotid an der 5'-Kohlenstoffposition des ersten 5'-terminalen Antisense-Nukleotids phosphoryliert ist, das erste 5'-terminale Antisense-Nukleotid weiter eine erste 2'-Kohlenstoff-terminale-Antisense-Modifikation umfasst, und das zweite 5'-terminale Antisense-Nukleotid eine zweite 2'-Kohlenstoff-terminale-Antisense-Modifikation umfasst;
(b) eine Sense-Region, worin die Sense-Region ein erstes 5'-terminales Sense-Nukleotid und ein zweites 5'-terminales Sense-Nukleotid umfasst, worin das erste 5'-terminale Sense-Nukleotid eine erste 2-Kohlenstoff-Sense-Modifikation umfasst, und das zweite 5'-terminale Sense-Nukleotid eine zweite 2'-Kohlenstoff-Sense-Modifikation umfasst, und
(c) eine Loop-Region, worin die Loop-Region zwischen der Sense-Region und der Antisense-Region lokalisiert ist, und worin alle Nukleotide, die von der Antisense-Region und der Sense-Region umfasst sind, ausgenommen das erste 5'-terminale Antisense-Nukleotid, das zweite 5'-terminale Antisense-Nukleotid, das erste 5'-terminale Sense-Nukleotid und das zweite 5'-terminale Sense-Nukleotid, nicht modifiziert sind.

8. siRNA, die eine unimolekulare siRNA mit der Fähigkeit zur Bildung einer Haarnadel ist, wobei die unimolekulare siRNA umfasst:
(a) eine Antisense-Region, worin die Antisense-Region ein erstes 5'-terminales Antisense-Nukleotid und ein zweites 5'-terminales Antisense-Nukleotid umfasst, und das erste 5'-terminale Antisense-Nukleotid an der 5'-Kohlenstoffposition des ersten 5'-terminalen Antisense-Nukleotids phosphoryliert ist, das erste 5'-terminale Antisense-Nukleotid weiter eine erste 2'-Kohlenstoff-terminale-Antisense-Modifikation umfasst, und das zweite 5'-terminale Antisense-Nukleotid eine zweite 2'-Kohlenstoff-terminale-Antisense-Modifikation umfasst;
(b) eine Sense-Region, worin die Sense-Region ein erstes 5'-terminales Sense-Nukleotid, ein zweites 5'-terminales Sense-Nukleotid und ein drittes 5'-terminales Sense-Nukleotid umfasst, wobei das erste 5'-terminale Sense-Nukleotid eine erste 2'-Kohlenstoff-Sense-Modifikation umfasst, das zweite 5'-terminale Sense-Nukleotid eine zweite 2'-Kohlenstoff-Sense-Modifikation umfasst, und das dritte 5'-terminale Sense-Nukleotid eine dritte 2'-Kohlenstoff-Sense-Modifikation umfasst, und
(c) eine Loop-Region, worin die Loop-Region zwischen der Sense-Region und der Antisense-Region lokalisiert ist, und worin alle Nukleotide, die von der Antisense-Region und der Sense-Region umfasst sind, ausgenommen das erste 5'-terminale Antisense-Nukleotid, das zweite 5'-terminale Antisense-Nukleotid, das erste 5'-terminale Sense-Nukleotid, das zweite 5'-terminale Sense-Nukleotid und das dritte 5'-terminale Sense-Nukleotid, nicht modifiziert sind.

9. siRNA nach einem der Ansprüche 1 bis 8, worin jeweils die erste 2'-Kohlenstoff-Sense-Modifikation und die zweite 2'-Kohlenstoff-Sense-Modifikation eine 2'-O-Alkylmodifikation sind, und bevorzugt worin mindestens eine und bevorzugt jede der 2'-O-Alkylmodifikationen eine 2'-O-Methylmodifikation sind.

10. siRNA nach einem der Ansprüche 1 bis 9, worin die siRNA 18 bis 30 Basenpaare und bevorzugt 19 bis 25 Basenpaare umfasst.

11. siRNA nach einem der Ansprüche 1, 2, 5 und 6, worin die siRNA 18 bis 30 Basenpaare und bevorzugt 19 bis 25 Basenpaare umfasst, und worin der Antisense-Strang und der Sense-Strang im Wesentlichen komplementär sind und eine Duplex bilden, die keine Überhänge enthält.

12. siRNA nach einem der Ansprüche 1, 2, 5 und 6, umfassend weiter ein Konjugat, das an das 5'- oder 3'-Ende des Sense-Strangs angefügt ist, worin das Konjugat bevorzugt Cholesterol oder Polyethylenglykol ist.

13. Verfahren zur Verringerung der Off-Target-Effekte während RNAi, wobei das Verfahren das Exponieren einer siRNA nach einem der Ansprüche 1 bis 12 gegenüber einer Zielnukleinsäure oder ein Zelle, die die Zielnukleinsäure exprimiert oder zu deren Expression in der Lage ist, umfasst, wobei das Verfahren kein Verfahren zur Behandlung des menschlichen oder tierischen Körpers durch Chirurgie oder Therapie oder ein am menschlichen oder tierischen Körper vorgenommenes diagnostisches Verfahren ist.

14. Pharmazeutische Zusammensetzung umfassend eine siRNA nach einem der Ansprüche 1 bis 12 und einen pharmazeutisch verträglichen Träger oder ein pharmazeutisch verträgliches Verdünnungsmittel.

15. siRNA nach einem der Ansprüche 1 bis 12 zur Verwendung in einem Verfahren zur Behandlung des menschlichen oder tierischen Körpers durch Therapie oder in einem diagnostischen Verfahren, das am menschlichen oder tierischen Körper vorgenommen wird.

## Revendications

1. Petit ARN interférent (pARNi), qui comprend :
a) un brin anti-sens, lequel brin anti-sens comprend un premier nucléotide anti-sens 5'-terminal, lequel premier nucléotide anti-sens 5'-terminal est phosphorylé au niveau de l'atome de carbone en 5' dudit premier nucléotide anti-sens 5'-terminal,
b) et un brin sens, lequel brin sens comprend un premier nucléotide sens 5'-terminal et un deuxième nucléotide sens 5'-terminal, lequel premier nucléotide sens 5'-terminal comporte une première modification sens sur l'atome de carbone en 2', et lequel deuxième nucléotide sens 5'-terminal comporte une deuxième modification sens sur l'atome de carbone en 2',
et dans lequel tous les nucléotides compris dans lesdits brin anti-sens et brin sens, autres que lesdits premier nucléotide sens 5'-terminal et deuxième nucléotide sens 5'-terminal, sont non-modifiés.

2. Petit ARN interférent (pARNi), qui comprend :
a) un brin anti-sens, lequel brin anti-sens comprend un premier nucléotide anti-sens 5'-terminal, lequel premier nucléotide anti-sens 5'-terminal est phosphorylé au niveau de l'atome de carbone en 5' dudit premier nucléotide anti-sens 5'-terminal,
b) et un brin sens, lequel brin sens comprend un premier nucléotide sens 5'-terminal, un deuxième nucléotide sens 5'-terminal et un troisième nucléotide sens 5'-terminal, lequel premier nucléotide sens 5'-terminal comporte une première modification sens sur l'atome de carbone en 2', lequel deuxième nucléotide sens 5'-terminal comporte une deuxième modification sens sur l'atome de carbone en 2', et lequel troisième nucléotide sens 5'-terminal comporte une modification de type O-alkyle en 2', chacune desdites première modification sens sur l'atome de carbone en 2' et deuxième modification sens sur l'atome de carbone en 2' étant une modification de type O-alkyle en 2',
et dans lequel tous les nucléotides compris dans lesdits brin anti-sens et brin sens, autres que lesdits premier nucléotide sens 5'-terminal, deuxième nucléotide sens 5'-terminal et troisième nucléotide sens 5'-terminal, sont non-modifiés.

3. Petit ARN interférent (pARNi) qui est un pARNi monomoléculaire capable de former une épingle à cheveux, lequel pARNi monomoléculaire comprend :
a) une région anti-sens, laquelle région anti-sens comprend un premier nucléotide anti-sens 5'-terminal, lequel premier nucléotide anti-sens 5'-terminal est phosphorylé au niveau de l'atome de carbone en 5' dudit premier nucléotide anti-sens 5'-terminal,
b) une région sens, laquelle région sens comprend un premier nucléotide sens 5'-terminal et un deuxième nucléotide sens 5'-terminal, lequel premier nucléotide sens 5'-terminal comporte une première modification sens sur l'atome de carbone en 2', et lequel deuxième nucléotide sens 5'-terminal comporte une deuxième modification sens sur l'atome de carbone en 2',
c) et une région boucle, laquelle région boucle est située entre ladite région sens et ladite région anti-sens,
et dans lequel tous les nucléotides compris dans lesdites région anti-sens et région sens, autres que lesdits premier nucléotide sens 5'-terminal et deuxième nucléotide sens 5'-terminal, sont non-modifiés.

4. Petit ARN interférent (pARNi) qui est un pARNi monomoléculaire capable de former une épingle à cheveux, lequel pARNi monomoléculaire comprend :
a) une région anti-sens, laquelle région anti-sens comprend un premier nucléotide anti-sens 5'-terminal, lequel premier nucléotide anti-sens 5'-terminal est phosphorylé au niveau de l'atome de carbone en 5' dudit premier nucléotide anti-sens 5'-terminal,
b) une région sens, laquelle région sens comprend un premier nucléotide sens 5'-terminal, un deuxième nucléotide sens 5'-terminal et un troisième nucléotide sens 5'-terminal, lequel premier nucléotide sens 5'-terminal comporte une première modification sens sur l'atome de carbone en 2', lequel deuxième nucléotide sens 5'-terminal comporte une deuxième modification sens sur l'atome de carbone en 2', et lequel troisième nucléotide sens 5'-terminal comporte une modification de type O-alkyle en 2',
c) et une région boucle, laquelle région boucle est située entre ladite région sens et ladite région anti-sens,
et dans lequel tous les nucléotides compris dans lesdites région anti-sens et région sens, autres que lesdits premier nucléotide sens 5'-terminal, deuxième nucléotide sens 5'-terminal et troisième nucléotide sens 5'-terminal, sont non-modifiés.

5. Petit ARN interférent (pARNi), qui comprend :
a) un brin anti-sens, lequel brin anti-sens comprend un premier nucléotide anti-sens 5'-terminal et un deuxième nucléotide anti-sens 5'-terminal, lequel premier nucléotide anti-sens 5'-terminal est phosphorylé au niveau de l'atome de carbone en 5' dudit premier nucléotide anti-sens 5'-terminal, lequel premier nucléotide anti-sens 5'-terminal comporte en outre une première modification anti-sens terminale sur l'atome de carbone en 2', et lequel deuxième nucléotide anti-sens 5'-terminal comporte une deuxième modification anti-sens terminale sur l'atome de carbone en 2',
b) et un brin sens, lequel brin sens comprend un premier nucléotide sens 5'-terminal et un deuxième nucléotide sens 5'-terminal, lequel premier nucléotide sens 5'-terminal comporte une première modification sens sur l'atome de carbone en 2', et lequel deuxième nucléotide sens 5'-terminal comporte une deuxième modification sens sur l'atome de carbone en 2',
et dans lequel tous les nucléotides compris dans lesdits brin anti-sens et brin sens, autres que lesdits premier nucléotide anti-sens 5'-terminal, deuxième nucléotide anti-sens 5'-terminal, premier nucléotide sens 5'-terminal et deuxième nucléotide sens 5'-terminal, sont non-modifiés.

6. Petit ARN interférent (pARNi), qui comprend :
a) un brin anti-sens, lequel brin anti-sens comprend un premier nucléotide anti-sens 5'-terminal et un deuxième nucléotide anti-sens 5'-terminal, lequel premier nucléotide anti-sens 5'-terminal est phosphorylé au niveau de l'atome de carbone en 5' dudit premier nucléotide anti-sens 5'-terminal, lequel premier nucléotide anti-sens 5'-terminal comporte en outre une première modification anti-sens terminale sur l'atome de carbone en 2', et lequel deuxième nucléotide anti-sens 5'-terminal comporte une deuxième modification anti-sens terminale sur l'atome de carbone en 2',
b) et un brin sens, lequel brin sens comprend un premier nucléotide sens 5'-terminal, un deuxième nucléotide sens 5'-terminal et un troisième nucléotide sens 5'-terminal, lequel premier nucléotide sens 5'-terminal comporte une première modification sens sur l'atome de carbone en 2', lequel deuxième nucléotide sens 5'-terminal comporte une deuxième modification sens sur l'atome de carbone en 2', et lequel troisième nucléotide sens 5'-terminal comporte une troisième modification sens sur l'atome de carbone en 2',
et dans lequel tous les nucléotides compris dans lesdits brin anti-sens et brin sens, autres que lesdits premier nucléotide anti-sens 5'-terminal, deuxième nucléotide anti-sens 5'-terminal, premier nucléotide sens 5'-terminal, deuxième nucléotide sens 5'-terminal et troisième nucléotide sens 5'-terminal, sont non-modifiés.

7. Petit ARN interférent (pARNi) qui est un pARNi monomoléculaire capable de former une épingle à cheveux, lequel pARNi monomoléculaire comprend :
a) une région anti-sens, laquelle région anti-sens comprend un premier nucléotide anti-sens 5'-terminal et un deuxième nucléotide anti-sens 5'-terminal, lequel premier nucléotide anti-sens 5'-terminal est phosphorylé au niveau de l'atome de carbone en 5' dudit premier nucléotide anti-sens 5'-terminal, lequel premier nucléotide anti-sens 5'-terminal comporte en outre une première modification anti-sens terminale sur l'atome de carbone en 2', et lequel deuxième nucléotide anti-sens 5'-terminal comporte une deuxième modification anti-sens terminale sur l'atome de carbone en 2',
b) une région sens, laquelle région sens comprend un premier nucléotide sens 5'-terminal et un deuxième nucléotide sens 5'-terminal, lequel premier nucléotide sens 5'-terminal comporte une première modification sens sur l'atome de carbone en 2', et lequel deuxième nucléotide sens 5'-terminal comporte une deuxième modification sens sur l'atome de carbone en 2',
c) et une région boucle, laquelle région boucle est située entre ladite région sens et ladite région anti-sens,
et dans lequel tous les nucléotides compris dans lesdites région anti-sens et région sens, autres que lesdits premier nucléotide anti-sens 5'-terminal, deuxième nucléotide anti-sens 5'-terminal, premier nucléotide sens 5'-terminal et deuxième nucléotide sens 5'-terminal, sont non-modifiés.

8. Petit ARN interférent (pARNi) qui est un pARNi monomoléculaire capable de former une épingle à cheveux, lequel pARNi monomoléculaire comprend :
a) une région anti-sens, laquelle région anti-sens comprend un premier nucléotide anti-sens 5'-terminal et un deuxième nucléotide anti-sens 5'-terminal, lequel premier nucléotide anti-sens 5'-terminal est phosphorylé au niveau de l'atome de carbone en 5' dudit premier nucléotide anti-sens 5'-terminal, lequel premier nucléotide anti-sens 5'-terminal comporte en outre une première modification anti-sens terminale sur l'atome de carbone en 2', et lequel deuxième nucléotide anti-sens 5'-terminal comporte une deuxième modification anti-sens terminale sur l'atome de carbone en 2',
b) une région sens, laquelle région sens comprend un premier nucléotide sens 5'-terminal, un deuxième nucléotide sens 5'-terminal et un troisième nucléotide sens 5'-terminal, lequel premier nucléotide sens 5'-terminal comporte une première modification sens sur l'atome de carbone en 2', lequel deuxième nucléotide sens 5'-terminal comporte une deuxième modification sens sur l'atome de carbone en 2', et lequel troisième nucléotide sens 5'-terminal comporte une troisième modification sens sur l'atome de carbone en 2',
c) et une région boucle, laquelle région boucle est située entre ladite région sens et ladite région anti-sens,
et dans lequel tous les nucléotides compris dans lesdites région anti-sens et région sens, autres que lesdits premier nucléotide anti-sens 5'-terminal, deuxième nucléotide anti-sens 5'-terminal, premier nucléotide sens 5'-terminal, deuxième nucléotide sens 5'-terminal et troisième nucléotide sens 5'-terminal, sont non-modifiés.

9. Petit ARN interférent, conforme à l'une des revendications 1 à 8, dans lequel chacune de ladite première modification sens sur l'atome de carbone en 2' et de ladite deuxième modification sens sur l'atome de carbone en 2' est une modification de type O-alkyle en 2', étant entendu que, de préférence, au moins l'une, et de préférence chacune, de ces modifications de type O-alkyle en 2' est une modification de type O-méthyle en 2'.

10. Petit ARN interférent, conforme à l'une des revendications 1 à 9, lequel pARNi est constitué de 18 à 30 paires de bases, et de préférence, de 19 à 25 paires de bases.

11. Petit ARN interférent, conforme à l'une des revendications 1, 2, 5 et 6, lequel pARNi est constitué de 18 à 30 paires de bases, et de préférence, de 19 à 25 paires de bases, et dans lequel pARNi ledit brin anti-sens et ledit brin sens sont sensiblement complémentaires et forment un duplex qui ne comporte pas de débordements.

12. Petit ARN interférent, conforme à l'une des revendications 1, 2, 5 et 6, qui comprend en outre un conjugué attaché à l'extrémité 3' ou 5' dudit brin sens, et dans lequel le conjugué est de préférence le cholestérol ou un polyéthylèneglycol.

13. Procédé visant à réduire les effets d'un ratage de cible au cours d'une opération d'interférence par ARN, lequel procédé comporte le fait d'exposer un petit ARN interférent, conforme à l'une des revendications 1 à 12, à un acide nucléique cible ou à une cellule qui exprime ou est capable d'exprimer ledit acide nucléique cible, étant entendu que ledit procédé n'est ni un procédé de traitement thérapeutique ou chirurgical du corps d'un humain ou d'un animal, ni un procédé de diagnostic pratiqué sur le corps d'un humain ou d'un animal.

14. Composition pharmaceutique comprenant un petit ARN interférent, conforme à l'une des revendications 1 à 12, et un véhicule ou diluant pharmacologiquement admissible.

15. Petit ARN interférent, conforme à l'une des revendications 1 à 12, pour utilisation dans un procédé de traitement thérapeutique ou chirurgical du corps d'un humain ou d'un animal, ou dans un procédé de diagnostic pratiqué sur le corps d'un humain ou d'un animal.
